# EUROPEAN PATENT SPECIFICATION

(11) **EP 3 890 737 B1**
(45) Date of publication and mention of the grant of the patent: **05.06.2024**
(21) Application number: 19821175.7
(22) Date of filing: 06.12.2019
(51) Int. Cl.: A61K 31/4545, A61K 31/496, A61K 31/513, A61K 31/5377, A61P 3/04, A61P 5/48, A61P 21/00, A61P 35/00

(54) **USP19 INHIBITORS FOR USE IN A METHOD OF TREATING OBESITY, INSULIN RESISTANCE AND TYPE II DIABETES**
USP19 INHIBITOREN ZUR VERWENDUNG IN EINEM VERFAHREN ZUR BEHANDLUNG VON OBESITAS, INSULINRESISTENZ UND TYP II DIABETES
INHIBITEURS DE LA USP19 POUR LEUR UTILISATION DANS UNE METHODE POUR TRAITER OBESITE, RESISTANCE A L'INSULINE ET DIABETE DE TYPE 2

(30) Priority: 06.12.2018 GB 201819936; 28.03.2019 GB 201904341
(43) Date of publication of application: 13.10.2021
(73) Proprietor: Almac Discovery Limited, Craigavon BT63 5QD (GB)
(72) Inventor: ROUNTREE, James Samuel Shane, Craigavon BT63 5QD (GB); HEWITT, Peter, Craigavon BT63 5QD (GB); MCFARLAND, Mary Melissa, Craigavon BT63 5QD (GB); BURKAMP, Frank, Craigavon BT63 5QD (GB); BELL, Christina, Craigavon BT63 5QD (GB); O'DOWD, Colin, Craigavon BT63 5QD (GB); HARRISON, Timothy, Craigavon BT63 5QD (GB); HELM, Matthew Duncan, Craigavon BT63 5QD (GB); ROZYCKA, Ewelina, Craigavon BT63 5QD (GB); CRANSTON, Aaron, Craigavon BT63 5QD (GB); JACQ, Xavier, Craigavon BT63 5QD (GB); PROCTOR, Lauren, Craigavon BT63 5QD (GB)
(74) Representative: Boult Wade Tennant LLP
(86) International application number: PCT/GB2019/053456
(87) International publication number: WO 2020/115500

(56) References cited:
- WO-A1-2018/020242
- WO-A1-2019/150119
- CN-A- 104 174 021
- COYNE ERIN S ET AL: "The deubiquitinating enzyme USP19 modulates adipogenesis and potentiates high-fat-diet-induced obesity and glucose intolerance in mice", DIABETOLOGIA, SPRINGER, BERLIN, DE, vol. 62, no. 1, 1 November 2018 (2018-11-01), pages 136-146, XP036914595, ISSN: 0012-186X, DOI: 10.1007/S00125-018-4754-4 [retrieved on 2018-11-01]
- COLIN R. O'DOWD ET AL: "Identification and Structure-Guided Development of Pyrimidinone Based USP7 Inhibitors", ACS MEDICINAL CHEMISTRY LETTERS, vol. 9, no. 3, 21 February 2018 (2018-02-21), pages 238-243, XP055565198, US ISSN: 1948-5875, DOI: 10.1021/acsmedchemlett.7b00512

## Description

### FIELD OF INVENTION

The present invention is directed to the treatment of obesity, insulin resistance and/or type II diabetes. In particular, the invention provides USP19 inhibitor compounds for use in methods of treating obesity, insulin resistance and/or type II diabetes.

### BACKGROUND

According to the World Health Organisation, the global obesity epidemic is a rapidly growing threat to public health. Obesity is associated with co-morbidities including cardiovascular diseases (e.g. coronary heart disease, hypertension and stroke), osteoarthritis, and pulmonary diseases (e.g. sleep apnoea). Obesity is also associated with metabolic disorders such as metabolic syndrome, type II diabetes and glucose intolerance.

The current primary treatments for obesity (diet and exercise) require behavioural change by the subject. However, maintaining such behavioural changes is frequently difficult and socio-economic factors can result in barriers to access. Bariatric surgery is effective, but not accessible or appropriate for many subjects. There is therefore a need to provide new therapies for obesity that can be used instead of or alongside existing interventions.

Insulin resistance is characterised by a reduced or refractory response to insulin produced by the body, resulting in symptoms such as hyperglycaemia, hyperlipidaemia and/or weight gain. Insulin resistance may be caused by obesity or viral infection, though is not confined to these conditions. Ultimately insulin resistance can progress to type II diabetes.

Management of insulin resistance is primarily through changes in diet and levels of activity. However, similarly to obesity, maintaining these behavioural changes can be challenging. In the context of type II diabetes, insulin resistance therapy with metformin or thiazolidinediones can lead to improvements in glycaemia, though efficacy is variable. Accordingly, there is a need for new therapies for treating insulin resistance, for example in type II diabetes patients.

Muscle wasting is a common complication of many prevalent diseases (e.g. cancer, heart failure, chronic obstructive lung disease, kidney failure, stroke) and a prominent feature of aging. Muscle loss causes weakness and limits mobility, thereby burdening both caregivers and the health care system. Severe loss of muscle mass results in death. Muscle wasting is an independent predictor of mortality in many conditions regardless of disease severity.

In gastrointestinal cancers, at initial presentation, it is already present in 41% of patients, but often masked by obesity. This muscle wastage predicts not only death, but increased dose limiting toxicity to chemotherapy as well as earlier relapse [7]. High quality animal studies have demonstrated that treatment of muscle wasting in cancer cachexia prolongs survival independently of any effect on tumor burden [9]. Treatment of muscle atrophy may therefore not only improve quality of life, but also survival.

Despite the wide prevalence of muscle wasting and its important medical and social consequences, there are no approved therapies. Such a therapeutic would have a major impact on quality and span of life as well as reduce costs for assisted living and institutionalized care. There is therefore a need for an effective therapy for muscular atrophy.

### SUMMARY OF INVENTION

USPs are the largest subfamily of the deubiquitinating enzymes (DUBs) family with over 60 family members reported to date (Komander D. et al., Nat. Rev. Mol. (2009), 10, 550-563; Claque M. et al., Physiol. Rev. (2013), 93, 1289-1315).

Amongst all USPs, USP19 is an important member due to its implications in pathological conditions including but not restricted to muscle atrophy disorders, cancer, neurodegeneration and degenerative diseases as well as antiviral immune response. USP19 expresses as multiple isoforms varying in length from 71.09 kDa (isoform 2) to 156.03 kDa (isoform 5) with the canonical sequence (isoform 1) of 145.65 kDa in size (uniprot.org). The cellular localisation of USP19 may be cytosolic or bound to the endoplasmic reticulum (Lee J. et al., J. Biol. Chem. (2014), 289, 3510-3507; Lee J. et al., Nat. Cell Biol. (2016), 18, 765-776). Localised to the endoplasmic reticulum, USP19 is a key component of the endoplasmic reticulum-associated degradation (ERAD) pathway (Hassink B. et al., EMBO J. (2009), 10, 755-761; Lee J. et al., J. Biol. Chem. (2014), 289, 3510-3507; Lee J. et al., Nat. Cell Biol. (2016), 18, 765-776). In particular, USP19 is involved in the latter steps of the protein quality-control machinery rescuing ERAD substrates that have been retro-translocated to the cytosol. USP19 has also been demonstrated to regulate the stability of the E3 ligases MARCH6 and HRD1 (Nakamura N. et al., Exp. Cell Res. (2014), 328, 207-216; Harada K. et al., Int. J. Mol. Sci. (2016), 17, E1829). In addition, USP19 has recently been implicated in the stabilisation of multiple and potentially important protein substrates. For instance, USP19 interacts with SIAH proteins to rescue HIF1α from degradation under hypoxic conditions (Altun M. et al., J. Biol. Chem. (2012), 287, 1962-1969; Velasco K. et al., Biochem. Biophys. Res. Commun. (2013), 433, 390-395). USP19 also stabilises the KPC1 ubiquitin ligase which is involved in the regulation of the p27^{Kip1} cyclin-dependent kinase inhibitor (Lu Y. et al., Mol. Cell Biol. (2009), 29, 547-558). Knock-out of USP19 by RNAi leads to p27^{Kip1} accumulation and inhibition of cell proliferation (Lu L. et al., PLoS ONE (2011), 6,e15936). USP19 was also found to interact with the inhibitors of apoptosis (lAPs) including c-IAP1 and c-IAP2 (Mei Y. et al., J. Biol. Chem. (2011), 286, 35380-35387). Knockdown of USP19 decreases the total levels of these c-IAPs whilst overexpression increases the levels of both BIRC2/cIAP1 and BIRC3/cIAP2. Knockdown of USP19 also enhances TNFα-induced caspase activation and apoptosis in a BIRC2/c-IAP1 and BIRC3/c-IAP2 dependent manner. In addition to some direct involvement in regulating hypoxia response and ER stress, USP19 has also been implicated recently as a positive regulator of autophagy and negative regulator of type I interferon signalling (IFN, antiviral immune response) by deubiquitinating Beclin-1. USP19 was found to stabilise Beclin-1 at the post-translational level by removing the K11-linked ubiquitin chains of Beclin-1 at Lysine 437 (Jin S. et al., EMBO J. (2016), 35, 866-880). USP19 negatively regulates type I IFN signalling pathway, by blocking RIG-I-MAVS interaction in a Beclin-1 dependent manner. Depletion of either USP19 or Beclin-1 inhibits autophagic flux and promotes type I IFN signalling as well as cellular antiviral immunity (Jin S. et al., EMBO J. (2016), 35, 866-880; Cui J. et al., Autophagy (2016), 12, 1210-1211). Recent findings also indicate USP19 may negatively affect the cellular antiviral type I IFN signalling by regulating the TRAF3 substrate (Gu Z. et al., Future Microbiol. (2017), 12, 767-779) USP19 has also been recently implicated in the Wnt signalling pathway by stabilising the coreceptor LRP6 (Perrody E. et al., eLife (2016), 5, e19083) and in the DNA repair processes, most particularly chromosomal stability and integrity, by regulating the HDAC1 and HDAC2 proteins (Wu M. et al., Oncotarget (2017), 8, 2197-2208).

USP19 is also implicated in muscular atrophy, muscle-wasting syndromes and other skeletal muscle atrophy disorders (Wing S., Int. J. Biochem. Cell Biol. (2013), 45, 2130-2135; Wing S. et al., Int. J. Biochem. Cell Biol. (2016), 79, 426-468; Wiles B. et al., Mol. Biol. Cell (2015), 26, 913-923; Combaret L. et al., Am. J. Physiol. Endocrinol. Metab. (2005), 288, E693-700). This was supported for instance by studies which demonstrated that USP19-silencing induced the expression of myofibrillar proteins and promoted myogenesis (Sundaram P. et al., Am. J. Physiol. Endocrinol. Metab. (2009), 297, E1283-90; Ogawa M. et al., J. Biol. Chem. (2011), 286, 41455-41465; Ogawa M. et al., J. Endocrinol. (2015), 225, 135-145).

*In vivo* studies have demonstrated that mice lacking the USP19 gene (USP19 KO mice) exhibited a decrease in fat mass when fed a high-fat diet (Coyne E, et al. Diabetologia. 2018 Nov 1. doi: 10.1007/s00125-018-4754-4.). USP19 KO mice also exhibited greater glucose tolerance and higher insulin sensitivity when fed a high-fat diet.

These gene knock-out studies describe a connection between USP19 and obesity, as well as USP19 and insulin sensitivity. However, no *in vivo* studies have been described demonstrating that pharmacological inhibitors of USP19 are an effective approach to the treatment of obesity. Similarly, no *in vivo* studies have demonstrated that pharmacological inhibitors of USP19 can be effective in increasing insulin sensitivity.

Previous studies have demonstrated that mice lacking the USP19 gene (USP19 KO mice) were resistant to muscle wasting in response to both glucocorticoids, a common systemic cause of muscle atrophy, as well as in response to denervation, a model of disuse atrophy (Bedard N. et al., FASEB J. (2015), 29, 3889-3898).

These gene knock-out studies describe a connection between USP19 and muscular atrophy. However, no *in vivo* studies have been described demonstrating that pharmacological inhibitors of USP19 are an effective approach to the treatment of muscle-wasting conditions (e.g. cachexia and sarcopenia).

As set out in the accompanying Examples, it is demonstrated herein for the first time that pharmacological treatment with a USP19 inhibitor can induce therapeutic effects in a wild-type *in vivo* model. In particular, it is demonstrated for the first time that USP19 inhibitors reduce fat deposition in an *in vivo* model, indicating that USP19 inhibitors can be an effective treatment for obesity.

Similarly, it is demonstrated herein for the first time that USP19 inhibitors can reduce loss of muscle mass in an *in vivo* model of muscular atrophy.

Similarly, it is demonstrated herein for the first time that USP19 inhibitors can treat the symptoms of insulin resistance, as indicated by an improved response to glucose.

WO2018/020242 provides for the first time a series of compounds which inhibit USP19, as determined by *in vitro* assay.

The Examples herein provide the first data demonstrating the efficacy of USP19 inhibitor compounds, for example those of WO2018/020242, in an *in vivo* model.

Thus, it is demonstrated in the accompanying Examples that the USP19 inhibitory compounds provided herein are capable of treating a number of disease conditions. Taken together, the *in vitro* and *in vivo* data demonstrate that compounds which potently inhibit USP19 activity can be effective therapeutic compounds.

Accordingly, in a first aspect the invention provides a compound, or a pharmaceutically acceptable salt, tautomer, stereoisomer or *N*-oxide derivative thereof, for use in a method of treatment according to the claims, wherein the compound is a compound according to formula (I): 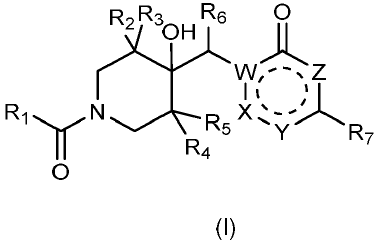 wherein:
R₁ is an optionally substituted alkyl, alkenyl, alkynyl, ether, cycloalkyl, alkylcycloalkyl, heterocycloalkyl, alkylheterocycloalkyl, aryl, arylalkyl, heteroaryl, or heteroarylalkyl group;
R₂ is H or an optionally substituted alkyl group,
R₃ is H or an optionally substituted alkyl group,
R₄ is H or an optionally substituted alkyl group,
R₅ is H or an optionally substituted alkyl group,
R₂ and R₄ may be joined to one another to form an optionally substituted cycloalkyl or heterocycloalkyl that includes the carbon to which they are attached,
R₄ and R₅ may be joined to one another to form an optionally substituted cycloalkyl or heterocycloalkyl that includes the carbon to which they are attached;
R₆ is H or an optionally substituted alkyl group;
W is C or N;
X is N or CR₈, wherein R₈ is H or optionally substituted C1-C6 alkyl,
Y is N or CR₉
Z is CH, or NH,
wherein R₉ is H or optionally substituted alkyl, amido, amino, aryl, heteroaryl, cycloalkyl, cycloheteroalkyl, halo, carbonyl, ester, aminoalkyl, or cyano;
R₇ is hydrogen, halo, or an optionally substituted alkyl, alkenyl, alkynyl, amino, aryl, cycloalkyl, cycloalkenyl, alkoxy, aryloxy, heteroaryl or heterocycloalkyl group;

or the compound 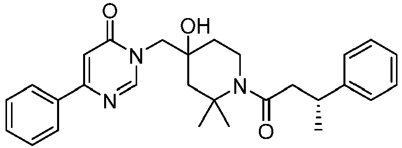
or a pharmaceutically acceptable salt, tautomer, stereoisomer or *N*-oxide derivative thereof.

In a further aspect is provided a compound as defined in relation to the first aspect, or a pharmaceutically acceptable salt, tautomer, stereoisomer or *N*-oxide derivative thereof, for use in a method of treating obesity.

Also provided is an effective amount of a pharmaceutical composition comprising a compound, pharmaceutically acceptable salt, tautomer, stereoisomer or *N*-oxide derivative for use as defined in relation to the first aspect.

In a further aspect of the invention is provided a compound as defined in relation to the first aspect of the invention, or a pharmaceutically acceptable salt, tautomer, stereoisomer or N-oxide derivative thereof, for use in a method of treating insulin resistance. In a further aspect of the invention is provided a compound as defined in relation to the first aspect of the invention, or a pharmaceutically acceptable salt, tautomer, stereoisomer or *N*-oxide derivative thereof, for use in a method of treating type II diabetes.

Presently disclosed but not claimed is a compound as defined in relation to the first aspect of the invention, or a pharmaceutically acceptable salt, tautomer, stereoisomer or *N*-oxide derivative thereof, for use in a method of treating muscular atrophy. Presently disclosed but not claimed is a compound as defined in relation to the first aspect, or a pharmaceutically acceptable salt, tautomer, stereoisomer or *N*-oxide derivative thereof, for use in a method of treating cachexia or sarcopenia.

It is further demonstrated herein that USP19 inhibitor compound as described in relation to the first aspect exhibit cell permeability and potent target engagement in cancer cell lines. The cell permeability and target engagement in cancer cells is comparable to that observed in muscle cells. As demonstrated herein, USP19 inhibitors exhibit potent *in vivo* therapeutic effects on muscle wasting. Thus, by extension, since similar target engagement is seen in cancer cells, it is expected that pharmacological USP19 inhibitors will be effective at exerting therapeutic effects in cancer, due to the association of USP19 and oncogenic processes described above.

Presently disclosed but not claimed is a compound as defined in relation to the first aspect of the invention, or a pharmaceutically acceptable salt, tautomer, stereoisomer or *N*-oxide derivative thereof, for use in a method of treating cancer.

The data presented herein demonstrate the efficacy of USP19 inhibitors as effective therapeutic drugs. This efficacy is shown in a variety of pathological conditions.

Other preferred embodiments of the invention appear throughout the specification and in particular in the examples. Particularly preferred are those named compounds having greater activity as tested. Compounds having higher activity are more preferred over those having lower activity.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

### FIGURES

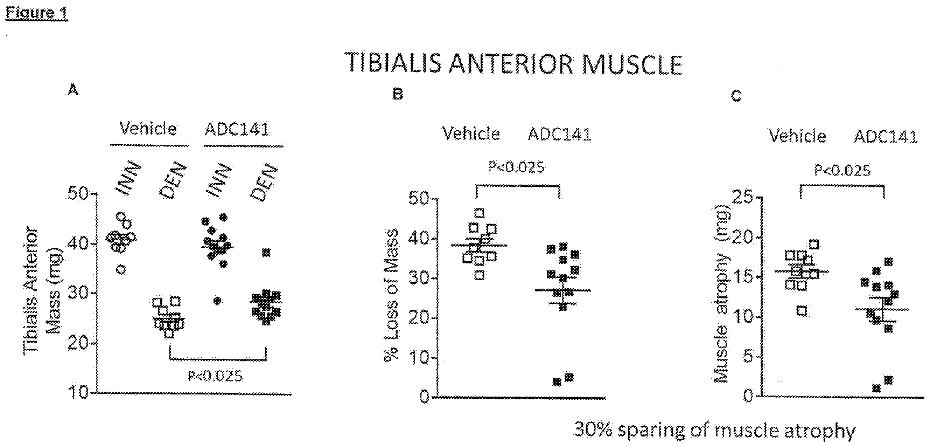

**Figure 1****:** Effect of USP19 pharmacological inhibition on tibialis anterior mass. (A) Tibialis anterior mass (mg) from mice treated with vehicle or USP19 inhibitor compound ADC-141. Mass is given for the muscle from limb that had undergone sciatic nerve denervation (DEN) and also from the innervated limb (INN). (B) Percentage loss of tibialis anterior muscle mass as a result of denervation in vehicle and USP19 inhibitor (ADC-141) treated mice. Percentage calculated as a proportion of the mass of the muscle from the innervated limb of the same mouse. (C) Loss of tibialis anterior muscle mass (in mg) as a result of denervation in vehicle treated and USP19 inhibitor (ADC-141) treated mice. P <0.025.

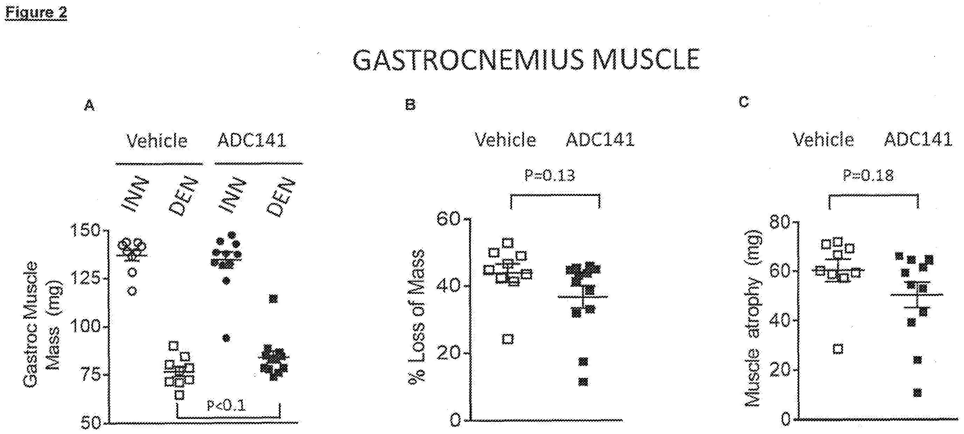

**Figure 2****:** Effect of USP19 pharmacological inhibition on gastrocnemius muscle mass. (A) gastrocnemius muscle mass (mg) from mice treated with vehicle or USP19 inhibitor compound ADC-141. Mass is given for the muscle from limb that had undergone sciatic nerve denervation (DEN) and also from the innervated limb (INN). (B) Percentage loss of gastrocnemius muscle mass as a result of denervation in vehicle and USP19 inhibitor (ADC-141) treated mice. Percentage calculated as a proportion of the mass of the muscle from the innervated limb of the same mouse. (C) Loss of gastrocnemius muscle mass (in mg) as a result of denervation in vehicle treated and USP19 inhibitor (ADC-141) treated mice.

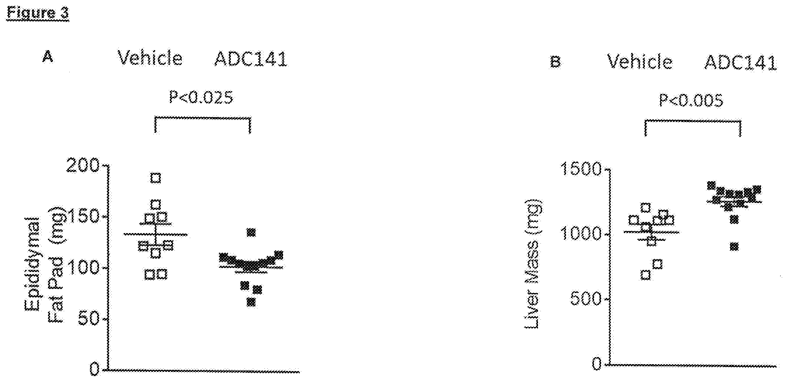

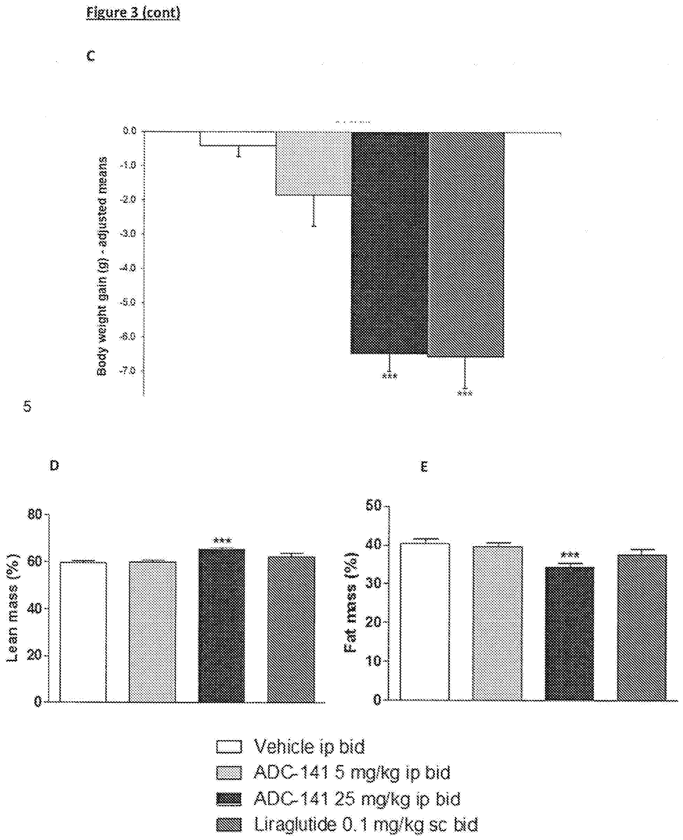

Figure 3: (A) Effect of USP19 pharmacological inhibition on fat mass. The epididymal fat pad was collected from vehicle and USP19 inhibitor (ADC-141) treated mice, with USP19 inhibitor treated mice showing a significant reduction in fat mass. (B) Effect of USP19 pharmacological inhibition on liver mass. The liver was collected from vehicle and USP19 inhibitor (ADC-141) treated mice. An increase in liver mass was observed, likely due to accumulation of drug compound in the liver. (C) Percentage change in overall body weight in vehicle-treated control DIO mice. USP19 inhibitor 5mg/kg ip BID, USP19 inhibitor 25mg/kg ip BID, or positive control liraglutide 0.1mg/kg sc BID (left to right bars, respectively); (D) Percentage change in overall lean mass and (E) percentage change in overall fat mass in vehicle, USP19 inhibitor 5mg/kg, USP19 inhibitor 25mg/kg, and liraglutide treated mice (left to right, respectively). ***p<0.001 vs vehicle.

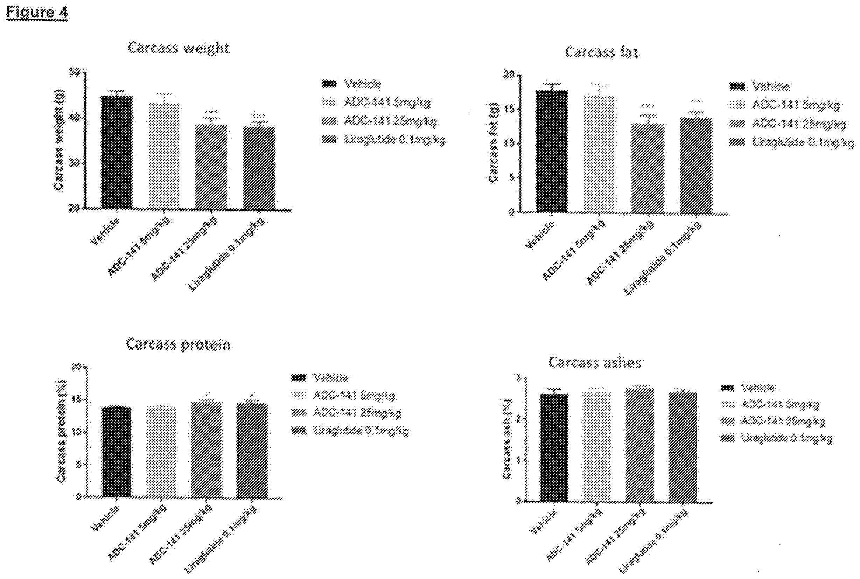

**Figure 4****:** Body composition analysis of mice in a dietary-induced obesity model, treated with USP19 inhibitor ACD-141 or liraglutide. All mice were fed a high-fat diet and treated as indicated. Results for total tissue mass, total body fat, and percentage body protein were determined. Percentage carcass ash was also determined. Means are adjusted for differences between treatment groups in Day 1 bodyweight. Error bars show SEM. *** p<0.001, ** p<0.01.

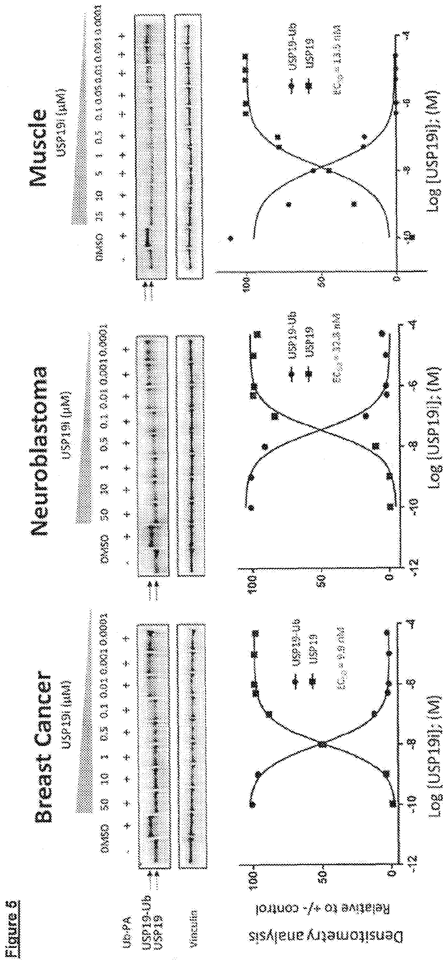

**Figure 5****:** Cell target engagement of USP19 inhibitor compound in breast cancer, neuroblastoma and skeletal muscle cell lines. EC₅₀ was determined by densitometry.

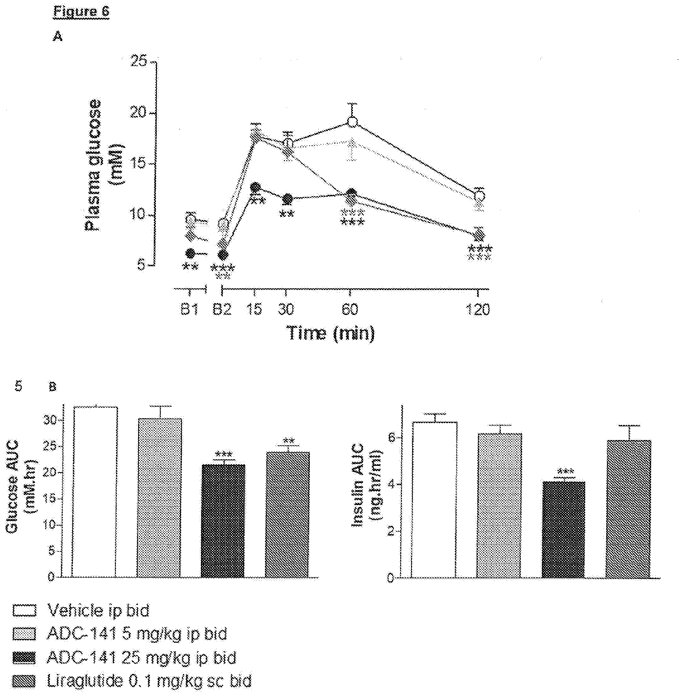

**Figure 6****:** Response to oral glucose tolerance test (OGTT) in obese mice. (A) Timeline of plasma glucose response in vehicle-treated control mice (circles), USP19 inhibitor 5mg/kg ip BID (triangle), USP19 inhibitor 25mg/kg ip BID (solid circle), or positive control liraglutide 0.1mg/kg sc BID (diamond); (B) Glucose AUC (mM.hr) and (C) insulin AUC (ng.hr/ml) for vehicle, USP19 inhibitor 5mg/kg, USP19 inhibitor 25mg/kg, and liraglutide (left to right, respectively). ** p<0.01 vs vehicle; ***p<0.001 vs vehicle.

### DETAILED DESCRIPTION

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedence over any dictionary or extrinsic definition.

As used in the specification and the appended claims, unless specified to the contrary, the following terms have the meaning indicated:
As used herein, a USP19 inhibitor refers to a compound which acts on USP19 so as to decrease the activity of the enzyme. Examples of USP19 inhibitors are exemplified compounds herein. Preferably a USP19 inhibitor exhibits an IC₅₀ of less than 5µM, preferably less than 0.5µM.

As used herein, "obesity" refers to the medical condition characterised by excess body fat. Obesity can be characterised by, for example, a body mass index (BMI) of greater than 30. Treatment of obesity may be indicated by, for example, the reduction of body fat, in percentage and/or absolute mass terms. Treatment of obesity may also be exemplified by a reduction in the rate of body fat accumulation by a subject compared to before treatment.

As used herein, "insulin resistance" refers to the medical condition characterised by an abnormally weak response to insulin. Since insulin resistance is typically not treated by exogenous insulin treatment, the resistance is typically to insulin produced by the body of the subject, though the subject may also be resistant to exogenous insulin. "Insulin resistance" encompasses the conditions "prediabetes" and Type II diabetes. Insulin resistance may be indicated, for example, by a glucose tolerance test (GTT) glycaemia of 7.8 mmol/L or greater. Type II diabetes is typically diagnosed following a glucose tolerance test (GTT) glycaemia of 11.1 mmol/L or greater.

Treatment of insulin resistance may be indicated by an improvement (i.e. reduction) in the subject's GTT glycaemia compared to before treatment. Treatment may also be indicated by a reduction in the subject's blood sugar concentration under normal conditions compared to before treatment.

As used herein, "muscular atrophy" and "muscle-wasting" are used interchangeably to refer to decrease in muscle mass in a subject, including in the context of cachexia or sarcopenia, for example. Muscular atrophy can be as a result of temporary or permanent disability, temporary or permanent immobilisation of a limb, extended bedrest, cachexia (for example as a result of cancer, heart failure, or COPD), or sarcopenia.

Treatment of muscular atrophy may be characterised as the slowing of the rate of atrophy - that is, treatment results in less muscle mass lost over a given period of time. Preferably, successful treatment results in no loss of muscle mass.

The term "alkyl group" (alone or in combination with another term(s)) means a straight-or branched-chain saturated hydrocarbon substituent typically containing 1 to 15 carbon atoms, such as 1 to 10, 1 to 8, 1 to 6, or 1 to 4 carbon atoms. A "Cₙ alkyl" group refers to an aliphatic group containing n carbon atoms. For example, a C₁-C₁₀ alkyl group contains 1, 2, 3, 4, 5, 6, 7, 8, 9 or 10 carbon atoms. Attachment to the alkyl group occurs through a carbon atom. Examples of such substituents include methyl, ethyl, n-propyl, isopropyl, n-butyl, isobutyl, sec-butyl, tert-butyl, pentyl (branched or unbranched), hexyl (branched or unbranched), heptyl (branched or unbranched), octyl (branched or unbranched), nonyl (branched or unbranched), and decyl (branched or unbranched).

The term "alkenyl group" (alone or in combination with another term(s)) means a straight-or branched-chain hydrocarbon substituent containing one or more double bonds and typically 2 to 15 carbon atoms; such as 2 to 10, 2 to 8, 2 to 6 or 2 to 4 carbon atoms. Examples of such substituents include ethenyl (vinyl), 1-propenyl, 3-propenyl, 1,4-pentadienyl, 1,4-butadienyl, 1-butenyl, 2-butenyl, 3-butenyl, pentenyl and hexenyl.

The term "alkynyl group" (alone or in combination with another term(s)) means a straight-or branched-chain hydrocarbon substituent containing one or more triple bonds and typically 2 to 15 carbon atoms; such as 2 to 10, 2 to 8, 2 to 6 or 2 to 4 carbon atoms. Examples of such substituents include ethynyl, 1-propynyl, 3-propynyl, 1-butynyl, 3-butynyl and 4-butynyl.

The term "heteroalkyl group" (alone or in combination with another term(s)) means a straight-or branched-chain saturated hydrocarbyl substituent typically containing 1 to 15 atoms, such as 1 to 10, 1 to 8, 1 to 6, or 1 to 4 atoms, wherein at least one of the atoms is a heteroatom (i.e. oxygen, nitrogen, or sulfur), with the remaining atoms being carbon atoms. A "Cₙ heteroalkyl" group refers to an aliphatic group containing n carbon atoms and one or more heteroatoms, for example one heteroatom. For example, a C₁-C₁₀ heteroalkyl group contains 1, 2, 3, 4, 5, 6, 7, 8, 9 or 10 carbon atoms in addition to one or more heteroatoms, for example one heteroatom. Attachment to the heteroalkyl group occurs through a carbon atom or through a heteroatom.

The term "heteroalkenyl group" (alone or in combination with another term(s)) means a straight-or branched-chain hydrocarbon substituent containing one or more carbon-carbon double bonds and typically 2 to 15 atoms; such as 2 to 10, 2 to 8, 2 to 6 or 2 to 4 atoms, wherein at least one of the atoms is a heteroatom (i.e. oxygen, nitrogen, or sulfur), with the remaining atoms being carbon atoms. A "Cₙ heteroalkenyl" group refers to an aliphatic group containing n carbon atoms and one or more heteroatoms, for example one heteroatom. For example, a C₂-C₁₀ heteroalkenyl group contains 2, 3, 4, 5, 6, 7, 8, 9 or 10 carbon atoms in addition to one or more heteroatoms, for example one heteroatom. Attachment to the heteroalkenyl group occurs through a carbon atom or through a heteroatom.

The term "heteroalkynyl group" (alone or in combination with another term(s)) means a straight-or branched-chain hydrocarbon substituent containing one or more carbon-carbon triple bonds and typically 2 to 15 carbon atoms; such as 2 to 10, 2 to 8, 2 to 6 or 2 to 4 carbon atoms, wherein at least one of the atoms is a heteroatom (i.e. oxygen, nitrogen, or sulfur), with the remaining atoms being carbon atoms. A "Cₙ heteroalkynyl" group refers to an aliphatic group containing n carbon atoms and one or more heteroatoms, for example one heteroatom. For example, a C₂-C₁₀ heteroalkynyl group contains 1, 2, 3, 4, 5, 6, 7, 8, 9 or 10 carbon atoms in addition to one or more heteroatoms, for example one heteroatom. Attachment to the heteroalkynyl group occurs through a carbon atom or through a heteroatom.

The term "carbocyclyl group" (alone or in combination with another term(s)) means a saturated cyclic (i.e. "cycloalkyl"), partially saturated cyclic (i.e. "cycloalkenyl"), or completely unsaturated (i.e. "aryl") hydrocarbon substituent containing from 3 to 14 carbon ring atoms ("ring atoms" are the atoms bound together to form the ring or rings of a cyclic substituent). A carbocyclyl may be a single-ring (monocyclic) or polycyclic ring structure.

A carbocyclyl may be a single ring structure, which typically contains 3 to 8 ring atoms, more typically 3 to 7 ring atoms, and more typically 5 to 6 ring atoms. Examples of such single-ring carbocyclyls include cyclopropyl (cyclopropanyl), cyclobutyl (cyclobutanyl), cyclopentyl (cyclopentanyl), cyclopentenyl, cyclopentadienyl, cyclohexyl (cyclohexanyl), cyclohexenyl, cyclohexadienyl, and phenyl. A carbocyclyl may alternatively be polycyclic (i.e. may contain more than one ring). Examples of polycyclic carbocyclyls include bridged, fused, and spirocyclic carbocyclyls. In a spirocyclic carbocyclyl, one atom is common to two different rings. An example of a spirocyclic carbocyclyl is spiropentanyl. In a bridged carbocyclyl, the rings share at least two common non-adjacent atoms. Examples of bridged carbocyclyls include bicyclo[2.2.1]heptanyl, bicyclo[2.2.1]hept-2-enyl, and adamantanyl. In a fused-ring carbocyclyl system, two or more rings may be fused together, such that two rings share one common bond. Examples of two- or three-fused ring carbocyclyls include naphthalenyl, tetrahydronaphthalenyl (tetralinyl), indenyl, indanyl (dihydroindenyl), anthracenyl, phenanthrenyl, and decalinyl.

The term "cycloalkyl group" (alone or in combination with another term(s)) means a saturated cyclic hydrocarbon substituent containing 3 to 14 carbon ring atoms. A cycloalkyl may be a single carbon ring, which typically contains 3 to 8 carbon ring atoms and more typically 3 to 6 ring atoms. It is understood that attachment to a cycloalkyl group is via a ring atom of the cycloalkyl group. Examples of single-ring cycloalkyls include cyclopropyl, cyclobutyl, cyclopentyl, and cyclohexyl. A cycloalkyl may alternatively be polycyclic or contain more than one ring. Polycyclic cycloalkyls include bridged, fused, and spirocyclic cycloalkyls.

The term "alkylcycloalkyl" refers to a cycloalkyl substituent attached via an alkyl chain. Examples of an alkylcycloalkyl substitent include cyclohexylethane, where the cyclohexane is attached via an ethane linker. Other examples include cyclopropylethane, cyclobutylethane, cyclopentylethane, cycloheptylethane, cyclohexylmethane. In a "Cₙ" alkylcycloalkyl, Cₙ includes the carbon atoms in the alkyl chain and in the cycloalkyl ring. For example, cyclohexylethane is a C8 alkylcycloalkyl.

The term "aryl group" (alone or in combination with another term(s)) means an aromatic carbocyclyl containing from 5 to 14 carbon ring atoms, optionally 5 to 8, 5 to 7, optionally 5 to 6 carbon ring atoms. A "Cₙ aryl" group refers to an aromatic group containing n carbon atoms. For example, a C₆-C₁₀ aryl group contains 6, 7, 8, 9 or 10 carbon atoms. Attachment to the aryl group occurs through a carbon atom. An aryl group may be monocyclic or polycyclic (i.e. may contain more than one ring). In the case of polycyclic aromatic rings, only one ring in the polycyclic system is required to be unsaturated while the remaining ring(s) may be saturated, partially saturated or unsaturated. Attachment to the aryl group occurs through a carbon atom contained in the ring. Examples of aryl groups include phenyl, naphthyl, acridinyl, indenyl, indanyl, and tetrahydronapthyl.

The term "arylalkyl" refers to an aryl substituent attached via an alkyl chain. Examples of an arylalkyl substitent include phenylethane/ethylbenzene, where the ethane chain links to a phenyl group to the point of attachment. In a "Cₙ" arylalkyl, Cₙ includes the carbon atoms in the alkyl chain and in the aryl group. For example, ethylbenzene is a C8 arylalkyl. The term "heterocyclyl group" (alone or in combination with another term(s)) means a saturated (i.e. "heterocycloalkyl"), partially saturated (i.e. "heterocycloalkenyl"), or completely unsaturated (i.e. "heteroaryl") ring structure containing a total of 3 to 14 ring atoms, wherein at least one of the ring atoms is a heteroatom (i.e. oxygen, nitrogen, or sulfur), with the remaining ring atoms being carbon atoms. A heterocyclyl group may, for example, contain one, two, three, four or five heteroatoms. Attachment to the heterocyclyl group may occur through a carbon atom and/or one or more heteroatoms that are contained in the ring. A heterocyclyl may be a single-ring (monocyclic) or polycyclic ring structure.

A heterocyclyl group may be a single ring, which typically contains from 3 to 7 ring atoms, more typically from 3 to 6 ring atoms, and even more typically 5 to 6 ring atoms. Examples of single-ring heterocyclyls include furanyl, dihydrofuranyl, tetrahydrofuranyl, thiophenyl (thiofuranyl), dihydrothiophenyl, tetrahydrothiophenyl, pyrrolyl, pyrrolinyl, pyrrolidinyl, imidazolyl, imidazolinyl, imidazolidinyl, pyrazolyl, pyrazolinyl, pyrazolidinyl, triazolyl, tetrazolyl, oxazolyl, oxazolidinyl, isoxazolidinyl, isoxazolyl, thiazolyl, isothiazolyl, thiazolinyl, isothiazolinyl, thiazolidinyl, isothiazolidinyl, thiodiazolyl, oxadiazolyl (including 1,2,3-oxadiazolyl, 1,2,4-oxadiazolyl, 1,2,5-oxadiazolyl (furazanyl) or 1,3,4-oxadiazolyl), oxatriazolyl, dioxazolyl oxathiolyl, pyranyl, dihydropyranyl, thiopyranyl, tetrahydrothiopyranyl, pyridinyl (azinyl), piperidinyl, diazinyl (including pyridazinyl (1,2-diazinyl), pyrimidinyl (1,3-diazinyl) or pyrazinyl (1,4-diazinyl)), piperazinyl, triazinyl (including 1,3,5-triazinyl,1,2,4-triazinyl and 1,2,3-triazinyl)), oxazinyl (including 1,2-oxazinyl, 1,3-oxazinyl or 1,4-oxazinyl)), oxadiazinyl (including 1,2,3-oxadiazinyl, 1,2,4-oxadiazinyl, 1,4,2-oxadiazinyl or 1,3,5-oxadiazinyl)), morpholinyl, azepinyl, oxepinyl, thiepinyl, and diazepinyl.

A heterocyclyl group may alternatively be polycyclic (i.e. may contain more than one ring). Examples of polycyclic heterocyclyl groups include bridged, fused, and spirocyclic heterocyclyl groups. In a spirocyclic heterocyclyl group, one atom is common to two different rings. In a bridged heterocyclyl group, the rings share at least two common non-adjacent atoms. In a fused-ring heterocyclyl group, two or more rings may be fused together, such that two rings share one common bond. Examples of fused ring heterocyclyl groups containing two or three rings include indolizinyl, pyranopyrrolyl, 4H-quinolizinyl, purinyl, naphthyridinyl, pyridopyridinyl (including pyrido[3,4-b]-pyridinyl, pyrido[3,2-b]-pyridinyl, or pyrido[4,3-b]-pyridinyl), and pteridinyl. Other examples of fused-ring heterocyclyl groups include benzo-fused heterocyclyl groups, such as indolyl, isoindolyl (isobenzazolyl, pseudoisoindolyl), indoleninyl (pseudoindolyl), isoindazolyl (benzpyrazolyl), benzazinyl (including quinolinyl (1-benzazinyl) or isoquinolinyl (2-benzazinyl)), phthalazinyl, quinoxalinyl, quinazolinyl, benzodiazinyl (including cinnolinyl (1,2-benzodiazinyl) or quinazolinyl (1,3-benzodiazinyl)), benzopyranyl (including chromanyl or isochromanyl), and benzisoxazinyl (including 1,2-benzisoxazinyl or 1,4-benzisoxazinyl).

The term "heterocycloalkyl group" (alone or in combination with another term(s)) means a saturated heterocyclyl. A "Cₙ heterocycloalkyl" group refers to a cyclic aliphatic group containing n carbon atoms in addition to at least one heteroatom, for example nitrogen. For example, a C₁-C₁₀ heterocycloalkyl group contains 1, 2, 3, 4, 5, 6, 7, 8, 9 or 10 carbon ring atoms in addition to the at least one heteroatom. Attachment to the heterocycloalkyl group occurs through a carbon atom or one of the at least one heteroatoms.

The term "alkylheterocycloalkyl" refers to a heterocycloalkyl substituent attached via an alkyl chain. In a "Cₙ" alkylheterocycloalkyl, Cₙ includes the carbon atoms in the alkyl chain and in the heterocycloalkyl ring. For example, ethylpiperidine is a C7 alkylheterocycloalkyl.

The term "heteroaryl group" (alone or in combination with another term(s)) means an aromatic heterocyclyl containing from 5 to 14 ring atoms. A "Cₙ heteroaryl" group refers to an aromatic group containing n carbon atoms and at least one heteroatom. For example, a C₂-C₁₀ aryl group contains 2, 3, 4, 5, 6, 7, 8, 9 or 10 carbon atoms in addition to at least one heteroatom. Attachment to the heteroaryl group occurs through a carbon atom or through a heteroatom. A heteroaryl group may be monocyclic or polycyclic. A heteroaryl may be a single ring or 2 or 3 fused rings. Examples of monocyclic heteroaryl groups include 6-membered rings such as pyridyl, pyrazinyl, pyrimidinyl, pyridazinyl, and 1,3,5-, 1,2,4- or 1,2,3-triazinyl; 5-membered rings such as imidazolyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl. Polycyclic heteroaryl groups may be 2 or 3 fused rings. Examples of polycyclic heteroaryl groups include 6/5-membered fused ring groups such as benzothiofuranyl, benzisoxazolyl, benzoxazolyl, and purinyl; and 6/6-membered fused ring groups such as benzopyranyl, quinolinyl, isoquinolinyl, cinnolinyl, quinazolinyl, and benzoxazinyl. In the case of polycyclic heteroaryl groups, only one ring in the polycyclic system is required to be unsaturated while the remaining ring(s) may be saturated, partially saturated or unsaturated.

A nitrogen-containing heteroaryl group is a heteroaryl group in which at least one of the one or more heteroatoms in the ring is nitrogen.

The term "heteroarylalkyl" refers to a heteroaryl substituent attached via an alkyl chain. Examples of a heteroarylalkyl substitent include ethylpyridine, where the ethane chain links a pyridine group to the point of attachment.

The term "amino group" refers to the -NR'R" group. The amino group can be optionally substituted. In an unsubstituted amino group, R' and R" are hydrogen. In a substituted amino group R' and R" each independently may be, but are not limited to, hydrogen, an alkyl, heteroalkyl, cycloalkyl, heterocycloalkyl, alkylcycloalkyl, alkylheterocycloalkyl, alkoxy, sulfonyl, alkenyl, alkanoyl, aryl, arylalkyl, or a heteroaryl group, provided R' and R" are not both hydrogen. In a substituted amino group R' and R" may cyclise to form a cyclic amino group, e.g. a pyrrolidine group or a piperidine group. Such a cyclic amino group may incorporate other heteroatoms, for example to form a piperazine or morpholine group. Such a cyclic amino group may be optionally substituted, e.g. with an amino group, a hydroxyl group or an oxo group.

The term "aminoalkyl" group refers to the -R^{a}NR'R" group, wherein R^{a} is an alkyl chain as defined above and NR'R" is an optionally substituted amino group as defined above. "Cₙ aminoalkyl" group refers to a group containing n carbon atoms. For example, a C₁-C₁₀ aminoalkyl group contains 1, 2, 3, 4, 5, 6, 7, 8, 9 or 10 carbon atoms. When the amino group of the aminoalkyl group is a substituted amino group, the number of carbon atoms includes any carbon atoms in the substituent groups. Attachment to the aminoalkyl group occurs through a carbon atom of the R^{a} alkyl group. Examples of aminoalkyl substituents include methylamine, ethylamine, methylaminomethyl, dimethylaminomethyl, methylaminoethyl, dimethylaminoethyl, methylpyrrolidine, and ethylpyrrolidine

The term "amido group" refers to the -C(=O)-NR- group. Attachment may be through the carbon and/or nitrogen atom. For example, the amido group may be attached as a substituent via the carbon atom only, in which case the nitrogen atom has two R groups attached (-C(=O)-NR₂). The amido group may be attached by the nitrogen atom only, in which case the carbon atom has an R group attached (-NR-C(=O)R).

The term "ether" refers to an an -O-alkyl group or an -alkyl-O-alkyl group, for example a methoxy group, a methoxymethyl group or an ethoxyethyl group. The alkyl chain(s) of an ether can be linear, branched or cyclic chains. The ether group can be optionally substituted (a "substituted ether") with one or more substituents. A Cₙ ether refers to an ether group having n carbons in all alkyl chains of the ether group. For example, a CH(CH3)-O-C6H11 ether is a C₈ ether group.

The term "alkoxy group" refers to an -O-alkyl group. The alkoxy group can refer to linear, branched, or cyclic, saturated or unsaturated oxy-hydrocarbon chains, including, for example, methoxyl, ethoxyl, propoxyl, isopropoxyl, butoxyl, t-butoxyl and pentoxyl. The alkoxy group can be optionally substituted (a "substituted alkoxy") with one or more alkoxy group substituents.

The term "aryloxy group" refers to an -O-aryl group, for example a phenoxy group. An aryloxy substituent may itself be optionally substituted, for example with a halogen.

The term "alkylester" refers to a -C(O)OR group, where R is an alkyl group as defined herein. An example of an alkylester is ethyl methanoate - i.e. R is an ethyl group.

The term "hydroxyl" refers to an -OH group.

The term "oxo group" refers to the (=O) group, i.e. a substituent oxygen atom connected to another atom by a double bond. For example, a carbonyl group (-C(=O)-) is a carbon atom connected by a double bond to an oxygen atom, i.e. an oxo group attached to a carbon atom. Examples of carbonyl substituents include aldehydes (-C(=O)H), acetyl (-C(=O)CH3) and carboxyl/carboxylic acid groups (-C(=O)OH).

The term "halo group" refers to a group selected from chlorine, fluorine, bromine and iodine. Preferably, the halo group is selected from chlorine and fluorine.

An alkyl, alkenyl, alkynyl, carbocyclyl (including cycloalkyl, cycloalkenyl and aryl), heterocyclyl (including heterocycloalkyl, heterocyloalkenyl, heteroaryl, nitrogen-containing heterocyclyl), amino, amido, ester, ether, alkoxy, or sulfonamide group can be optionally substituted with one or more substituents, which can be the same or different. A substituent can be attached through a carbon atom and/or a heteroatom in the alkyl, alkenyl, alkynyl, carbocyclyl (including cycloalkyl, cycloalkenyl and aryl), heterocyclyl (including heterocycloalkyl, heterocyloalkenyl, heteroaryl, nitrogen-containing heterocyclyl, nitrogen-containing heteroaryl), amino, amido, ester, ether, alkoxy, or sulfonamide group. The term "substituent" (or "radical") includes but is not limited to alkyl, substituted alkyl, heteroalkyl, substituted heteroalkyl, aralkyl, substituted aralkyl, alkenyl, substituted alkenyl, alkynyl, substituted alkynyl, halo, hydroxyl, cyano, amino, amido, alkylamino, arylamino, carbocyclyl, cycloalkyl, substituted cycloalkyl, heterocycloalkyl, substituted heterocycloalkyl, cycloalkenyl, substituted cycloalkenyl, aryl, substituted aryl, heteroaryl, substituted heteroaryl, nitro, thio, alkanoyl, hydroxyl, aryloxyl, alkoxyl, alkylthio, arylthio, aralkyloxyl, aralkylthio, carboxyl, alkoxycarbonyl, oxo, alkylsulfonyl, arylsulfonyl and sulfoximinyl.

In certain aspects, the substituent is alkyl, substituted alkyl, heteroalkyl, substituted heteroalkyl, alkenyl, substituted alkenyl, alkynyl, substituted alkynyl, halo, hydroxyl, cyano, amino, amido, alkylamino, arylamino, carbocyclyl, cycloalkyl, substituted cycloalkyl, heterocycloalkyl, substituted heterocycloalkyl, cycloalkenyl, substituted cycloalkenyl, aryl, substituted aryl, heteroaryl, substituted heteroaryl, nitro, thio, alkanoyl, hydroxyl, aryloxyl, alkoxyl, alkylthio, arylthio, aralkyloxyl, aralkylthio, carboxyl, alkoxycarbonyl, oxo, alkylsulfonyl and arylsulfonyl.

If a group, for example an alkyl group, is "optionally substituted", it is understood that the group has one or more substituents attached (substituted) or does not have any substituents attached (unsubstituted).

If a group is substituted with a further optionally substituted group, it is understood that the first substituent may itself be either unsubstituted or substituted.

For completeness, it is also noted that certain chemical formulae used herein define delocalized systems. This definition is known in the art as a definition of aromaticity and may indicate the presence of, for example, a planar mono-, di- or tri-cyclic system that contains (4*n*+2) electrons where n is an integer. In other words, these systems may display Hückel aromaticity.

In whatever aspect, the compounds of the present invention may possess some aspect of stereochemistry. For example, the compounds may possess chiral centres and/or planes and/or axes of symmetry. As such, the compounds may be provided as single stereoisomers, single diastereomers, mixtures of stereoisomers or as racemic mixtures, unless otherwise specified. Stereoisomers are known in the art to be molecules that have the same molecular formula and sequence of bonded atoms, but which differ in their spatial orientations of their atoms and/or groups.

In addition, the compounds of the present invention may exhibit tautomerism. Each tautomeric form is intended to fall within the scope of the invention.

In addition, it will be understood that the elements described herein may be the common isotope or an isotope other than the common isotope. For example, a hydrogen atom may be ¹H, ²H (deuterium) or ³H (tritium).

In addition, the compounds of the present invention may be provided in the form of their pharmaceutically acceptable salts or as co-crystals.

The term "pharmaceutically acceptable salt" refers to ionic compounds formed by the addition of an acid to a base. The term refers to such salts that are considered in the art as being suitable for use in contact with a patient, for example *in vivo* and pharmaceutically acceptable salts are generally chosen for their non-toxic, non-irritant characteristics.

The term "co-crystal" refers to a multi- component molecular crystal, which may comprise non-ionic interactions.

Pharmaceutically acceptable salts and co-crystals may be prepared by ion exchange chromatography or by reacting the free base or acidic form of a compound with stoichiometric amounts or with an excess of the desired salt-forming inorganic or organic acid or base in one or more suitable solvents, or by mixing the compound with another pharmaceutically acceptable compound capable of forming a co-crystal.

Salts known in the art to be generally suitable for use in contact with a patient include salts derived from inorganic and/or organic acids, including the hydrobromide, hydrochloride, sulfate, bisulfate, nitrate, acetate, oxalate, oleate, palmitate, stearate, laurate, benzoate, lactate, phosphate, tosylate, citrate, maleate, fumarate, succinate and tartrate. These may include cations based on the alkali and alkaline earth metals, such as sodium, potassium, calcium and magnesium, as well as ammonium, tetramethylammonium, tetraethylammonium. Further reference is made to the number of literature sources that survey suitable pharmaceutically acceptable salts, for example the handbook of pharmaceutical salts published by IUPAC.

In addition, the compounds for use in accordance with the present invention may sometimes exist as zwitterions, which are considered as part of the invention.

It is demonstrated in the accompanying Examples that compounds provided herein are potent USP19 inhibitors and further that potent USP19 inhibitory compounds effectively treat a number of disease conditions. Taken together, the *in vitro* and *in vivo* data demonstrate that compounds which potently inhibit USP19 activity can be effective therapeutic compounds.

Accordingly, in a first aspect the invention provides a compound, or a pharmaceutically acceptable salt, tautomer, stereoisomer or *N*-oxide derivative thereof, for use in a method of treatment according to the claims, wherein the compound is a compound according to formula (I): 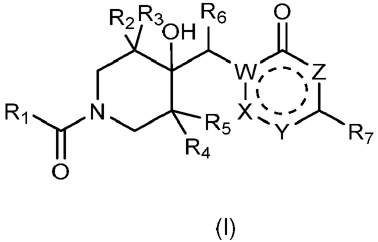 wherein:
R₁ is an optionally substituted alkyl, alkenyl, alkynyl, ether, cycloalkyl, alkylcycloalkyl, heterocycloalkyl, alkylheterocycloalkyl, aryl, arylalkyl, heteroaryl, or heteroarylalkyl group;
R₂ is H or an optionally substituted alkyl group,
R₃ is H or an optionally substituted alkyl group,
R₄ is H or an optionally substituted alkyl group,
R₅ is H or an optionally substituted alkyl group,
R₂ and R₄ may be joined to one another to form an optionally substituted cycloalkyl or heterocycloalkyl that includes the carbon to which they are attached,
R₄ and R₅ may be joined to one another to form an optionally substituted cycloalkyl or heterocycloalkyl that includes the carbon to which they are attached;
R₆ is H or an optionally substituted alkyl group;
W is C or N;
X is N or CR₈, wherein R₈ is H or optionally substituted C1-C6 alkyl,
Y is N or CR₉
Z is CH, or NH,
wherein R₉ is H or optionally substituted alkyl, amido, amino, aryl, heteroaryl, cycloalkyl, cycloheteroalkyl, halo, carbonyl, ester, aminoalkyl, or cyano;
R₇ is hydrogen, halo, or an optionally substituted alkyl, alkenyl, alkynyl, amino, aryl, cycloalkyl, cycloalkenyl, alkoxy, aryloxy, heteroaryl or heterocycloalkyl group;

or the compound 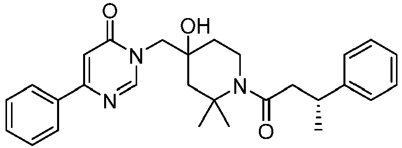
or a pharmaceutically acceptable salt, tautomer, stereoisomer or *N*-oxide derivative thereof.

It is demonstrated herein that the potent USP19 inhibitory compounds provided herein effectively reduce fat accumulation *in vivo.* Gene knockout studies have described a possible association between USP19 and fat accumulation (Coyne et al, Diabetologia. 2018 Nov 1. doi: 10.1007/s00125-018-4754-4.). However, the effects seen in these studies need to be considered alongside the possible confounding factors inherent in knockout studies such as altered developmental or underlying physiological processes. For these reasons, acute or chronic pharmacological inhibition of an enzyme does not always result in similar physiological outcomes to genetic ablation.

The data provided herein is the first demonstration that pharmacological inhibition of USP19 can reduce fat accumulation in a wild-type background. Taken together, the *in vitro* and *in vivo* data demonstrate that compounds which potently inhibit USP19 activity can effectively treat obesity.

Accordingly, in a further aspect is provided a USP19 inhibitor for use in a method of treating obesity.

In a further aspect is provided a compound as defined in relation to the first aspect, or a pharmaceutically acceptable salt, tautomer, stereoisomer or *N*-oxide derivative thereof, for use in a method of treating obesity.

It is further demonstrated herein that the potent USP19 inhibitory compounds provided herein can effectively treat insulin resistance. Gene knockout studies have described an association between USP19 and insulin sensitivity (Coyne *et al*, *supra*). Coyne *et al.* describe an improvement in insulin sensitivity in USP19 knockout mice but, as noted above, it could not be assumed that the effects would translate to pharmacological inhibition of USP19 in wild-type subjects.

The data provided herein is the first demonstration that pharmacological inhibition of USP19 can effectively treat insulin resistance.

Accordingly, in a further aspect of the invention is provided a compound as defined in relation to the first aspect of the invention, or a pharmaceutically acceptable salt, tautomer, stereoisomer or *N*-oxide derivative thereof, for use in a method of treating insulin resistance. In a further aspect of the invention is provided a compound as defined in relation to the first aspect of the invention, or a pharmaceutically acceptable salt, tautomer, stereoisomer or *N*-oxide derivative thereof, for use in a method of treating type II diabetes.

It is demonstrated in the accompanying Examples that compounds provided herein are potent USP19 inhibitors and further that potent USP19 inhibitory compounds effectively treat muscle loss in an *in vivo* disease model. Taken together, the *in vitro* and *in vivo* data demonstrate that compounds which potently inhibit USP19 activity can effectively treat muscular atrophy.

Presently disclosed but not claimed is a USP19 inhibitor for use in a method of treating muscular atrophy.

Presently disclosed but not claimed is a compound as defined in relation to the first aspect of the invention, or a pharmaceutically acceptable salt, tautomer, stereoisomer or *N*-oxide derivative thereof, for use in a method of treating muscular atrophy. Presently disclosed but not claimed is a compound as defined in relation to the first aspect, or a pharmaceutically acceptable salt, tautomer, stereoisomer or *N*-oxide derivative thereof, for use in a method of treating cachexia or sarcopenia.

### Compounds for use according to the invention

In all aspects, the compound (or pharmaceutically acceptable salt, tautomer, stereoisomer or *N*-oxide derivative thereof) for use in methods of therapy according to the invention is a compound according to formula (I): 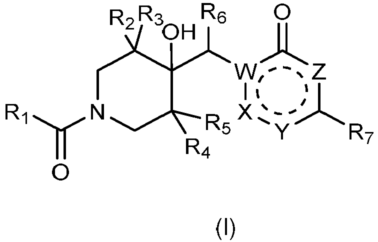 wherein:
R₁ is an optionally substituted alkyl, alkenyl, alkynyl, ether, cycloalkyl, alkylcycloalkyl, heterocycloalkyl, alkylheterocycloalkyl, aryl, arylalkyl, heteroaryl, or heteroarylalkyl group;
R₂ is H or an optionally substituted alkyl group,
R₃ is H or an optionally substituted alkyl group,
R₄ is H or an optionally substituted alkyl group,
R₅ is H or an optionally substituted alkyl group,
R₂ and R₄ may be joined to one another to form an optionally substituted cycloalkyl or heterocycloalkyl that includes the carbon to which they are attached,
R₄ and R₅ may be joined to one another to form an optionally substituted cycloalkyl or heterocycloalkyl that includes the carbon to which they are attached;
R₆ is H or an optionally substituted alkyl group;
W is C or N;
X is N or CR₈, wherein R₈ is H or optionally substituted C1-C6 alkyl,
Y is N or CR₉
Z is CH, or NH,
wherein R₉ is H or optionally substituted alkyl, amido, amino, aryl, heteroaryl, cycloalkyl, cycloheteroalkyl, halo, carbonyl, ester, aminoalkyl, or cyano;
R₇ is hydrogen, halo, or an optionally substituted alkyl, alkenyl, alkynyl, amino, aryl, cycloalkyl, cycloalkenyl, alkoxy, aryloxy, heteroaryl or heterocycloalkyl group;
or the compound

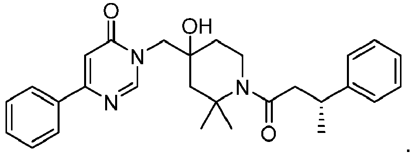

In certain embodiments of all aspects of the invention, R₁ is optionally substituted C1-C6 alkyl, optionally substituted C4-C10 alkylcycloalkyl, optionally substituted C7-C10 arylalkyl, optionally substituted C3-C6 cycloalkyl, optionally substituted C4-C5 heterocycloalkyl, or optionally substituted C3-C6 heteroaryl,
wherein each optional substituent is independently selected from C1-C6 alkyl, C2-C6 alkenyl, hydroxymethyl, methoxymethyl, benzyloxy methyl, phenyl, C3-C6 cycloalkyl, CF₃, CHF₂, OH, or halo.

In certain embodiments, R₁ is optionally substituted C7-C10 cyclohexylalkane, optionally substituted C4-C6 cyclopropylalkane, optionally substituted C5-C6 cyclobutylalkane, optionally substituted C7-C10 alkylbenzene, optionally substituted 2,2 difluorobutane, or optionally substituted 3,3,3-trifluoropropane,
wherein each optional substituent is independently selected from C1-C6 alkyl, C2-C6 alkenyl, hydroxymethyl, hydroxyethyl, methoxymethyl, OH, or halo.

In certain embodiments, R₁ is substituted and the substituent is C1-C6 alkyl, optionally methyl.

In certain preferred embodiments, R₁ is methyl substituted cyclohexylethane or methyl substituted ethylbenzene.

In certain preferred embodiments, R₁ is:

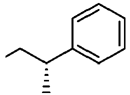

In certain preferred embodiments, R₁ is:

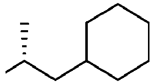

In certain preferred embodiments, R₁ is methyl substituted 2,2 difluorobutane, or methyl substituted 3,3,3-trifluoropropane.

In certain preferred embodiments, R₁ is:

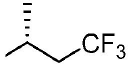

In certain preferred embodiments, R₁ is:

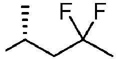

In certain preferred embodiments, R₁ is C4-C5 heterocycloalkyl, optionally substituted with phenyl.

In certain preferred embodiments R₁ is phenyl-substituted pyrrolidine or phenyl-substituted piperidine.

In certain preferred embodiments, R₁ forms the following group together with the carbonyl to which it is attached:

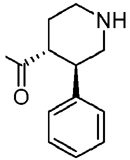

In certain preferred embodiments, R₁ forms the following group together with the carbonyl to which it is attached:

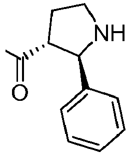

In certain preferred embodiments, R₁ forms the following group together with the carbonyl to which it is attached:

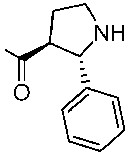

In certain preferred embodiments, R₁ forms the following group together with the carbonyl to which it is attached:

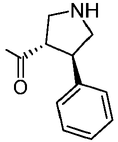

In certain preferred embodiments, R₁ forms the following group together with the carbonyl to which it is attached:

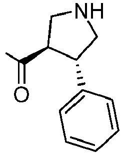

In certain embodiments of all aspects of the invention R₇ is optionally substituted C6-C10 aryl, C1-C12 heteroaryl, C1-C10 alkyl, C2-C10 alkenyl, C3-C10 cycloalkyl, amino, C1-C3 alkoxy, or halo,
wherein the optional substituent is selected from C1-C6 alkyl, hydroxysubstituted C1-C6 alkyl, C3-C6 cycloalkyl, C1-C6 heterocycloalkyl, C1-C6 alkoxy, halo-substituted C1-C6 alkoxy, amido, cyano or halo.

In certain preferred embodiments, R₇ is cyclopropyl, thiophene, or optionally substituted phenyl,
wherein the optional substituent is selected from C1-C6 alkyl, hydroxysubstituted C1-C6 alkyl, C1-C6 alkylamine, and halo.

In certain preferred embodiments, R₇ is phenyl.

In certain preferred embodiments, R₇ is fluoro-substituted phenyl. In certain preferred such embodiments, the phenyl has a single fluoro substituent. In certain preferred such embodiments, the fluoro substituent is in the *ortho* or *meta* position,

In certain embodiments of all aspects of the invention, R₂, R₃, R₄, R₅ are independently selected from H and C1-C6 alkyl.

In certain preferred embodiments, R₂ and R₃ are H, and R₄ and R₅ are methyl.

In certain preferred embodiments, R₄ and R₅ are joined to one another to form an optionally substituted C3-C6 cycloalkyl or C3-C6 heterocycloalkyl that includes the carbon to which they are attached and R₂ and R₃ are independently selected from H and C1-C6 alkyl.

In certain preferred embodiments, R₂ and R₃ are H and R₄ and R₅ are joined to one another to form a C3-C6 cycloalkyl that includes the carbon to which they are attached.

In certain preferred embodiments, R₄ and R₅ are joined to one another to form a C4 cycloalkyl that includes the carbon to which they are attached.

In certain preferred embodiments, R₄ and R₅ are joined to one another to form a C5 cycloalkyl that includes the carbon to which they are attached.

In certain preferred embodiments, R₆ is H or C1-C6 alkyl.

In certain embodiments, R₂ and R₄ are joined to one another to form a C5 cycloalkyl that includes the carbons to which they are attached, and wherein R₃, R₅ and R₆ are independently H or C1-C6 alkyl.

In certain preferred embodiments, W is N and X is CR₈, wherein R₈ is H or methyl.

In certain preferred embodiments, Y is N or CR₉, wherein R₉ is H, C1-C6 alkyl, NR'R", C(O)NR'R", cyano, carboxyl, halo, C1-C6 alkylamine, C3-C6 alkylester, optionally substituted C6-C10 aryl or optionally substituted C2-C6 heteroaryl, wherein the one or more heteroatoms are selected from N and O, and the one or more optional substituents of the aryl or heteroaryl are selected from C1-C6 alkyl, C1-C6 alkylamine, amido, and cyano,
wherein R' and R" are independently selected from H, C1-C6 alkyl optionally substituted with OH, C3-C7 cycloalkyl, C1-C7 heterocycloalkyl, C4-C7 alkylcycloalkyl, C3-C7 alkylheterocycloalkyl, benzyl, phenyl, and methoxy, or wherein R' and R" are joined to one another to form a C2-C7 heterocycle that includes the N to which they are attached, wherein the heterocycle is optionally hydroxyl-substituted, oxo-substituted, methyl-substituted, CH2OH-substituted, or acetyl-substituted.

In certain preferred embodiments, Y is N or CR₉, wherein R₉ is selected from: phenyl optionally substituted with amido, cyano or methyl amine; pyridine; oxazole; pyrazole; carboxyl; C(O)NR'R"; or NR'R";
wherein R' and R" are independently selected from H, C1-C6 alkyl, C3-C7 cycloalkyl, C3-C7 heterocycloalkyl wherein the heteroatom is N or O, or wherein R' and R" are joined to one another to form a C2-C7 heterocycloalkyl that includes the N to which they are attached, wherein the heterocycloalkyl is optionally hydroxyl-substituted, oxo-substituted, methyl-substituted, CH2OH-substituted, or acetyl-substituted.

In a preferred embodiment of all aspects of the invention, the compound (or pharmaceutically acceptable salt, tautomer, stereoisomer or N-oxide derivative thereof) for use in methods according to the invention is a compound according to formula (I), wherein
R₁ is selected from optionally substituted ethylcyclohexane, optionally substituted ethylbenzene, optionally substituted 2,2 difluorobutane, and optionally substituted 3,3,3-trifluoropropane, wherein the optional substituent is one or more of methyl, ethyl, propyl, propenyl, hydroxymethyl and methoxymethyl, or wherein R₁ is phenyl-substituted pyrrolidine,
R₂ and R₃ are independently selected from H, methyl,
R₄ and R₅ are independently selected from H, methyl, or are joined to one another to form a C3-C6 cycloalkyl or heterocycloalkyl that includes the carbon to which they are attached, optionally a C4 or C5 cycloalkyl that includes the carbon to which they are attached
R₆ is H or methyl,
R₇ is selected from optionally substituted phenyl, indazole, thiophene, or pyrazole, wherein the optional substituent is selected from C1-C6 alkyl, hydroxysubstituted C1-C6 alkyl, C3-C6 cycloalkyl, C1-C6 heterocycloalkyl, C1-C6 alkoxy, halo-substituted C1-C6 alkoxy, C1-C6 alkylamine, amido, cyano or halo,
W is N or C
X is CH or N
Z is C or NH
Y is N or CR₉, wherein R₉ is selected from: phenyl optionally substituted with amido, cyano or methyl amine; pyridine; oxazole; pyrazole; C(O)NR'R"; or NR'R";
wherein R' and R" are independently selected from H, C1-C6 alkyl, C3-C7 cycloalkyl, C3-C7 heterocycloalkyl wherein the heteroatom is N or O, or wherein R' and R" are joined to one another to form a C2-C7 heterocycloalkyl that includes the N to which they are attached, wherein the heterocycloalkyl is optionally hydroxyl-substituted, oxo-substituted, methyl-substituted, hydroxymethyl-substituted, or acetyl-substituted.

In certain preferred embodiments of all aspects of the invention Y is CR₉, wherein R₉ is C(O)NR'R" and wherein R' and R" are joined to one another to form an optionally substituted pyrrolidine, piperidine, piperazine or morpholine that includes the N to which they are attached, wherein the piperidine, pyrrolidine, piperazine or morpholine is optionally hydroxyl-substituted, oxo-substituted, methyl-substituted, hydroxymethyl-substituted, or acetyl-substituted.

In certain preferred embodiments Y is CR₉, wherein R₉ is C(O)NR'R" and wherein R' and R" are joined to one another to form a piperazinyl ring.

In certain preferred embodiments, W is N and Y is N, such that the ring comprising W, X, Y, Z is a substituted pyrimidinone ring.

It will be appreciated by the skilled person that some compounds according to formula I for use according to the invention will be chiral at the carbon of the piperadinyl ring bound to the hydroxyl group. In certain preferred embodiments of all aspects of the invention, when the compound is chiral at this position, the compound is the S enantiomer. In certain alternative preferred embodiments of all aspects of the invention, when the compound is chiral at this position, the compound is the R enantiomer.

In certain embodiments of all aspects of the invention the compound is:
(R)-5-Bromo-1-((4-hydroxy-1-(3-phenylbutanoyl)piperidin-4-yl)methyl)-4-phenylpyridin-2(1*H*)-one;
6-(2-Fluorophenyl)-3-((4-hydroxy-1-(3-phenylpropanoyl)piperidin-4-yl)methyl)pyrimidin-4(3*H*)-one;
3-((1-(3-Cyclobutylpropanoyl)-4-hydroxypiperidin-4-yl)methyl)-6-(2-fluorophenyl)pyrimidin-4(3*H*)-one;
3-((1-(3-Cyclopentylpropanoyl)-4-hydroxypiperidin-4-yl)methyl)-6-(2-fluorophenyl)pyrimidin-4(3*H*)-one;
(*R*,*S*)-6-(2-Fluorophenyl)-3-((4-hydroxy-1-(2-methyl-3-phenylpropanoyl)piperidin-4-yl)methyl)pyrimidin-4(3*H*)-one;
(*R*)-*N*-(3-(1-((4-Hydroxy-1-(3-phenylbutanoyl)piperidin-4-yl)methyl)-6-oxo-4-phenyl-1,6-dihydropyridin-3-yl)phenyl)acetamide;
(*R*)-3-(1-((4-Hydroxy-1-(3-phenylbutanoyl)piperidin-4-yl)methyl)6-oxo-4-phenyl-1,6-dihydropyridin-3-yl)benzamide;
(*R*)-5-(Furan-2-yl)-1-((4-hydroxy-1-(3-phenylbutanoyl)piperidin-4-yl)methyl)-4-phenylpyridin-2(1*H*)-one;
(*R*)-1-((4-Hydroxy-1-(3-phenylbutanoyl)piperidin-4-yl)methyl)-5-(1-methyl-1*H*-pyrazol-5-yl)-4-phenylpyridin-2(1*H*)-one;
(*R*)-4-Chloro-1-((4-hydroxy-1(3-phenylbutanoyl)piperidin-4-yl)methyl)-5-phenylpyridin-2(1*H*)-one;
(*R*)-4-(Dimethylamino)-1-((4-hydroxy-1-(3-phenylbutanoyl)piperidin-4-yl)methyl)-5-phenylpyridin-2(1*H*)-one;
(*R*)-1-((4-Hydroxy-1-(3-phenylbutanoyl)piperidin-4-yl)methyl)-5-phenyl-4-(prop-1-en-2-yl)pyridine-2(1*H*)-one;
(*R*)-Ethyl 1-((4-hydroxy-1-(3-phenylbutanoyl)piperidin-4-yl)methyl)-6-oxo-4-phenyl-1,6-dihydropyridine-3-carboxylate;
(*R*,*S*)-4-(2-Fluorophenyl)-1-((4-hydroxy-1-(2-methyl-1,2,3,4-tetrahydronaphthalene-2-carbonyl)piperidin-4-yl)methyl)-*N*-isopropyl-*N*-methyl-6-oxo-1,6-dihydropyridine-3-carboxamide;
(*R*,*S*)-1-((1-(3-Cyclobutyl-2-methylpropanoyl)-4-hydroxypiperidin-4-yl)methyl)-4-phenyl-5-(piperidine-1-carbonyl)pyridin-2(1*H*)-one;
(*R*)-3-((4-Hydroxy-1-(3-phenylbutanoyl)piperidin-4-yl)methyl)-6-(1-methyl-1*H*-indazol-7-yl)pyrimidin-4(3*H*)-one;
(*R*)-3-((4-Hydroxy-1-(3-phenylbutanoyl)piperidin-4-yl)methyl)-6-(2-methoxyphenyl)pyrimidin-4(3*H*)-one;
(*R*)-3-((4-Hydroxy-1-(3-phenylbutanoyl)piperidin-4-yl)methyl)-2-methyl-6-phenylpyrimidin-4(3*H*)-one;
(*R*)-2-((4-Hydroxy-1-(3-phenylbutanoyl)piperidin-4-yl)methyl)-5,6-diphenylpyridazin-3(2*H*)-one;
(*R*)-3-((4-Hydroxy-1-(3-phenylbutanoyl)piperidin-4-yl)methyl)-6-(naphthalen-1-yl)pyrimidin-4(3*H*)-one;
(*R*)-6-(3-(1,3-Dioxolan-2-yl)phenyl)-3-((4-hydroxy-1 -(3-phenylbutanoyl)piperidin-4-yl)methyl)pyrimidin-4(3*H*)-one;
(*R*)-3-((4-Hydroxy-1-(3-phenylbutanoyl)piperidin-4-yl)methyl)-6-(thiophen-3-yl)pyrimidin-4(3*H*)-one;
4-(2-Fluorophenyl)-1-((4-hydroxy-1-(1-methylcyclopentane-1-carbonyl)piperidin-4-yl)methyl)-*N*-isopropyl-*N*-methyl-6-oxo-1,6-dihydropyridine-3-carboxamide;
3-((1-(1-Ethylcyclohexane-1-carbonyl)-4-hydroxypiperidin-4-yl)methyl)-6-(2-fluorophenyl)pyrimidin-4(3*H*)-one;
3-((1-(2-(Cyclohexylmethyl)-3-methylbutanoyl)-4-hydroxy-3,3-dimethylpiperidin-4-yl)methyl)-6-(2-fluorophenyl)pyrimidin-4(3*H*)-one;
(*R*)-6-(Furan-2-yl)-3-((4-hydroxy-1-(3-phenylbutanoyl)piperidin-4-yl)methyl)pyrimidin-4(3*H*)-one;
(*R*)-1-((4-Hydroxy-1-(3-phenylbutanoyl)piperidin-4-yl)methyl)-4-phenylpyridin-2(1*H*)-one;
(*R*)-6-(Cyclohex-1-en-1-yl)-3-((4-hydroxy-1-(3-phenylbutanoyl)piperidin-4-yl)methyl)pyrimidin-4(3*H*)-one;
(R)-3-((4-Hydroxy-1-(3-phenylbutanoyl)piperidin-4-yl)methyl)-6-(prop-1-en-2-yl)pyrimidin-4(3*H*)-one;
(*R*)-3-((4-Hydroxy-1-(3-phenylbutanoyl)piperidin-4-yl)methyl)-6-(2-(trifluoromethoxy)phenyl)pyrimidin-4(3*H*)-one;
3-(((1*R*,5*S*)-3-((*R*)-3-Cyclohexyl-2-methylpropanoyl)-8-hydroxy-3-azabicyclo[3.2.1]octan-8-yl)methyl)-6-(2-fluorophenyl)pyrimidin-4(3*H*)-one;
(*R*)-6-(2-Fluorophenyl)-3-((4-hydroxy-1-(3-phenylbutanoyl)piperidin-4-yl)methyl)pyrimidin-4(3*H*)-one;
(*R*)-5-((4-Hydroxy-1-(3-phenylbutanoyl)piperidin-4-yl)methyl)-2-phenylpyrimidin-4(3*H*)-one;
3-((4-Hydroxy-1-(3-phenylpropanoyl)piperidin-4-yl)methyl)-2-methyl-6-phenylpyrimidin-4(3*H*)-one;
(*R*)-3-((4-Hydroxy-1-(3-phenylbutanoyl)piperidin-4-yl)methyl)-6-(isobutylamino)pyrimidin-4(3*H*)-one;
(*R*)-1-((1-(3-Cyclohexyl-2-methylpropanoyl)-4-hydroxypiperidin-4-yl)methyl)-4-phenyl-5-(piperidine-1-carbonyl)pyridin-2(1*H*)-one;
(*R*)-2-((4-Hydroxy-1-(3-phenylbutanoyl)piperidin-4-yl)methyl)-5-phenylpyridazin-3(2*H*)-one;
(*R*)-4-(2-Cyanophenyl)-1-((4-hydroxy-1-(3-phenylbutanoyl)piperidin-4-yl)methyl)-*N-*isopropyl-*N*-methyl-6-oxo-1,6-dihydropyridine-3-carboxamide;
(*R*)-3-((1-(3-Cyclohexyl-2-methylpropanoyl)-4-hydroxypiperidin-4-yl)methyl)-6-(2-fluorophenyl)pyrimidin-4(3*H*)-one;
3-((1-(2-(Cyclohexylmethyl)butanoyl)-4-hydroxypiperidin-4-yl)methyl)-6-(2-fluorophenyl)pyrimidin-4(3*H*)-one;
*rac*-6-(2-Fluorophenyl)-3-((4-hydroxy-1-(cis-2-phenylcyclopropanecarbonyl)piperidin-4-yl)methyl)pyrimidin-4(3*H*)-one;
6-(2-Fluorophenyl)-3-((4-hydroxy-1-(1-methylcyclohexanecarbonyl)piperidin-4-yl)methyl)pyrimidin-4(3*H*)-one;
3-((*S*)-1-(1-((*R*)-3-Cyclohexyl-2-methylpropanoyl)-4-hydroxypiperidin-4-yl)propyl)-6-phenylpyrimidin-4(3*H*)-one;
3-((*R*)-1-(1-((*R*)-3-Cyclohexyl-2-methylpropanoyl)-4-hydroxypiperidin-4-yl)propyl)-6-phenylpyrimidin-4(3*H*)-one;
(*R,S*)-3-((1-(3-Cyclohexyl-2-hydroxypropanoyl)-4-hydroxypiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-6-oxo-4-phenyl-1,6-dihydropyridine-3-carbaldehyde;
(*R*)-5-((Dimethylamino)methyl)-1-((4-hydroxy-1-(3-phenylbutanoyl)piperidin-4-yl)methyl)-4-phenylpyridin-2(1*H*)-one;
3-((*R*)-1-(4-Hydroxy-1-((*R*)-3-phenylbutanoyl)piperidin-4-yl)ethyl)-6-phenylpyrimidin-4(3*H*)-one;
3-((*S*)-1-(4-Hydroxy-1-((*R*)-3-phenylbutanoyl)piperidin-4-yl)ethyl)-6-phenylpyrimidin-4(3*H*)-one;
(*R*)-6-(2-Fluorophenyl)-3-((4-hydroxy-1-(4,4,4-trifluoro-3-phenylbutanoyl)piperidin-4-yl)methyl)pyrimidin-4(3*H*)-one;
(*R*,*S*)-3-((1-(3-Cyclohexyl-2-fluoropropanoyl)-4-hydroxypiperidin-4-yl)methyl)-6-(2-fluorophenyl)pyrimidin-4(3*H*)-one;
3-(((*R*)-1-((*R*)-3-Cyclohexyl-2-methylpropanoyl)-4-hydroxy-3,3-dimethylpiperidin-4-yl)methyl)-6-phenylpyrimidin-4(3*H*)-one;
3-(((*S*)-1-((*R*)-3-Cyclohexyl-2-methylpropanoyl)-4-hydroxy-3,3-dimethylpiperidin-4-yl)methyl)-6-phenylpyrimidin-4(3*H*)-one;
3-(((*R*)-6-((*R*)-3-Cyclohexyl-2-methylpropanoyl)-9-hydroxy-6-azaspiro[3.5]nonan-9-yl)methyl)-6-(2-fluorophenyl)pyrimidin-4(3*H*)-one;
3-(((*S*)-6-((*R*)-3-Cyclohexyl-2-methylpropanoyl)-9-hydroxy-6-azaspiro[3.5]nonan-9-yl)methyl)-6-(2-fluorophenyl)pyrimidin-4(3*H*)-one;
(*R*)-5-(3-(Aminomethyl)phenyl)-1-((1-(3-cyclohexyl-2-methylpropanoyl)-4-hydroxypiperidin-4-yl)methyl)-4-(2-fluorophenyl)pyridin-2(1*H*)-one;
3-(((*S*)-4-Hydroxy-3,3-dimethyl-1-((*R*)-3-phenylbutanoyl)piperidin-4-yl)methyl)-6-phenylpyrimidin-4(3*H*)-one;
3-(((*R*)-4-Hydroxy-3,3-dimethyl-1-((*R*)-3-phenylbutanoyl)piperidin-4-yl)methyl)-6-phenylpyrimidin-4(3*H*)-one;
(*R*)-6-(4-Fluorophenoxy)-3-((4-hydroxy-1-(3-phenylbutanoyl)piperidin-4-yl)methyl)pyrimidin-4(3*H*)-one;
(*R*)-1-((4-Hydroxy-1-(3-phenylbutanoyl)piperidin-4-yl)methyl)-5-(2-oxopyrrolidin-1-yl)-4-phenylpyridin-2(1*H*)-one;
6-(2-Fluorophenyl)-3-((4-hydroxy-1-(1-methyl-4-methylenecyclohexanecarbonyl)piperidin-4-yl)methyl)pyrimidin-4(3*H*)-one;
(*R*,*S*)-3-((1-(3-Cyclobutyl-2-methylpropanoyl)-4-hydroxypiperidin-4-yl)methyl)-6-(2-fluorophenyl)pyrimidin-4(3*H*)-one;
(*R*,*S*)-3-((1-(3-Cycloheptyl-2-methylpropanoyl)-4-hydroxypiperidin-4-yl)methyl)-6-(2-fluorophenyl)pyrimidin-4(3*H*)-one;
3-((1-(3-Cyclobutyl-2,2-dimethylpropanoyl)-4-hydroxypiperidin-4-yl)methyl)-6-(2-fluorophenyl)pyrimidin-4(3*H*)-one;
6-(2-Fluorophenyl)-3-(((1*R*,5*S*)-8-hydroxy-3-((*R*)-3-phenylbutanoyl)-3-azabicyclo[3.2.1]octan-8-yl)methyl)pyrimidin-4(3*H*)-one;
3-(((*R*)-4-Hydroxy-2,2-dimethyl-1-((R)-3-phenylbutanoyl)piperidin-4-yl)methyl)-6-phenylpyrimidin-4(3*H*)-one;
3-(((*S*)-4-Hydroxy-2,2-dimethyl-1-((*R*)-3-phenylbutanoyl)piperidin-4-yl)methyl)-6-phenylpyrimidin-4(3*H*)-one;
(*R*)-1-((4-Hydroxy-1-(3-phenylbutanoyl)piperidin-4-yl)methyl)-5-(4-hydroxypiperidine-1-carbonyl)-4-phenylpyridin-2(1*H*)-one;
(*R*)-1-((4-Hydroxy-1-(3-phenylbutanoyl)piperidin-4-yl)methyl)-4-phenyl-5-(pyrrolidine-1-carbonyl)pyridin-2(1*H*)-one;
(*R*)-1-((4-Hydroxy-1-(3-phenylbutanoyl)piperidin-4-yl)methyl)-4-phenyl-5-(1,4-dioxa-8-azaspiro[4.5]decane-8-carbonyl)pyridin-2(1*H*)-one;
(*R*)-*N*-Cyclohexyl-1-((4-hydroxy-1-(3-phenylbutanoyl)piperidin-4-yl)methyl)-6-oxo-4-phenyl-1,6-dihydropyridine-3-carboxamide;
6-(2-Fluorophenyl)-3-((4-hydroxy-3,3-dimethyl-1-(*cis*-2-phenylcyclopropane-1-carbonyl)piperidin-4-yl)methyl)pyrimidin-4(3*H*)-one;
(*R*,*S*)-3-((1-(3-Cyclohexyl-1H-pyrazole-4-carbonyl)-4-hydroxy-3,3-dimethylpiperidin-4-yl)methyl)-6-(2-fluorophenyl)pyrimidin-4(3*H*)-one;
(*R*,*S*)-6-(2-Fluorophenyl)-3-((4-hydroxy-3,3-dimethyl-1-(3-phenylpropanoyl)piperidin-4-yl)methyl)pyrimidin-4(3*H*)-one;
(*R*,*S*)-6-(2-Fluorophenyl)-3-((4-hydroxy-1-(1-isobutyl cyclopropane-1-carbonyl)-3,3-dimethylpiperidin-4-yl)methyl)pyrimidin-4(3*H*)-one;
(*R*,*S*)-6-(2-Fluorophenyl)-3-((4-hydroxy-3,3-dimethyl-1-(3-methyl-3-phenylbutanoyl)piperidin-4-yl)methyl)pyrimidin-4(3*H*)-one;
(*S*)-1-((1-(4,4-Difluoro-3-phenylbutanoyl)-4-hydroxypiperidin-4-yl)methyl)-*N*-isopropyl-*N-*methyl-6-oxo-4-phenyl-1,6-dihydropyridine-3-carboxamide;
1-((4-Hydroxy-1-(1-(thiophen-2-yl)cyclopropane-1-carbonyl)piperidin-4-yl)methyl)-*N-*isopropyl-*N*-methyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((S)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
(*R*)-3-((4-Hydroxy-1-(3-phenylbutanoyl)piperidin-4-yl)methyl)-6-(2-(hydroxymethyl)phenyl)pyrimidin-4(3*H*)-one;
(*R*)-*N*,*N*-Diethyl-1-((4-hydroxy-1-(3-phenylbutanoyl)piperidin-4-yl)methyl)-6-oxo-4-phenyl-1,6-dihydropyridine-3-carboxamide;
3-((1-(3-Cyclohexylbutanoyl)-4-hydroxy-3,3-dimethylpiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-5-(morpholine-4-carbonyl)-4-phenylpyridin-2(1*H*)-one;
(*R*,*S*)-3-((1-(3-Cyclohexylpropanoyl)-4-hydroxy-3,3-dimethylpiperidin-4-yl)methyl)-6-(2-fluorophenyl)pyrimidin-4(3*H*)-one;
(*R*,*S*)-1-((1-(3-Cyclobutylpropanoyl)-4-hydroxy-3,3-dimethylpiperidin-4-yl)methyl)-*N-*isopropyl-*N*-methyl-6-oxo-4-phenyl-1,6-dihydropyridine-3-carboxamide;
(*R*,*S*)-1-((1-(2-Ethylhexanoyl)-4-hydroxypiperidin-4-yl)methyl)-*N*-isopropyl-*N*-methyl-6-oxo-4-phenyl-1,6-dihydropyridine-3-carboxamide;
(*R*,*S*)-6-(2-Fluorophenyl)-3-((4-hydroxy-3,3-dimethyl-1-(1-methylcyclohexane-1-carbonyl)piperidin-4-yl)methyl)pyrimidin-4(3*H*)-one;
6-(2-Fluorophenyl)-3-((1-(3-(4-fluorophenyl)propanoyl)-4-hydroxypiperidin-4-yl)methyl)pyrimidin-4(3*H*)-one;
(*R*,*S*)-3-((1-(3-Cyclopropylpropanoyl)-4-hydroxy-3,3-dimethylpiperidin-4-yl)methyl)-6-(2-fluorophenyl)pyrimidin-4(3*H*)-one;
1-((1-(2,2-Dimethylbutanoyl)-4-hydroxypiperidin-4-yl)methyl)-*N*-isopropyl-*N*-methyl-6-oxo-4-phenyl-1,6-dihydropyridine-3-carboxamide;
(*R*)-*N*-(2-(1-((4-Hydroxy-1-(3-phenylbutanoyl)piperidin-4-yl)methyl)-6-oxo-1,6-dihydropyrimidin-4-yl)phenyl)acetamide;
(*R*)-5-(4-(Aminomethyl)phenyl)-1-((4-hydroxy-1-(3-phenylbutanoyl)piperidin-4-yl)methyl)-4-phenylpyridin-2(1*H*)-one;
(*R*)-1-((1-(3-Cyclohexyl-2-methylpropanoyl)-4-hydroxypiperidin-4-yl)methyl)-*N*-isopropyl-*N-*methyl-6-oxo-4-phenyl-1,6-dihydropyridine-3-carboxamide;
(*R*)-1-((4-Hydroxy-1-(3-phenylbutanoyl)piperidin-4-yl)methyl)-4-phenyl-5-(piperidine-1-carbonyl)pyridin-2(1*H*)-one;
(*R*)-1-((4-Hydroxy-1-(3-phenylbutanoyl)piperidin-4-yl)methyl)-*N*-isopropyl-4-(2-methoxyphenyl)-*N*-methyl-6-oxo-1,6-dihydropyridine-3-carboxamide;
(*R*)-4-(2-Fluorophenyl)-1-((4-hydroxy-1-(3-phenylbutanoyl)piperidin-4-yl)methyl)-*N-*isopropyl-*N*-methyl-6-oxo-1,6-dihydropyridine-3-carboxamide;
(*R*)-1'-((4-Hydroxy-1 -(3-phenylbutanoyl)piperidin-4-yl)methyl)-4'-phenyl-[2,3'-bipyridin]-6'(1'*H*)-one;
1-((1-(3-Cyclohexylpropanoyl)-4-hydroxypiperidin-4-yl)methyl)-*N*-isopropyl-*N*-methyl-6-oxo-4-phenyl-1,6-dihydropyridine-3-carboxamide;
(*R*)-tert-Butyl 4-(1-((4-hydroxy-1-(3-phenylbutanoyl)piperidin-4-yl)methyl)-6-oxo-4-phenyl-1,6-dihydropyridine-3-carbonyl)piperazine-1-carboxylate;
(*R*)-1-((4-Hydroxy-1-(3-phenylbutanoyl)piperidin-4-yl)methyl)-4-phenyl-5-(piperazine-1-carbonyl)pyridin-2(1*H*)-one;
(*R*)-1-((4-Hydroxy-1-(3-phenylbutanoyl)piperidin-4-yl)methyl)-*N*,*N*-dimethyl-6-oxo-4-phenyl-1,6-dihydropyridine-3-carboxamide;
(*R*)-1-((4-Hydroxy-1-(3-phenylbutanoyl)piperidin-4-yl)methyl)-*N*-isopropyl-*N*-methyl-6-oxo-4-(p-tolyl)-1,6-dihydropyridine-3-carboxamide;
(*R*)-1-((4-Hydroxy-1-(3-phenylbutanoyl)piperidin-4-yl)methyl)-*N*-methoxy-*N*-methyl-6-oxo-4-phenyl-1,6-dihydropyridine-3-carboxamide;
(*R*)-1-((4-Hydroxy-1-(3-phenylbutanoyl)piperidin-4-yl)methyl)-4,5-diphenylpyridin-2(1*H*)-one;
(*R*)-1-((4-Hydroxy-1-(3-phenylbutanoyl)piperidin-4-yl)methyl)-4-phenyl-[3,3'-bipyridin]-6(1*H*)-one;
(*R*)-5-(3-(Aminomethyl)phenyl)-1-((4-hydroxy-1-(3-phenylbutanoyl)piperidin-4-yl)methyl)-4-phenylpyridin-2(1*H*)-one;
(*R*)-1-((4-Hydroxy-1-(3-phenylbutanoyl)piperidin-4-yl)methyl)-*N*-isopropyl-*N*-methyl-6-oxo-4-phenyl-1,6-dihydropyridine-3-carboxamide;
(*R*)-1-((4-Hydroxy-1-(3-phenylbutanoyl)piperidin-4-yl)methyl)-*N*-isopropyl-*N*-methyl-6-oxo-4-(m-tolyl)-1,6-dihydropyridine-3-carboxamide;
(*R*)-1-((4-Hydroxy-1-(3-phenylbutanoyl)piperidin-4-yl)methyl)-4-phenyl-5-(pyrimidin-5-yl)pyridin-2(1*H*)-one;
(*R*)-*N*-(Cyclopropylmethyl)-1-((4-hydroxy-1-(3-phenylbutanoyl)piperidin-4-yl)methyl)-6-oxo-4-phenyl-1,6-dihydropyridine-3-carboxamide;
(*R*)-*N*-Benzyl-1-((4-hydroxy-1-(3-phenylbutanoyl)piperidin-4-yl)methyl)-*N*-methyl-6-oxo-4-phenyl-1,6-dihydropyridine-3-carboxamide;
(*R*)-2-(1-((4-Hydroxy-1-(3-phenylbutanoyl)piperidin-4-yl)methyl)-6-oxo-4-phenyl-1,6-dihydropyridin-3-yl)benzonitrile;
(*R*)-1-((4-Hydroxy-1-(3-phenylbutanoyl)piperidin-4-yl)methyl)-*N*-methyl-6-oxo-*N*,4-diphenyl-1,6-dihydropyridine-3-carboxamide;
3-(1-(1-((*R*)-3-Cyclohexyl-2-methylpropanoyl)-4-hydroxypiperidin-4-yl)ethyl)-6-phenylpyrimidin-4(3*H*)-one;
(*R*)-1-((4-Hydroxy-1-(3-phenylbutanoyl)piperidin-4-yl)methyl)-5-(1-methyl-1H-pyrazol-4-yl)-4-phenylpyridin-2(1H)-one;
(*R*)-3-((1-(2-(Cyclohexylmethyl)pent-4-enoyl)-4-hydroxypiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-5-methyl-4-phenylpyridin-2(1*H*)-one;
(*R*)-6-(1,5-Dimethyl-1*H*-pyrazol-4-yl)-3-((4-hydroxy-1 -(3-phenylbutanoyl)piperidin-4-yl)methyl)pyrimidin-4(3*H*)-one;
3-(((1*R*,5*S*)-3-(3-Cyclohexylpropanoyl)-8-hydroxy-3-azabicyclo[3.2.1]octan-8-yl)methyl)-6-(2-fluorophenyl)pyrimidin-4(3*H*)-one;
(*R*)-1-((4-Hydroxy-1-(3-phenylbutanoyl)piperidin-4-yl)methyl)-6-oxo-4-phenyl-1,6-dihydropyridine-3-carbonitrile;
(*R*)-1-((4-Hydroxy-1-(3-phenylbutanoyl)piperidin-4-yl)methyl)-*N*-(2-hydroxyethyl)-6-oxo-4-phenyl-1,6-dihydropyridine-3-carboxamide;
1-((4-Hydroxy-1-((*R*)-3-phenylbutanoyl)piperidin-4-yl)methyl)-5-((*S*)-2-methylpyrrolidine-1-carbonyl)-4-phenylpyridin-2(1*H*)-one;
(*R*)-1-((4-Hydroxy-1-(3-phenylbutanoyl)piperidin-4-yl)methyl)-5-(4-(hydroxymethyl)piperidine-1-carbonyl)-4-phenylpyridin-2(1*H*)-one;
3-((1-(2-(Cyclohexylmethyl)butanoyl)-4-hydroxy-3,3-dimethylpiperidin-4-yl)methyl)-6-(2-fluorophenyl)pyrimidin-4(3*H*)-one;
3-((7-((*R*)-3-Cyclohexyl-2-methylpropanoyl)-10-hydroxy-7-azaspiro[4.5]decan-10-yl)methyl)-6-(2-fluorophenyl)pyrimidin-4(3*H*)-one;
1-(((*S*)-1-((R)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((*S*)-1-((R)-3-Cyclohexyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*S*)-1-((R)-3-Cyclohexyl-2-methylpropanoyl)-4-hydroxy-3,3-dimethylpiperidin-4-yl)methyl)-4-phenyl-5-(pyrrolidine-1-carbonyl)pyridin-2(1*H*)-one;
1-(((*S*)-1-((R)-3-Cyclohexyl-2-methylpropanoyl)-4-hydroxy-3,3-dimethylpiperidin-4-yl)methyl)-4-phenyl-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-4-phenyl-5-(piperazine-1-carbonyl)pyridin-2(1*H*)-one;
3-((1-(2-(Cyclohexyloxy)propanoyl)-4-hydroxy-3,3-dimethylpiperidin-4-yl)methyl)-6-(2-fluorophenyl)pyrimidin-4(3*H*)-one;
1-((7-((*R*)-3-Cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((1-((*R*)-3-Cyclobutyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-((1-((*R*)-3-Cyclopropyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-((1-((*R*)-3-Cyclohexyl-2-methylpropanoyl)-4-hydroxy-3,3-dimethylpiperidin-4-yl)methyl)-[4,5'-bipyrimidin]-6(1*H*)-one;
3-((1-((*R*)-3-Cyclohexyl-2-methylpropanoyl)-4-hydroxy-3,3-dimethylpiperidin-4-yl)methyl)-6-(1-methyl-1*H*-pyrazol-5-yl)pyrimidin-4(3*H*)-one;
3-((1-((*R*)-3-Cyclohexyl-2-methylpropanoyl)-4-hydroxy-3,3-dimethylpiperidin-4-yl)methyl)-6-(1*H*-pyrazol-4-yl)pyrimidin-4(3*H*)-one;
3-((1-((*R*)-3-Cyclohexyl-2-methylpropanoyl)-4-hydroxy-3,3-dimethylpiperidin-4-yl)methyl)-6-(1*H*-pyrazol-5-yl)pyrimidin-4(3*H*)-one;
3-((7-((*R*)-3-Cyclohexyl-2-methylpropanoyl)-10-hydroxy-7-azaspiro[4.5]decan-10-yl)methyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(thiophen-3-yl)pyrimidin-4(3*H*)-one;
1-((1-((*R*)-3-Cyclohexyl-2-methylpropanoyl)-4-hydroxy-3,3-dimethylpiperidin-4-yl)methyl)-5-(oxazol-2-yl)-4-phenylpyridin-2(1*H*)-one;
3-((1-((*R*)-3-Cyclohexyl-2-methylpropanoyl)-4-hydroxy-3,3-dimethylpiperidin-4-yl)methyl)-6-(2-(hydroxymethyl)phenyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(3-(hydroxymethyl)phenyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(4-(hydroxymethyl)phenyl)pyrimidin-4(3*H*)-one;
6-(4-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
6-(2-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
6-(2-Fluorophenyl)-3-((4-hydroxy-3,3-dimethyl-1-(2,4,4-trimethylpentanoyl)piperidin-4-yl)methyl)pyrimidin-4(3*H*)-one;
6-(2-Fluorophenyl)-3-((4-hydroxy-3,3-dimethyl-1-(4,4,4-trifluoro-2-methylbutanoyl)piperidin-4-yl)methyl)pyrimidin-4(3*H*)-one;
4-Chloro-1-((7-((*R*)-3-cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(dimethylamino)-*N*,*N*-dimethyl-6-oxo-1,6-dihydropyridine-3-carboxamide;
1-((1-((*S*)-3-(Benzyloxy)-2-(cyclohexylmethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-((10-Hydroxy-7-isobutyryl-7-azaspiro[4.5]decan-10-yl)methyl)-*N*,*N*-dimethyl-6-oxo-4-phenyl-1,6-dihydropyridine-3-carboxamide;
1-((10-Hydroxy-7-((*R*)-2-methylbutanoyl)-7-azaspiro[4.5]decan-10-yl)methyl)-*N*,*N*-dimethyl-6-oxo-4-phenyl-1,6-dihydropyridine-3-carboxamide;
1-((7-((*R*)-3-Cyclopropyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
6-(2-Fluorophenyl)-3-((4-hydroxy-3,3-dimethyl-1-(2-methyl-3-(1*H*-pyrazol-1-yl)propanoyl)piperidin-4-yl)methyl)pyrimidin-4(3*H*)-one;
1-((7-((*R*)-3-Cyclohexyl-2-methylpropanoyl)-10-hydroxy-7-azaspiro[4.5]decan-10-yl)methyl)-4-(2-(hydroxymethyl)phenyl)-*N*,*N*-dimethyl-6-oxo-1,6-dihydropyridine-3-carboxamide;
1-(((*S*)-1-((*S*)-3-Cyclohexyl-2-(hydroxymethyl)propanoyl)-4-hydroxy-3,3-dimethylpiperidin-4-yl)methyl)-*N,N*-dimethyl-6-oxo-4-phenyl-1,6-dihydropyridine-3-carboxamide;
1-(((*R*)-1-((*S*)-3-Cyclohexyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1'-((1-((*R*)-3-Cyclohexyl-2-methylpropanoyl)-4-hydroxy-3,3-dimethylpiperidin-4-yl)methyl)-4'-(2-fluorophenyl)-[2,3'-bipyridin]-6'(1'*H*)-one;
1-((7-((*R*)-3-Cyclohexyl-2-methylpropanoyl)-10-hydroxy-7-azaspiro[4.5]decan-10-yl)methyl)-*N*,*N*-dimethyl-6-oxo-4-(thiophen-3-yl)-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(3-(hydroxymethyl)phenyl)-*N*,*N*-dimethyl-6-oxo-1,6-dihydropyridine-3-carboxamide;
1-((7-(3-Cyclohexyl-2-hydroxypropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-((10-Hydroxy-7-(2-methyl-3-(piperidin-1-yl)propanoyl)-7-azaspiro[4.5]decan-10-yl)methyl)-*N*,*N*-dimethyl-6-oxo-4-phenyl-1,6-dihydropyridine-3-carboxamide;
6-(3-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
1-(((*S*)-1-((*S*)-3-Cyclohexyl-2-(methoxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
4-(2-(Aminomethyl)phenyl)-1-((7-((*R*)-3-cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(4-(hydroxymethyl)phenyl)-*N*,*N*-dimethyl-6-oxo-1,6-dihydropyridine-3-carboxamide;
1-((10-Hydroxy-7-(2-methyl-3-morpholinopropanoyl)-7-azaspiro[4.5]decan-10-yl)methyl)-*N*,*N*-dimethyl-6-oxo-4-phenyl-1,6-dihydropyridine-3-carboxamide;
1-(((*S*)-1-((S)-2-(Cyclohexylmethyl)-4-hydroxybutanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*S*)-2-(Cyclohexylmethyl)-4-hydroxybutanoyl)-4-hydroxy-3,3-dimethylpiperidin-4-yl)methyl)-*N,N*-dimethyl-6-oxo-4-phenyl-1,6-dihydropyridine-3-carboxamide;
1-(((*S*)-1-((*S*)-3-Cyclobutyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*S*)-3-Cyclobutyl-2-(hydroxymethyl)propanoyl)-4-hydroxy-3,3-dimethylpiperidin-4-yl)methyl)-*N,N*-dimethyl-6-oxo-4-phenyl-1,6-dihydropyridine-3-carboxamide;
1-((1-((*R*)-3-Cyclohexyl-2-methylpropanoyl)-4-hydroxy-3,3-dimethylpiperidin-4-yl)methyl)-5-(morpholine-4-carbonyl)-4-phenylpyridin-2(1*H*)-one;
5-(4-Acetylpiperazine-1-carbonyl)-1-((1-((*R*)-3-cyclohexyl-2-methylpropanoyl)-4-hydroxy-3,3-dimethylpiperidin-4-yl)methyl)-4-phenylpyridin-2(1*H*)-one;
1-((1-((*R*)-3-Cyclohexyl-2-methylpropanoyl)-4-hydroxy-3,3-dimethylpiperidin-4-yl)methyl)-5-(4-methylpiperazine-1-carbonyl)-4-phenylpyridin-2(1*H*)-one;
1-((10-Hydroxy-7-(4,4,4-trifluoro-3-methylbutanoyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-(4,4,4-trifluorobutanoyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-(3-(tetrahydrofuran-3-yl)propanoyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-((1*R*,2*S*)-2-phenylcyclopropane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(Cyclopropanecarbonyl)-10-hydroxy-7-azaspiro[4.5]decan-10-yl)methyl)-*N*,*N-*dimethyl-6-oxo-4-phenyl-1,6-dihydropyridine-3-carboxamide;
1-(10-Hydroxy-7-(1-methylcyclohexane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(3-Fluorocyclopentane-1-carbonyl)-10-hydroxy-7-azaspiro[4.5]decan-10-yl)methyl)-*N*,*N*-dimethyl-6-oxo-4-phenyl-1,6-dihydropyridine-3-carboxamide;
1-((7-(1-(2,2-Difluoroethyl)cyclopropane-1-carbonyl)-10-hydroxy-7-azaspiro[4.5]decan-10-yl)methyl)-*N,N*-dimethyl-6-oxo-4-phenyl-1,6-dihydropyridine-3-carboxamide;
1-((7-(Bicyclo[2.2.1]heptane-1-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-((*S*)-4,4-Difluoro-3-phenylbutanoyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-((*R*)-4,4-Difluoro-3-phenylbutanoyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-(3-Ethoxypropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-((10-Hydroxy-7-((*S*)-3-phenylbutanoyl)-7-azaspiro[4.5]decan-10-yl)methyl)-*N,N*-dimethyl-6-oxo-4-phenyl-1,6-dihydropyridine-3-carboxamide;
1-((7-(3-(4-Fluorophenyl)propanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((10-Hydroxy-7-((*R*)-3-phenylbutanoyl)-7-azaspiro[4.5]decan-10-yl)methyl)-*N,N*-dimethyl-6-oxo-4-phenyl-1,6-dihydropyridine-3-carboxamide;
1-((10-Hydroxy-7-(1-methylcyclopentane-1-carbonyl)-7-azaspiro [4.5]decan-10-yl)methyl)-*N*,*N*-dimethyl-6-oxo-4-phenyl-1,6-dihydropyridine-3-carboxamide;
1-((10-Hydroxy-7-(1*H*-pyrazole-3-carbonyl)-7-azaspiro[4.5]decan-10-yl)methyl)-*N*,*N-*dimethyl-6-oxo-4-phenyl-1,6-dihydropyridine-3-carboxamide;
1-((10-Hydroxy-7-(1*H*-indazole-3-carbonyl)-7-azaspiro[4.5]decan-10-yl)methyl)-*N*,*N-*dimethyl-6-oxo-4-phenyl-1,6-dihydropyridine-3-carboxamide;
1-((7-(3-Cyclohexyl-1*H*-pyrazole-4-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((10-Hydroxy-7-(1*H*-indole-3-carbonyl)-7-azaspiro[4.5]decan-10-yl)methyl)-*N*,*N*-dimethyl-6-oxo-4-phenyl-1,6-dihydropyridine-3-carboxamide;
1-((7-(Bicyclo[1.1.1]pentane-1-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((10-Hydroxy-7-(5-phenyloxazole-4-carbonyl)-7-azaspiro[4.5]decan-10-yl)methyl)-*N*,*N-*dimethyl-6-oxo-4-phenyl-1,6-dihydropyridine-3-carboxamide;
1-((7-(5-Cyclopropyloxazole-4-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-(2-(Cyclohexyloxy)acetyl)-10-hydroxy-7-azaspiro[4.5]decan-10-yl)methyl)-*N,N-*dimethyl-6-oxo-4-phenyl-1,6-dihydropyridine-3-carboxamide;
1-((10-Hydroxy-7-(spiro[2.2]pentane-1-carbonyl)-7-azaspiro[4.5]decan-10-yl)methyl)-*N,N-*dimethyl-6-oxo-4-phenyl-1,6-dihydropyridine-3-carboxamide;
1-((10-Hydroxy-7-(3-(trifluoromethyl)cyclopentane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(2,4-Dimethylpentanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(1-Benzyl-1*H*-pyrrole-2-carbonyl)-10-hydroxy-7-azaspiro[4.5]decan-10-yl)methyl)-*N,N-*dimethyl-6-oxo-4-phenyl-1,6-dihydropyridine-3-carboxamide;
1-((7-(2-(Cyclohexyloxy)propanoyl)-10-hydroxy-7-azaspiro[4.5]decan-10-yl)methyl)-*N,N-*dimethyl-6-oxo-4-phenyl-1,6-dihydropyridine-3-carboxamide;
3-((1-((*S*)-3-Cyclobutyl-2-(hydroxymethyl)propanoyl)-4-hydroxy-3,3-dimethylpiperidin-4-yl)methyl)-6-(2-fluorophenyl)pyrimidin-4(3*H*)-one;
1-((7-(2-Cyclobutoxyacetyl)-10-hydroxy-7-azaspiro[4.5]decan-10-yl)methyl)-*N*,*N*-dimethyl-6-oxo-4-phenyl-1,6-dihydropyridine-3-carboxamide;
1-((7-(3,3-Difluorocyclopentane-1-carbonyl)-10-hydroxy-7-azaspiro[4.5]decan-10-yl)methyl)-*N*,*N*-dimethyl-6-oxo-4-phenyl-1,6-dihydropyridine-3-carboxamide;
1-((10-Hydroxy-7-(2-hydroxy-3-phenylpropanoyl)-7-azaspiro[4.5]decan-10-yl)methyl)-*N*,*N-*dimethyl-6-oxo-4-phenyl-1,6-dihydropyridine-3-carboxamide;
1-(((*S*)-4-Hydroxy-3,3-dimethyl-1-((*R*)-4,4,4-trifluoro-2-methylbutanoyl)piperidin-4-yl)methyl)-*N*,*N*-dimethyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-(2-fluorophenyl)-*N*,*N*-dimethyl-6-oxo-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
(*S*)-1-((4-Hydroxy-3,3-dimethyl-1-(4,4,4-trifluoro-2-(2,2,2-trifluoroethyl)butanoyl)piperidin-4-yl)methyl)-*N*,*N*-dimethyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-5-((*S*)-2-(hydroxymethyl)piperazine-1-carbonyl)-4-phenylpyridin-2(1*H*)-one;
1-(((*R*)-7-((*R*)-3-Cyclohexyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-1-((*R*)-3-Cyclohexyl-2-methylpropanoyl)-4-hydroxy-3,3-dimethylpiperidin-4-yl)methyl)-5-((*R*)-3-methylpiperazine-1-carbonyl)-4-phenylpyridin-2(1*H*)-one;
1-(((*S*)-7-((*R*)-3-Cyclobutyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*R*)-4,4,4-trifluoro-2-methylbutanoyl)-7-azaspiro[4.5]decan-10-yl)methyl)-4-phenyl-5-(piperazine-1-carbonyl)pyridin-2(1*H*)-one;
(*S*)-1-((10-Hydroxy-7-(4,4,4-trifluoro-2-(2,2,2-trifluoroethyl)butanoyl)-7-azaspiro[4.5]decan-10-yl)methyl)-*N*,*N*-dimethyl-6-oxo-4-phenyl-1,6-dihydropyridine-3-carboxamide;
(*S*)-1-((10-Hydroxy-7-(4,4,4-trifluoro-2-(2,2,2-trifluoroethyl)butanoyl)-7-azaspiro[4.5]decan-10-yl)methyl)-4-phenyl-5-(piperazine-1-carbonyl)pyridin-2(1*H*-one;
1-(((*S*)-1-((*R*)-3-Cyclohexyl-2-methylpropanoyl)-4-hydroxy-3,3-dimethylpiperidin-4-yl)methyl)-4-cyclopropyl-*N*,*N*-dimethyl-6-oxo-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-cyclopropyl-5-(piperazine-1-carbonyl)pyridin-2(1*H*)-one;
1-(((*S*)-7-((*R*)-3-Cyclobutyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
4-(2-Fluorophenyl)-1-(((*S*)-10-hydroxy-7-((R)-4,4,4-trifluoro-2-methylbutanoyl)-7-azaspiro[4.5]decan-10-yl)methyl)-*N*,*N*-dimethyl-6-oxo-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
4-(2-Fluorophenyl)-1-(((*S*)-10-hydroxy-7-((R)-4,4,4-trifluoro-2-methylbutanoyl)-7-azaspiro[4.5]decan-10-yl)methyl)-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
1-(((*S*)-7-((*R*)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
1-(((*S*)-1-((R)-3-Cyclohexyl-2-methylpropanoyl)-4-hydroxy-3,3-dimethylpiperidin-4-yl)methyl)-5-(4-hydroxypiperidine-1-carbonyl)-4-phenylpyridin-2(1*H*)-one;
1-((10-Hydroxy-7-(3-(trifluoromethyl)cyclobutane-1-carbonyl)-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-cyclopropyl-*N*,*N*-dimethyl-6-oxo-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-4-cyclopropyl-*N*,*N*-dimethyl-6-oxo-1,6-dihydropyridine-3-carboxamide;
1-(((*S*)-7-((*R*)-3-Cyclohexyl-2-methylpropanoyl)-1 0-hydroxy-7-azaspiro[4.5]decan-1 0-yl)methyl)-4-cyclopropyl-5-(piperazine-1-carbonyl)pyridin-2(1*H*)-one;
1-((2-((*R*)-3-Cyclohexyl-2-methylpropanoyl)-5-hydroxy-9-oxa-2-azaspiro[5.5]undecan-5-yl)methyl)-*N,N*-dimethyl-6-oxo-4-phenyl-1,6-dihydropyridine-3-carboxamide;
1-(((*S*)-1-((*R*)-3-Cyclobutyl-2-methylpropanoyl)-4-hydroxy-3,3-dimethylpiperidin-4-yl)methyl)-4-(2-fluorophenyl)-*N*,*N*-dimethyl-6-oxo-1,6-dihydropyridine-3-carboxamide;
1-(((*S*)-1-((*R*)-3-Cyclobutyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
1-((2-((*R*)-3-Cyclohexyl-2-methylpropanoyl)-5-hydroxy-2-azaspiro[5.5]undecan-5-yl)methyl)-*N*,*N*-dimethyl-6-oxo-4-phenyl-1,6-dihydropyridine-3-carboxamide;
1-((2-((*R*)-3-Cyclohexyl-2-methylpropanoyl)-5-hydroxy-2-azaspiro[5.5]undecan-5-yl)methyl)-4-phenyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-5-(morpholine-4-carbonyl)-4-phenylpyridin-2(1*H*)-one;
1-(((*S*)-7-((*R*)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1*H*)-one;
1-((1-((*R*)-3-Cyclohexyl-2-methylpropanoyl)-4-hydroxy-3,3-dimethylpiperidin-4-yl)methyl)-6-oxo-4-phenyl-1,6-dihydropyridine-3-carboxylic acid;
1-((7-((*R*)-3-Cyclohexyl-2-methylpropanoyl)-10-hydroxy-7-azaspiro[4.5]decan-10-yl)methyl)-4-methoxy-*N*,*N*-dimethyl-6-oxo-1,6-dihydropyridine-3-carboxamide;
1-((7-(4,4-Difluoro-2-methylbutanoyl)-10-hydroxy-7-azaspiro[4.5]decan-10-yl)methyl)-*N*,*N* dimethyl-6-oxo-4-phenyl-1,6-dihydropyridine-3-carboxamide;
1-((7-(4,4-Difluoro-2,2-dimethylbutanoyl)-10-hydroxy-7-azaspiro[4.5]decan-10-yl)methyl)-*N*,*N*-dimethyl-6-oxo-4-phenyl-1,6-dihydropyridine-3-carboxamide;
1-((10-Hydroxy-7-(4-(trifluoromethyl)thiazole-2-carbonyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-(2-(trifluoromethyl)thiazole-4-carbonyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*S*)-4,4,4-trifluoro-2-(hydroxymethyl)butanoyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*S*)-4,4,4-trifluoro-2-(hydroxymethyl)butanoyl)-7-azaspiro[4.5]decan-10-yl)methyl)-5-(morpholine-4-carbonyl)-4-phenylpyridin-2(1*H*)-one;
1-(((*S*)-10-Hydroxy-7-((*S*)-4,4,4-trifluoro-2-(hydroxymethyl)butanoyl)-7-azaspiro[4.5]decan-10-yl)methyl)-4-phenyl-5-(piperazine-1-carbonyl)pyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-5-(morpholine-4-carbonyl)-4-phenylpyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-5-(4-methylpiperazine-1-carbonyl)-4-phenylpyridin-2(1*H*)-one;
Ethyl 1-(((*S*)-7-((*R*)-3-cyclohexyl-2-methylpropanoyl)-10-hydroxy-7-azaspiro[4.5]decan-10-yl)methyl)-6-oxo-4-phenyl-1,6-dihydropyridine-3-carboxylate;
1-(((*S*)-7-((*R*)-3-Cyclohexyl-2-methylpropanoyl)-10-hydroxy-7-azaspiro[4.5]decan-10-yl)methyl)-6-oxo-4-phenyl-1,6-dihydropyridine-3-carboxylic acid;
1-(((*R*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-phenyl-5-(pyrrolidine-1-carbonyl)pyridin-2(1*H*)-one;
1-(((*R*)-1-((*S*)-3-Cyclohexyl-2-(methoxymethyl)propanoyl)-4-hydroxy-3,3-dimethylpiperidin-4-yl)methyl)-*N,N*-dimethyl-6-oxo-4-phenyl-1,6-dihydropyridine-3-carboxamide;
1-(((*R*)-4-Hydroxy-3,3-dimethyl-1-((*R*)-4,4,4-trifluoro-2-methylbutanoyl)piperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-(2-fluorophenyl)-*N*,*N*-dimethyl-6-oxo-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
(*R*)-1-((4-Hydroxy-3,3-dimethyl-1-(4,4,4-trifluoro-2-(2,2,2-trifluoroethyl)butanoyl)piperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-5-((*S*)-2-(hydroxymethyl)piperazine-1-carbonyl)-4-phenylpyridin-2(1*H*)-one;
1-(((*R*)-1-((*R*)-3-Cyclohexyl-2-methylpropanoyl)-4-hydroxy-3,3-dimethylpiperidin-4-yl)methyl)-5-((*R*)-3-methylpiperazine-1-carbonyl)-4-phenylpyridin-2(1*H*)-one;
1-(((R)-7-((*R*)-3-Cyclobutyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*R*)-7-((R)-3-Cyclobutyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-*N*,*N*-dimethyl-6-oxo-4-phenyl-1,6-dihydropyridine-3-carboxamide;
1-(((*R*)10-Hydroxy-7-((*R*)-4,4,4-trifluoro-2-methylbutanoyl)-7-azaspiro[4.5]decan-10-yl)methyl)-4-phenyl-5-(piperazine-1-carbonyl)pyridin-2(1*H*)-one;
(*R*)-1-((10-Hydroxy-7-(4,4,4-trifluoro-2-(2,2,2-trifluoroethyl)butanoyl)-7-azaspiro[4.5]decan-10-yl)methyl)-*N*,*N*-dimethyl-6-oxo-4-phenyl-1,6-dihydropyridine-3-carboxamide;
(*R*)-1-((10-Hydroxy-7-(4,4,4-trifluoro-2-(2,2,2-trifluoroethyl)butanoyl)-7-azaspiro[4.5]decan-10-yl)methyl)-4-phenyl-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-4-cyclopropyl-*N*,*N*-dimethyl-6-oxo-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-cyclopropyl-5-(piperazine-1-carbonyl)pyridin-2(1*H*)-one;
1-(((*R*)-7-((*R*)-3-Cyclobutyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
4-(2-Fluorophenyl)-1-(((*R*)-10-hydroxy-7-((*R*)-4,4,4-trifluoro-2-methylbutanoyl)-7-azaspiro[4.5]decan-10-yl)methyl)-*N*,*N*-dimethyl-6-oxo-1,6-dihydropyridine-3-carboxamide;
1-(((*R*)-7-((*R*)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
4-(2-Fluorophenyl)-1-(((*R*)-10-hydroxy-7-((*R*)-4,4,4-trifluoro-2-methylbutanoyl)-7-azaspiro[4.5]decan-10-yl)methyl)-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-5-(4-hydroxypiperidine-1-carbonyl)-4-phenylpyridin-2(1*H*)-one;
1-(((*R*)-7-((*R*)-3-Cyclobutyl-2-methylpropanoyl)-10-hydroxy-7-azaspiro[4.5]decan-10-yl)methyl)-4-cyclopropyl-*N*,*N*-dimethyl-6-oxo-1,6-dihydropyridine-3-carboxamide;
1-(((*R*)-7-((*R*)-3-Cyclobutyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-4-cyclopropyl-*N*,*N*-dimethyl-6-oxo-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*R*)-1-((*R*)-3-Cyclobutyl-2-methylpropanoyl)-4-hydroxy-3,3-dimethylpiperidin-4-yl)methyl)-4-(2-fluorophenyl)-*N*,*N*-dimethyl-6-oxo-1,6-dihydropyridine-3-carboxamide;
1-(((*R*)-1-((*R*)-3-Cyclobutyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-5-(morpholine-4-carbonyl)-4-phenylpyridin-2(1*H*)-one;
1-(((*R*)-7-((*R*)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1*H*)-one;
1-(((*R*)-10-Hydroxy-7-((*S*)-4,4,4-trifluoro-2-(hydroxymethyl)butanoyl)-7-azaspiro[4.5]decan-10-yl)methyl)-*N*,*N*-dimethyl-6-oxo-4-phenyl-1,6-dihydropyridine-3-carboxamide;
1-(((*R*)-10-Hydroxy-7-((*S*)-4,4,4-trifluoro-2-(hydroxymethyl)butanoyl)-7-azaspiro[4.5]decan-10-yl)methyl)-5-(morpholine-4-carbonyl)-4-phenylpyridin-2(1*H*)-one;
1-(((*R*)-10-Hydroxy-7-((*S*)-4,4,4-trifluoro-2-(hydroxymethyl)butanoyl)-7-azaspiro[4.5]decan-10-yl)methyl)-4-phenyl-5-(piperazine-1-carbonyl)pyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-5-(morpholine-4-carbonyl)-4-phenylpyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-5-(4-methylpiperazine-1-carbonyl)-4-phenylpyridin-2(1*H*)-one;
Ethyl 1-(((*R*)-7-((*R*)-3-cyclohexyl-2-methylpropanoyl)-10-hydroxy-7-azaspiro[4.5]decan-10-yl)methyl)-6-oxo-4-phenyl-1,6-dihydropyridine-3-carboxylate;
1-(((*R*)-7-((*R*)-3-Cyclohexyl-2-methylpropanoyl)-10-hydroxy-7-azaspiro[4.5]decan-10-yl)methyl)-6-oxo-4-phenyl-1,6-dihydropyridine-3-carboxylic acid;
3-((10-Hydroxy-7-((3*R*,4*R*)-3-phenylpiperidine-4-carbonyl)-7-azaspiro[4.5]decan-10-yl)methyl)-6-phenylpyrimidin-4(3*H*)-one;
3-(((*S*)-10-Hydroxy-7-((*R*)-4,4,4-trifluoro-2-methylbutanoyl)-7-azaspiro[4.5]decan-10-yl)methyl)-6-phenylpyrimidin-4(3*H*)-one;
6-(2-Fluorophenyl)-3-(((*S*)-10-hydroxy-7-((R)-4,4,4-trifluoro-2-methylbutanoyl)-7-azaspiro[4.5]decan-10-yl)methyl)pyrimidin-4(3*H*)-one;
6-(3-Fluorophenyl)-3-(((*S*)-10-hydroxy-7-((R)-4,4,4-trifluoro-2-methylbutanoyl)-7-azaspiro[4.5]decan-10-yl)methyl)pyrimidin-4(3*H*)-one;
3-(((*S*)-7-((*R*)-4,4-Difluoro-2-methylpentanoyl)-10-hydroxy-7-azaspiro[4.5]decan-10-yl)methyl)-6-phenylpyrimidin-4(3*H*)-one;
3-(((*S*)-10-Hydroxy-7-((3*S*,4*R*)-4-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((3*R*,4*S*)-4-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((2*R*,3*R*)-2-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((2*S*,3*S*)-2-phenylpyrrolidine-3-carbonyl)-7-azaspiro[4.5]decan-10-yl)methyl)-6-phenylpyrimidin-4(3*H*)-one;
or a pharmaceutically acceptable salt, tautomer, stereoisomer or *N*-oxide derivative thereof.

In a preferred embodiment, the compound for use in the methods of treatment in accordance with the invention is the compound of Example 212 herein: 1-(((S)-7-((R)-3-Cyclohexyl-2-methylpropanoyl)-10-hydroxy-7-azaspiro[4.5]decan-10-yl)methyl)-4-phenyl-5-(piperazine-1-carbonyl)pyridin-2(1H)-one:

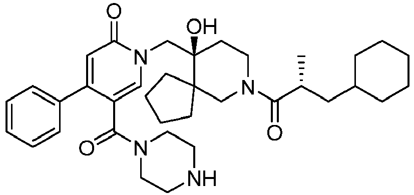

This compound and its synthesis is provided as Example 212 of WO2018/020242 (page 217).

In a further aspect is provided the compound 1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one for use in a method of treating obesity.

In a further aspect is provided the compound 1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one for use in a method of treating insulin resistance.

In a further aspect is provided the compound 1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one for use in a method of treating type II diabetes.

### Pharmaceutical compositions for use according to the invention

In a further aspect the present invention provides a pharmaceutical composition comprising a compound, pharmaceutically acceptable salt, tautomer, stereoisomer or *N*-oxide derivative as defined herein, for use in a method of obesity.

Also provided in accordance with the invention is a pharmaceutical composition comprising a compound, pharmaceutically acceptable salt, tautomer, stereoisomer or *N*-oxide derivative as defined herein, for use in a method of treating insulin resistance.

Also provided in accordance with the invention is a pharmaceutical composition comprising a compound, pharmaceutically acceptable salt, tautomer, stereoisomer or *N*-oxide derivative as defined herein, for use in a method of treating type II diabetes.

In certain embodiments of the invention, the pharmaceutical composition for use in methods of the invention further comprises a pharmaceutically acceptable diluent, excipient or carrier.

Pharmaceutical compositions may be formulated according to their particular use and purpose by mixing, for example, excipient, binding agent, lubricant, disintegrating agent, coating material, emulsifier, suspending agent, solvent, stabilizer, absorption enhancer and/or ointment base. The composition may be suitable for oral, injectable, rectal or topical administration.

Suitable pharmaceutically acceptable excipients would be known by the person skilled in the art, for example: fats, water, physiological saline, alcohol (e.g. ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose; and other conventionally used additives such as gelatin, talc, plant oil and gum arabic.

For parenteral injection for, for example, intravenous, intramuscular or subcutaneous use, a sterile aqueous solution may be provided that may contain other substances including, for example, salts and/or glucose to make to solution isotonic.

For example, the pharmaceutical composition may be administered orally, such as in the form of tablets, coated tablets, hard or soft gelatine capsules, solutions, emulsions, or suspensions. Administration can also be carried out rectally, for example using suppositories, locally or percutaneously, for example using ointments, creams, gels or solution, or parenterally, for example using injectable solutions.

For the preparation of tablets, coated tablets or hard gelatine capsules, the compounds of the present invention may be admixed with pharmaceutically inert, inorganic or organic excipients. Examples of suitable excipients include lactose, mize starch or derivatives thereof, talc or stearic acid or salts thereof. Suitable excipients for use with soft gelatine capsules include, for example, vegetable oils, waxes, fats and semi-solid or liquid polyols.

For the preparation of solutions and syrups, excipients include, for example, water, polyols, saccharose, invert sugar and glucose.

For injectable solutions, excipients include, for example, water, alcohols, polyols, glycerine and vegetable oil.

For suppositories and for local and percutaneous application, excipients include, for example, natural or hardened oils, waxes, fats and semi-solid or liquid polyols.

The pharmaceutical compositions may also contain preserving agents, solublizing agents, stabilizing agents, wetting agents, emulsifiers, sweeteners, colorants, odorants, buffers, coating agents and/or antioxidants.

For combination therapies, the second drug may be provided in pharmaceutical composition with the present invention or may be provided separately.

### Routes of administration

In certain preferred embodiments, of the methods according to the invention, the method comprises administering the therapeutic agent (that is, the compound, pharmaceutically acceptable salt, tautomer, stereoisomer or *N*-oxide derivative, or pharmaceutical composition for use according to the invention) parenterally.

In certain preferred embodiments, the therapeutic agent is administered orally.

In certain preferred embodiments the therapeutic agent is administered intravenously. In certain preferred embodiments, the therapeutic agent is administered intraperitoneally. In certain preferred embodiments, the therapeutic agent is administered subcutaneously.

### Dosage regimen

In certain preferred embodiments of the methods according to the invention comprises administering the therapeutic agent (that is, the compound, pharmaceutically acceptable salt, tautomer, stereoisomer or *N*-oxide derivative, or pharmaceutical composition for use according to the invention) at a dose in the range of from 10 to 150 mg/kg. In such embodiments, the dose refers to the amount of the active ingredient administered to the subject per single administration.

In certain preferred embodiments, the method comprises administering the therapeutic agent at a dose in the range of from 25 to 125mg/kg. In certain preferred embodiments, the method comprises administering the therapeutic agent at a dose in the range of from 50 to 100mg/kg.

In certain preferred embodiments, the method comprises administering the therapeutic agent at a dose of 75mg/kg.

In certain preferred embodiments, the method comprises administering the therapeutic agent (that is, the compound, pharmaceutically acceptable salt, tautomer, stereoisomer or *N*-oxide derivative, or pharmaceutical composition for use according to the invention) 1, 2, 3 or 4 times daily. In certain preferred embodiments, the therapeutic agent is administered once or twice daily, most preferably twice daily.

In certain preferred embodiments, the therapeutic agent is administered at a daily dosage in the range of from 10 to 300 mg/kg. That is, the total amount of active agent administered to the subject in one day is in the range of from 10-300 mg/kg. In such embodiments, the therapeutic agent may be administered once or multiple times per day as described herein, provided the total daily dosage is in the indicated range.

In certain preferred embodiments, the therapeutic agent is administered at a daily dosage in the range of from 50 to 250 mg/kg. In certain preferred embodiments, the therapeutic agent is administered at a daily dosage in the range of from 75 to 250 mg/kg. In certain preferred embodiments, the therapeutic agent is administered at a daily dosage in the range of from 100 to 200 mg/kg. In certain preferred embodiments, the therapeutic agent is administered at a daily dosage of 150mg/kg.

In certain preferred embodiments, the therapeutic agent (for example a compound as provided herein) is administered at a dose of 75mg/kg twice daily.

In a preferred aspect of all aspects of the invention, the subject or patient to be treated is a human.

### EXAMPLES

The compounds described herein were first described in WO2018/020242.

### In vitro activity

As described in WO2018/020242, the compounds were assessed *in vitro* for their ability to inhibit USP19 activity. The results are shown in Table 1.

**Table 1. USP19 inhibition by exemplified compounds**

| **Example Number** | **USP19 IC₅₀ activity** | **Example Number** | **USP19 IC₅₀ activity** | **Example Number** | **USP19 IC₅₀ activity** | **Example Number** | **USP19 IC₅₀ activity** |
|---|---|---|---|---|---|---|---|
| **1** | * | **67** | ** | **133** | *** | **199** | ** |
| **2** | * | **68** | ** | **134** | ** | **200** | *** |
| **3** | * | **69** | ** | **135** | **** | **201** | ** |
| **4** | * | **70** | ** | **136** | *** | **202** | * |
| **5** | ** | **71** | ** | **137** | *** | **203** | *** |
| **6** | ** | **72** | * | **138** | **** | **204** | *** |
| **7** | *** | **73** | **** | **139** | **** | **205** | **** |
| **8** | ** | **74** | * | **140** | *** | **206** | **** |
| **9** | ** | **75** | *** | **141** | **** | **207** | ** |
| **10** | * | **76** | *** | **142** | * | **208** | **** |
| **11** | * | **77** | *** | **143** | ** | **209** | ** |
| **12** | ** | **78** | *** | **144** | **** | **210** | ** |
| **13** | * | **79** | *** | **145** | **** | **211** | **** |
| **14** | *** | **80** | ** | **146** | *** | **212** | **** |
| **15** | ** | **81** | * | **147** | * | **213** | **** |
| **16** | ** | **82** | * | **148** | ** | **214** | **** |
| **17** | ** | **83** | * | **149** | **** | **215** | **** |
| **18** | ** | **84** | * | **150** | * | **216** | **** |
| **19** | ** | **85** | * | **151** | ** | **217** | **** |
| **20** | ** | **86** | ** | **152** | **** | **218** | *** |
| **21** | ** | **87** | *** | **153** | ** | **219** | *** |
| **22** | ** | **88** | *** | **154** | ** | **220** | *** |
| **23** | ** | **89** | *** | **155** | **** | **221** | *** |
| **24** | ** | **90** | *** | **156** | **** | **222** | **** |
| **25** | *** | **91** | *** | **157** | **** | **223** | *** |
| **26** | * | **92** | *** | **158** | * | **224** | **** |
| **27** | * | **93** | ** | **159** | *** | **225** | *** |
| **28** | * | **94** | *** | **160** | **** | **226** | **** |
| **29** | * | **95** | ** | **161** | ** | **227** | **** |
| **30** | * | **96** | ** | **162** | **** | **228** | **** |
| **31** | *** | **97** | ** | **163** | ** | **229** | ** |
| **32** | ** | **98** | ** | **164** | **** | **230** | *** |
| **33** | ** | **99** | ** | **165** | ** | **231** | *** |
| **34** | * | **100** | ** | **166** | **** | **232** | **** |
| **35** | * | **101** | ** | **167** | ** | **233** | **** |
| **36** | *** | **102** | *** | **168** | **** | **234** | *** |
| **37** | * | **103** | ** | **169** | **** | **235** | *** |
| **38** | * | **104** | ** | **170** | **** | **236** | **** |
| **39** | ** | **105** | ** | **171** | * | **237** | **** |
| **40** | ** | **106** | ** | **172** | ** | **238** | **** |
| **41** | ** | **107** | ** | **173** | * | **239** | **** |
| **42** | ** | **108** | *** | **174** | **** | **240** | **** |
| **43** | ** | **109** | * | **175** | * | **241** | *** |
| **44** | ** | **110** | *** | **176** | ** | **242** | *** |
| **45** | * | **111** | * | **177** | * | **243** | ** |
| **46** | * | **112** | * | **178** | * | **244** | ** |
| **47** | * | **113** | * | **179** | * | **245** | ** |
| **48** | * | **114** | ** | **180** | ** | **246** | ** |
| **49** | * | **115** | ** | **181** | *** | **247** | **** |
| **50** | **** | **116** | *** | **182** | ** | **248** | **** |
| **51** | *** | **117** | *** | **183** | ** | **249** | **** |
| **52** | *** | **118** | *** | **184** | *** | **250** | **** |
| **53** | *** | **119** | **** | **185** | *** | **251** | **** |
| **54** | * | **120** | **** | **186** | * | **252** | **** |
| **55** | ** | **121** | **** | **187** | * | **253** | **** |
| **56** | ** | **122** | * | **188** | * | **254** | **** |
| **57** | ** | **123** | **** | **189** | *** | **255** | **** |
| **58** | ** | **124** | **** | **190** | * | **256** | **** |
| **59** | ** | **125** | ** | **191** | * | **257** | **** |
| **60** | ** | **126** | * | **192** | ** | **258** | **** |
| **61** | * | **127** | *** | **193** | ** | **259** | **** |
| **62** | *** | **128** | *** | **194** | ** | **260** | **** |
| **63** | *** | **129** | ** | **195** | ** | | |
| **64** | ** | **130** | *** | **196** | *** | | |
| **65** | ** | **131** | *** | **197** | **** | | |
| **66** | N.D. | **132** | ** | **198** | ** | | |

For representative examples in **Table 1**, USP19 inhibitory activities are classified as the following:

| | **** | *** | ** | * | N.D. |
|---|---|---|---|---|---|
| USP19 IC₅₀ [µM] | IC₅₀<0.5 | 0.5≤IC₅₀<5 | 0.5≤IC₅₀<50 | 0.5≤IC₅₀<250 | Not determined |

USP19 activity was determined in a fluorescence polarisation (FP) homogeneous assay using the isopeptide Ubiquitin-Lys-TAMRA substrate (either AUB-101, Almac Sciences Scotland Limited, or U-558, Boston Biochem, both of which gave identical results). Full-length USP19 was purchased from Boston Biochem (E-576). Unless otherwise stated, all other reagents were purchased from Sigma. Enzymatic reactions were conducted in black flat bottom polystyrene 384-well plates (Nunc) and 30 µL total volume. USP19 (2.5 nM, 10 µL) was incubated in assay buffer (50 mM HEPES (pH 7.4), 150 mM NaCl, 5 mM DTT, 0.05% BSA (w/v), 0.05% CHAPS) in the presence or absence of inhibitor (10 µL). Inhibitors were stored as 10 mM DMSO stocks in an inert environment (low humidity, dark, low oxygen, room temperature) using the Storage Pod System and serial dilutions were prepared in buffer just prior to the assay (from 200 µM to 2 pM, 8-18 data point curve).

Following incubation at RT for 30 min, the enzymatic reactions were initiated by dispensing the Ub substrate (500 nM, 10 µL). FP was measured every 15 min over a period of 90 min (within the linear range of the assay) using a Synergy 4 plate reader (BioTek) exciting at 530 nm and measuring the amount of parallel and perpendicular light at 575 nm. The FP signal was subsequently normalised to the no compound control. Data were plotted and fitted, and the concentrations resulting in 50% inhibition (IC₅₀) were calculated using the non-linear regression curve fitting model using GraphPad (Prism). IC₅₀ values for the inhibitors of the invention are compiled in Table 1 and represent the average of at least two duplicate experiments.

### Cell target engagement

Cells from a breast cancer cell line, a neuroblastoma cell line and a mouse skeletal muscle cell line were treated with a USP19 inhibitor compound (ADC-141, corresponding to Example 212 provided herein) for 2 hrs, lysed (lysis buffer: 50mM Tris pH 7.4; 150mM NaCl; 5mM MgCl2; 0.5mM EDTA; 0.5% NP40; 10% Glycerol; 2mM DTT) and Ubiquitin-propargylamine (Ub-PA; UbiQ) or Ubiquitin-vinyl methyl ester (Ub-VME; Almac Sciences Scotland Limited) was then added. Samples were analysed by western blotting probing for USP19 (EC₅₀ determined by densitometry).

In each cell line, the USP19 inhibitor compound showed good cell permeability and exhibited a low nanomolar EC₅₀. The results for each cell line are shown in Figure 5.

### In vivo activity

The *in vitro* activity data described in WO2018/020242 and reproduced above indicated that the compounds provided in that document and herein could have therapeutic utility due to their ability to inhibit USP19.

The following data from an *in vivo* model is the first demonstration that a USP19 inhibitor can be used to treat muscle loss, to reduce fat deposition and to improve insulin sensitivity. These data demonstrate that compounds which potently inhibit USP19 activity can effectively treat muscular atrophy, obesity and/or insulin resistance.

### Methods:

To induce muscle wasting, a 1 cm segment of the sciatic nerve in the thigh was removed from mice (male C57bl/6 mice at 8-10 weeks of age; n=10 per group) under isoflurane anaesthesia and analgesia with carprofen. A sham operation was carried out in the opposite leg as a control.

Mice were randomised into Vehicle or Test groups, with all animals weighed to ensure a similar mean weight in each group. A USP19 inhibitory compound (ADC-141 , which is 1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one, corresponding to exemplary compound 212 provided herein) at 75 mg/kg or Vehicle was administered IP twice daily starting from the evening post-operation.

Mice were sacrificed 14 days later. Fat pads, liver, gastrocnemius and tibialis anterior muscles were harvested. Tissue mass were measured in both groups.

To assess obesity and insulin resistance, a diet-induced obesity mouse model was used. The diet-induced obese (DIO) mouse is a well characterised model of obesity which exhibits increased adiposity, insulin resistance and glucose intolerance.

Male C57BL6/J mice were continuously provided with high-fat diet (D12451, 45% kcal as fat; Research Diets, New Jersey, USA) and filtered tap water *ad libitum* for the duration of the study. From day 0, mice were administered vehicle i.p. BID, USP19 inhibitor (ADC-141) i.p. BID at 5mg/kg or 25mg/kg, or positive control liraglutide 0.1mg/kg s.c. BID. Mice were allocated to treatment groups to balance the groups on the basis of body weight, food and water intake prior to the start of treatment.

Body weight was measured daily. On Day 13, body composition was assessed by DEXA. On Day 15, fasting glucose and insulin levels were measured before and during an oral glucose tolerance test (OGTT) to assess improvements in glucose control. The OGTT was performed following an overnight fast. Hence, on Day 14 food (but not water) was removed beginning at approximately 16:45, immediately after the PM dose. An OGTT was performed the following morning (approx. 16h post fast). Mice were dosed with vehicle or test compound (starting at 08.45) to a timed schedule 30 minutes prior to the administration of the glucose challenge (2.0 g/kg po). Blood samples were taken immediately prior to dosing (B1), immediately prior to glucose administration (B2) and 15, 30, 60 and 120 minutes after glucose administration.

After OGTT, mice were humanely killed and carcass composition was assessed. The carcass was weighed and stored frozen and the chemical composition of each carcass (fat, protein, water and ash) was determined using classical techniques. Carcass water was determined by freeze-drying the carcasses to constant weight for 2 weeks. Carcass fat was determined on samples of the dry powdered carcasses using a modified Soxhlet extraction protocol (petroleum ether at 40-60°C) with a Tecator Soxtec 2050 system (Foss UK Ltd, Wheldrake, UK) according to the manufacturer's recommended protocol. Carcass protein was determined using a micro-Kjeldahl procedure on samples of the dry powdered carcasses using a Tecator 2012 digestion block and 2200 distilling unit (Foss UK Ltd). Residual carcass ash was determined by firing samples of the dry powdered carcasses at high temperatures using a muffle ashing furnace (Carbolite OAF 11/1). Repeat determinations of the chemical analysis parameters were performed if necessary (e.g. if the duplicate samples differed by more than 1%). Data for each body composition parameter (fat, protein, water and ash) was determined as g/carcass and % total. Final carcass weights were also analysed as a direct comparison.

### Results:

### Muscular atrophy

As shown in Figure 1, mice receiving a USP19 inhibitor had a significantly lower loss of muscle mass in the tibialis anterior muscle compared to mice receiving vehicle only. The sparing of muscle atrophy was evident both in terms of percentage mass (Figure 1B) and absolute muscle mass (Figure 1C).

Muscle wasting was also reduced in the gastrocnemius muscle (Figure 2), though the trend did not reach significance. Again, mice receiving a USP19 inhibitor exhibited less muscle wasting both in terms of percentage mass (Figure 2B) and absolute muscle mass (Figure 2C).

These data demonstrate the pharmacological inhibition of USP19 *in vivo* can reduce muscular atrophy. The data indicate that pharmacological inhibition of USP19 will be especially effective at reducing muscle wasting as a result of inactivity, immobilisation or other disuse. On the basis of the results provided herein, pharmacological USP19 inhibition is also expected to be effective in treating muscular atrophy as a result of cachexia or sarcopenia.

### Obesity

Figure 3A shows the mass of the epididymal fat pad in mice following 2 weeks of receiving a USP19 inhibitor or vehicle alone. As shown in Figure 3, mice which received the USP19 inhibitor had significantly smaller fat pads compared to vehicle treated mice.

Figure 3B shows an increase in liver mass in mice treated with a USP19 inhibitor. This is thought to be as a result of drug accumulation in the liver.

Figure 3C shows that mice receiving USP19 inhibitor exhibited a reduction in overall body weight gain when on a high-fat diet. Average weekly body weight gain was significantly decreased by USP19 inhibitor (25 mg/kg ip bid) in week 1 and 2 (p<0.001 and p<0.01 respectively). In contrast, Liraglutide significantly decreased body weight gain in week 1 (p<0.001) but not week 2 (p>0.05).

Figures 3D and 3E show USP19 inhibitor treated mice exhibited a reduction in fat mass by 24% compared to the vehicle treated controls (p<0.001), but that lean body mass does not change significantly (-3%; p>0.05. Similarly, Liraglutide (0.1 mg/kg sc bid) significantly reduced fat mass (g) by 19% compared to the vehicle treated controls (p<0.001), but in contrast it also significantly reduced lean mass (g) by 8% (p<0.01).

When fat and lean mass were expressed as a percentage of total tissue mass, USP19 inhibitor (25 mg/kg ip bid) significantly decreased fat mass % and increased lean mass % (p<0.001). In contrast, Liraglutide did not significantly change fat or lean mass % (p=0.069).

Thus, DEXA analysis showed that administration of a USP19 inhibitor (ADC-141) caused a significant reduction in total tissue mass, which was primarily attributed to a reduction in fat mass, with no significant change in lean mass.

Figure 4 shows body composition data determined based on carcass material. USP19 inhibitor (25 mg/kg ip bid) and Liraglutide (0.1 mg/kg sc bid) significantly decreased carcass weight compared to that of vehicle-treated controls (c. -12%; p<0.001). Reductions in carcass weight observed following two weeks administration of USP19 inhibitor (25 mg/kg ip bid) and Liraglutide (0.1 mg/kg sc bid) closely reflected differences in body weights on the final day of the study (c. 13%).

USP19 inhibitor (5 and 25 mg/kg ip bid) had no significant effect on carcass water content, whereas Liraglutide caused a significant reduction in carcass water content (g; -8.6%; p<0.001). When expressed as a percentage of total carcass mass, USP19 inhibitor (25 mg/kg ip bid) increased the relative water content of the carcass (+ 11.4%; p<0.001) whereas ADC-141 (5 mg/kg ip bid) and Liraglutide (0.1 mg/kg sc bid) had no effect on this parameter.

USP19 inhibitor ADC-141 (25 mg/kg ip bid) produced a 24.9% reduction in carcass fat (g; p<0.001) from controls. Liraglutide (0.1 mg/kg sc bid) produced a 17.4% reduction in carcass fat (g; p<0.01) (Figure 4). When expressed as a percentage, only ADC-141 (25 mg/kg ip bid) significantly reduced the carcass percentage fat (-14.6%; p<0.001). Overall, the loss of fat accounted for 79% of the total weight lost for ADC-141 (25 mg/kg ip bid) and 60% for Liraglutide (0.1 mg/kg sc bid).

Carcass protein content (g) was significantly decreased by USP19 inhibitor ADC-141 (25 mg/kg ip bid; -7.2%; p<0.05) and Liraglutide (0.1 mg/kg sc bid; -7.9%; p<0.05). However, when expressed as a percentage of total carcass mass, percent protein was significantly increased (6.0% and 5.7% respectively; p<0.05; Figure 4). The lowest dose of ADC-141 (5 mg/kg ip bid) produced no significant changes in carcass protein when compared to vehicle-treated animals.

Carcass ash content (g) was significantly reduced by USP19 inhibitor ADC-141 (25 mg/kg ip bid; -9.6%; p<0.05) and Liraglutide (0.1 mg/kg sc bid; -11.6%; p<0.01). However, when expressed as a percentage of total carcass mass, there was no significant difference in carcass ash for any of the treatment groups in comparison to control values (Figure 4).

DIO mice treated with USP19 inhibitor also exhibited a reduction in cumulative and average food intake compared to vehicle control mice (p<0.001).

In conclusion, administration of USP19 inhibitor (25 mg/kg ip bid) produced a marked reduction in carcass weight, which was primarily attributed to a significant loss of fat mass, with a small contribution from the loss of protein and ash. Effective weight loss agents typically produce weight loss consisting of 70-90% from fat, with the remainder consisting of a small compensatory decrease in protein, ash and water. Therefore, the effect of the USP19 inhibitor ADC-141 to target the loss of fat mass *per se* is consistent with the desirable effects of anti-obesity agents in the DIO mouse model.

The data shown in Figures 3 and 4 is the first demonstration that pharmacological inhibition of USP19 can reduce fat accumulation in a wild-type background. Gene knockout studies have described a possible association between USP19 and fat accumulation (Coyne et al, Diabetologia. 2018 Nov 1. doi: 10.1007/s00125-018-4754-4., incorporated herein by reference). However, acute or chronic pharmacological inhibition of an enzyme does not always result in similar physiological outcomes to genetic ablation.

It is notable also that UP19 inhibition is able to reduce fat accumulation while preserving or increasing relative body protein and ash content.

The *in vivo* pharmacological inhibition data provided herein demonstrate that compounds which potently inhibit USP19 activity can effectively treat obesity.

### Insulin resistance

Figure 6 shows the results of an oral glucose tolerance test (OGTT) in mice with diet-induced obesity.

Mice responded to the glucose load as expected, with a sharp increase in plasma glucose and insulin which diminished by 120 minutes post-glucose. USP19 inhibitor ADC-141 (25 mg/kg ip bid) significantly reduced plasma glucose at all time points pre- and post-glucose (Figure 6A) and glucose AUC (Figure 6B) and AUCB2 (0-120 minutes only), compared to the vehicle group. USP19 inhibitor ADC-141 (25 mg/kg ip bid) also reduced plasma insulin at 30, 60 and 120 minutes post-glucose, and insulin AUC (0-60 and 0-120 minutes; Figure 6C).

Following 2 weeks of treatment, USP19 inhibition was effective at decreasing fasting plasma glucose whilst maintaining plasma insulin levels similar to that of the controls, indicating improved insulin sensitivity. Consistent with these observations in the fasted state, when challenged with a glucose load USP19 inhibition led to improved glucose disposal and stimulated a diminished increase in plasma insulin (at 30, 60 and 120 minutes) compared to that of the controls. Therefore, treatment with a USP19 inhibitor was effective at improving insulin sensitivity and glucose tolerance in the DIO mouse model of insulin resistance.

The data shown in Figure 6 is the first demonstration that pharmacological inhibition of USP19 can reduce insulin resistance in a wild-type background. Gene knockout studies have also described an association between USP19 and insulin sensitivity (Coyne *et al, supra*)*.* Coyne *et al.* describe an improvement in insulin sensitivity in USP19 knockout mice but, as noted above, it could not be assumed that the effects would translate to pharmacological inhibition of USP19 in wild-type subjects.

The data provided herein is the first demonstration that pharmacological inhibition of USP19 effectively treats insulin resistance.

The data presented herein is the first demonstration of the therapeutic effects of pharmacological inhibition of USP19. Accordingly, USP19 inhibitors, for example those compounds provided herein and disclosed in WO2018/020242, can effectively treat muscular atrophy, obesity, insulin resistance and/or cancer.

### Experimental

The following USP19 inhibitory compounds were first described in WO2018/020242, where their synthesis is provided in detail. The Example numbers provided below correspond to the Examples of WO2018/020242.

### Example 1: (R)-5-Bromo-1-((-hydroxy-1-(3-phenylbutanoyl)piperidin-4-yl)methyl)-4-phenylpyridin-2(1H)-one

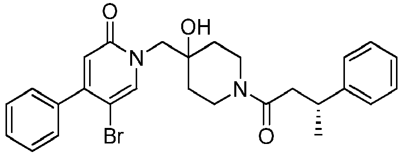

### Example 2: 6-(2-Fluorophenyl)-3-((4-hydroxy-1-(3-phenylpropanoyl)piperidin-4-yl)methyl)pyrimidin-4(3H)-one

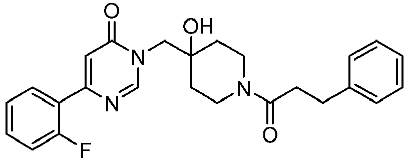

### Example 3: 3-((1-(3-Cyclobutylpropanoyl)-4-hydroxypiperidin-4-yl)methyl)-6-(2-fluorophenyl)pyrimidin-4(3H)-one

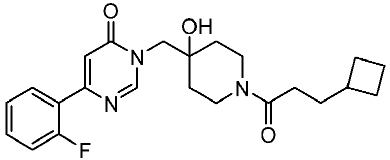

### Example 4: 3-((1-(3-Cyclopentylpropanoyl)-4-hydroxypiperidin-4-yl)methyl)-6-(2-fluorophenyl)pyrimidin-4(3H)-one

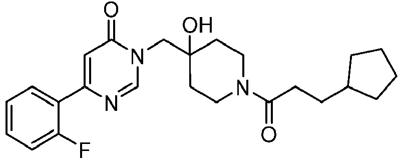

### Example 5: (R,S)-6-(2-Fluorophenyl)-3-((4-hydroxy-1-(2-methyl-3-phenylpropanoyl)piperidin-4-yl)methyl)pyrimidin-4(3H)-one

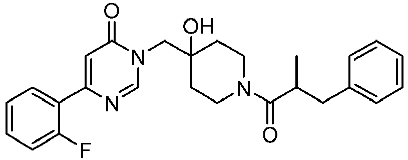

### Example 6: (R)-N-(3-(1-((4-Hydroxy-1-(3-phenylbutanoyl)piperidin-4-yl)methyl)-6-oxo-4-phenyl-1,6-dihydropyridin-3-yl)phenyl)acetamide

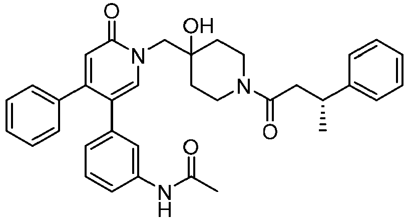

### Example 7: (R)-3-(1-((4-Hydroxy-1-(3-phenylbutanoyl)piperidin-4-yl)methyl)6-oxo-4-phenyl-1,6-dihydropyridin-3-yl)benzamide

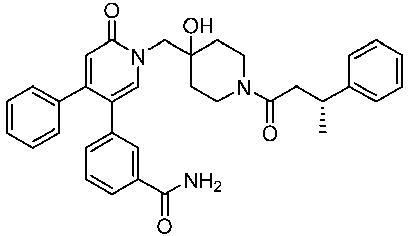

### Example 8: (R)-5-(Furan-2-yl)-1-((4-hydroxy-1-(3-phenylbutanoyl)piperidin-4-yl)methyl)-4-phenylpyridin-2(1H)-one

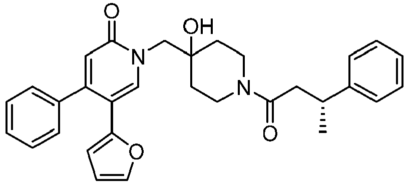

### Example 9: (R)-1-((4-Hydroxy-1-(3-phenylbutanoyl)piperidin-4-yl)methyl)-5-(1-methyl-1H-pyrazol-5-yl)-4-phenylpyridin-2(1H)-one

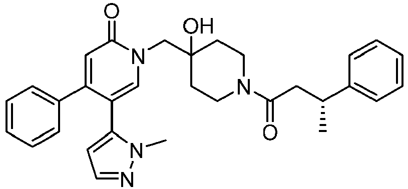

### Example 10: (R)-4-Chloro-1-((4-hydroxy-1(3-phenylbutanoyl)piperidin-4-yl)methyl)-5-phenylpyridin-2(1H)-one

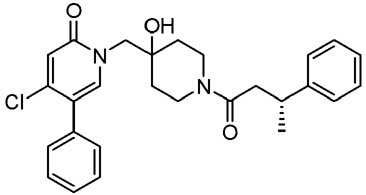

### Example 11: (R)-4-(Dimethylamino)-1-((4-hydroxy-1-(3-phenylbutanoyl)piperidin-4-yl)methyl)-5-phenylpyridin-2(1H)-one

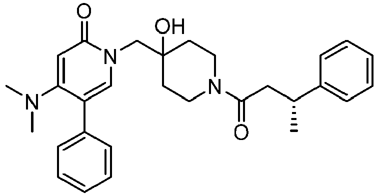

### Example 12: (R)-1-((4-Hydroxy-1-(3-phenylbutanoyl)piperidin-4-yl)methyl)-5-phenyl-4-(prop-1-en-2-yl)pyridine-2(1H)-one

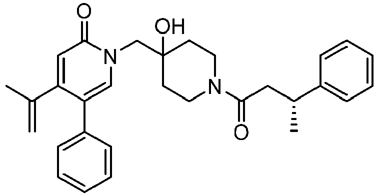

### Example 13: (R)-Ethyl 1-((4-hydroxy-1-(3-phenylbutanoyl)piperidin-4-yl)methyl)-6-oxo-4-phenyl-1,6-dihydropyridi ne-3-carboxylate

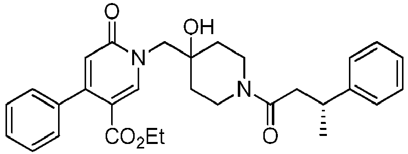

### Example 14: (R,S)-4-(2-Fluorophenyl)-1-((4-hydroxy-1-(2-methyl-1,2,3,4-tetrahydronaphthalene-2-carbonyl)piperidin-4-yl)methyl)-N-isopropyl-N-methyl-6-oxo-1,6-dihydropyridine-3-carboxamide

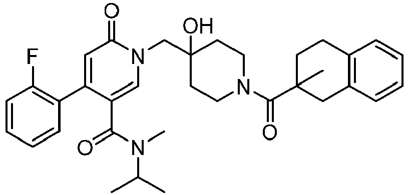

### Example 15: (R,S)-1-((1-(3-Cyclobutyl-2-methylpropanoyl)-4-hydroxypiperidin-4-yl)methyl)-4-phenyl-5-(piperidine-1-carbonyl)pyridin-2(1H)-one

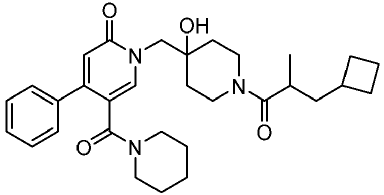

### Example 16: (R)-3-((4-Hydroxy-1-(3-phenylbutanoyl)piperidin-4-yl)methyl)-6-(1-methyl-1H-indazol-7-yl)pyrimidin-4(3H)-one

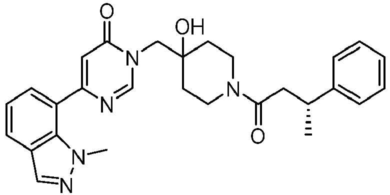

### Example 17: (R)-3-((4-Hydroxy-1-(3-phenylbutanoyl)piperidin-4-yl)methyl)-6-(2-methoxyphenyl)pyrimidin-4(3H)-one

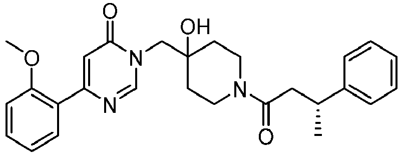

### Example 18: (R)-3-((4-Hydroxy-1-(3-phenylbutanoyl)piperidin-4-yl)methyl)-2-methyl-6-phenylpyrimidin-4(3H)-one

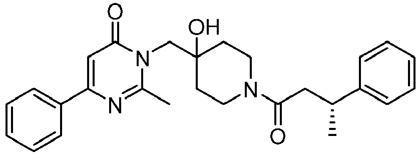

### Example 19: (R)-2-((4-Hydroxy-1-(3-phenylbutanoyl)piperidin-4-yl)methyl)-5,6-diphenylpyridazin-3(2H)-one

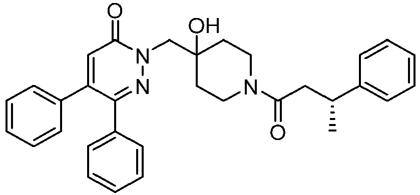

### Example 20: (R)-3-((4-Hydroxy-1-(3-phenylbutanoyl)piperidin-4-yl)methyl)-6-(naphthalen-1-yl)pyrimidin-4(3H)-one

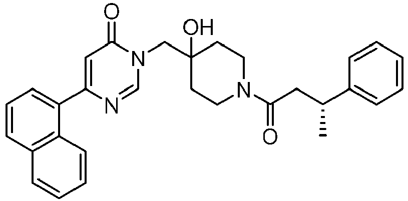

### Example 21: (R)-6-(3-(1,3-Dioxolan-2-yl)phenyl)-3-((4-hydroxy-1-(3-phenylbutanoyl)piperidin-4-yl)methyl)pyrimidin-4(3H)-one

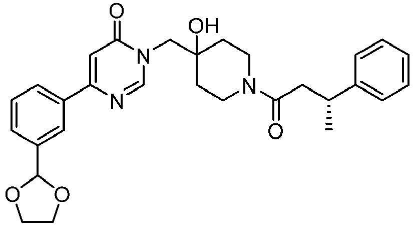

### Example 22: (R)-3-((4-Hydroxy-1-(3-phenylbutanoyl)piperidin-4-yl)methyl)-6-(thiophen-3-yl)pyrimidin-4(3H)-one

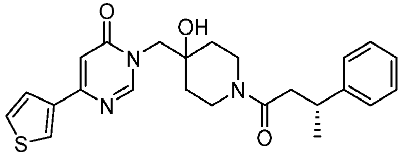

### Example 23: 4-(2-Fluorophenyl)-1-((4-hydroxy-1-(1-methylcyclopentane-1-carbonyl)piperidin-4-yl)methyl)-N-isopropyl-N-methyl-6-oxo-1,6-dihydropyridine-3-carboxamide

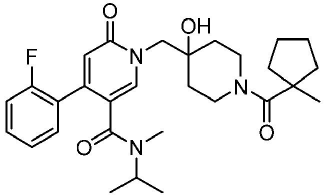

### Example 24: 3-((1-(1-Ethylcyclohexane-1-carbonyl)-4-hydroxypiperidin-4-yl)methyl)-6-(2-fluorophenyl)pyrimidin-4(3H)-one

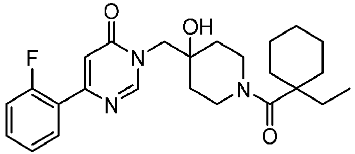

### Example 25: 3-((1-(2-(Cyclohexylmethyl)-3-methylbutanoyl)-4-hydroxy-3,3-dimethylpiperidin-4-yl)methyl)-6-(2-fluorophenyl)pyrimidin-4(3H)-one

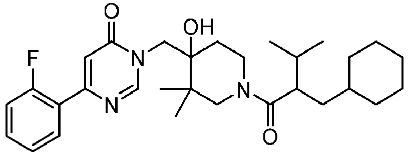

### Example 26: (R)-6-(Furan-2-yl)-3-((4-hydroxy-1-(3-phenylbutanoyl)piperidin-4-yl)methyl)pyrimidin-4(3H)-one

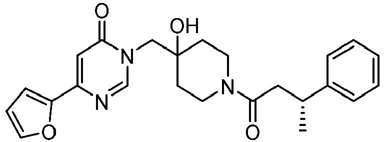

### Example 27: (R)-1-((4-Hydroxy-1-(3-phenylbutanoyl)piperidin-4-yl)methyl)-4-phenylpyridin-2(1H)-one

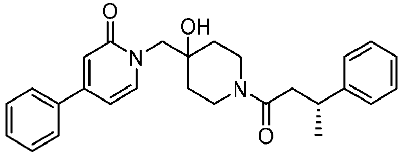

### Example 28: (R)-6-(Cyclohex-1-en-1-yl)-3-((4-hydroxy-1-(3-phenylbutanoyl)piperidin-4-yl)methyl)pyrimidin-4(3H)-one

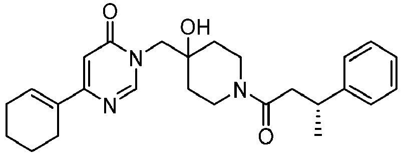

### Example 29: (R)-3-((4-Hydroxy-1-(3-phenylbutanoyl)piperidin-4-yl)methyl)-6-(prop-1-en-2-yl)pyrimidin-4(3H)-one

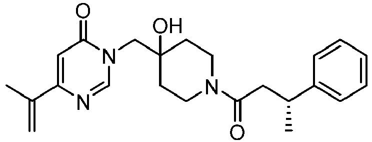

### Example 30: (R)-3-((4-Hydroxy-1-(3-phenylbutanoyl)piperidin-4-yl)methyl)-6-(2-(trifluoromethoxy)phenyl)pyrimidin-4(3H)-one

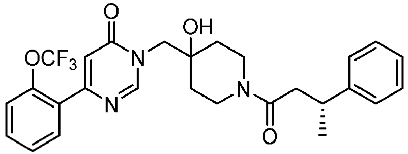

### Example 31: 3-(((1R,5S)-3-((R)-3-Cyclohexyl-2-methylpropanoyl)-8-hydroxy-3-azabicyclo[3.2.1]octan-8-yl)methyl)-6-(2-fluorophenyl)pyrimidin-4(3H)-one

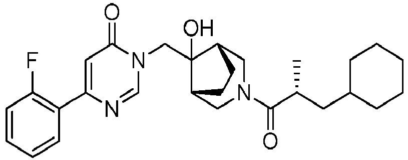

### Example 32: (R)-6-(2-Fluorophenyl)-3-((4-hydroxy-1-(3-phenylbutanoyl)piperidin-4-yl)methyl)pyrimidin-4(3H)-one

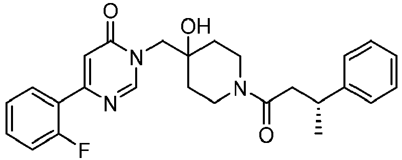

### Example 33: (R)-5-((4-Hydroxy-1-(3-phenylbutanoyl)piperidin-4-yl)methyl)-2-phenylpyrimidin-4(3H)-one

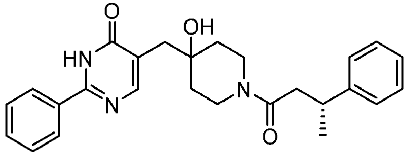

### Example 34: 3-((4-Hydroxy-1-(3-phenylpropanoyl)piperidin-4-yl)methyl)-2-methyl-6-phenylpyrimidin-4(3H)-one

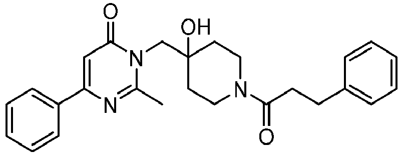

### Example 35: (R)-3-((4-Hydroxy-1-(3-phenylbutanoyl)piperidin-4-yl)methyl)-6-(isobutylamino)pyrimidin-4(3H)-one

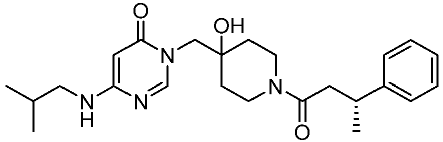

### Example 36: (R)-1-((1-(3-Cyclohexyl-2-methylpropanoyl)-4-hydroxypiperidin-4-yl)methyl)-4-phenyl-5-(piperidine-1-carbonyl)pyridin-2(1H)-one

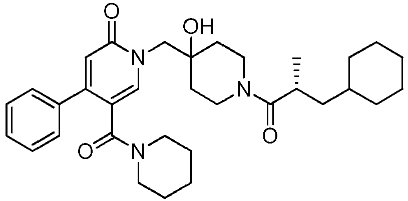

### Example 37: (R)-2-((4-Hydroxy-1-(3-phenylbutanoyl)piperidin-4-yl)methyl)-5-phenylpyridazin-3(2H)-one

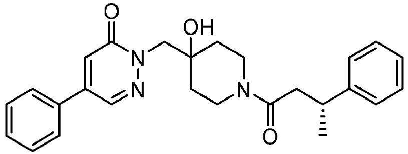

### Example 38: (R)-4-(2-Cyanophenyl)-1-((4-hydroxy-1-(3-phenylbutanoyl)piperidin-4-yl)methyl)-N-isopropyl-N-methyl-6-oxo-1,6-dihydropyridine-3-carboxamide

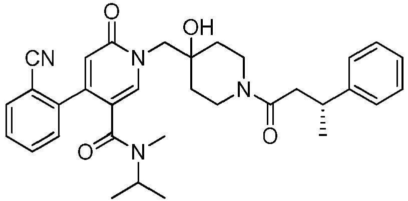

### Example 39: (R)-3-((1-(3-Cyclohexyl-2-methylpropanoyl)-4-hydroxypiperidin-4-yl)methyl)-6-(2-fluorophenyl)pyrimidin-4(3H)-one

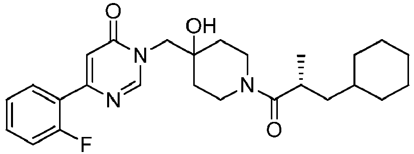

### Example 40: 3-((1-(2-(Cyclohexylmethyl)butanoyl)-4-hydroxypiperidin-4-yl)methyl)-6-(2-fluorophenyl)pyrimidin-4(3H)-one

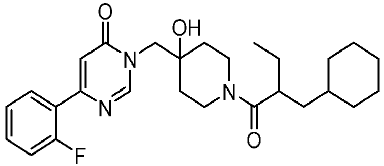

### Example 41: rac-6-(2-Fluorophenyl)-3-((4-hydroxy-1-(cis-2-phenylcyclopropanecarbonyl)piperidin-4-yl)methyl)pyrimidin-4(3H)-one

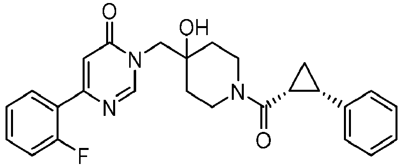

### Example 42: 6-(2-Fluorophenyl)-3-((4-hydroxy-1-(1-methylcyclohexanecarbonyl)piperidin-4-yl)methyl)pyrimidin-4(3H)-one

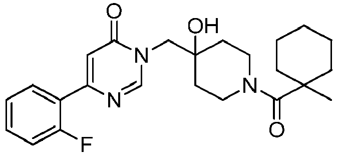

### Example 43: 3-((S)-1-(1-((R)-3-Cyclohexyl-2-methylpropanoyl)-4-hydroxypiperidin-4-yl)propyl)-6-phenylpyrimidin-4(3H)-one and 3-((R)-1-(1-((R)-3-cyclohexyl-2-methylpropanoyl)-4-hydroxypiperidin-4-yl)propyl)-6-phenylpyrimidin-4(3H)-one

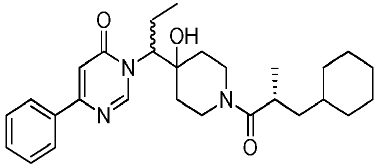

### Example 44: (R,S)-3-((1-(3-Cyclohexyl-2-hydroxypropanoyl)-4-hydroxypiperidin-4-yl)methyl)-6-(2-fluorophenyl)pyrimidin-4(3H)-one

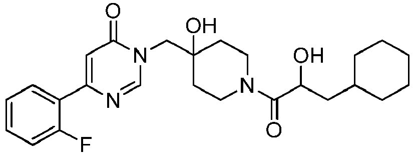

### Example 45: (R)-1-((4-Hydroxy-1-(3-phenylbutanoyl)piperidin-4-yl)methyl)-6-oxo-4-phenyl-1,6-dihydropyridine-3-carbaldehyde

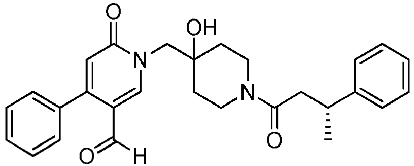

### Example 46: (R)-5-((Dimethylamino)methyl)-1-((4-hydroxy-1-(3-phenylbutanoyl)piperidin-4-yl)methyl)-4-phenylpyridin-2(1H)-one

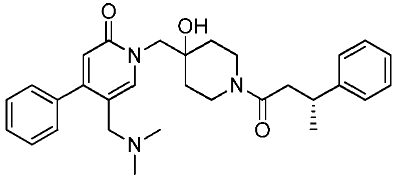

### Example 47: 3-((R)-1-(4-Hydroxy-1-((R)-3-phenylbutanoyl)piperidin-4-yl)ethyl)-6-phenylpyrimidin-4(3H)-one and 3-((S)-1-(4-hydroxy-1-((R)-3-phenylbutanoyl)piperidin-4-yl)ethyl)-6-phenylpyrimidin-4(3H)-one

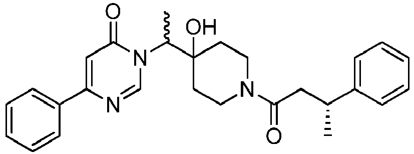

### Example 48: (R)-6-(2-Fluorophenyl)-3-((4-hydroxy-1-(4,4,4-trifluoro-3-phenylbutanoyl)piperidin-4-yl)methyl)pyrimidin-4(3H)-one

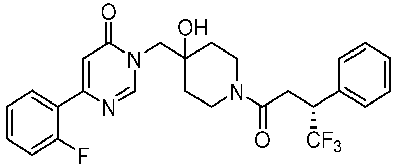

### Example 49: (R,S)-3-((1-(3-Cyclohexyl-2-fluoropropanoyl)-4-hydroxypiperidin-4-yl)methyl)-6-(2-fluorophenyl)pyrimidin-4(3H)-one

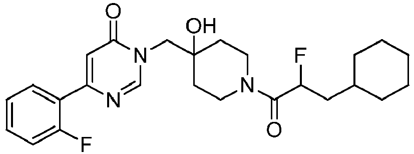

### Example 50: 3-(((R)-1-((R)-3-Cyclohexyl-2-methylpropanoyl)-4-hydroxy-3,3-dimethylpiperidin-4-yl)methyl)-6-phenylpyrimidin-4(3H)-one and 3-(((S)-1-((R)-3-Cyclohexyl-2-methylpropanoyl)-4-hydroxy-3,3-dimethylpiperidin-4-yl)methyl)-6-phenylpyrimidin-4(3H)-one

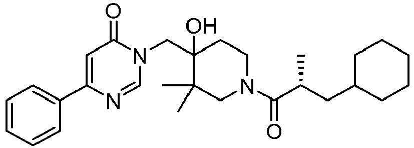

### Example 51: 3-(((R)-6-((R)-3-Cyclohexyl-2-methylpropanoyl)-9-hydroxy-6-azaspiro[3.5]nonan-9-yl)methyl)-6-(2-fluorophenyl)pyrimidin-4(3H)-one and 3-(((S)-6-((R)-3-Cyclohexyl-2-methylpropanoyl)-9-hydroxy-6-azaspiro[3.5]nonan-9-yl)methyl)-6-(2-fluorophenyl)pyrimidin-4(3H)-one

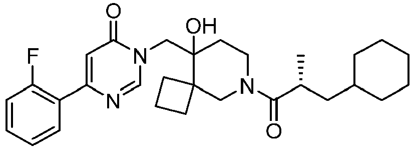

### Example 52: (R)-5-(3-(Aminomethyl)phenyl)-1-((1-(3-cyclohexyl-2-methylpropanoyl)-4-hydroxypiperidin-4-yl)methyl)-4-(2-fluorophenyl)pyridin-2(1H)-one

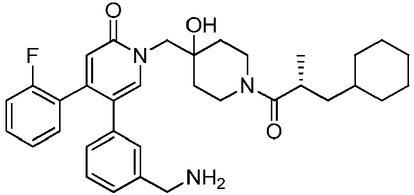

### Example 53: 3-(((S)-4-Hydroxy-3,3-dimethyl-1-((R)-3-phenylbutanoyl)piperidin-4-yl)methyl)-6-phenylpyrimidin-4(3H)-one and 3-((R)-4-hydroxy-3,3-dimethyl-1-((R)-3-phenylbutanoyl)piperidin-4-yl)methyl)-6-phenylpyrimidin-4(3H)-one

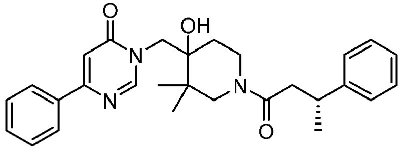

### Example 54: (R)-6-(4-Fluorophenoxy)-3-((4-hydroxy-1-(3-phenylbutanoyl)piperidin-4-yl)methyl)pyrimidin-4(3H)-one

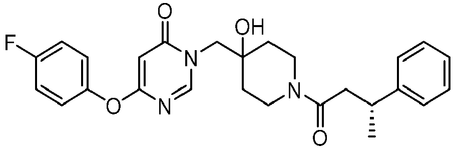

### Example 55: (R)-1-((4-Hydroxy-1-(3-phenylbutanoyl)piperidin-4-yl)methyl)-5-(2-oxopyrrolidin-1-yl)-4-phenylpyridin-2(1H)-one

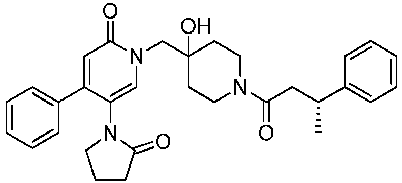

### Example 56: 6-(2-Fluorophenyl)-3-((4-hydroxy-1-(1-methyl-4-methylenecyclohexanecarbonyl)piperidin-4-yl)methyl)pyrimidin-4(3H)-one

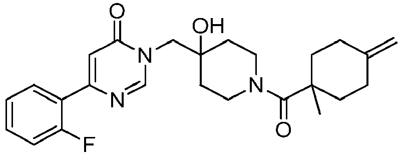

### Example 57: (R,S)-3-((1-(3-Cyclobutyl-2-methylpropanoyl)-4-hydroxypiperidin-4-yl)methyl)-6-(2-fluorophenyl)pyrimidin-4(3H)-one

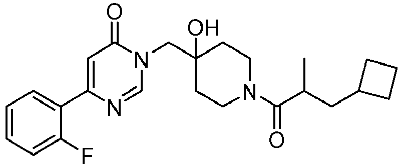

### Example 58: (R,S)-3-((1-(3-Cycloheptyl-2-methylpropanoyl)-4-hydroxypiperidin-4-yl)methyl)-6-(2-fluorophenyl)pyrimidin-4(3H)-one

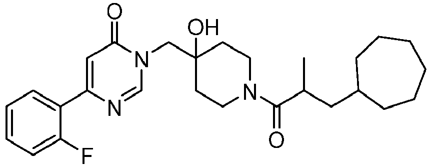

### Example 59: 3-((1-(3-Cyclobutyl-2,2-dimethylpropanoyl)-4-hydroxypiperidin-4-yl)methyl)-6-(2-fluorophenyl)pyrimidin-4(3H)-one

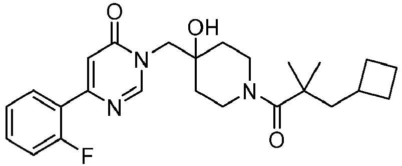

### Example 60: 6-(2-Fluorophenyl)-3-(((1R,5S)-8-hydroxy-3-((R)-3-phenylbutanoyl)-3-azabicyclo[3.2.1]octan-8-yl)methyl)pyrimidin-4(3H)-one

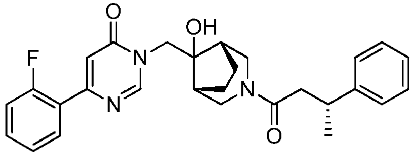

### Example 61: 3-(((R)-4-Hydroxy-2,2-dimethyl-1-((R)-3-phenylbutanoyl)piperidin-4-yl)methyl)-6-phenylpyrimidin-4(3H)-one and 3-(((S)-4-Hydroxy-2,2-dimethyl-1-((R)-3-phenylbutanoyl)piperidin-4-yl)methyl)-6-phenylpyrimidin-4(3H)-one

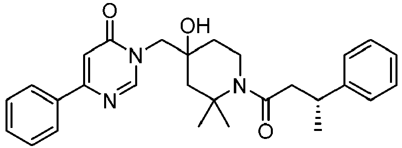

### Example 62: (R)-1-((4-Hydroxy-1-(3-phenylbutanoyl)piperidin-4-yl)methyl)-5-(4-hydroxypiperidine-1-carbonyl)-4-phenylpyridin-2(1H)-one

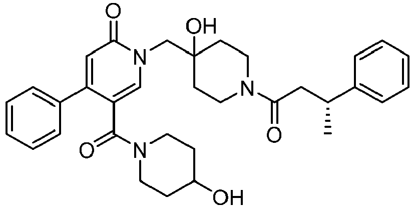

### Example 63: (R)-1-((4-Hydroxy-1-(3-phenylbutanoyl)piperidin-4-yl)methyl)-4-phenyl-5-(pyrrolidine-1-carbonyl)pyridin-2(1H)-one

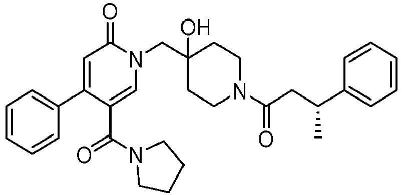

### Example 64: (R)-1-((4-Hydroxy-1-(3-phenylbutanoyl)piperidin-4-yl)methyl)-4-phenyl-5-(1,4-dioxa-8-azaspiro[4.5]decane-8-carbonyl)pyridin-2(1H)-one

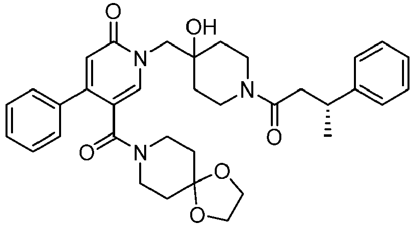

### Example 65: (R)-N-Cyclohexyl-1-((4-hydroxy-1-(3-phenylbutanoyl)piperidin-4-yl)methyl)-6-oxo-4-phenyl-1,6-dihydropyridine-3-carboxamide

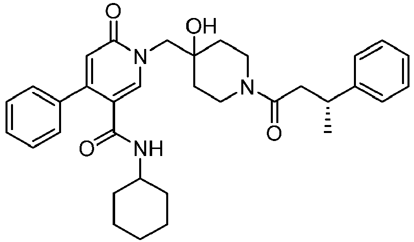

### Example 66: 6-(2-Fluorophenyl)-3-((4-hydroxy-3,3-dimethvl-1-(cis-2-phenylcyclopropane-1-carbonyl)piperidin-4-yl)methyl)pyrimidin-4(3H)-one

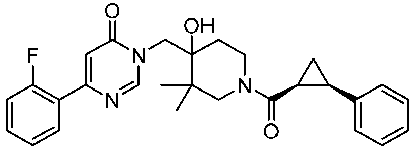

### Example 67: (R,S)-3-((1-(3-Cyclohexyl-1H-pyrazole-4-carbonyl)-4-hydroxy-3,3-dimethylpiperidin-4-yl)methyl)-6-(2-fluorophenyl)pyrimidin-4(3H)-one

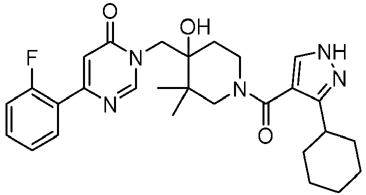

### Example 68: (R,S)-6-(2-Fluorophenyl)-3-((4-hydroxy-3,3-dimethyl-1-(3-phenylpropanoyl)piperidin-4-yl)methyl)pyrimidin-4(3H)-one

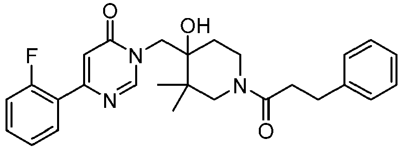

### Example 69: (R,S)-6-(2-Fluorophenyl)-3-((4-hydroxv-1-(1-isobutyl cyclopropane-1-carbonyl)-3,3-dimethylpiperidin-4-yl)methyl)pyrimidin-4(3H)-one

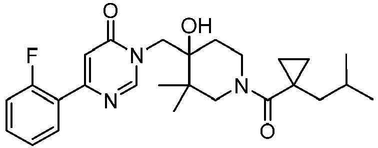

### Example 70: (R,S)-6-(2-Fluorophenyl)-3-((4-hydroxy-3,3-dimethyl-1-(3-methyl-3-phenylbutanoyl)piperidin-4-yl)methyl)pyrimidin-4(3H)-one

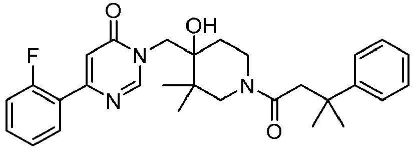

### Example 71: (S)-1-((1-(4,4-Difluoro-3-phenylbutanoyl)-4-hydroxypiperidin-4-yl)methyl)-N-isopropyl-N-methyl-6-oxo-4-phenyl-1,6-dihydropyridine-3-carboxamide

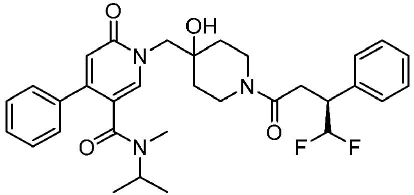

### Example 72: 1-((4-Hydroxy-1-(1-(thiophen-2-yl)cyclopropane-1-carbonyl)piperidin-4-yl)methyl)-N-isopropyl-N-methyl-6-oxo-4-phenyl-1,6-dihydropyridine-3-carboxamide

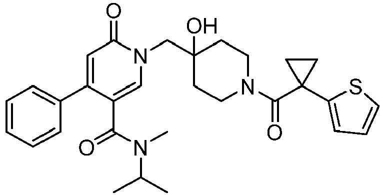

### Example 73: 1-(((R)-1-((R)-3-Cyclohexyl-2-methylpropanoyl)-4-hydroxy-3,3-dimethylpiperidin-4-yl)methyl)-N-isopropyl-N-methyl-6-oxo-4-phenyl-1,6-dihydropyridine-3-carboxamide and 1-(((S)-1-((R)-3-Cyclohexyl-2-methylpropanoyl)-4-hydroxy-3,3-dimethylpiperidin-4-yl)methyl)-N-isopropyl-N-methyl-6-oxo-4-phenyl-1,6-dihydropyridine-3-carboxamide

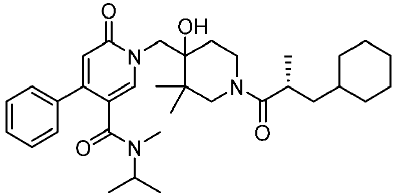

### Example 74: (R)-3-((4-Hydroxy-1-(3-phenylbutanoyl)piperidin-4-yl)methyl)-6-(2-(hydroxymethyl)phenyl)pyrimidin-4(3H)-one

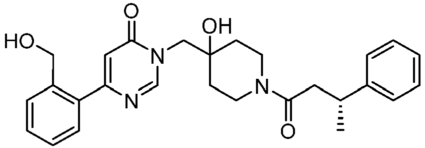

### Example 75: (R)-N,N-Diethyl-1-((4-hydroxy-1-(3-phenylbutanoyl)piperidin-4-yl)methyl)-6-oxo-4-phenyl-1,6-dihydropyridine-3-carboxamide

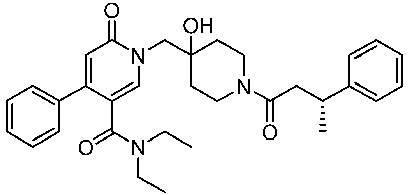

### Example 76: 3-((1-(3-Cyclohexylbutanoyl)-4-hydroxy-3,3-dimethylpiperidin-4-yl)methyl)-6-(2-fluorophenyl)pyrimidin-4(3H)-one

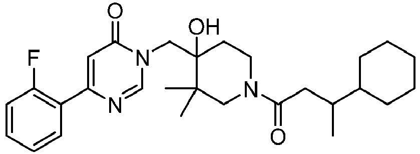

### Example 77: (R)-1-((4-Hydroxy-1-(3-phenylbutanoyl)piperidin-4-yl)methyl)-5-(morpholine-4-carbonyl)-4-phenylpyridin-2(1H)-one

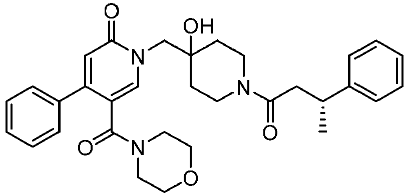

### Example 78: (R,S)-3-((1-(3-Cyclohexylpropanoyl)-4-hydroxy-3,3-dimethylpiperidin-4-yl)methyl)-6-(2-fluorophenyl)pyrimidin-4(3H)-one

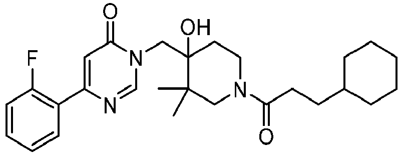

### Example 79: (R,S)-1-((1-(3-Cyclobutylpropanoyl)-4-hydroxy-3,3-dimethylpiperidin-4-yl)methyl)-N-isopropyl-N-methyl-6-oxo-4-phenyl-1,6-dihydropyridine-3-carboxamide

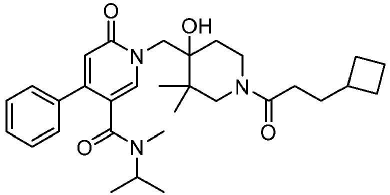

### Example 80: (R,S)-1-((1-(2-Ethylhexanoyl)-4-hydroxypiperidin-4-yl)methyl)-N-isopropyl-N-methyl-6-oxo-4-phenyl-1,6-dihydropyridine-3-carboxamide

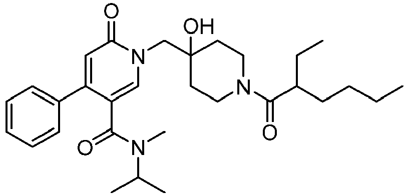

### Example 81: (R,S)-6-(2-Fluorophenyl)-3-((4-hydroxy-3,3-dimethyl-1-(1-methylcyclohexane-1-carbonyl)piperidin-4-yl)methyl)pyrimidin-4(3H)-one

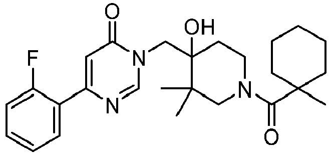

### Example 82: 6-(2-Fluorophenyl)-3-((1-(3-(4-fluorophenyl)propanoyl)-4-hydroxypiperidin-4-yl)methyl)pyrimidin-4(3H)-one

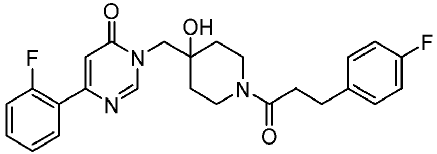

### Example 83: (R,S)-3-((1-(3-Cyclopropylpropanoyl)-4-hydroxy-3,3-dimethylpiperidin-4-yl)methyl)-6-(2-fluorophenyl)pyrimidin-4(3H)-one

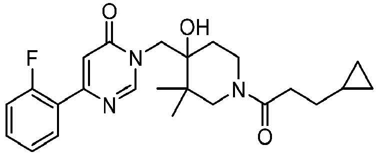

### Example 84: 1-((1-(2,2-Dimethylbutanoyl)-4-hydroxypiperidin-4-yl)methyl)-N-isopropyl-N-methyl-6-oxo-4-phenyl-1,6-dihydropyridine-3-carboxamide

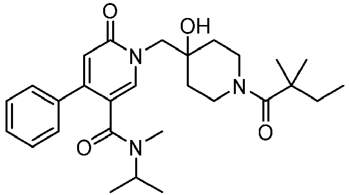

### Example 85: (R)-N-(2-(1-((4-Hydroxy-1-(3-phenylbutanoyl)piperidin-4-yl)methyl)-6-oxo-1,6-dihydropyrimidin-4-yl)phenyl)acetamide

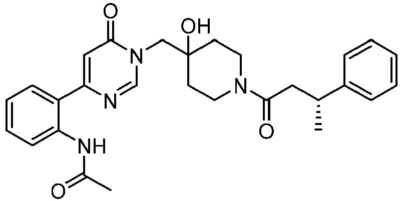

### Example 86: (R)-5-(4-(Aminomethyl)phenyl)-1-((4-hydroxy-1-(3-phenylbutanoyl)piperidin-4-yl)methyl)-4-phenylpyridin-2(1H)-one

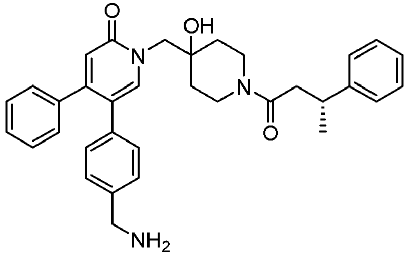

### Example 87: (R)-1-((1-(3-Cyclohexyl-2-methylpropanoyl)-4-hydroxypiperidin-4-yl)methyl)-N-isopropyl-N-methyl-6-oxo-4-phenyl-1,6-dihvdropyridine-3-carboxamide

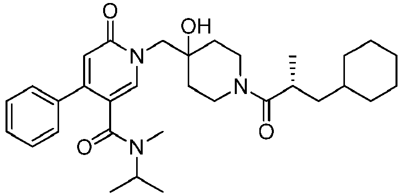

### Example 88: (R)-1-((4-Hydroxy-1-(3-phenylbutanoyl)piperidin-4-yl)methyl)-4-phenyl-5-(piperidine-1-carbonyl)pyridin-2(1H)-one

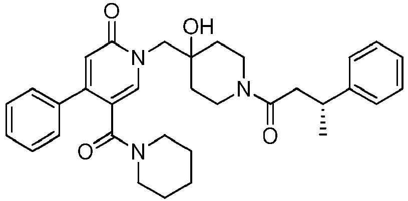

### Example 89: (R)-1-((4-Hydroxy-1-(3-phenylbutanoyl)piperidin-4-yl)methyl)-N-isopropyl-4-(2-methoxyphenyl)-N-methyl-6-oxo-1,6-dihydropyridine-3-carboxamide

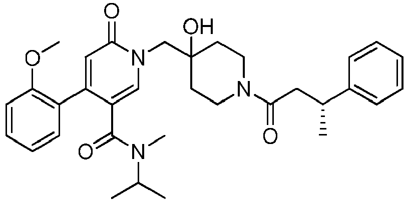

### Example 90: (R)-4-(2-Fluorophenyl)-1-((4-hydroxy-1-(3-phenylbutanoyl)piperidin-4-yl)methyl)-N-isopropyl-N-methyl-6-oxo-1,6-dihydropyridine-3-carboxamide

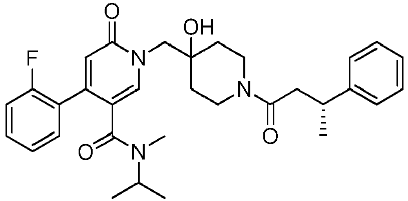

### Example 91: (R)-1'-((4-Hydroxy-1-(3-phenylbutanoyl)piperidin-4-yl)methyl)-4'-phenyl-[2,3'-bipyridin1-6'(1'H)-one

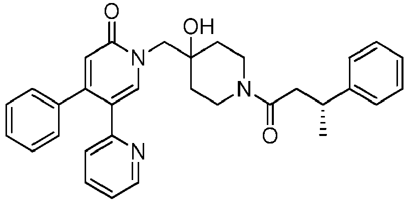

### Example 92: 1-((1-(3-Cyclohexylpropanoyl)-4-hydroxypiperidin-4-yl)methyl)-N-isopropyl-N-methyl-6-oxo-4-phenyl-1,6-dihydropyridine-3-carboxamide

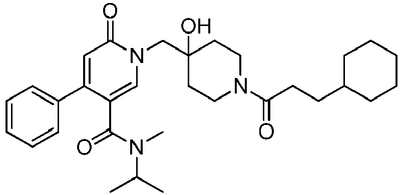

### Example 93: (R)-tert-Butyl 4-(1-((4-hydroxy-1-(3-phenylbutanoyl)piperidin-4-yl)methyl)-6-oxo-4-phenyl-1,6-dihydropyridine-3-carbonyl)piperazine-1-carboxylate

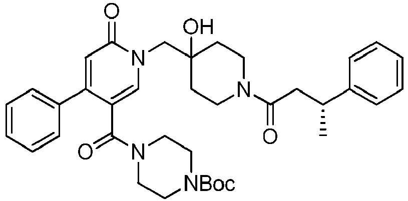

### Example 94: (R)-1-((4-Hydroxy-1-(3-phenylbutanoyl)piperidin-4-yl)methyl)-4-phenyl-5-(piperazine-1-carbonyl)pyridin-2(1H)-one

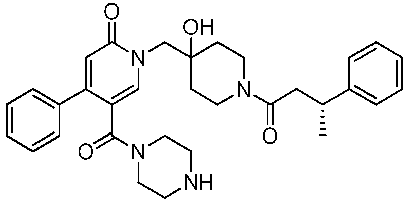

### Example 95: (R)-1-((4-Hydroxy-1-(3-phenylbutanoyl)piperidin-4-yl)methyl)-N,N-dimethyl-6-oxo-4-phenyl-1,6-dihydropyridine-3-carboxamide

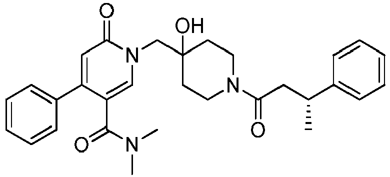

### Example 96: (R)-1-((4-Hydroxy-1-(3-phenylbutanoyl)piperidin-4-yl)methyl)-N-isopropyl-N-methyl-6-oxo-4-(p-tolyl)-1,6-dihydropyridine-3-carboxamide

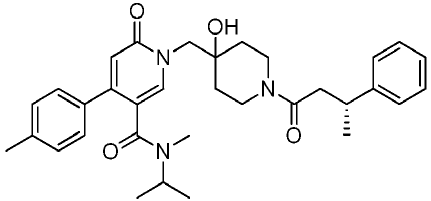

### Example 97: (R)-1-((4-Hydroxy-1-(3-phenylbutanoyl)piperidin-4-yl)methyl)-N-methoxy-N-methyl-6-oxo-4-phenyl-1,6-dihydropyridine-3-carboxamide

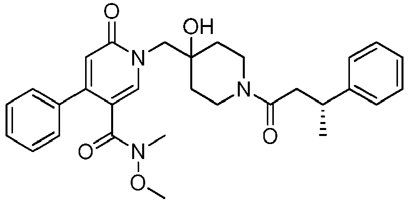

### Example 98: (R)-1-((4-Hydroxy-1-(3-phenylbutanoyl)piperidin-4-yl)methyl)-4,5-diphenylpyridin-2(1H)-one

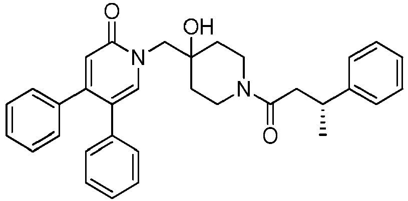

### Example 99: (R)-1-((4-Hydroxy-1-(3-phenylbutanoyl)piperidin-4-yl)methyl)-4-phenyl-[3,3'-bipyridin]-6(1H)-one

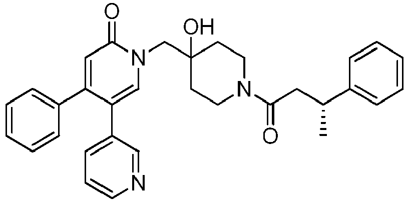

### Example 100: (R)-5-(3-(Aminomethyl)phenyl)-1-((4-hydroxy-1-(3-phenylbutanoyl)piperidin-4-yl)methyl)-4-phenylpyridin-2(1H)-one

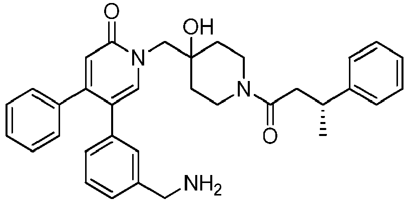

### Example 101: (R)-1-((4-Hydroxy-1-(3-phenylbutanoyl)piperidin-4-yl)methyl)-N-isopropyl-N-methyl-6-oxo-4-phenyl-1,6-dihydropyridine-3-carboxamide

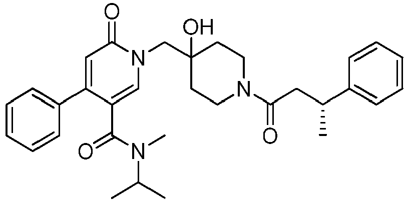

### Example 102: (R)-1-((4-Hydroxy-1-(3-phenylbutanoyl)piperidin-4-yl)methyl)-N-isopropyl-N-methyl-6-oxo-4-(m-tolyl)-1,6-dihydropyridine-3-carboxamide

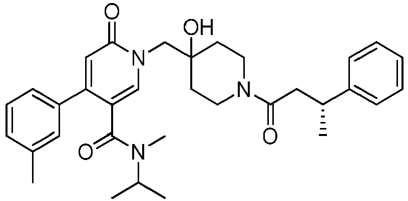

### Example 103: (R)-1-((4-Hydroxy-1-(3-phenylbutanoyl)piperidin-4-yl)methyl)-4-phenyl-5-(pyrimidin-5-yl)pyridin-2(1H)-one

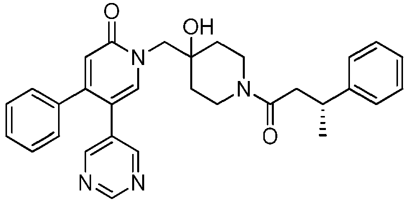

### Example 104: (R)-N-(Cyclopropylmethyl)-1-((4-hydroxy-1-(3-phenylbutanoyl)piperidin-4-vl)methyl)-6-oxo-4-phenyl-1,6-dihvdropyridine-3-carboxamide

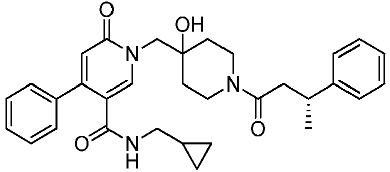

### Example 105: (R)-N-Benzyl-1-((4-hydroxy-1-(3-phenylbutanoyl)piperidin-4-yl)methyl)-N-methyl-6-oxo-4-phenyl-1,6-dihydropyridine-3-carboxamide

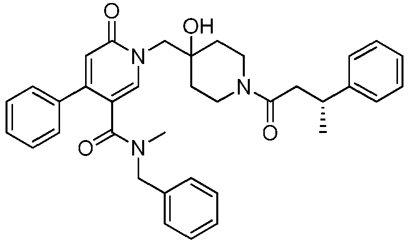

### Example 106: (R)-2-(1-((4-Hydroxy-1-(3-phenylbutanoyl)piperidin-4-yl)methyl)-6-oxo-4-phenyl-1,6-dihydropyridin-3-yl)benzonitrile

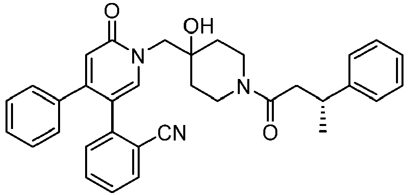

### Example 107: (R)-1-((4-Hydroxy-1-(3-phenylbutanoyl)piperidin-4-yl)methyl)-N-methyl-6-oxo-N,4-diphenyl-1,6-dihydropyridine-3-carboxamide

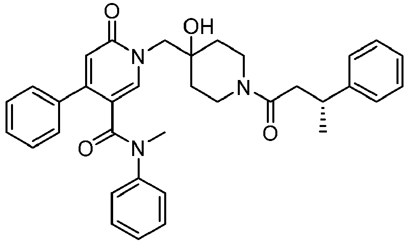

### Example 108: 3-(1-(1-((R)-3-Cyclohexyl-2-methylpropanoyl)-4-hydroxypiperidin-4-yl)ethyl)-6-phenylpyrimidin-4(3H)-one

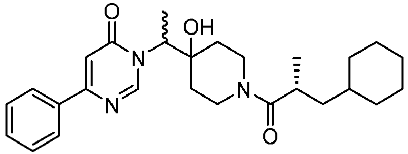

### Example 109: (R)-1-((4-Hydroxy-1-(3-phenylbutanoyl)piperidin-4-yl)methyl)-5-(1-methyl-1H-pyrazol-4-yl)-4-phenylpyridin-2(1H)-one

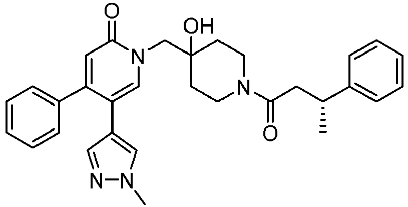

### Example 110: (R)-3-((1-(2-(Cyclohexylmethyl)pent-4-enoyl)-4-hydroxypiperidin-4-yl)methyl)-6-(2-fluorophenyl)pyrimidin-4(3H)-one

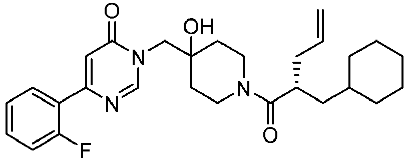

### Example 111: (R)-1-((4-Hydroxy-1-(3-phenylbutanoyl)piperidin-4-yl)methyl)-5-methyl-4-phenylpyridin-2(1H)-one

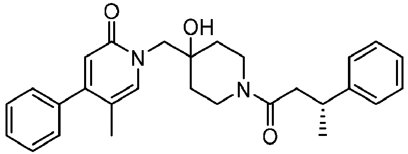

### Example 112: (R)-6-(1,5-Dimethyl-1H-pyrazol-4-yl)-3-((4-hydroxy-1-3-phenylbutanoyl)piperidin-4-yl)methyl)pyrimidin-4(3H)-one

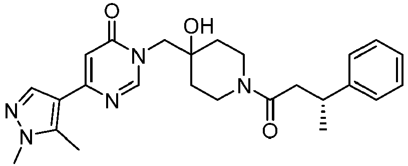

### Example 113: 3-(((1R,5S)-3-(3-Cyclohexylpropanoyl)-8-hydroxy-3-azabicyclo[3.2.1]octan-8-yl)methyl)-6-(2-fluorophenyl)pyrimidin-4(3H)-one

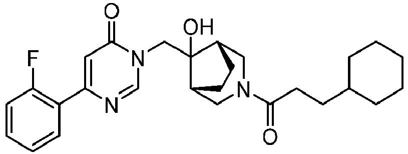

### Example 114: (R)-1-((4-Hydroxy-1-(3-phenylbutanoyl)piperidin-4-yl)methyl)-6-oxo-4-phenyl-1,6-dihydropyridine-3-carbonitrile

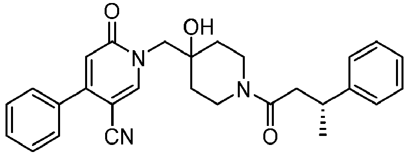

### Example 115: (R)-1-((4-Hydroxy-1-(3-phenylbutanoyl)piperidin-4-yl)methyl)-N-(2-hydroxyethyl)-6-oxo-4-phenyl-1,6-dihydropyridine-3-carboxamide

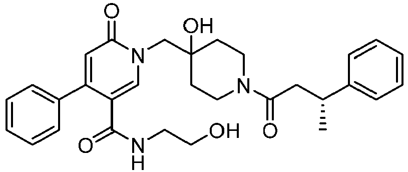

### Example 116: 1-((4-Hydroxy-1-((R)-3-phenylbutanoyl)piperidin-4-yl)methyl)-5-((S)-2-methylpyrrolidine-1-carbonyl)-4-phenylpyridin-2(1H)-one

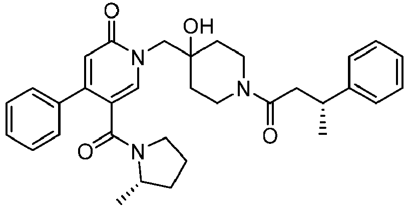

### Example 117: (R)-1-((4-Hydroxy-1-(3-phenylbutanoyl)piperidin-4-yl)methyl)-5-(4-(hydroxymethyl)piperidine-1-carbonyl)-4-phenylpyridin-2(1H)-one

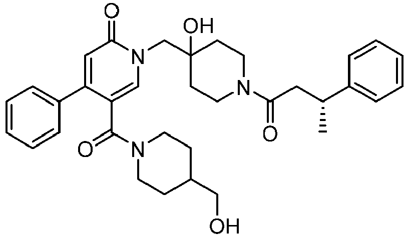

### Example 118: 3-((1-(2-(Cyclohexylmethyl)butanoyl)-4-hydroxy-3,3-dimethylpiperidin-4-yl)methyl)-6-(2-fluorophenyl)pyrimidin-4(3H)-one

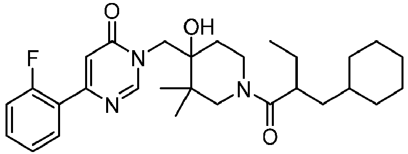

### Example 119: 3-((7-((R)-3-Cyclohexyl-2-methylpropanoyl)-10-hydroxy-7-azaspiro[4.5]decan-10-yl)methyl)-6-(2-fluorophenyl)pyrimidin-4(3H)-one

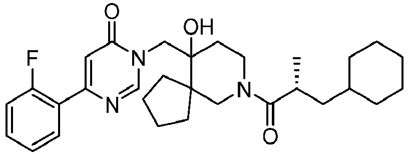

### Example 120: 1-(((S)-1-((R)-3-Cvclohexyl-2-methylpropanoyl)-4-hydroxy-3,3-dimethylpiperidin-4-yl)methyl)-N-isopropyl-N-methyl-6-oxo-4-phenyl-1,6-dihydropyridine-3-carboxamide

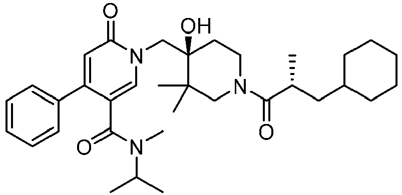

### Example 121: 1-(((S)-1-((R)-3-Cyclohexyl-2-methylpropanoyl)-4-hydroxy-3,3-dimethylpiperidin-4-yl)methyl)-N,N-dimethyl-6-oxo-4-phenyl-1,6-dihydropyridine-3-carboxamide

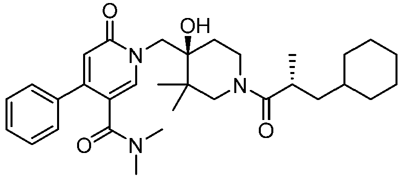

### Example 122: 1-(((R)-1-((R)-3-Cyclohexyl-2-methylpropanoyl)-4-hydroxy-3,3-dimethylpiperidin-4-yl)methyl)-N,N-dimethyl-6-oxo-4-phenyl-1,6-dihydropyridine-3-carboxamide

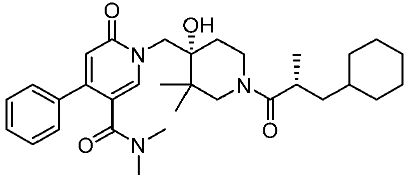

### Example 123: 1-(((S)-1-((R)-3-Cyclohexyl-2-methylpropanoyl)-4-hydroxy-3,3-dimethylpiperidin-4-yl)methyl)-4-phenyl-5-(pyrrolidine-1-carbonyl)pyridin-2(1H)-one

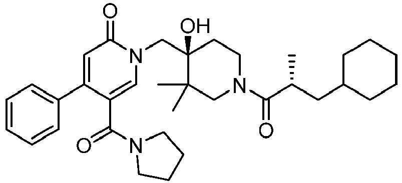

### Example 124: 1-(((S)-1-((R)-3-Cyclohexyl-2-methylpropanoyl)-4-hydroxy-3,3-dimethylpiperidin-4-yl)methyl)-4-phenyl-5-(piperazine-1-carbonyl)pyridin-2(1H)-one

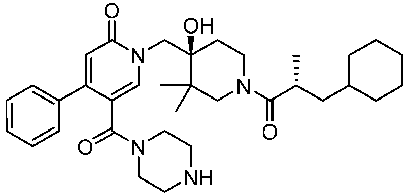

### Example 125: 1-(((R)-1-((R)-3-Cyclohexyl-2-methylpropanoyl)-4-hydroxy-3,3-dimethylpiperidin-4-yl)methyl)-4-phenyl-5-(piperazine-1-carbonyl)pyridin-2(1H)-one

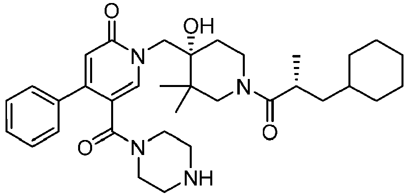

### Example 126: 3-((1-(2-(Cyclohexyloxy)propanoyl)-4-hydroxy-3,3-dimethylpiperidin-4-yl)methyl)-6-(2-fluorophenyl)pyrimidin-4(3H)-one

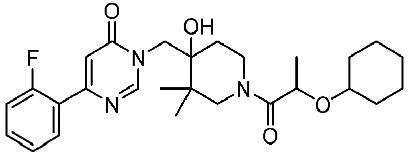

### Example 127: 1-((7-((R)-3-Cyclohexyl-2-methylpropanoyl)-10-hydroxy-7-azaspiror[4.5]decan-10-yl)methyl)-N,N-dimethyl-6-oxo-1,6-dihydropyridine-3-carboxamide

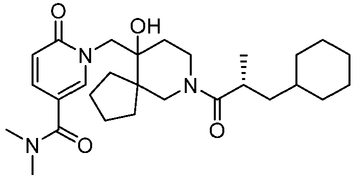

### Example 128: 1-((1-((R)-3-Cyclobutyl-2-methylpropanoyl)-4-hydroxy-3,3-dimethylpiperidin-4-yl)methyl)-N,N-dimethyl-6-oxo-4-phenyl-1,6-dihydropyridine-3-carboxamide

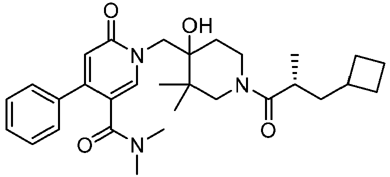

### Example 129: 1-((1-((R)-3-Cyclopropyl-2-methylpropanoyl)-4-hydroxy-3,3-dimethylpiperidin-4-yl)methyl)-N,N-dimethyl-6-oxo-4-phenyl-1,6-dihydropyridine-3-carboxamide

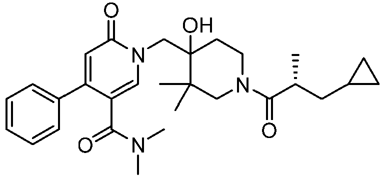

### Example 130: 1-((1-((R)-3-Cyclohexyl-2-methylpropanoyl)-4-hydroxy-3,3-dimethylpiperidin-4-yl)methyl)-[4,5'-b]pyrimidin]-6(1H)-one

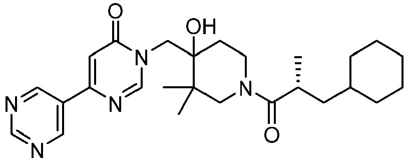

### Example 131: 3-((1-((R)-3-Cvclohexyl-2-methylpropanoyl)-4-hydroxy-3,3-dimethylpiperidin-4-yl)methyl)-6-(1-methyl-1H-pyrazol-5-yl)pyrimidin-4(3H)-one

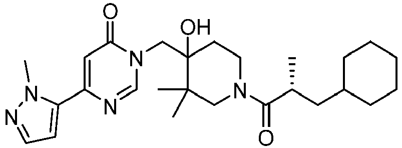

### Example 132: 3-((1-((R)-3-Cyclohexyl-2-methylpropanoyl)-4-hydroxy-3,3-dimethylpiperidin-4-yl)methyl)-6-(1H-pyrazol-4-yl)pyrimidin-4(3H)-one

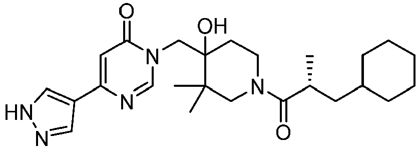

### Example 133: 3-((1-((R)-3-Cyclohexyl-2-methylpropanoyl)-4-hydroxy-3,3-dimethylpiperidin-4-yl)methyl)-6-(1H-pyrazol-5-yl)pyrimidin-4(3H)-one

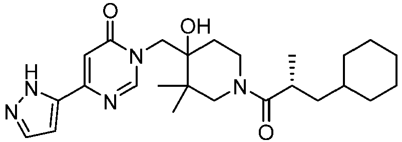

### Example 134: 3-((7-((R)-3-Cyclohexyl-2-methylpropanoyl)-10-hydroxy-7-azaspiro[4.5]decan-10-yl)methyl)pyrimidin-4(3H)-one

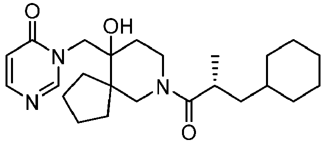

### Example 135: 3-((1-((R)-3-Cyclohexyl-2-methylpropanoyl)-4-hydroxy-3,3-dimethylpiperidin-4-yl)methyl)-6-(thiophen-3-yl)pyrimidin-4(3H)-one

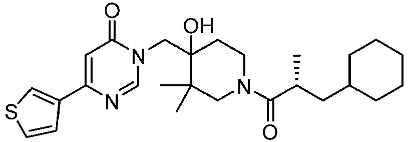

### Example 136: 1-((1-((R)-3-Cyclohexyl-2-methylpropanoyl)-4-hydroxy-3,3-dimethylpiperidin-4-yl)methyl)-5-(oxazol-2-yl)-4-phenylpyridin-2(1H)-one

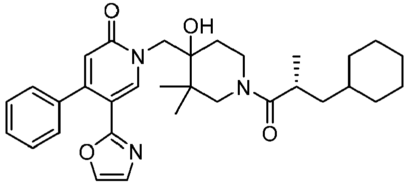

### Example 137: 3-((1-((R)-3-Cyclohexyl-2-methylpropanoyl)-4-hydroxy-3,3-dimethylpiperidin-4-yl)methyl)-6-(2-(hydroxymethyl)phenyl)pyrimidin-4(3H)-one

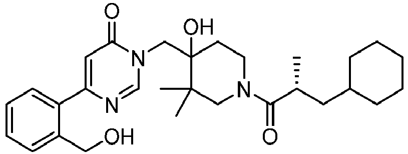

### Example 138: 3-((1-((R)-3-Cyclohexyl-2-methylpropanoyl)-4-hydroxy-3,3-dimethylpiperidin-4-yl)methyl)-6-(3-(hydroxymethyl)phenyl)pyrimidin-4(3H)-one

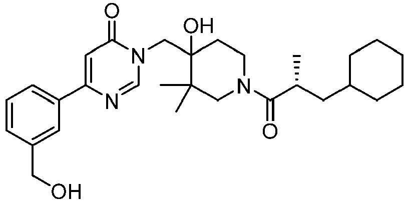

### Example 139: 3-((1-((R)-3-Cvclohexyl-2-methylpropanoyl)-4-hydroxy-3,3-dimethylpiperidin-4-yl)methyl)-6-(3-(hydroxymethyl)phenyl)pyrimidin-4(3H)-one

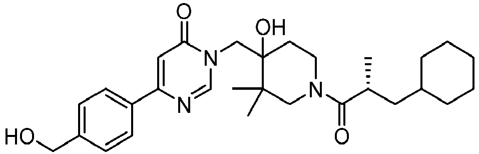

### Example 140: 6-(4-(Aminomethyl)phenyl)-3-((1-((R)-3-cyclohexyl-2-methylpropanoyl)-4-hydroxy-3,3-dimethylpiperidin-4-yl)methyl)pyrimidin-4(3H)-one

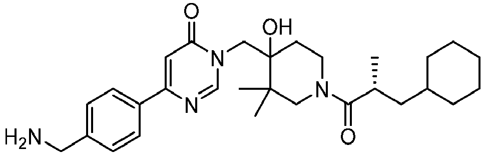

### Example 141: 6-(2-(Aminomethyl)phenyl)-3-((1-((R)-3-cyclohexyl-2-methylpropanoyl)-4-hydroxy-3,3-dimethylpiperidin-4-yl)methyl)pyrimidin-4(3H)-one

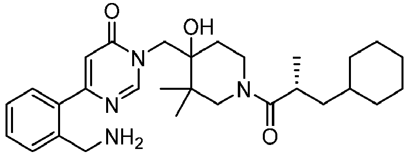

### Example 142: 6-(2-Fluorophenyl)-3-((4-hydroxy-3,3-dimethyl-1-(2,4,4-trimethylpentanoyl)piperidin-4-yl)methyl)pyrimidin-4(3H)-one

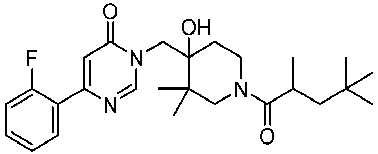

### Example 143: 6-(2-Fluorophenyl)-3-((4-hydroxy-3,3-dimethyl-1-(4,4,4-trifluoro-2-methylbutanoyl)piperidin-4-yl)methyl)pyrimidin-4(3H)-one

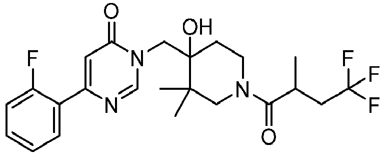

### Example 144: 4-Chloro-1-((7-((R)-3-cyclohexyl-2-methylpropanoyl)-10-hydroxy-7-azaspiro[4.5]decan-10-yl)methyl)-N,N-dimethyl-6-oxo-1,6-dihydropyridine-3-carboxamide

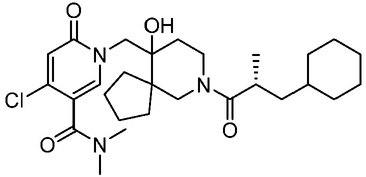

### Example 145: 1-((7-((R)-3-Cyclohexyl-2-methylpropanoyl)-10-hydroxy-7-azaspirof4.51decan-10-yl)methyl)-4-(dimethylamino)-N,N-dimethyl-6-oxo-1,6-dihydropyridine-3-carboxamide

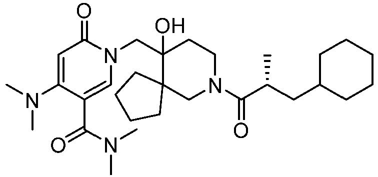

### Example 146: 1-((1-((S)-3-(Benzyloxy)-2-(cyclohexylmethyl)propanoyl)-4-hydroxy-3,3-dimethylpiperidin-4-yl)methyl)-N,N-dimethyl-6-oxo-4-phenyl-1,6-dihydropyridine-3-carboxamide

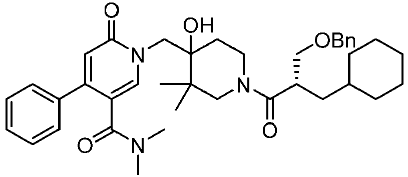

### Example 147: 1-((10-Hydroxy-7-isobutyryl-7-azaspiro[4.5]decan-10-yl)methyl)-N,N-dimethyl-6-oxo-4-phenyl-1,6-dihydropyridine-3-carboxamide

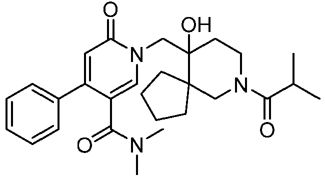

### Example 148: 1-((10-Hydroxy-7-((R)-2-methylbutanoyl)-7-azaspiro[4.5]decan-10-yl)methyl)-N,N-dimethyl-6-oxo-4-phenyl-1,6-dihvdropyridine-3-carboxamide

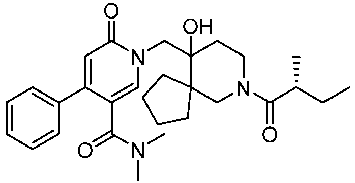

### Example 149: 1-((7-((R)-3-Cyclopropyl-2-methylpropanoyl)-10-hydroxy-7-azaspiro[4.5]decan-10-yl)methyl)-N,N-dimethyl-6-oxo-4-phenyl-1,6-dihydropyridine-3-carboxamide

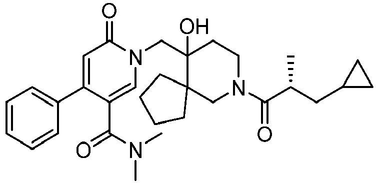

### Example 150: 6-(2-Fluorophenyl)-3-((4-hydroxy-3,3-dimethyl-1-(2-methyl-3-(1H-pyrazol-1-yl)propanoyl)piperidin-4-yl)methyl)pyrimidin-4(3H)-one

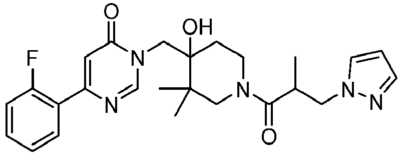

### Example 151: 1-((7-((R)-3-Cyclohexyl-2-methylpropanoyl)-10-hydroxy-7-azaspiro[4.5]decan-10-yl)methyl)-4-(2-(hydroxymethyl)phenyl)-N,N-dimethyl-6-oxo-1,6-dihydropyridine-3-carboxamide

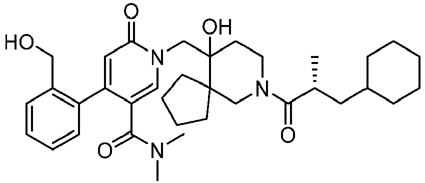

### Example 152 and Example 153: 1-(((S)-1-((S)-3-Cyclohexyl-2-(hydroxymethyl)propanoyl)-4-hydroxy-3,3-dimethylpiperidin-4-yl)methyl)-N,N-dimethyl-6-oxo-4-phenyl-1,6-dihydropyridine-3-carboxamide and 1-(((R)-1-((S)-3-Cyclohexyl-2-(hydroxymethyl)propanoyl)-4-hydroxy-3,3-dimethylpiperidin-4-yl)methyl)-N,N-dimethyl-6-oxo-4-phenyl-1,6-dihydropyridine-3-carboxamide

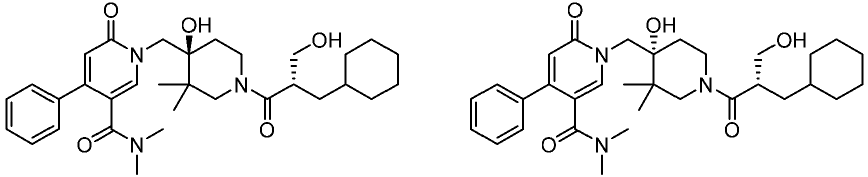

### Example 154: 1-((1-((R)-3-Cyclohexyl-2-methylpropanoyl)-4-hydroxy-3,3-dimethylpiperidin-4-yl)methyl)-4'-(2-fluorophenyl)-[2,3'-bipyridin]-6'(1H)-one

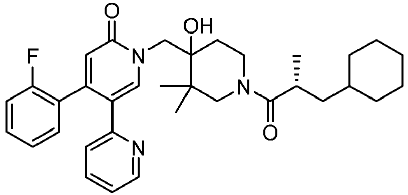

### Example 155: 1-((7-((R)-3-Cyclohexyl-2-methylpropanoyl)-10-hydroxy-7-azaspiro[4.5]decan-10-yl)methyl)-N,N-dimethyl-6-oxo-4-(thiophen-3-yl)-1,6-dihydropyridine-3-carboxamide

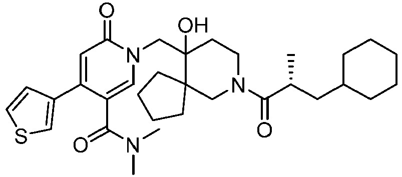

### Example 156: 1-((7-((R)-3-Cyclohexyl-2-methylpropanoyl)-10-hydroxy-7-azaspiro[4.5]decan-10-yl)methyl)-4-(3-(hydroxymethyl)phenyl)-N,N-dimethyl-6-oxo-1,6-dihydropyridine-3-carboxamide

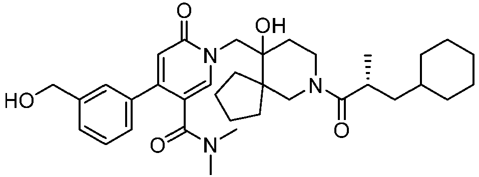

### Example 157: 1-((7-(3-Cyclohexyl-2-hydroxypropanoyl)-10-hydroxy-7-azaspiro[4.5]decan-10-yl)methyl)-N,N-dimethyl-6-oxo-4-phenyl-1,6-dihydropyridine-3-carboxamide

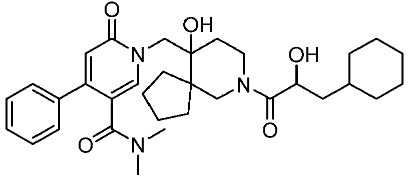

### Example 158: 1-((10-Hydroxy-7-(2-methyl-3-(piperidin-1-yl)propanoyl)-7-azaspiro[4.5]decan-10-yl)methyl)-N,N-dimethyl-6-oxo-4-phenyl-1,6-dihydropyridine-3-carboxamide

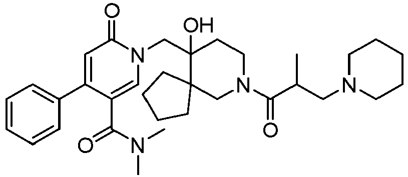

### Example 159: 6-(3-(Aminomethyl)phenyl)-3-((1-((R)-3-cyclohexyl-2-methylpropanoyl)-4-hydroxy-3,3-dimethylpiperidin-4-yl)methyl)pyrimidin-4(3H)-one

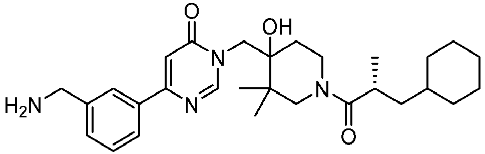

### Example 160: 1-(((S)-1-((S)-3-Cyclohexyl-2-(methoxymethyl)propanoyl)-4-hydroxy-3,3-dimethylpiperidin-4-yl)methyl)-N,N-dimethyl-6-oxo-4-phenyl-1,6-dihydropyridine-3-carboxamide

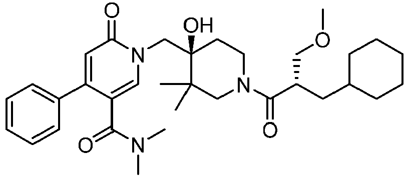

### Example 161: 4-(2-(Aminomethyl)phenyl)-1-((7-((R)-3-cyclohexyl-2-methylpropanoyl)-10-hydroxy-7-azaspiro[4.5]decan-10-yl)methyl)-N,N-dimethyl-6-oxo-1,6-dihydropyridine-3-carboxamide

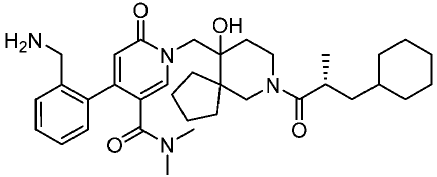

### Example 162: 1-((7-((R)-3-Cyclohexyl-2-methylpropanoyl)-10-hydroxy-7-azaspiro[4.5]decan-10-yl)methyl)-4-(4-(hydroxymethyl)phenyl)-N,N-dimethyl-6-oxo-1,6-dihydropyridine-3-carboxamide

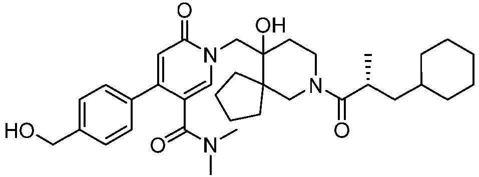

### Example 163: 1-((10-Hydroxy-7-(2-methyl-3-morpholinopropanoyl)-7-azaspiro[4.5]decan-10-yl)methyl)-N,N-dimethyl-6-oxo-4-phenyl-1,6-dihydropyridine-3-carboxamide

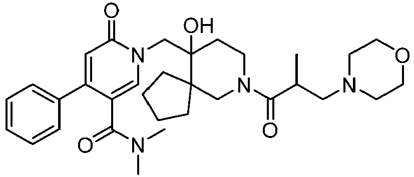

### Example 164 and Example 165: 1-(((S)-1-((S)-2-(Cyclohexylmethyl)-4-hydroxybutanoyl)-4-hydroxy-3,3-dimethylpiperidin-4-yl)methyl)-N,N-dimethyl-6-oxo-4-phenyl-1,6-dihydropyridine-3-carboxamide and 1-(((R)-1-((S)-2-(Cyclohexylmethyl)-4-hydroxybutanoyl)-4-hydroxy-3,3-dimethylpiperidin-4-yl)methyl)-N,N-dimethyl-6-oxo-4-phenyl-1,6-dihydropyridine-3-carboxamide

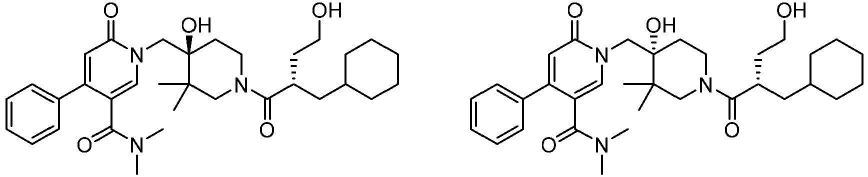

### Example 166 and Example 167: 1-(((S)-1-((S)-3-Cyclobutyl-2-(hydroxymethyl)propanoyl)-4-hydroxy-3,3-dimethylpiperidin-4-yl)methyl)-N,N-dimethyl-6-oxo-4-phenyl-1,6-dihydropyridine-3-carboxamide and 1-(((R)-1-((S)-3-Cyclobutyl-2-(hydroxymethyl)propanoyl)-4-hydroxy-3,3-dimethylpiperidin-4-yl)methyl)-N,N-dimethyl-6-oxo-4-phenyl-1,6-dihydropyridine-3-carboxamide

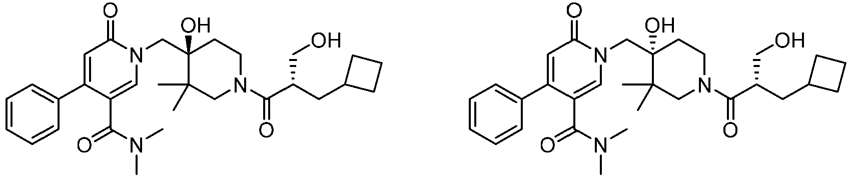

### Example 168: 1-((1-((R)-3-Cyclohexyl-2-methylpropanoyl)-4-hydroxy-3,3-dimethylpiperidin-4-yl)methyl)-5-(morpholine-4-carbonyl)-4-phenylpyridin-2(1/7)-one

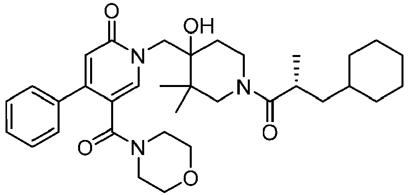

### Example 169: 5-(4-Acetylpiperazine-1-carbonyl)-1-((1-((R)-3-cyclohexyl-2-methylpropanoyl)-4-hydroxy-3,3-dimethylpiperidin-4-yl)methyl)-4-phenylpyridin-2(1H)-one

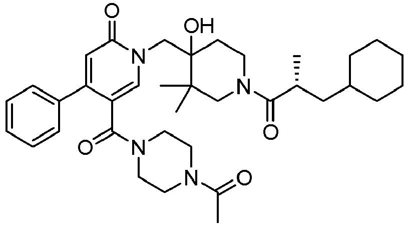

### Example 170: 1-((1-((R)-3-Cyclohexyl-2-methylpropanoyl)-4-hydroxy-3,3-dimethylpiperidin-4-yl)methyl)-5-(4-methylpiperazine-1-carbonyl)-4-phenylpyridin-2(1H)-one

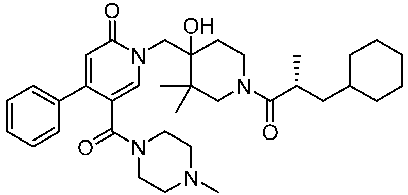

| Example | Structure | Name | LCMS (Method A): R_{T}, *m*/*z* |
|---|---|---|---|
| 171 | 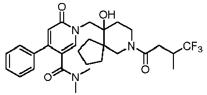 | 1-((10-Hydroxy-7-(4,4,4-trifluoro-3-methylbutanoyl)-7-azaspiro[4.5]decan-10-yl)methyl)-*N,N*-dimethyl-6-oxo-4-phenyl-1,6-dihydropyridine-3-carboxamide | 1.38 min, 548 [M+H]⁺ |
| 172 | 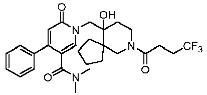 | 1-((10-Hydroxy-7-(4,4,4-trifluorobutanoyl)-7-azaspiro[4.5]decan-10-yl)methyl)-*N,N-*dimethyl-6-oxo-4-phenyl-1,6-dihydropyridine-3-carboxamide | 1.28 min, 534 [M+H]⁺ |
| 173 | 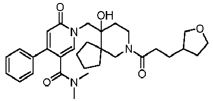 | 1-((10-Hydroxy-7-(3-(tetrahydrofuran-3-yl)propanoyl)-7-azaspiro [4.5]decan-10-yl)methyl)-*N,N*-dimethyl-6-oxo-4-phenyl-1,6-dihydropyridine-3-carboxamide | 1.05 min, 536 [M+H]⁺ |
| 174 | 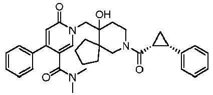 | 1-((10-Hydroxy-7-((1*R*,2*S*)-2-phenylcyclopropane-1-carbonyl)-7-azaspiro[4.5]decan-10-yl)methyl)-*N,N-*dimethyl-6-oxo-4-phenyl-1,6-dihydropyridine-3-carboxamide | 1.32 min, 554 [M+H]⁺ |
| 175 | 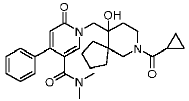 | 1-((7-(Cyclopropanecarbonyl)-10-hydroxy-7-azaspiro[4.5]decan-10-yl)methyl)-*N,N-*dimethyl-6-oxo-4-phenyl-1,6-dihydropyridine-3-carboxamide | 1.09 min, 478 [M+H]⁺ |
| 176 | 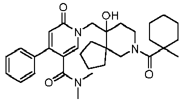 | 1-((10-Hydroxy-7-(1-methylcyclohexane-1-carbonyl)-7-azaspiro[4.5]decan-10-yl)methyl)-*N,N*-dimethyl-6-oxo-4-phenyl-1,6-dihydropyridine-3-carboxamide | 1.50 min, 534 [M+H]⁺ |
| 177 | 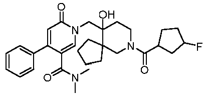 | 1-((7-(3-Fluorocyclopentane-1-carbonyl)-10-hydroxy-7-azaspiro[4.5]decan-10-yl)methyl)-*N,N*-dimethyl-6-oxo-4-phenyl-1,6-dihydropyridine-3-carboxamide | 1.18 min, 524 [M+H]⁺ |
| 178 | 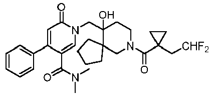 | 1-((7-(1-(2,2-Difluoroethyl)cyclopropane-1-carbonyl)-10-hydroxy-7-azaspiro[4.5]decan-10-yl)methyl)-*N*,*N*-dimethyl-6-oxo-4-phenyl-1,6-dihydropyridine-3-carboxamide | 1.18 min, 542 [M+H]⁺ |
| 179 | 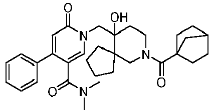 | 1-((7-(Bicyclo[2.2.1]heptane-1-carbonyl)-10-hydroxy-7-azaspiro [4.5]decan-10-yl)methyl)-*N,N*-dimethyl-6-oxo-4-phenyl-1,6-dihydropyridine-3-carboxamide | 1.38 min, 532 [M+H]⁺ |
| 180 | 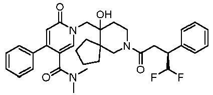 | 1-((7-((S)-4,4-Difluoro-3-phenylbutanoyl)-10-hydroxy-7-azaspiro [4.5]decan-10-yl)methyl)-*N,N*-dimethyl-6-oxo-4-phenyl-1,6-dihydropyridine-3-carboxamide | 1.41 min, 592 [M+H]⁺ |
| 181 | 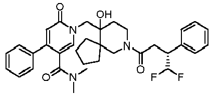 | 1-((7-((R)-4,4-Difluoro-3-phenylbutanoyl)-10-hydroxy-7-azaspiro [4.5]decan-10-yl)methyl)-*N,N*-dimethyl-6-oxo-4-phenyl-1,6-dihydropyridine-3-carboxamide | 1.41 min, 592 [M+H]⁺ |
| 182 | 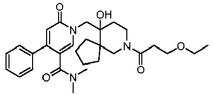 | 1-((7-(3-Ethoxypropanoyl)-10-hydroxy-7-azaspiro[4.5]decan-10-yl)methyl)-*N,N-*dimethyl-6-oxo-4-phenyl-1,6-dihydropyridine-3-carboxamide | 1.08 min, 510 [M+H]⁺ |
| 183 | 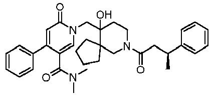 | 1-((10-Hydroxy-7-((*S*)-3-phenylbutanoyl)-7-azaspiro[4.5]decan-10-yl)methyl)-*N,N-*dimethyl-6-oxo-4-phenyl-1,6-dihydropyridine-3-carboxamide | 1.41 min, 556 [M+H]⁺ |
| 184 | 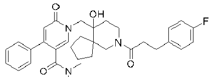 | 1-((7-(3-(4-Fluorophenyl)propanoyl)-10-hydroxy-7-azaspiro[4.5]decan-10-yl)methyl)-*N,N*-dimethyl-6-oxo-4-phenyl-1,6-dihydropyridine-3-carboxamide | 1.38 min, 560 [M+H]⁺ |
| 185 | 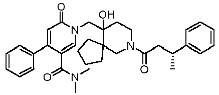 | 1-((10-Hydroxy-7-((*R*)-3-phenylbutanoyl)-7-azaspiro[4.5]decan-10-yl)methyl)-*N,N-*dimethyl-6-oxo-4-phenyl-1,6-dihydropyridine-3-carboxamide | 1.41 min, 556 [M+H]⁺ |
| 186 | 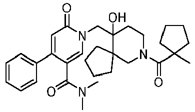 | 1-((10-Hydroxy-7-(1-methylcyclopentane-1-carbonyl)-7-azaspiro[4.5]decan-10-yl)methyl)-*N,N*-dimethyl-6-oxo-4-phenyl-1,6-dihydropyridine-3-carboxamide | 1.37 min, 520 [M+H]⁺ |
| 187 | 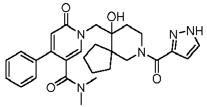 | 1-((10-Hydroxy-7-(1/7-pyrazole-3-carbonyl)-7-azaspiro[4.5]decan-10-yl)methyl)-*N,N-*dimethyl-6-oxo-4-phenyl-1,6-dihydropyridine-3-carboxamide | 0.864 min, 504 [M+H]⁺ |
| 188 | 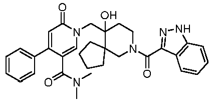 | 1-((10-Hydroxy-7-(1*H*-indazole-3-carbonyl)-7-azaspiro[4.5]decan-10-yl)methyl)-*N,N-*dimethyl-6-oxo-4-phenyl-1,6-dihydropyridine-3-carboxamide | 1.09 min, 554 [M+H]⁺ |
| 189 | 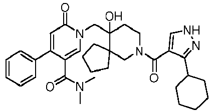 | 1-((7-(3-Cyclohexyl-1*H*-pyrazole-4-carbonyl)-10-hydroxy-7-azaspiro [4.5]decan-10-yl)methyl)-*N,N*-dimethyl-6-oxo-4-phenyl-1,6-dihydropyridine-3-carboxamide | 1.10 min, 586 [M+H]⁺ |
| 190 | 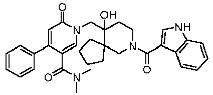 | 1-((10-Hydroxy-7-(1*H*-indole-3-carbonyl)-7-azaspiro[4.5]decan-10-yl)methyl)-*N,N-*dimethyl-6-oxo-4-phenyl-1,6-dihydropyridine-3-carboxamide | 1.10 min, 553 [M+H]⁺ |
| 191 | 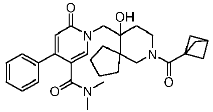 | 1-((7-(Bicyclo[1.1.1]pentane-1-carbonyl)-10-hydroxy-7-azaspiro [4.5]decan-10-yl)methyl)-*N,N*-dimethyl-6-oxo-4-phenyl-1,6-dihydropyridine-3-carboxamide | 1.14 min, 504 [M+H]⁺ |
| 192 | 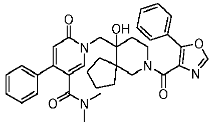 | 1-((10-Hydroxy-7-(5-phenyloxazole-4-carbonyl)-7-azaspiro[4.5]decan-10-yl)methyl)-*N,N*-dimethyl-6-oxo-4-phenyl-1,6-dihydropyridine-3-carboxamide | 1.23 min, 581 [M+H]⁺ |
| 193 | 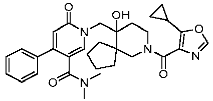 | 1-((7-(5-Cyclopropyloxazole-4-carbonyl)-10-hydroxy-7-azaspiro [4.5]decan-10-yl)methyl)-*N,N*-dimethyl-6-oxo-4-phenyl-1,6-dihydropyridine-3-carboxamide | 1.11 min, 545 [M+H]⁺ |
| 194 | 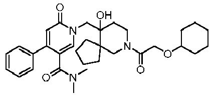 | 1-((7-(2-(Cyclohexyloxy)acetyl)-10-hydroxy-7-azaspiro[4.5]decan-10-yl)methyl)-*N*,*N-*dimethyl-6-oxo-4-phenyl-1,6-dihydropyridine-3-carboxamide | 1.30 min, 550 [M+H]⁺ |
| 195 | 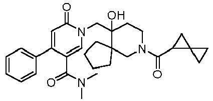 | 1-((10-Hydroxy-7-(spiro[2.2]pentane-1-carbonyl)-7-azaspiro[4.5]decan-10-yl)methyl)-*N,N*-dimethyl-6-oxo-4-phenyl-1,6-dihydropyridine-3-carboxamide | 1.14 min, 504 [M+H]⁺ |
| 196 | 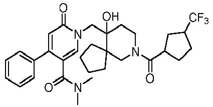 | 1-((10-Hydroxy-7-(3-(trifluoromethyl)cyclopentane-1-carbonyl)-7-azaspiro[4.5]decan-10-yl)methyl)-*N,N-*dimethyl-6-oxo-4-phenyl-1,6-dihydropyridine-3-carboxamide | 1.33 min, 574 [M+H]⁺ |
| 197 | 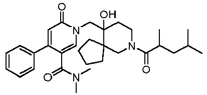 | 1-((7-(2,4-Dimethylpentanoyl)-10-hydroxy-7-azaspiro[4.5]decan-10-yl)methyl)-*N,N-*dimethyl-6-oxo-4-phenyl-1,6-dihydropyridine-3-carboxamide | 1.37 min, 522 [M+H]⁺ |
| 198 | 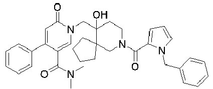 | 1-((7-(1-Benzyl-1*H*-pyrrole-2-carbonyl)-10-hydroxy-7-azaspiro[4.5]decan-10-yl)methyl)-*N,N*-dimethyl-6-oxo-4-phenyl-1,6-dihydropyridine-3-carboxamide | 1.41 min, 593 [M+H]⁺ |
| 199 | 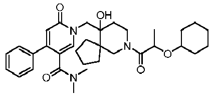 | 1-((7-(2-(Cyclohexyloxy)propanoyl)-10-hydroxy-7-azaspiro[4.5]decan-10-yl)methyl)-*N,N*-dimethyl-6-oxo-4-phenyl-1,6-dihydropyridine-3-carboxamide | 1.32 min, 564 [M+H]⁺ |

### Example 200: 3-((1-((S)-3-Cyclobutyl-2-(hydroxymethyl)propanoyl)-4-hydroxy-3,3-dimethylpiperidin-4-yl)methyl)-6-(2-fluorophenyl)pyrimidin-4(3H)-one

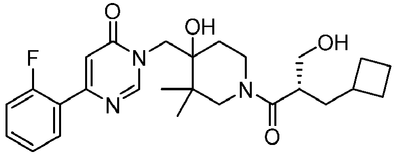

### Example 201: 1-((7-(2-Cyclobutoxyacetyl)-10-hydroxy-7-azaspiro[4.5]decan-10-yl)methyl)-N,N-dimethyl-6-oxo-4-phenyl-1,6-dihydropyridine-3-carboxamide

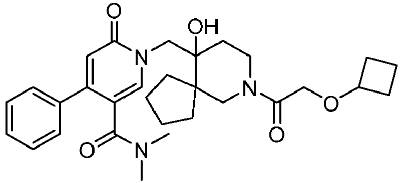

### Example 202: 1-((7-(3,3-Difluorocyclopentane-1-carbonyl)-10-hydroxy-7-azaspiro[4.5]decan-10-yl)methyl)-N,N-dimethyl-6-oxo-4-phenyl-1,6-dihydropyridine-3-carboxamide

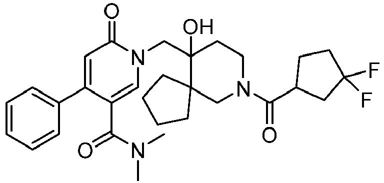

### Example 203: 1-((10-Hydroxy-7-(2-hydroxy-3-phenylpropanoyl)-7-azaspiro[4.5]decan-10-yl)methyl)-N,N-dimethyl-6-oxo-4-phenyl-1,6-dihydropyridine-3-carboxamide

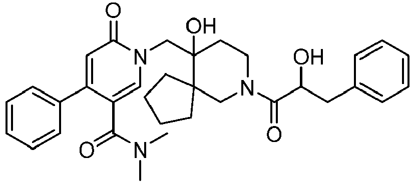

### Example 204: 1-(((S)-4-Hydroxy-3,3-dimethyl-1-((R)-4,4,4-trifluoro-2-methylbutanoyl)piperidin-4-yl)methyl)-N,N-dimethyl-6-oxo-4-phenyl-1,6-dihydropyridine-3-carboxamide

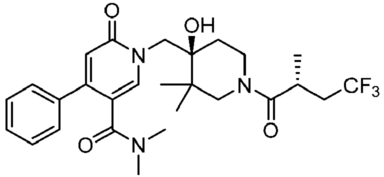

### Example 205: 1-(((S)-1-((R)-3-Cyclohexyl-2-methylpropanoyl)-4-hydroxy-3,3-dimethylpiperidin-4-yl)methyl)-4-(2-fluorophenyl)-N,N-dimethyl-6-oxo-1,6-dihydropyridine-3-carboxamide

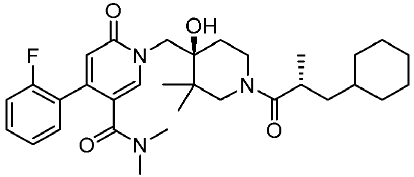

### Example 206: 1-(((S)-1-((R)-3-Cyclohexyl-2-methylpropanoyl)-4-hydroxy-3,3-dimethylpiperidin-4-yl)methyl)-4-(2-fluorophenyl)-5-(piperazine-1-carbonyl)pyridin-2(1H)-one

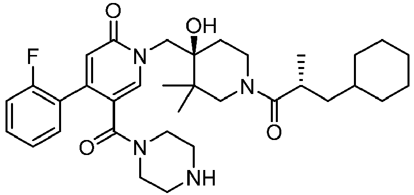

### Example 207: (S)-1-((4-Hydroxy-3,3-dimethyl-1-(4,4,4-trifluoro-2-(2,2,2-trifluoroethyl)butanoyl)piperidin-4-yl)methyl)-N,N-dimethyl-6-oxo-4-phenyl-1,6-dihydropyridine-3-carboxamide

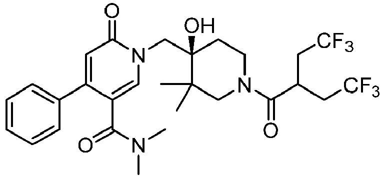

### Example 208: 1-(((S)-1-((R)-3-Cyclohexyl-2-methylpropanoyl)-4-hydroxy-3,3-dimethylpiperidin-4-yl)methyl)-5-((S)-2-(hydroxymethyl)piperazine-1-carbonyl)-4-phenylpyridin-2(1H)-one

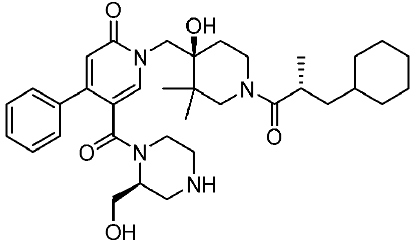

### Example 209: 1-(((R)-7-((R)-3-Cyclohexyl-2-methylpropanoyl)-10-hydroxy-7-azaspiro[4.5]decan-10-yl)methyl)-N,N-dimethyl-6-oxo-4-phenyl-1,6-dihydropyridine-3-carboxamide

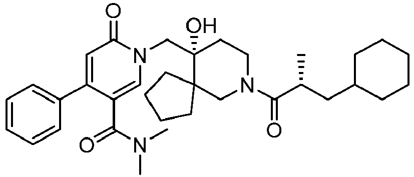

### Example 210: 1-(((R)-7-((R)-3-Cyclohexyl-2-methylpropanoyl)-10-hydroxy-7-azaspiro[4.5]decan-10-yl)methyl)-4-phenyl-5-(piperazine-1-carbonyl)pyridin-2(1H)-one

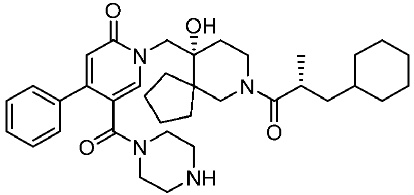

### Example 211: 1-(((S)-7-((R)-3-Cyclohexyl-2-methylpropanoyl)-10-hydroxy-7-azaspiro[4.5]decan-10-yl)methyl)-N,N-dimethyl-6-oxo-4-phenyl-1,6-dihydropyridine-3-carboxamide

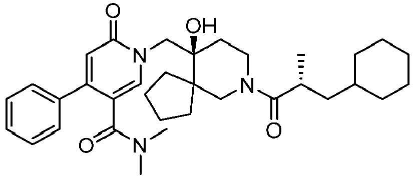

### Example 212: 1-(((S)-7-((R)-3-Cyclohexyl-2-methylpropanoyl)-10-hydroxy-7-azaspiro[4.5]decan-10-yl)methyl)-4-phenyl-5-(piperazine-1-carbonyl)pyridin-2(1H)-one

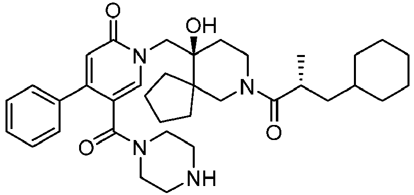

### Example 213: 1-(((S)-1-((R)-3-Cyclohexyl-2-methylpropanoyl)-4-hydroxy-3,3-dimethylpiperidin-4-yl)methyl)-5-((R)-3-methylpiperazine-1-carbonyl)-4-phenylpyridin-2(1H)-one

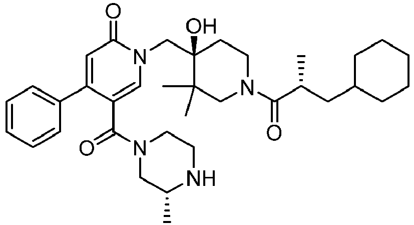

### Example 214: 1-(((S)-7-((R)-3-Cyclobutyl-2-methylpropanoyl)-10-hydroxy-7-azaspiro[4.5]decan-10-yl)methyl)-N,N-dimethyl-6-oxo-4-phenyl-1,6-dihydropyridine-3-carboxamide

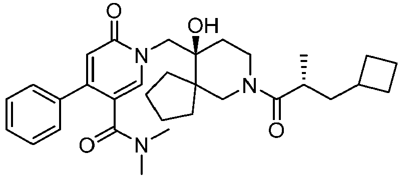

### Example 215: 1-(((S)-7-((R)-3-Cyclobutyl-2-methylpropanoyl)-10-hydroxy-7-azaspiro[4.5]decan-10-yl)methyl)-4-phenyl-5-(piperazine-1-carbonyl)pyridin-2(1H)-one

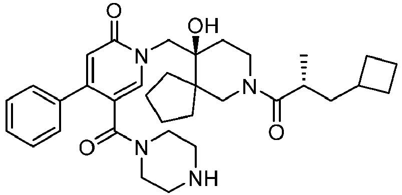

### Example 216: 1-(((S)-10-Hydroxy-7-((R)-4,4,4-trifluoro-2-methylbutanoyl)-7-azaspiro[4.5]decan-10-yl)methyl)-N,N-dimethyl-6-oxo-4-phenyl-1,6-dihydropyridine-3-carboxamide

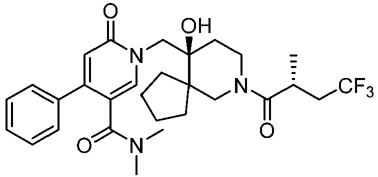

### Example 217: 1-(((S)-10-Hydroxy-7-((R)-4,4,4-trifluoro-2-methylbutanoyl)-7-azaspiro[4.5]decan-10-yl)methyl)-4-phenyl-5-(piperazine-1-carbonyl)pyridin-2(1H)-one

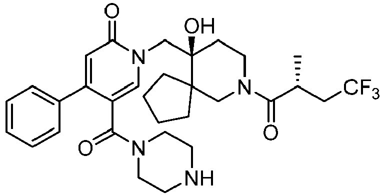

### Example 218: (S)-1-((10-Hydroxy-7-(4,4,4-trifluoro-2-(2,2,2-trifluoroethyl)butanoyl)-7-azaspiro[4.5]decan-10-yl)methyl)-N,N-dimethyl-6-oxo-4-phenyl-1,6-dihydropyridine-3-carboxamide

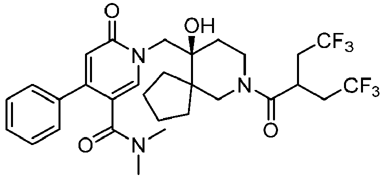

### Example 219: (S)-1-((10-Hydroxy-7-(4,4,4-trifluoro-2-(2,2,2-trifluoroethyl)butanoyl)-7-azaspiro[4.5]decan-10-yl)methyl)-4-phenyl-5-(piperazine-1-carbonyl)pyridin-2(1H)-one

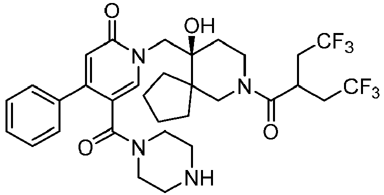

### Example 220: 1-(((S)-1-((R)-3-Cyclohexyl-2-methylpropanoyl)-4-hydroxy-3,3-dimethylpiperidin-4-yl)methyl)-4-cyclopropyl-N,N-dimethyl-6-oxo-1,6-dihydropyridine-3-carboxamide

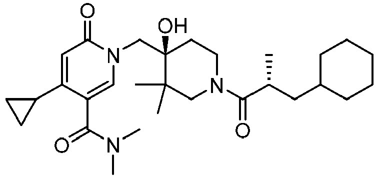

### Example 221: 1-(((S)-1-((R)-3-Cvclohexyl-2-methylpropanoyl)-4-hydroxy-3,3-dimethylpiperidin-4-yl)methyl)-4-cyclopropyl-5-(piperazine-1-carbonyl)pyridin-2(1H)-one

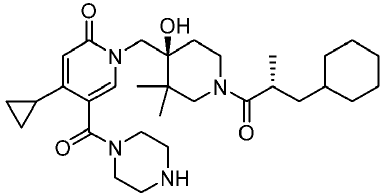

### Example 222: 1-(((S)-7-((R)-3-Cyclobutyl-2-methylpropanoyl)-10-hydroxy-7-azaspiro[4.5]decan-10-yl)methyl)-4-(2-fluorophenyl)-N,N-dimethyl-6-oxo-1,6-dihydropyridine-3-carboxamide

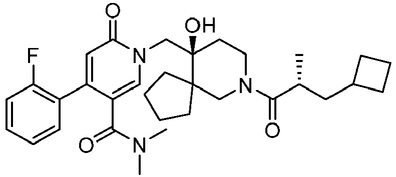

### Example 223: 4-(2-Fluorophenyl)-1-(((S)-10-hydroxy-7-((R)-4,4,4-trifluoro-2-methylbutanoyl)-7-azaspiro[4.5]decan-10-yl)methyl)-N,N-dimethyl-6-oxo-1,6-dihydropyridine-3-carboxamide

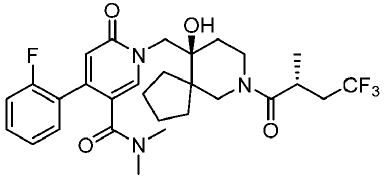

### Example 224: 1-(((S)-7-((R)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1H)-one

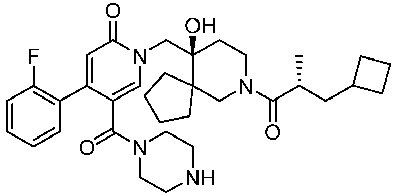

### Example 225: 4-(2-Fluorophenyl)-1-(((S)-10-hydroxy-7-((R)-4,4,4-trifluoro-2-methylbutanoyl)-7-azaspirof4.51decan-10-yl)methyl)-5-(piperazine-1-carbonyl)pyridin-2(1H)-one

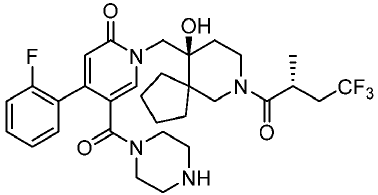

### Example 226: 1-(((S)-7-((R)-3-Cyclohexyl-2-methylpropanoyl)-10-hydroxy-7-azaspiro[4.5]decan-10-yl)methyl)-4-(2-fluorophenyl)-N,N-dimethyl-6-oxo-1,6-dihydropyridine-3-carboxamide

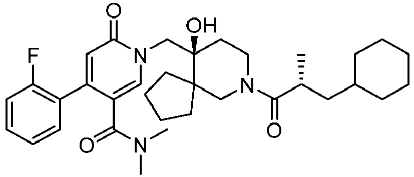

### Example 227: 1-(((S)-7-((R)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1H)-one

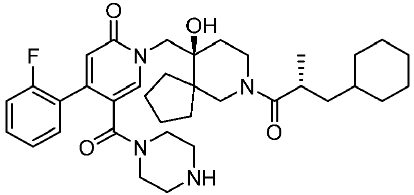

### Example 228: 1-(((S)-1-((R)-3-Cvclohexyl-2-methylpropanoyl)-4-hydroxy-3,3-dimethylpiperidin-4-yl)methyl)-5-(4-hydroxypiperidine-1-carbonyl)-4-phenylpyridin-2(1H)-one

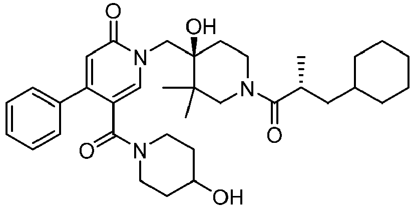

### Example 229: 1-((10-Hydroxy-7-(3-(trifluoromethyl)cyclobutane-1-carbonyl)-7-azaspiro[4.5]decan-10-yl)methyl)-N,N-dimethyl-6-oxo-4-phenyl-1,6-dihydropyridine-3-carboxamide

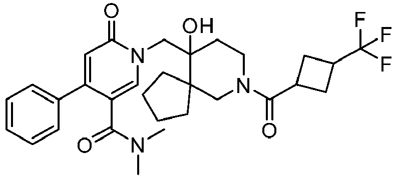

### Example 230: 1-(((S)-7-((R)-3-Cyclobutyl-2-methylpropanoyl)-10-hydroxy-7-azaspiro[4.5]decan-10-yl)methyl)-4-cyclopropyl-N,N-dimethyl-6-oxo-1,6-dihydropyridine-3-carboxamide

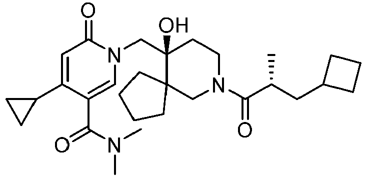

### Example 231: 1-(((S)-7-((R)-3-Cyclobutyl-2-methyl-propanoyl)-10-hydroxy-7-azaspiro[4.5]decan-10-yl)methyl)-4-cyclopropyl-5-(piperazine-1-carbonyl)pyridin-2(1H)-one

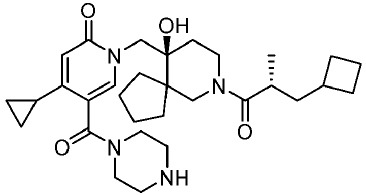

### Example 232: 1-(((S)-7-((R)-3-Cyclohexyl-2-methylpropanoyl)-10-hydroxy-7-azaspiro[4.5]decan-10-yl)methyl)-4-cyclopropyl-N,N-dimethyl-6-oxo-1,6-dihydropyridine-3-carboxamide

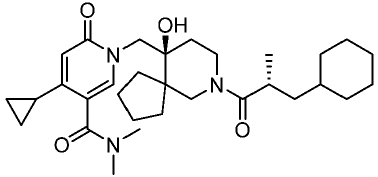

### Example 233: 1-(((S)-7-((R)-3-Cyclohexyl-2-methylpropanoyl)-10-hydroxy-7-azaspiro[4.5]decan-10-yl)methyl)-4-cyclopropyl-5-(piperazine-1-carbonyl)pyridin-2(1H)-one

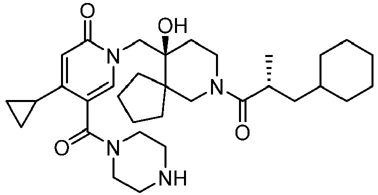

### Example 234: 1-((2-((R)-3-Cyclohexyl-2-methylpropanoyl)-5-hydroxy-9-oxa-2-azaspiro[5.5]undecan-5-yl)methyl)-N,N-dimethyl-6-oxo-4-phenyl-1,6-dihydropyridine-3-carboxamide

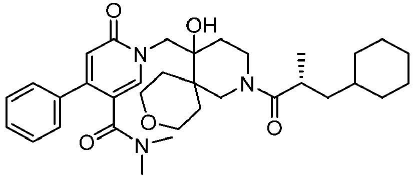

### Example 235: 1-(((S)-1-((R)-3-Cyclobutyl-2-methylpropanoyl)-4-hydroxy-3,3-dimethylpiperidin-4-yl)methyl)-4-(2-fluorophenyl)-N,N-dimethyl-6-oxo-1,6-dihydropyridine-3-carboxamide

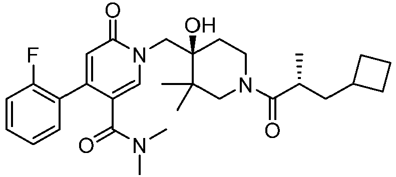

### Example 236: 1-(((S)-1-((R)-3-Cyclobutyl-2-methylpropanoyl)-4-hydroxy-3,3-dimethylpiperidin-4-yl)methyl)-4-(2-fluorophenyl)-5-(piperazine-1-carbonyl)pyridin-2(1H)-one

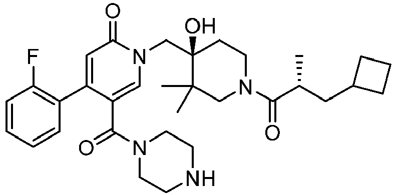

### Example 237: 1-((2-((R)-3-Cyclohexyl-2-methylpropanoyl)-5-hydroxy-2-azaspiro[5.5]undecan-5-yl)methyl)-N,N-dimethyl-6-oxo-4-phenyl-1,6-dihydropyridine-3-carboxamide

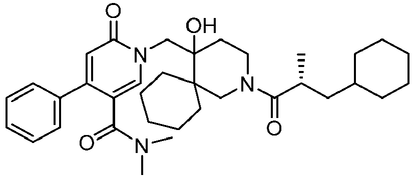

### Example 238: 1-((2-((R)-3-Cyclohexyl-2-methylpropanoyl)-5-hydroxy-2-azaspiro[5.5]undecan-5-yl)methyl)-4-phenyl-5-(piperazine-1-carbonyl)pyridin-2(1H)-one

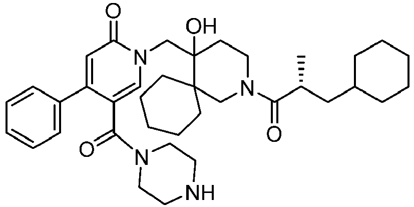

### Example 239: 1-(((S)-7-((R)-3-Cyclohexyl-2-methylpropanoyl)-10-hydroxy-7-azaspiro[4.5]decan-10-yl)methyl)-5-(morpholine-4-carbonyl)-4-phenylpyridin-2(1H)-one

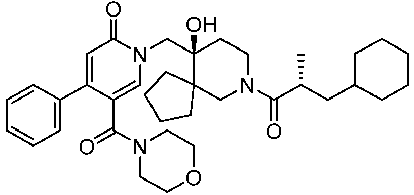

### Example 240: 1-(((S)-7-((R)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1H)-one

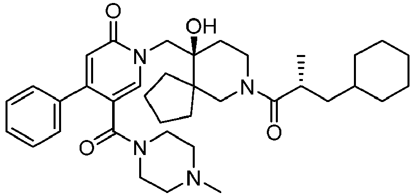

### Example 241: 1-((1-((R)-3-Cyclohexyl-2-methylpropanoyl)-4-hydroxy-3,3-dimethylpiperidin-4-yl)methyl)-6-oxo-4-phenyl-1,6-dihydropyridine-3-carboxylic acid

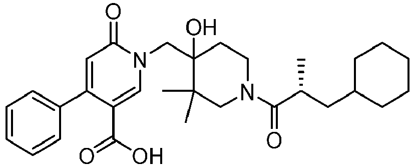

### Example 242: 1-((7-((R)-3-Cyclohexyl-2-methylpropanoyl)-10-hydroxy-7-azaspiro[4.5]decan-10-yl)methyl)-4-methoxy-N,N-dimethyl-6-oxo-1,6-dihydropyridine-3-carboxamide

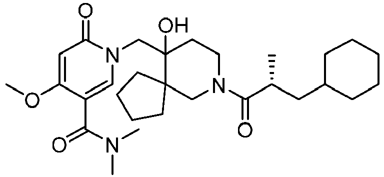

### Example 243: 1-((7-(4,4-Difluoro-2-methylbutanoyl)-10-hydroxy-7-azaspiro[4.5]decan-10-yl)methyl)-N,N-dimethyl-6-oxo-4-phenyl-1,6-dihydropyridine-3-carboxamide

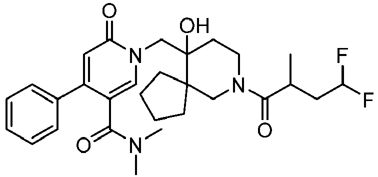

### Example 244: 1-((7-(4,4-Difluoro-2,2-dimethylbutanoyl)-10-hydroxy-7-azaspiro[4.5]decan-10-yl)methyl)-N,N-dimethyl-6-oxo-4-phenyl-1,6-dihydropyridine-3-carboxamide

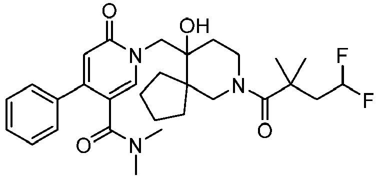

### Example 245: 1-((10-Hydroxy-7-(4-(trifluoromethyl)thiazole-2-carbonyl)-7-azaspiro[4.5]decan-10-yl)methyl)-N,N-dimethyl-6-oxo-4-phenyl-1,6-dihydropyridine-3-carboxamide

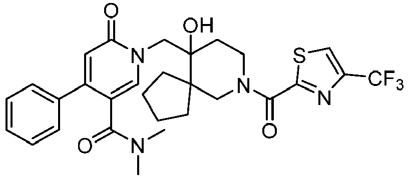

### Example 246: 1-((10-Hydroxy-7-(2-(trifluoromethyl)thiazole-4-carbonyl)-7-azaspiro[4.5]decan-10-yl)methyl)-N,N-dimethyl-6-oxo-4-phenyl-1,6-dihydropyridine-3-carboxamide

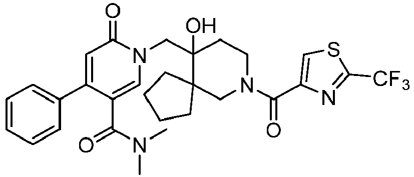

### Example 247: 1-(((S)-10-Hydroxy-7-((S)-4,4,4-trifluoro-2-(hydroxymethyl)butanoyl)-7-azaspiro[4.5]decan-10-yl)methyl)-N,N-dimethyl-6-oxo-4-phenyl-1,6-dihydropyridine-3-carboxamide

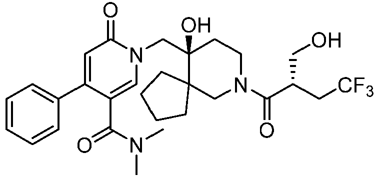

### Example 248: 1-(((S)-10-Hydroxy-7-((S)-4,4,4-trifluoro-2-(hydroxymethyl)butanoyl)-7-azaspiro[4.5]decan-10-yl)methyl)-5-(morpholine-4-carbonyl)-4-phenylpyridin-2(1H)-one

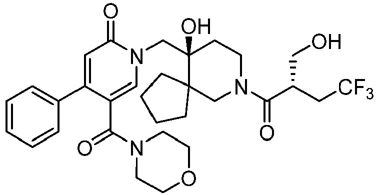

### Example 249: 1-(((S)-10-Hydroxy-7-((S)-4,4,4-trifluoro-2-(hydroxymethyl)butanoyl)-7-azaspiro[4.5]decan-10-yl)methyl)-4-phenyl-5-(piperazine-1-carbonyl)pyridin-2(1H)-one

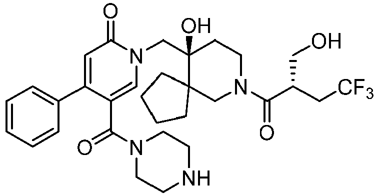

### Example 250: 1-(((S)-10-Hydroxy-7-((R)-4,4,4-trifluoro-2-methylbutanoyl)-7-azaspiro[4.5]decan-10-yl)methyl)-5-(morpholine-4-carbonyl)-4-phenylpyridin-2(1H)-one

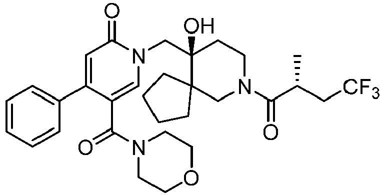

### Example 251: 1-(((S)-10-Hydroxy-7-((R)-4,4,4-trifluoro-2-methylbutanoyl)-7-azaspiro[4.5]decan-10-yl)methyl)-5-(4-methylpiperazine-1-carbonyl)-4-phenylpyridin-2(1H)one

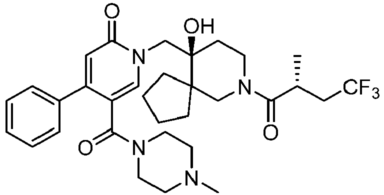

### Example 252: Ethyl 1-(((S)-7-((R)-3-cyclohexyl-2-methylpropanoyl)-10-hydroxy-7-azaspiro[4.5]decan-10-yl)methyl)-6-oxo-4-phenyl-1,6-dihydropyridine-3-carboxylate

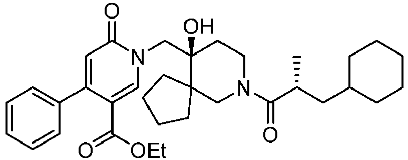

### Example 253: 1-(((S)-7-((R)-3-Cyclohexyl-2-methylpropanoyl)-10-hydroxy-7-azaspiro[4.5]decan-10-yl)methyl)-6-oxo-4-phenyl-1,6-dihydropyridine-3-carboxylic acid

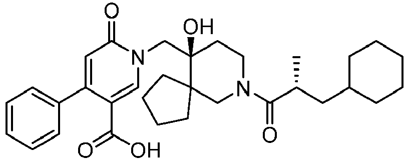

The following USP19 inhibitory compounds were prepared similarly to the above compounds first described in WO2018/020242 and in addition, in some cases using intermediates that were first described in WO2019/150119. Example 254 was prepared using the intermediate described in WO2019/150119 Example 193, Step 3' and (3*R*,4*R*)-1-(*tert*-butoxycarbonyl)-3-phenylpiperidine-4-carboxylic acid that is described in WO2009/072643.

Examples 255, 258, 259 and 260 were prepared using the intermediate described in WO2019/150119 Example 210, Step 3'. Example 256 may be prepared using the intermediate (*S*)-6-(2-fluorophenyl)-3-((10-hydroxy-7-azaspiro[4.5]decan-10-yl)methyl)pyrimidin-4(3*H*)-one that can be prepared similarly to the intermediate described in WO2019/150119 Example 210, Step 3', except using (2-fluorophenyl)boronic acid in Step 2. Example 257 may be prepared using the intermediate (*S*)-6-(3-fluorophenyl)-3-((10-hydroxy-7-azaspiro[4.5]decan-10-yl)methyl)pyrimidin-4(3*H*)-one that can be prepared similarly to the intermediate described in WO2019/150119 Example 210, Step 3', except using (3-fluorophenyl)boronic acid in Step 2. In addition, Examples 255, 256 and 257 used WO2018/020242 'Acid 11' and Examples 258 used commercially available (*R*)-4,4-difluoro-2-methylpentanoic acid. In the cases of Example 259 and 260, commercially available *rel-*(3*R*,4*S*)-1-(*tert*-butoxycarbonyl)-4-phenylpyrrolidine-3-carboxylic acid and *rel-*(2*R*,3*R*)-1-(*tert*-butoxycarbonyl)-2-phenylpyrrolidine-3-carboxylic acid were used, respectively, followed by Boc protecting group removal after HATU coupling (e.g. as described in WO2018/020242 General Procedures 3 and 7). In all cases, the compounds were purified by flash chromatography and freeze-dried.

### Example 254: 3-((10-Hydroxy-7-((3R,4R)-3-phenylpiperidine-4-carbonyl)-7-azaspiro[4.5]decan-10-yl)methyl)-6-phenylpyrimidin-4(3H)-one

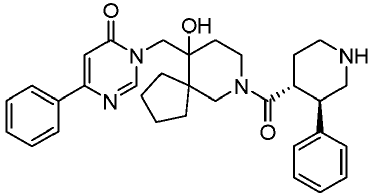

LCMS (Method B): R_{T} = 0.87 min, *m*/*z* = 527 [M+H]⁺. ¹H NMR (500 MHz, DMSO-*d*₆): δ 8.45 - 8.38 (m, 1H), 8.14 - 7.99 (m, 2H), 7.61 - 7.40 (m, 3H), 7.35 - 7.05 (m, 5H), 6.99 - 6.92 (m, 1H), 4.83 - 4.68 (m, 1H), 4.62 - 4.55 (m, 1H), 4.39 - 4.33 (m, 1H), 3.76 - 3.60 (m, 1H), 3.60 - 3.46 (m, 1H), 3.42 - 3.16 (m, 2H (signals overlap with HDO)), 3.14 - 3.04 (m, 1H), 2.99 - 2.84 (m, 3H), 2.70 - 2.53 (m, 2H), 2.20 (br s, 1H), 1.83 - 1.18 (m, 9H), 1.11 - 0.86 (m, 2H), 0.66 - 0.47 (m, 1H).

### Example 255: 3-(((S)-10-Hydroxy-7-((R)-4,4,4-trifluoro-2-methylbutanoyl)-7-azaspiro[4.5]decan-10-yl)methyl)-6-phenylpyrimidin-4(3H)-one

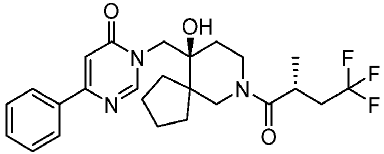

LCMS (Method A): R_{T} = 1.43 min, *m*/*z* = 478 [M+H]⁺. ¹H NMR (500 MHz, DMSO-*d*₆): δ 8.47 (s, 1H), 8.18 - 7.99 (m, 2H), 7.63 - 7.35 (m, 3H), 6.99 (s, 0.4H), 6.98 (s, 0.6H), 4.92 (s, 0.4H), 4.90 (s, 0.6H), 4.60 (d, *J* = 13.6 Hz, 0.6H), 4.51 (d, *J* = 13.5 Hz, 0.4H), 4.00 - 3.92 (m, 0.4H), 3.76 - 3.67 (m, 1H), 3.64 (d, *J* = 13.6 Hz, 0.6H), 3.53 - 3.21 (m, 1.4H (signal overlaps with HDO)), 3.18 - 2.98 (m, 1.6H), 2.83 - 2.65 (m, 1H), 2.31 - 2.20 (m, 1H), 2.06 - 1.99 (m, 0.4H), 1.95 - 1.87 (m, 0.6H), 1.80 - 1.50 (m, 5H), 1.50 - 1.12 (m, 5H), 1.09 (d, *J* = 6.9 Hz, 1.8H), 1.07 (d, *J* = 6.8 Hz, 1.2H).

### Example 256: 6-(2-Fluorophenyl)-3-(((S)-10-hydroxy-7-((R)-4,4,4-trifluoro-2-methylbutanoyl)-7-azaspiro[4.5]decan-10-yl)methyl)pyrimidin-4(3H)-one

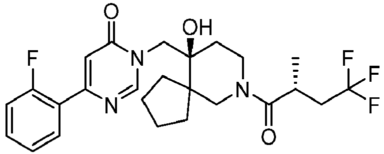

LCMS (Method A): R_{T} = 1.45 min, *m*/*z* = 496 [M+H]⁺. ¹H NMR (500 MHz, DMSO-*d*₆): δ 8.49 (s, 1H), 8.07 - 7.99 (m, 1H), 7.60 - 7.51 (m, 1H), 7.40 - 7.30 (m, 2H), 6.81 (s, 1H), 4.92 (s, 1H), 4.60 (d, *J* = 13.6 Hz, 0.6H), 4.50 (d, *J* = 13.7 Hz, 0.4H), 4.00 - 3.92 (m, 0.4H), 3.77 - 3.67 (m, 1H), 3.65 (d, *J* = 13.6 Hz, 0.6H), 3.53 - 3.22 (m, 1.4H (signal overlaps with HDO)), 3.19 - 3.00 (m, 1.6H), 2.83 - 2.65 (m, 1H), 2.32 - 2.19 (m, 1H), 2.05 - 1.98 (m, 0.4H), 1.94 - 1.87 (m, 0.6H), 1.79 - 1.20 (m, 9H), 1.20 - 1.12 (m, 1H), 1.10 (d, *J* = 6.8 Hz, 1.8H), 1.07 (d, *J* = 6.9 Hz, 1.2H).

### Example 257: 6-(3-Fluorophenyl)-3-(((S)-10-hydroxy-7-((R)-4,4,4-trifluoro-2-methylbutanoyl)-7-azaspiro[4.5]decan-10-yl)methyl)pyrimidin-4(3H)-one

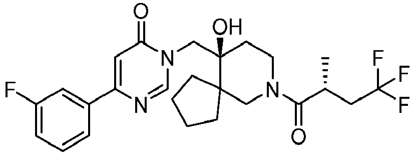

LCMS (Method A): R_{T} = 1.48 min, *m*/*z* = 496 [M+H]⁺. ¹H NMR (500 MHz, DMSO-*d*₆): δ 8.48 (s, 1H), 7.95 (d, *J* = 7.9 Hz, 1H), 7.92 - 7.86 (m, 1H), 7.60 - 7.50 (m, 1H), 7.39 - 7.29 (m, 1H), 7.08 (s, 0.4H), 7.06 (s, 0.6H), 4.92 (s, 1H), 4.60 (d, *J* = 13.5 Hz, 0.6H), 4.51 (d, *J* = 13.5 Hz, 0.4H), 3.99 - 3.93 (m, 0.4H), 3.76 - 3.67 (m, 1H), 3.64 (d, *J=* 13.6 Hz, 0.6H), 3.54 - 3.21 (m, 1.4H (signals overlap with HDO)), 3.20 - 2.99 (m, 1.6H), 2.83 - 2.65 (m, 1H), 2.32 - 2.19 (m, 1H), 2.05 - 2.00 (m, 0.4H), 1.94 - 1.87 (m, 0.6H), 1.76 - 1.20 (m, 9H), 1.19 - 1.12 (m, 1H), 1.09 (d, *J* = 6.9 Hz, 1.8H), 1.07 (d, *J* = 6.9 Hz, 1.2H).

### Example 258: 3-(((S)-7-((R)-4,4-Difluoro-2-methylpentanoyl)-10-hydroxy-7-azaspiro[4.5]decan-10-yl)methyl)-6-phenylpyrimidin-4(3H)-one

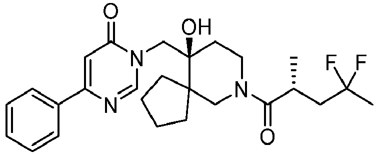

LCMS (Method A): R_{T} = 1.38 min, *m*/*z =* 474 [M+H]⁺. ¹H NMR (500 MHz, DMSO-*d*₆): δ 8.47 (s, 1H), 8.11 - 8.04 (m, 2H), 7.56 - 7.45 (m, 3H), 7.01 - 6.95 (m, 1H), 4.93 - 4.85 (m, 1H), 4.65 - 4.49 (m, 1H), 3.94 - 3.59 (m, 2H), 3.53 - 3.20 (m, 2H, overlapping HDO signal), 3.16 - 2.99 (m, 1H), 2.47 - 2.37 (m, 1H, overlapping solvent signal), 2.05 - 1.76 (m, 2H), 1.74 - 0.75 (m, 16H).

### Example 259: 3-(((S)-10-Hydroxy-7-((3S,4R)-4-phenylpyrrolidine-3-carbonyl)-7-azaspiro[4.5]decan-10-yl)methyl)-6-phenylpyrimidin-4(3H)-one and 3-(((S)-10-Hydroxy-7-((3R,4S)-4-phenylpyrrolidine-3-carbonyl)-7-azaspirof4.51decan-10-yl)methyl)-6-phenylpyrimidin-4(3H)-one

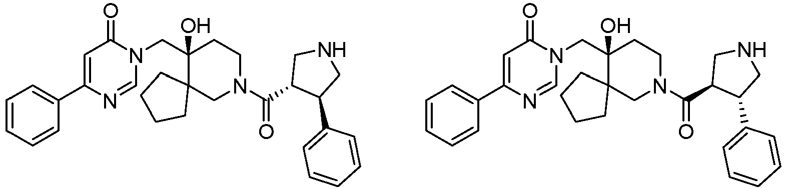

LCMS (Method B): R_{T} = 0.84 min, *m*/*z =* 513 [M+H]⁺. ¹H NMR (500 MHz, DMSO-*d*₆): δ 8.50 - 8.36 (m, 1H), 8.25 - 7.92 (m, 2H), 7.70 - 7.37 (m, 3H), 7.35 - 7.00 (m, 5H), 7.00 - 6.89 (m, 1H), 4.90 - 4.76 (m, 1H), 4.61 - 4.38 (m, 1H), 3.87 - 2.66 (m, 11H (signals overlap with HDO)), 1.94 - 1.79 (m, 1H), 1.75 - 1.38 (m, 4H), 1.36 - 1.20 (m, 2H), 1.18 - 0.99 (m, 2H), 0.91 - 0.70 (m, 1H). NH not visible.

### Example 260: 3-(((S)-10-Hydroxy-7-((2R,3R)-2-phenylpyrrolidine-3-carbonyl)-7-azaspiro[4.5]decan-10-yl)methyl)-6-phenylpyrimidin-4(3H)-one and 3-(((S)-10-Hydroxy-7-((2S,3S)-2-phenylpyrrolidine-3-carbonyl)-7-azaspiro[4.5]decan-10-yl)methyl)-6-phenylpyrimidin-4(3H)-one

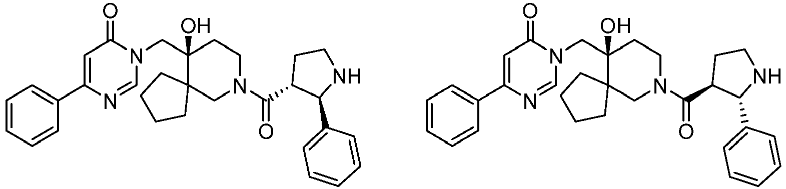

LCMS (Method B): R_{T} = 0.84 min, *m*/*z =* 513 [M+H]⁺. ¹H NMR (500 MHz, DMSO-*d*₆): δ 8.45 - 8.39 (m, 1H), 8.12 - 8.00 (m, 2H), 7.58 - 7.42 (m, 3H), 7.38 - 7.12 (m, 5H), 7.00 - 6.92 (m, 1H), 4.88 - 4.74 (m, 1H), 4.59 - 4.16 (m, 3H), 3.88 - 3.66 (m, 1H), 3.62 - 3.53 (m, 1H), 3.52 - 2.89 (m, 5H (signals overlap with HDO)), 2.69 (br s, 1H), 2.19 - 2.04 (m, 1H), 1.93 - 1.74 (m, 2H), 1.68 - 1.58 (m, 2H), 1.55 - 1.46 (m, 1H), 1.43 - 1.31 (m, 1H), 1.30 - 1.17 (m, 2H), 1.15 - 0.97 (m, 2H), 0.89 - 0.66 (m, 1H).

## Claims

1. A compound, or a pharmaceutically acceptable salt, tautomer, stereoisomer or *N-*oxide derivative thereof, for use in a method of treating obesity, insulin resistance, or type II diabetes, wherein the compound is a compound according to formula (I): 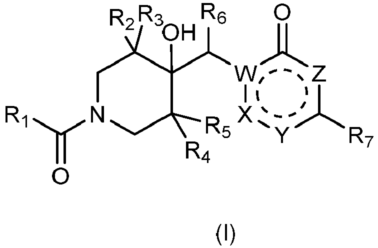 wherein:
R₁ is an optionally substituted alkyl, alkenyl, alkynyl, ether, cycloalkyl, alkylcycloalkyl, heterocycloalkyl, alkylheterocycloalkyl, aryl, arylalkyl, heteroaryl, or heteroarylalkyl group;
R₂ is H or an optionally substituted alkyl group,
R₃ is H or an optionally substituted alkyl group,
R₄ is H or an optionally substituted alkyl group,
R₅ is H or an optionally substituted alkyl group,
R₂ and R₄ may be joined to one another to form an optionally substituted cycloalkyl or heterocycloalkyl that includes the carbon to which they are attached,
R₄ and R₅ may be joined to one another to form an optionally substituted cycloalkyl or heterocycloalkyl that includes the carbon to which they are attached;
R₆ is H or an optionally substituted alkyl group;
W is C or N;
X is N or CR₈, wherein R₈ is H or optionally substituted C1-C6 alkyl,
Y is N or CR₉
Z is CH, or NH,
wherein R₉ is H or optionally substituted alkyl, amido, amino, aryl, heteroaryl, cycloalkyl, cycloheteroalkyl, halo, carbonyl, ester, aminoalkyl, or cyano;
R₇ is hydrogen, halo, or an optionally substituted alkyl, alkenyl, alkynyl, amino, aryl, cycloalkyl, cycloalkenyl, alkoxy, aryloxy, heteroaryl or heterocycloalkyl group;
or the compound 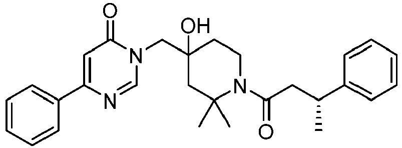
or a pharmaceutically acceptable salt, tautomer, stereoisomer or *N-*oxide derivative thereof.

2. A compound, pharmaceutically acceptable salt, tautomer, stereoisomer or *N-*oxide derivative for use according to claim 1, wherein R₁ is optionally substituted C1-C6 alkyl, optionally substituted C4-C10 alkylcycloalkyl, optionally substituted C4-C5 heterocycloalkyl, optionally substituted C7-C10 arylalkyl, optionally substituted C3-C6 cycloalkyl, or optionally substituted C3-C6 heteroaryl,
wherein each optional substituent is independently selected from C1-C6 alkyl, C2-C6 alkenyl, hydroxymethyl, methoxymethyl, benzyloxy methyl, phenyl, C3-C6 cycloalkyl, CF₃, CHF₂, OH, or halo.

3. A compound, pharmaceutically acceptable salt, tautomer, stereoisomer or *N-*oxide derivative for use according to claim 1 or claim 2, wherein R₁ is optionally substituted C7-C10 cyclohexylalkane, optionally substituted C4-C6 cyclopropylalkane, optionally substituted C5-C6 cyclobutylalkane, optionally substituted C7-C10 alkylbenzene, optionally substituted 2,2 difluorobutane, or optionally substituted 3,3,3-trifluoropropane,
wherein each optional substituent is independently selected from C1-C6 alkyl, C2-C6 alkenyl, hydroxymethyl, hydroxyethyl, methoxymethyl, OH, or halo, optionally wherein R₁ is substituted and the substituent is C1-C6 alkyl, optionally methyl.

4. A compound, pharmaceutically acceptable salt, tautomer, stereoisomer or *N-*oxide derivative for use according to any one of claims 1-3, wherein R₁ is methyl substituted cyclohexylethane, methyl substituted ethylbenzene, methyl substituted 2,2 difluorobutane, methyl substituted 3,3,3-trifluoropropane, C4-C5 heterocycloalkyl, or C4-C5 heterocycloalkyl substituted with phenyl, optionally phenyl-substituted pyrrolidine or phenyl-substituted piperidine.

5. A compound, pharmaceutically acceptable salt, tautomer, stereoisomer or *N-*oxide derivative for use according to any of claims 1-4, wherein R₇ is optionally substituted C6-C10 aryl, C1-C12 heteroaryl, C1-C10 alkyl, C2-C10 alkenyl, C3-C10 cycloalkyl, amino, C1-C3 alkoxy, or halo,
wherein the optional substituent is selected from C1-C6 alkyl, hydroxysubstituted C1-C6 alkyl, C3-C6 cycloalkyl, C1-C6 heterocycloalkyl, C1-C6 alkoxy, halo-substituted C1-C6 alkoxy, amido, cyano or halo, optionally wherein R₇ is cyclopropyl, thiophene, or optionally substituted phenyl,
wherein the optional substituent is selected from C1-C6 alkyl, hydroxysubstituted C1-C6 alkyl, C1-C6 alkylamine, and halo, optionally wherein R₇ is phenyl or fluoro-substituted phenyl.

6. A compound, pharmaceutically acceptable salt, tautomer, stereoisomer or *N-*oxide derivative for use according to any of claims 1-5, wherein R₂, R₃, R₄, R₅ are independently selected from H and C1-C6 alkyl, optionally wherein R₂ and R₃ are H, and R₄ and R₅ are methyl.

7. A compound, pharmaceutically acceptable salt, tautomer, stereoisomer or *N-*oxide derivative for use according to any of claims 1-5, wherein R₄ and R₅ are joined to one another to form an optionally substituted C3-C6 cycloalkyl or C3-C6 heterocycloalkyl that includes the carbon to which they are attached and R₂ and R₃ are independently selected from H and C1-C6 alkyl, optionally wherein R₂ and R₃ are H and R₄ and R₅ are joined to one another to form a C3-C6 cycloalkyl that includes the carbon to which they are attached, optionally wherein R₄ and R₅ are joined to one another to form a C4 or C5 cycloalkyl that includes the carbon to which they are attached.

8. A compound, pharmaceutically acceptable salt, tautomer, stereoisomer or *N-*oxide derivative for use according to any one of claims 1-7, wherein R₆ is H or C1-C6 alkyl.

9. A compound, pharmaceutically acceptable salt, tautomer, stereoisomer or *N-*oxide derivative for use according to any of claims 1-5, wherein R₂ and R₄ are joined to one another to form a C5 cycloalkyl that includes the carbons to which they are attached, and wherein R₃, R₅ and R₆ are independently H or C1-C6 alkyl.

10. A compound, pharmaceutically acceptable salt, tautomer, stereoisomer or *N-*oxide derivative for use according to any of claims 1-9, wherein W is N and X is CR₈, wherein R₈ is H or methyl.

11. A compound, pharmaceutically acceptable salt, tautomer, stereoisomer or *N-*oxide derivative for use according to any of claims 1-10, wherein Y is N or CR₉, wherein R₉ is H, C1-C6 alkyl, NR'R", C(O)NR'R", cyano, carboxyl, halo, C1-C6 alkylamine, C3-C6 alkylester, optionally substituted C6-C10 aryl or optionally substituted C2-C6 heteroaryl, wherein the one or more heteroatoms are selected from N and O, and the one or more optional substituents of the aryl or heteroaryl are selected from C1-C6 alkyl, C1-C6 alkylamine, amido, and cyano,
wherein R' and R" are independently selected from H, C1-C6 alkyl optionally substituted with OH, C3-C7 cycloalkyl, C1-C7 heterocycloalkyl, C4-C7 alkylcycloalkyl, C3-C7 alkylheterocycloalkyl, benzyl, phenyl, and methoxy, or wherein R' and R" are joined to one another to form a C2-C7 heterocycle that includes the N to which they are attached, wherein the heterocycle is optionally hydroxyl-substituted, oxo-substituted, methyl-substituted, CH2OH-substituted, or acetyl-substituted.

12. A compound, pharmaceutically acceptable salt, tautomer, stereoisomer or *N*-oxide derivative for use according to any of claims 1-11, wherein Y is N or CR₉, wherein R₉ is selected from: phenyl optionally substituted with amido, cyano or methyl amine; pyridine; oxazole; pyrazole; carboxyl; C(O)NR'R"; or NR'R";
wherein R' and R" are independently selected from H, C1-C6 alkyl, C3-C7 cycloalkyl, C3-C7 heterocycloalkyl wherein the heteroatom is N or O, or wherein R' and R" are joined to one another to form a C2-C7 heterocycloalkyl that includes the N to which they are attached, wherein the heterocycloalkyl is optionally hydroxyl-substituted, oxo-substituted, methyl-substituted, CH2OH-substituted, or acetyl-substituted.

13. A compound, pharmaceutically acceptable salt, tautomer, stereoisomer or *N-*oxide derivative for use according to claim 1, wherein
R₁ is selected from optionally substituted ethylcyclohexane, optionally substituted ethylbenzene, optionally substituted 2,2 difluorobutane, and optionally substituted 3,3,3-trifluoropropane, wherein the optional substituent is one or more of methyl, ethyl, propyl, propenyl, hydroxymethyl and methoxymethyl, or wherein R₁ is phenyl-substituted pyrrolidine;
R₂ and R₃ are independently selected from H, methyl,
R₄ and R₅ are independently selected from H, methyl, or are joined to one another to form a C3-C6 cycloalkyl or heterocycloalkyl that includes the carbon to which they are attached, optionally a C4 or C5 cycloalkyl that includes the carbon to which they are attached
R₆ is H or methyl,
R₇ is selected from optionally substituted phenyl, indazole, thiophene, or pyrazole, wherein the optional substituent is selected from C1-C6 alkyl, hydroxysubstituted C1-C6 alkyl, C3-C6 cycloalkyl, C1-C6 heterocycloalkyl, C1-C6 alkoxy, halo-substituted C1-C6 alkoxy, C1-C6 alkylamine, amido, cyano or halo,
W is N or C
X is CH or N
Z is C or NH
Y is N or CR₉, wherein R₉ is selected from: phenyl optionally substituted with amido, cyano or methyl amine; pyridine; oxazole; pyrazole; C(O)NR'R"; or NR'R";
wherein R' and R" are independently selected from H, C1-C6 alkyl, C3-C7 cycloalkyl, C3-C7 heterocycloalkyl wherein the heteroatom is N or O, or wherein R' and R" are joined to one another to form a C2-C7 heterocycloalkyl that includes the N to which they are attached, wherein the heterocycloalkyl is optionally hydroxyl-substituted, oxo-substituted, methyl-substituted, hydroxymethyl-substituted, or acetyl-substituted.

14. A compound, pharmaceutically acceptable salt, tautomer, stereoisomer or *N-*oxide derivative for use according to any one of claims 11-13, wherein Y is CR₉, wherein R₉ is C(O)NR'R" and wherein R' and R" are joined to one another to form an optionally substituted pyrrolidine, piperidine, piperazine or morpholine that includes the N to which they are attached, wherein the piperidine, pyrrolidine, piperazine or morpholine is optionally hydroxyl-substituted, oxo-substituted, methyl-substituted, hydroxymethyl-substituted, or acetyl-substituted, optionally wherein Y is CR₉, wherein R₉ is C(O)NR'R" and wherein R' and R" are joined to one another to form a piperazinyl ring.

15. A compound, pharmaceutically acceptable salt, tautomer, stereoisomer or *N-*oxide derivative for use according to any one of claims 1-13, wherein W is N and Y is N, such that the ring comprising W, X, Y, Z is a substituted pyrimidinone ring.

16. A compound, pharmaceutically acceptable salt, tautomer, stereoisomer or *N-*oxide derivative for use according to any one of claims 1-15, wherein when the compound is chiral at the hydroxyl position of formula (I), the compound is the S enantiomer.

17. A compound, or pharmaceutically acceptable salt, tautomer, stereoisomer or *N-*oxide derivative thereof, for use according to claim 1, wherein the compound is:
(*R*)-5-Bromo-1-((4-hydroxy-1-(3-phenylbutanoyl)piperidin-4-yl)methyl)-4-phenylpyridin-2(1*H*)-one;
6-(2-Fluorophenyl)-3-((4-hydroxy-1-(3-phenylpropanoyl)piperidin-4-yl)methyl)pyrimidin-4(3*H*)-one;
3-((1-(3-Cyclobutylpropanoyl)-4-hydroxypiperidin-4-yl)methyl)-6-(2-fluorophenyl)pyrimidin-4(3*H*)-one;
3-((1-(3-Cyclopentylpropanoyl)-4-hydroxypiperidin-4-yl)methyl)-6-(2-fluorophenyl)pyrimidin-4(3*H*)-one;
(*R*,*S*)-6-(2-Fluorophenyl)-3-((4-hydroxy-1-(2-methyl-3-phenylpropanoyl)piperidin-4-yl)methyl)pyrimidin-4(3*H*)-one;
(*R*)-*N*-(3-(1-((4-Hydroxy-1-(3-phenylbutanoyl)piperidin-4-yl)methyl)-6-oxo-4-phenyl-1,6-dihydropyridin-3-yl)phenyl)acetamide;
(*R*)-3-(1-((4-Hydroxy-1-(3-phenylbutanoyl)piperidin-4-yl)methyl)6-oxo-4-phenyl-1,6-dihydropyridin-3-yl)benzamide;
(*R*)-5-(Furan-2-yl)-1-((4-hydroxy-1-(3-phenylbutanoyl)piperidin-4-yl)methyl)-4-phenylpyridin-2(1*H*)-one;
(*R*)-1-((4-Hydroxy-1-(3-phenylbutanoyl)piperidin-4-yl)methyl)-5-(1-methyl-1*H*-pyrazol-5-yl)-4-phenylpyridin-2(1*H*)-one;
(*R*)-4-Chloro-1-((4-hydroxy-1(3-phenylbutanoyl)piperidin-4-yl)methyl)-5-phenylpyridin-2(1*H*)-one;
(*R*)-4-(Dimethylamino)-1-((4-hydroxy-1-(3-phenylbutanoyl)piperidin-4-yl)methyl)-5-phenylpyridin-2(1*H*)-one;
(*R*)-1-((4-Hydroxy-1-(3-phenylbutanoyl)piperidin-4-yl)methyl)-5-phenyl-4-(prop-1-en-2-yl)pyridine-2(1*H*)-one;
(*R*)-Ethyl 1-((4-hydroxy-1-(3-phenylbutanoyl)piperidin-4-yl)methyl)-6-oxo-4-phenyl-1,6-dihydropyridine-3-carboxylate;
(*R*,*S*)-4-(2-Fluorophenyl)-1-((4-hydroxy-1-(2-methyl-1,2,3,4-tetrahydronaphthalene-2-carbonyl)piperidin-4-yl)methyl)-*N*-isopropyl-*N*-methyl-6-oxo-1,6-dihydropyridine-3-carboxamide;
(*R*,*S*)-1-((1-(3-Cyclobutyl-2-methylpropanoyl)-4-hydroxypiperidin-4-yl)methyl)-4-phenyl-5-(piperidine-1-carbonyl)pyridin-2(1*H*)-one;
(*R*)-3-((4-Hydroxy-1-(3-phenylbutanoyl)piperidin-4-yl)methyl)-6-(1-methyl-1*H*-indazol-7-yl)pyrimidin-4(3*H*)-one;
(*R*)-3-((4-Hydroxy-1-(3-phenylbutanoyl)piperidin-4-yl)methyl)-6-(2-methoxyphenyl)pyrimidin-4(3*H*)-one;
(*R*)-3-((4-Hydroxy-1-(3-phenylbutanoyl)piperidin-4-yl)methyl)-2-methyl-6-phenylpyrimidin-4(3*H*)-one;
(*R*)-2-((4-Hydroxy-1-(3-phenylbutanoyl)piperidin-4-yl)methyl)-5,6-diphenylpyridazin-3(2*H*)-one;
(*R*)-3-((4-Hydroxy-1-(3-phenylbutanoyl)piperidin-4-yl)methyl)-6-(naphthalen-1-yl)pyrimidin-4(3*H*)-one;
(*R*)-6-(3-(1,3-Dioxolan-2-yl)phenyl)-3-((4-hydroxy-1-(3-phenylbutanoyl)piperidin-4-yl)methyl)pyrimidin-4(3*H*)-one;
(*R*)-3-((4-Hydroxy-1-(3-phenylbutanoyl)piperidin-4-yl)methyl)-6-(thiophen-3-yl)pyrimidin-4(3*H*)-one;
4-(2-Fluorophenyl)-1-((4-hydroxy-1-(1-methylcyclopentane-1-carbonyl)piperidin-4-yl)methyl)-*N-*isopropyl-*N-*methyl-6-oxo-1,6-dihydropyridine-3-carboxamide;
3-((1-(1-Ethylcyclohexane-1-carbonyl)-4-hydroxypiperidin-4-yl)methyl)-6-(2-fluorophenyl)pyrimidin-4(3*H*)-one;
3-((1-(2-(Cyclohexylmethyl)-3-methylbutanoyl)-4-hydroxy-3,3-dimethylpiperidin-4-yl)methyl)-6-(2-fluorophenyl)pyrimidin-4(3*H*)-one;
(*R*)-6-(Furan-2-yl)-3-((4-hydroxy-1-(3-phenylbutanoyl)piperidin-4-yl)methyl)pyrimidin-4(3*H*)-one;
(*R*)-1-((4-Hydroxy-1-(3-phenylbutanoyl)piperidin-4-yl)methyl)-4-phenylpyridin-2(1*H*)-one;
(*R*)-6-(Cyclohex-1-en-1-yl)-3-((4-hydroxy-1-(3-phenylbutanoyl)piperidin-4-yl)methyl)pyrimidin-4(3*H*)-one;
(*R*)-3-((4-Hydroxy-1-(3-phenylbutanoyl)piperidin-4-yl)methyl)-6-(prop-1-en-2-yl)pyrimidin-4(3*H*)-one;
(*R*)-3-((4-Hydroxy-1-(3-phenylbutanoyl)piperidin-4-yl)methyl)-6-(2-(trifluoromethoxy)phenyl)pyrimidin-4(3*H*)-one;
3-(((1*R*,5*S*)-3-((*R*)-3-Cyclohexyl-2-methylpropanoyl)-8-hydroxy-3-azabicyclo[3.2.1]octan-8-yl)methyl)-6-(2-fluorophenyl)pyrimidin-4(3*H*)-one;
(R)-6-(2-Fluorophenyl)-3-((4-hydroxy-1-(3-phenylbutanoyl)piperidin-4-yl)methyl)pyrimidin-4(3*H*)-one;
(*R*)-5-((4-Hydroxy-1-(3-phenylbutanoyl)piperidin-4-yl)methyl)-2-phenylpyrimidin-4(3*H*)-one;
3-((4-Hydroxy-1-(3-phenylpropanoyl)piperidin-4-yl)methyl)-2-methyl-6-phenylpyrimidin-4(3*H*)-one;
(*R*)-3-((4-Hydroxy-1-(3-phenylbutanoyl)piperidin-4-yl)methyl)-6-(isobutylamino)pyrimidin-4(3*H*)-one;
(*R*)-1-((1-(3-Cyclohexyl-2-methylpropanoyl)-4-hydroxypiperidin-4-yl)methyl)-4-phenyl-5-(piperidine-1-carbonyl)pyridin-2(1*H*)-one;
(*R*)-2-((4-Hydroxy-1-(3-phenylbutanoyl)piperidin-4-yl)methyl)-5-phenylpyridazin-3(2*H*)-one;
(*R*)-4-(2-Cyanophenyl)-1-((4-hydroxy-1-(3-phenylbutanoyl)piperidin-4-yl)methyl)-*N-*isopropyl-*N*-methyl-6-oxo-1,6-dihydropyridine-3-carboxamide;
(*R*)-3-((1-(3-Cyclohexyl-2-methylpropanoyl)-4-hydroxypiperidin-4-yl)methyl)-6-(2-fluorophenyl)pyrimidin-4(3*H*)-one;
3-((1-(2-(Cyclohexylmethyl)butanoyl)-4-hydroxypiperidin-4-yl)methyl)-6-(2-fluorophenyl)pyrimidin-4(3*H*)-one;
*rac*-6-(2-Fluorophenyl)-3-((4-hydroxy-1-(*cis*-2-phenylcyclopropanecarbonyl)piperidin-4-yl)methyl)pyrimidin-4(3*H*)-one;
6-(2-Fluorophenyl)-3-((4-hydroxy-1-(1-methylcyclohexanecarbonyl)piperidin-4-yl)methyl)pyrimidin-4(3*H*)-one;
3-((*S*)-1-(1-((*R*)-3-Cyclohexyl-2-methylpropanoyl)-4-hydroxypiperidin-4-yl)propyl)-6-phenylpyrimidin-4(3*H*)-one;
3-((*R*)-1-(1-((*R*)-3-Cyclohexyl-2-methylpropanoyl)-4-hydroxypiperidin-4-yl)propyl)-6-phenylpyrimidin-4(3*H*)-one;
(*R*,*S*)-3-((1-(3-Cyclohexyl-2-hydroxypropanoyl)-4-hydroxypiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-6-oxo-4-phenyl-1,6-dihydropyridine-3-carbaldehyde;
(*R*)-5-((Dimethylamino)methyl)-1-((4-hydroxy-1-(3-phenylbutanoyl)piperidin-4-yl)methyl)-4-phenylpyridin-2(1*H*)-one;
3-((*R*)-1-(4-Hydroxy-1-((*R*)-3-phenylbutanoyl)piperidin-4-yl)ethyl)-6-phenylpyrimidin-4(3*H*)-one;
3-((*S*)-1-(4-Hydroxy-1-((*R*)-3-phenylbutanoyl)piperidin-4-yl)ethyl)-6-phenylpyrimidin-4(3*H*)-one;
(*R*)-6-(2-Fluorophenyl)-3-((4-hydroxy-1-(4,4,4-trifluoro-3-phenylbutanoyl)piperidin-4-yl)methyl)pyrimidin-4(3*H*)-one;
(*R*,*S*)-3-((1-(3-Cyclohexyl-2-fluoropropanoyl)-4-hydroxypiperidin-4-yl)methyl)-6-(2-fluorophenyl)pyrimidin-4(3*H*)-one;
3-(((*R*)-1-((*R*)-3-Cyclohexyl-2-methylpropanoyl)-4-hydroxy-3,3-dimethylpiperidin-4-yl)methyl)-6-phenylpyrimidin-4(3*H*)-one;
3-(((*S*)-1-((*R*)-3-Cyclohexyl-2-methylpropanoyl)-4-hydroxy-3,3-dimethylpiperidin-4-yl)methyl)-6-phenylpyrimidin-4(3*H*)-one;
3-(((*R*)-6-((*R*)-3-Cyclohexyl-2-methylpropanoyl)-9-hydroxy-6-azaspiro[3.5]nonan-9-yl)methyl)-6-(2-fluorophenyl)pyrimidin-4(3*H*)-one;
3-(((*S*)-6-((*R*)-3-Cyclohexyl-2-methylpropanoyl)-9-hydroxy-6-azaspiro[3.5]nonan-9-yl)methyl)-6-(2-fluorophenyl)pyrimidin-4(3*H*)-one;
(*R*)-5-(3-(Aminomethyl)phenyl)-1-((1-(3-cyclohexyl-2-methylpropanoyl)-4-hydroxypiperidin-4-yl)methyl)-4-(2-fluorophenyl)pyridin-2(1*H*)-one;
3-(((*S*)-4-Hydroxy-3,3-dimethyl-1-((*R*)-3-phenylbutanoyl)piperidin-4-yl)methyl)-6-phenylpyrimidin-4(3*H*)-one;
3-(((*R*)-4-Hydroxy-3,3-dimethyl-1-((*R*)-3-phenylbutanoyl)piperidin-4-yl)methyl)-6-phenylpyrimidin-4(3*H*)-one;
(*R*)-6-(4-Fluorophenoxy)-3-((4-hydroxy-1-(3-phenylbutanoyl)piperidin-4-yl)methyl)pyrimidin-4(3*H*)-one;
(*R*)-1-((4-Hydroxy-1-(3-phenylbutanoyl)piperidin-4-yl)methyl)-5-(2-oxopyrrolidin-1-yl)-4-phenylpyridin-2(1*H*)-one;
6-(2-Fluorophenyl)-3-((4-hydroxy-1-(1-methyl-4-methylenecyclohexanecarbonyl)piperidin-4-yl)methyl)pyrimidin-4(3*H*)-one;
(*R*,*S*)-3-((1-(3-Cyclobutyl-2-methylpropanoyl)-4-hydroxypiperidin-4-yl)methyl)-6-(2-fluorophenyl)pyrimidin-4(3*H*)-one;
(*R*,*S*)-3-((1-(3-Cycloheptyl-2-methylpropanoyl)-4-hydroxypiperidin-4-yl)methyl)-6-(2-fluorophenyl)pyrimidin-4(3*H*)-one;
3-((1-(3-Cyclobutyl-2,2-dimethylpropanoyl)-4-hydroxypiperidin-4-yl)methyl)-6-(2-fluorophenyl)pyrimidin-4(3*H*)-one;
6-(2-Fluorophenyl)-3-(((1*R*,5*S*)-8-hydroxy-3-((*R*)-3-phenylbutanoyl)-3-azabicyclo[3.2.1]octan-8-yl)methyl)pyrimidin-4(3*H*)-one;
3-(((*R*)-4-Hydroxy-2,2-dimethyl-1-((*R*)-3-phenylbutanoyl)piperidin-4-yl)methyl)-6-phenylpyrimidin-4(3*H*)-one;
3-(((*S*)-4-Hydroxy-2,2-dimethyl-1-((*R*)-3-phenylbutanoyl)piperidin-4-yl)methyl)-6-phenylpyrimidin-4(3*H*)-one;
(*R*)-1-((4-Hydroxy-1-(3-phenylbutanoyl)piperidin-4-yl)methyl)-5-(4-hydroxypiperidine-1-carbonyl)-4-phenylpyridin-2(1*H*)-one;
(*R*)-1-((4-Hydroxy-1-(3-phenylbutanoyl)piperidin-4-yl)methyl)-4-phenyl-5-(pyrrolidine-1-carbonyl)pyridin-2(1*H*)-one;
(*R*)-1-((4-Hydroxy-1-(3-phenylbutanoyl)piperidin-4-yl)methyl)-4-phenyl-5-(1,4-dioxa-8-azaspiro[4.5]decane-8-carbonyl)pyridin-2(1*H*)-one;
(*R*)-*N*-Cyclohexyl-1-((4-hydroxy-1-(3-phenylbutanoyl)piperidin-4-yl)methyl)-6-oxo-4-phenyl-1,6-dihydropyridine-3-carboxamide;
6-(2-Fluorophenyl)-3-((4-hydroxy-3,3-dimethyl-1-(*cis*-2-phenylcyclopropane-1-carbonyl)piperidin-4-yl)methyl)pyrimidin-4(3*H*)-one;
(*R*,*S*)-3-((1-(3-Cyclohexyl-1H-pyrazole-4-carbonyl)-4-hydroxy-3,3-dimethylpiperidin-4-yl)methyl)-6-(2-fluorophenyl)pyrimidin-4(3*H*)-one;
(*R*,*S*)-6-(2-Fluorophenyl)-3-((4-hydroxy-3,3-dimethyl-1-(3-phenylpropanoyl)piperidin-4-yl)methyl)pyrimidin-4(3*H*)-one;
(*R*,*S*)-6-(2-Fluorophenyl)-3-((4-hydroxy-1-(1-isobutyl cyclopropane-1-carbonyl)-3,3-dimethylpiperidin-4-yl)methyl)pyrimidin-4(3*H*)-one;
(*R*,*S*)-6-(2-Fluorophenyl)-3-((4-hydroxy-3,3-dimethyl-1-(3-methyl-3-phenylbutanoyl)piperidin-4-yl)methyl)pyrimidin-4(3*H*)-one;
(*S*)-1-((1-(4,4-Difluoro-3-phenylbutanoyl)-4-hydroxypiperidin-4-yl)methyl)-*N*-isopropyl-*N-*methyl-6-oxo-4-phenyl-1,6-dihydropyridine-3-carboxamide;
1-((4-Hydroxy-1-(1-(thiophen-2-yl)cyclopropane-1-carbonyl)piperidin-4-yl)methyl)-*N-*isopropyl-*N*-methyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
(*R*)-3-((4-Hydroxy-1-(3-phenylbutanoyl)piperidin-4-yl)methyl)-6-(2-(hydroxymethyl)phenyl)pyrimidin-4(3*H*)-one;
(*R*)-*N*,*N*-Diethyl-1-((4-hydroxy-1-(3-phenylbutanoyl)piperidin-4-yl)methyl)-6-oxo-4-phenyl-1,6-dihydropyridine-3-carboxamide;
3-((1-(3-Cyclohexylbutanoyl)-4-hydroxy-3,3-dimethylpiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-5-(morpholine-4-carbonyl)-4-phenylpyridin-2(1*H*)-one;
(*R*,*S*)-3-((1-(3-Cyclohexylpropanoyl)-4-hydroxy-3,3-dimethylpiperidin-4-yl)methyl)-6-(2-fluorophenyl)pyrimidin-4(3*H*)-one;
(*R*,*S*)-1-((1-(3-Cyclobutylpropanoyl)-4-hydroxy-3,3-dimethylpiperidin-4-yl)methyl)-*N-*isopropyl-*N*-methyl-6-oxo-4-phenyl-1,6-dihydropyridine-3-carboxamide;
(*R*,*S*)-1-((1-(2-Ethylhexanoyl)-4-hydroxypiperidin-4-yl)methyl)-*N*-isopropyl-*N*-methyl-6-oxo-4-phenyl-1,6-dihydropyridine-3-carboxamide;
(*R*,*S*)-6-(2-Fluorophenyl)-3-((4-hydroxy-3,3-dimethyl-1-(1-methylcyclohexane-1-carbonyl)piperidin-4-yl)methyl)pyrimidin-4(3*H*)-one;
6-(2-Fluorophenyl)-3-((1-(3-(4-fluorophenyl)propanoyl)-4-hydroxypiperidin-4-yl)methyl)pyrimidin-4(3*H*)-one;
(*R*,*S*)-3-((1-(3-Cyclopropylpropanoyl)-4-hydroxy-3,3-dimethylpiperidin-4-yl)methyl)-6-(2-fluorophenyl)pyrimidin-4(3*H*)-one;
1-((1-(2,2-Dimethylbutanoyl)-4-hydroxypiperidin-4-yl)methyl)-*N*-isopropyl-*N*-methyl-6-oxo-4-phenyl-1,6-dihydropyridine-3-carboxamide;
(*R*)-*N*-(2-(1-((4-Hydroxy-1-(3-phenylbutanoyl)piperidin-4-yl)methyl)-6-oxo-1,6-dihydropyrimidin-4-yl)phenyl)acetamide;
(*R*)-5-(4-(Aminomethyl)phenyl)-1-((4-hydroxy-1-(3-phenylbutanoyl)piperidin-4-yl)methyl)-4-phenylpyridin-2(1*H*)-one;
(*R*)-1-((1-(3-Cyclohexyl-2-methylpropanoyl)-4-hydroxypiperidin-4-yl)methyl)-*N*-isopropyl-*N-*methyl-6-oxo-4-phenyl-1,6-dihydropyridine-3-carboxamide;
(*R*)-1-((4-Hydroxy-1-(3-phenylbutanoyl)piperidin-4-yl)methyl)-4-phenyl-5-(piperidine-1-carbonyl)pyridin-2(1*H*)-one;
(*R*)-1-((4-Hydroxy-1-(3-phenylbutanoyl)piperidin-4-yl)methyl)-*N*-isopropyl-4-(2-methoxyphenyl)-*N*-methyl-6-oxo-1,6-dihydropyridine-3-carboxamide;
(*R*)-4-(2-Fluorophenyl)-1-((4-hydroxy-1-(3-phenylbutanoyl)piperidin-4-yl)methyl)-*N-*isopropyl-*N*-methyl-6-oxo-1,6-dihydropyridine-3-carboxamide;
(*R*)-1'-((4-Hydroxy-1-(3-phenylbutanoyl)piperidin-4-yl)methyl)-4'-phenyl-[2,3'-bipyridin]-6'(1'*H*)-one;
1-((1-(3-Cyclohexylpropanoyl)-4-hydroxypiperidin-4-yl)methyl)-*N*-isopropyl-*N*-methyl-6-oxo-4-phenyl-1,6-dihydropyridine-3-carboxamide;
(*R*)-*tert*-Butyl 4-(1-((4-hydroxy-1-(3-phenylbutanoyl)piperidin-4-yl)methyl)-6-oxo-4-phenyl-1,6-dihydropyridine-3-carbonyl)piperazine-1-carboxylate;
(*R*)-1-((4-Hydroxy-1-(3-phenylbutanoyl)piperidin-4-yl)methyl)-4-phenyl-5-(piperazine-1-carbonyl)pyridin-2(1*H*)-one;
(*R*)-1-((4-Hydroxy-1-(3-phenylbutanoyl)piperidin-4-yl)methyl)-*N*,*N*-dimethyl-6-oxo-4-phenyl-1,6-dihydropyridine-3-carboxamide;
(*R*)-1-((4-Hydroxy-1-(3-phenylbutanoyl)piperidin-4-yl)methyl)-*N*-isopropyl-*N*-methyl-6-oxo-4-(*p*-tolyl)-1,6-dihydropyridine-3-carboxamide;
(*R*)-1-((4-Hydroxy-1-(3-phenylbutanoyl)piperidin-4-yl)methyl)-*N*-methoxy-*N*-methyl-6-oxo-4-phenyl-1,6-dihydropyridine-3-carboxamide;
(*R*)-1-((4-Hydroxy-1-(3-phenylbutanoyl)piperidin-4-yl)methyl)-4,5-diphenylpyridin-2(1*H*)-one;
(*R*)-1-((4-Hydroxy-1-(3-phenylbutanoyl)piperidin-4-yl)methyl)-4-phenyl-[3,3'-bipyridin]-6(1*H*)-one;
(*R*)-5-(3-(Aminomethyl)phenyl)-1-((4-hydroxy-1-(3-phenylbutanoyl)piperidin-4-yl)methyl)-4-phenylpyridin-2(1*H*)-one;
(*R*)-1-((4-Hydroxy-1-(3-phenylbutanoyl)piperidin-4-yl)methyl)-*N*-isopropyl-*N*-methyl-6-oxo-4-phenyl-1,6-dihydropyridine-3-carboxamide;
(*R*)-1-((4-Hydroxy-1-(3-phenylbutanoyl)piperidin-4-yl)methyl)-*N*-isopropyl-*N*-methyl-6-oxo-4-(*m*-tolyl)-1,6-dihydropyridine-3-carboxamide;
(*R*)-1-((4-Hydroxy-1-(3-phenylbutanoyl)piperidin-4-yl)methyl)-4-phenyl-5-(pyrimidin-5-yl)pyridin-2(1*H*)-one;
(*R*)-*N*-(Cyclopropylmethyl)-1-((4-hydroxy-1-(3-phenylbutanoyl)piperidin-4-yl)methyl)-6-oxo-4-phenyl-1,6-dihydropyridine-3-carboxamide;
(*R*)-*N*-Benzyl-1-((4-hydroxy-1-(3-phenylbutanoyl)piperidin-4-yl)methyl)-*N*-methyl-6-oxo-4-phenyl-1,6-dihydropyridine-3-carboxamide;
(*R*)-2-(1-((4-Hydroxy-1-(3-phenylbutanoyl)piperidin-4-yl)methyl)-6-oxo-4-phenyl-1,6-dihydropyridin-3-yl)benzonitrile;
(*R*)-1-((4-Hydroxy-1-(3-phenylbutanoyl)piperidin-4-yl)methyl)-*N*-methyl-6-oxo-*N*,4-diphenyl-1,6-dihydropyridine-3-carboxamide;
3-(1-(1-((*R*)-3-Cyclohexyl-2-methylpropanoyl)-4-hydroxypiperidin-4-yl)ethyl)-6-phenylpyrimidin-4(3*H*)-one;
*(R)*-1-((4-Hydroxy-1-(3-phenylbutanoyl)piperidin-4-yl)methyl)-5-(1-methyl-1H-pyrazol-4-yl)-4-phenylpyridin-2(1H)-one;
(*R*)-3-((1-(2-(Cyclohexylmethyl)pent-4-enoyl)-4-hydroxypiperidin-4-yl)methyl)-6-(2-fluorophenyl)pyrimidin-4(3*H*)-one;
(*R*)-1-((4-Hydroxy-1-(3-phenylbutanoyl)piperidin-4-yl)methyl)-5-methyl-4-phenylpyridin-2(1*H*)-one;
(*R*)-6-(1,5-Dimethyl-1*H*-pyrazol-4-yl)-3-((4-hydroxy-1-(3-phenylbutanoyl)piperidin-4-yl)methyl)pyrimidin-4(3*H*)-one;
3-(((1*R*,5*S*)-3-(3-Cyclohexylpropanoyl)-8-hydroxy-3-azabicyclo[3.2.1]octan-8-yl)methyl)-6-(2-fluorophenyl)pyrimidin-4(3*R*)-one;
(*R*)-1-((4-Hydroxy-1-(3-phenylbutanoyl)piperidin-4-yl)methyl)-6-oxo-4-phenyl-1,6-dihydropyridine-3-carbonitrile;
(*R*)-1-((4-Hydroxy-1-(3-phenylbutanoyl)piperidin-4-yl)methyl)-*N*-(2-hydroxyethyl)-6-oxo-4-phenyl-1,6-dihydropyridine-3-carboxamide;
1-((4-Hydroxy-1-((*R*)-3-phenylbutanoyl)piperidin-4-yl)methyl)-5-((*S*)-2-methylpyrrolidine-1-carbonyl)-4-phenylpyridin-2(1*H*)-one;
(*R*)-1-((4-Hydroxy-1-(3-phenylbutanoyl)piperidin-4-yl)methyl)-5-(4-(hydroxymethyl)piperidine-1-carbonyl)-4-phenylpyridin-2(1*H*)-one;
3-((1-(2-(Cyclohexylmethyl)butanoyl)-4-hydroxy-3,3-dimethylpiperidin-4-yl)methyl)-6-(2-fluorophenyl)pyrimidin-4(3*H*)-one;
3-((7-((*R*)-3-Cyclohexyl-2-methylpropanoyl)-10-hydroxy-7-azaspiro[4.5]decan-10-yl)methyl)-6-(2-fluorophenyl)pyrimidin-4(3*H*)-one;
1-(((*S*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-phenyl-5-(pyrrolidine-1-carbonyl)pyridin-2(1*H*)-one;
1-(((*S*)-1-((*R*)-3-Cyclohexyl-2-methylpropanoyl)-4-hydroxy-3,3-dimethylpiperidin-4-yl)methyl)-4-phenyl-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-4-phenyl-5-(piperazine-1-carbonyl)pyridin-2(1*H*)-one;
3-((1-(2-(Cyclohexyloxy)propanoyl)-4-hydroxy-3,3-dimethylpiperidin-4-yl)methyl)-6-(2-fluorophenyl)pyrimidin-4(3*H*)-one;
1-((7-((*R*)-3-Cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((1-((*R*)-3-Cyclobutyl-2-methylpropanoyl)-4-hydroxy-3,3-dimethylpiperidin-4-yl)methyl)-*N*,*N-*dimethyl-6-oxo-4-phenyl-1,6-dihydropyridine-3-carboxamide;
1-((1-((*R*)-3-Cyclopropyl-2-methylpropanoyl)-4-hydroxy-3,3-dimethylpiperidin-4-yl)methyl)-*N*,*N-*dimethyl-6-oxo-4-phenyl-1,6-dihydropyridine-3-carboxamide;
1-((1-((*R*)-3-Cyclohexyl-2-methylpropanoyl)-4-hydroxy-3,3-dimethylpiperidin-4-yl)methyl)-[4,5'-bipyrimidin]-6(1*H*)-one;
3-((1-((*R*)-3-Cyclohexyl-2-methylpropanoyl)-4-hydroxy-3,3-dimethylpiperidin-4-yl)methyl)-6-(1-methyl-1*H*-pyrazol-5-yl)pyrimidin-4(3*H*)-one;
3-((1-((*R*)-3-Cyclohexyl-2-methylpropanoyl)-4-hydroxy-3,3-dimethylpiperidin-4-yl)methyl)-6-(1*H*-pyrazol-4-yl)pyrimidin-4(3*H*)-one;
3-((1-((*R*)-3-Cyclohexyl-2-methylpropanoyl)-4-hydroxy-3,3-dimethylpiperidin-4-yl)methyl)-6-(1*H*-pyrazol-5-yl)pyrimidin-4(3*H*)-one;
3-((7-((*R*)-3-Cyclohexyl-2-methylpropanoyl)-10-hydroxy-7-azaspiro[4.5]decan-10-yl)methyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(thiophen-3-yl)pyrimidin-4(3*H*)-one;
1-((1-((*R*)-3-Cyclohexyl-2-methylpropanoyl)-4-hydroxy-3,3-dimethylpiperidin-4-yl)methyl)-5-(oxazol-2-yl)-4-phenylpyridin-2(1*H*)-one;
3-((1-((*R*)-3-Cyclohexyl-2-methylpropanoyl)-4-hydroxy-3,3-dimethylpiperidin-4-yl)methyl)-6-(2-(hydroxymethyl)phenyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(3-(hydroxymethyl)phenyl)pyrimidin-4(3*H*)-one;
3-((1-((*R*)-3-Cyclohexyl-2-methylpropanoyl)-4-hydroxy-3,3-dimethylpiperidin-4-yl)methyl)-6-(4-(hydroxymethyl)phenyl)pyrimidin-4(3*H*)-one;
6-(4-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
6-(2-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
6-(2-Fluorophenyl)-3-((4-hydroxy-3,3-dimethyl-1-(2,4,4-trimethylpentanoyl)piperidin-4-yl)methyl)pyrimidin-4(3*H*)-one;
6-(2-Fluorophenyl)-3-((4-hydroxy-3,3-dimethyl-1-(4,4,4-trifluoro-2-methylbutanoyl)piperidin-4-yl)methyl)pyrimidin-4(3*H*)-one;
4-Chloro-1-((7-((*R*)-3-cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(dimethylamino)-*N*,*N-*dimethyl-6-oxo-1,6-dihydropyridine-3-carboxamide;
1-((1-((*S*)-3-(Benzyloxy)-2-(cyclohexylmethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-((10-Hydroxy-7-isobutyryl-7-azaspiro[4.5]decan-10-yl)methyl)-*N,N*-dimethyl-6-oxo-4-phenyl-1,6-dihydropyridine-3-carboxamide;
1-((10-Hydroxy-7-((*R*)-2-methylbutanoyl)-7-azaspiro[4.5]decan-10-yl)methyl)-*N*,*N*-dimethyl-6-oxo-4-phenyl-1,6-dihydropyridine-3-carboxamide;
1-((7-((*R*)-3-Cyclopropyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
6-(2-Fluorophenyl)-3-((4-hydroxy-3,3-dimethyl-1-(2-methyl-3-(1*H*-pyrazol-1-yl)propanoyl)piperidin-4-yl)methyl)pyrimidin-4(3*H*)-one;
1-((7-((*R*)-3-Cyclohexyl-2-methylpropanoyl)-10-hydroxy-7-azaspiro[4.5]decan-10-yl)methyl)-4-(2-(hydroxymethyl)phenyl)-*N*,*N*-dimethyl-6-oxo-1,6-dihydropyridine-3-carboxamide;
1-(((*S*)-1-((*S*)-3-Cyclohexyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*S*)-3-Cyclohexyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1'-((1-((*R*)-3-Cyclohexyl-2-methylpropanoyl)-4-hydroxy-3,3-dimethylpiperidin-4-yl)methyl)-4'-(2-fluorophenyl)-[2,3'-bipyridin]-6'(1'*H*)-one;
1-((7-((*R*)-3-Cyclohexyl-2-methylpropanoyl)-10-hydroxy-7-azaspiro[4.5]decan-10-yl)methyl)-*N*,*N*-dimethyl-6-oxo-4-(thiophen-3-yl)-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(3-(hydroxymethyl)phenyl)-*N*,*N*-dimethyl-6-oxo-1,6-dihydropyridine-3-carboxamide;
1-((7-(3-Cyclohexyl-2-hydroxypropanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((10-Hydroxy-7-(2-methyl-3-(piperidin-1-yl)propanoyl)-7-azaspiro[4.5]decan-10-yl)methyl)-*N*,*N*-dimethyl-6-oxo-4-phenyl-1,6-dihydropyridine-3-carboxamide;
6-(3-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-one;
1-(((*S*)-1-((*S*)-3-Cyclohexyl-2-(methoxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
4-(2-(Aminomethyl)phenyl)-1-((7-((*R*)-3-cyclohexyl-2-methylpropanoyl)-10-hydroxy-7-azaspiro[4.5]decan-10-yl)methyl)-*N*,*N*-dimethyl-6-oxo-1,6-dihydropyridine-3-carboxamide;
1-((7-((*R*)-3-Cyclohexyl-2-methylpropanoyl)-10-hydroxy-7-azaspiro[4.5]decan-10-yl)methyl)-4-(4-(hydroxymethyl)phenyl)-*N*,*N*-dimethyl-6-oxo-1,6-dihydropyridine-3-carboxamide;
1-((10-Hydroxy-7-(2-methyl-3-morpholinopropanoyl)-7-azaspiro[4.5]decan-10-yl)methyl)-*N*,*N-*dimethyl-6-oxo-4-phenyl-1,6-dihydropyridine-3-carboxamide;
1-(((*S*)-1-((*S*)-2-(Cyclohexylmethyl)-4-hydroxybutanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((*S*)-2-(Cyclohexylmethyl)-4-hydroxybutanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((S)-1-((S)-3-Cyclobutyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-1-((S)-3-Cyclobutyl-2-(hydroxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-((1-((*R*)-3-Cyclohexyl-2-methylpropanoyl)-4-hydroxy-3,3-dimethylpiperidin-4-yl)methyl)-5-(morpholine-4-carbonyl)-4-phenylpyridin-2(1*H*)-one;
5-(4-Acetylpiperazine-1-carbonyl)-1-((1-((*R*)-3-cyclohexyl-2-methylpropanoyl)-4-hydroxy-3,3-dimethylpiperidin-4-yl)methyl)-4-phenylpyridin-2(1*H*)-one;
1-((1-((*R*)-3-Cyclohexyl-2-methylpropanoyl)-4-hydroxy-3,3-dimethylpiperidin-4-yl)methyl)-5-(4-methylpiperazine-1-carbonyl)-4-phenylpyridin-2(1*H*)-one;
1-((10-Hydroxy-7-(4,4,4-trifluoro-3-methylbutanoyl)-7-azaspiro[4.5]decan-10-yl)methyl)-*N*,*N-*dimethyl-6-oxo-4-phenyl-1,6-dihydropyridine-3-carboxamide;
1-((10-Hydroxy-7-(4,4,4-trifluorobutanoyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-(3-(tetrahydrofuran-3-yl)propanoyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-((1*R*,2*S*)-2-phenylcyclopropane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(Cyclopropanecarbonyl)-10-hydroxy-7-azaspiro[4.5]decan-10-yl)methyl)-*N*,*N-*dimethyl-6-oxo-4-phenyl-1,6-dihydropyridine-3-carboxamide;
1-(10-Hydroxy-7-(1-methylcyclohexane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(3-Fluorocyclopentane-1-carbonyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(1-(2,2-Difluoroethyl)cyclopropane-1-carbonyl)-10-hydroxy-7-azaspiro[4.5]decan-10-yl)methyl)-*N*,*N*-dimethyl-6-oxo-4-phenyl-1,6-dihydropyridine-3-carboxamide;
1-((7-(Bicyclo[2.2.1]heptane-1-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-((*S*)-4,4-Difluoro-3-phenylbutanoyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-((*R*)-4,4-Difluoro-3-phenylbutanoyl)-10-hydroxy-7-azaspiro[4.5]decan-10-yl)methyl)-*N*,*N*-dimethyl-6-oxo-4-phenyl-1,6-dihydropyridine-3-carboxamide;
1-((7-(3-Ethoxypropanoyl)-10-hydroxy-7-azaspiro[4.5]decan-10-yl)methyl)-*N,N*-dimethyl-6-oxo-4-phenyl-1,6-dihydropyridine-3-carboxamide;
1-((10-Hydroxy-7-((*S*)-3-phenylbutanoyl)-7-azaspiro[4.5]decan-10-yl)methyl)-*N*,*N-*dimethyl-6-oxo-4-phenyl-1,6-dihydropyridine-3-carboxamide;
1-((7-(3-(4-Fluorophenyl)propanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((10-Hydroxy-7-((*R*)-3-phenylbutanoyl)-7-azaspiro[4.5]decan-10-yl)methyl)-*N*,*N-*dimethyl-6-oxo-4-phenyl-1,6-dihydropyridine-3-carboxamide;
1-((10-Hydroxy-7-(1-methylcyclopentane-1-carbonyl)-7-azaspiro [4.5]decan-10-yl)methyl)-*N*,*N-*dimethyl-6-oxo-4-phenyl-1,6-dihydropyridine-3-carboxamide;
1-((10-Hydroxy-7-(1*H*-pyrazole-3-carbonyl)-7-azaspiro[4.5]decan-10-yl)methyl)-*N*,*N-*dimethyl-6-oxo-4-phenyl-1,6-dihydropyridine-3-carboxamide;
1-((10-Hydroxy-7-(1*H*-indazole-3-carbonyl)-7-azaspiro[4.5]decan-10-yl)methyl)-*N*,*N-*dimethyl-6-oxo-4-phenyl-1,6-dihydropyridine-3-carboxamide;
1-((7-(3-Cyclohexyl-1*H*-pyrazole-4-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((10-Hydroxy-7-(1*H*-indole-3-carbonyl)-7-azaspiro[4.5]decan-10-yl)methyl)-*N*,*N*-dimethyl-6-oxo-4-phenyl-1,6-dihydropyridine-3-carboxamide;
1-((7-(Bicyclo[1.1.1]pentane-1-carbonyl)-10-hydroxy-7-azaspiro [4.5]decan-10-yl)methyl)-*N*,*N-*dimethyl-6-oxo-4-phenyl-1,6-dihydropyridine-3-carboxamide;
1-((10-Hydroxy-7-(5-phenyloxazole-4-carbonyl)-7-azaspiro[4.5]decan-10-yl)methyl)-*N*,*N-*dimethyl-6-oxo-4-phenyl-1,6-dihydropyridine-3-carboxamide;
1-((7-(5-Cyclopropyloxazole-4-carbonyl)-10-hydroxy-7-azaspiro [4.5]decan-10-yl)methyl)-*N*,*N*-dimethyl-6-oxo-4-phenyl-1,6-dihydropyridine-3-carboxamide;
1-((7-(2-(Cyclohexyloxy)acetyl)-10-hydroxy-7-azaspiro[4.5]decan-10-yl)methyl)-*N*,*N-*dimethyl-6-oxo-4-phenyl-1,6-dihydropyridine-3-carboxamide;
1-((10-Hydroxy-7-(spiro[2.2]pentane-1-carbonyl)-7-azaspiro[4.5]decan-10-yl)methyl)-*N*,*N-*dimethyl-6-oxo-4-phenyl-1,6-dihydropyridine-3-carboxamide;
1-((10-Hydroxy-7-(3-(trifluoromethyl)cyclopentane-1-carbonyl)-7-azaspiro[4.5]decan-10-yl)methyl)-*N*,*N*-dimethyl-6-oxo-4-phenyl-1,6-dihydropyridine-3-carboxamide;
1-((7-(2,4-Dimethylpentanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(1-Benzyl-1*H*-pyrrole-2-carbonyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(2-(Cyclohexyloxy)propanoyl)-10-hydroxy-7-azaspiro[4.5]decan-10-yl)methyl)-*N*,*N-*dimethyl-6-oxo-4-phenyl-1,6-dihydropyridine-3-carboxamide;
3-((1-((S)-3-Cyclobutyl-2-(hydroxymethyl)propanoyl)-4-hydroxy-3,3-dimethylpiperidin-4-yl)methyl)-6-(2-fluorophenyl)pyrimidin-4(3*H*)-one;
1-((7-(2-Cyclobutoxyacetyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(3,3-Difluorocyclopentane-1-carbonyl)-10-hydroxy-7-azaspiro[4.5]decan-10-yl)methyl)-*N*,*N*-dimethyl-6-oxo-4-phenyl-1,6-dihydropyridine-3-carboxamide;
1-((10-Hydroxy-7-(2-hydroxy-3-phenylpropanoyl)-7-azaspiro[4.5]decan-10-yl)methyl)-*N*,*N-*dimethyl-6-oxo-4-phenyl-1,6-dihydropyridine-3-carboxamide;
1-(((*S*)-4-Hydroxy-3,3-dimethyl-1-((*R*)-4,4,4-trifluoro-2-methylbutanoyl)piperidin-4-yl)methyl)-*N*,*N*-dimethyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-(2-fluorophenyl)-*N*,*N-*dimethyl-6-oxo-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
(*S*)-1-((4-Hydroxy-3,3-dimethyl-1-(4,4,4-trifluoro-2-(2,2,2-trifluoroethyl)butanoyl)piperidin-4-yl)methyl)-*N*,*N*-dimethyl-6-oxo-4-phenyl-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-5-((*S*)-2-(hydroxymethyl)piperazine-1-carbonyl)-4-phenylpyridin-2(*H*)-one;
1-(((*R*)-7-((*R*)-3-Cyclohexyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclohexyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-1-((*R*)-3-Cyclohexyl-2-methylpropanoyl)-4-hydroxy-3,3-dimethylpiperidin-4-yl)methyl)-5-((*R*)-3-methylpiperazine-1-carbonyl)-4-phenylpyridin-2(1*H*)-one;
1-(((*S*)-7-((*R*)-3-Cyclobutyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*R*)-4,4,4-trifluoro-2-methylbutanoyl)-7-azaspiro[4.5]decan-10-yl)methyl)-4-phenyl-5-(piperazine-1-carbonyl)pyridin-2(1*H*)-one;
(*S*)-1-((10-Hydroxy-7-(4,4,4-trifluoro-2-(2,2,2-trifluoroethyl)butanoyl)-7-azaspiro[4.5]decan-10-yl)methyl)-*N*,*N-*dimethyl-6-oxo-4-phenyl-1,6-dihydropyridine-3-carboxamide;
(*S*)-1-((10-Hydroxy-7-(4,4,4-trifluoro-2-(2,2,2-trifluoroethyl)butanoyl)-7-azaspiro[4.5]decan-10-yl)methyl)-4-phenyl-5-(piperazine-1-carbonyl)pyridin-2(1*H*)-one;
1-(((*S*)-1-((*R*)-3-Cyclohexyl-2-methylpropanoyl)-4-hydroxy-3,3-dimethylpiperidin-4-yl)methyl)-4-cyclopropyl-*N*,*N*-dimethyl-6-oxo-1,6-dihydropyridine-3-carboxamide;
1-(((*S*)-1-((*R*)-3-Cyclohexyl-2-methylpropanoyl)-4-hydroxy-3,3-dimethylpiperidin-4-yl)methyl)-4-cyclopropyl-5-(piperazine-1-carbonyl)pyridin-2(1*H*)-one;
1-(((*S*)-7-((*R*)-3-Cyclobutyl-2-methylpropanoyl)-10-hydroxy-7-azaspiro[4.5]decan-10-yl)methyl)-4-(2-fluorophenyl)-*N*,*N-*dimethyl-6-oxo-1,6-dihydropyridine-3-carboxamide;
4-(2-Fluorophenyl)-1-(((*S*)-10-hydroxy-7-((*R*)-4,4,4-trifluoro-2-methylbutanoyl)-7-azaspiro[4.5]decan-10-yl)methyl)-*N*,*N-*dimethyl-6-oxo-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
4-(2-Fluorophenyl)-1-(((*S*)-10-hydroxy-7-((*R*)-4,4,4-trifluoro-2-methylbutanoyl)-7-azaspiro[4.5]decan-10-yl)methyl)-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
1-(((*S*)-7-((*R*)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
1-(((*S*)-1-((*R*)-3-Cyclohexyl-2-methylpropanoyl)-4-hydroxy-3,3-dimethylpiperidin-4-yl)methyl)-5-(4-hydroxypiperidine-1-carbonyl)-4-phenylpyridin-2(1*H*)-one;
1-((10-Hydroxy-7-(3-(trifluoromethyl)cyclobutane-1-carbonyl)-7-azaspiro[4.5]decan-10-yl)methyl)-*N*,*N*-dimethyl-6-oxo-4-phenyl-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-cyclopropyl-*N*,*N-*dimethyl-6-oxo-1,6-dihydropyridine-3-carboxamide;
1-(((*S*)-7-((*R*)-3-Cyclobutyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-4-cyclopropyl-*N*,*N-*dimethyl-6-oxo-1,6-dihydropyridine-3-carboxamide;
1 -(((*S*)-7-((*R*)-3-Cyclohexyl-2-methylpropanoyl)-1 0-hydroxy-7-azaspiro[4.5]decan-1 0-yl)methyl)-4-cyclopropyl-5-(piperazine-1-carbonyl)pyridin-2(1*H*)-one;
1-((2-((*R*)-3-Cyclohexyl-2-methylpropanoyl)-5-hydroxy-9-oxa-2-azaspiro[5.5]undecan-5-yl)methyl)-*N*,*N*-dimethyl-6-oxo-4-phenyl-1,6-dihydropyridine-3-carboxamide;
1-(((*S*)-1-((*R*)-3-Cyclobutyl-2-methylpropanoyl)-4-hydroxy-3,3-dimethylpiperidin-4-yl)methyl)-4-(2-fluorophenyl)-*N*,*N-*dimethyl-6-oxo-1,6-dihydropyridine-3-carboxamide;
1-(((*S*)-1-((*R*)-3-Cyclobutyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
1-((2-((R)-3-Cyclohexyl-2-methylpropanoyl)-5-hydroxy-2-azaspiro[5.5]undecan-5-yl)methyl)-*N*,*N*-dimethyl-6-oxo-4-phenyl-1,6-dihydropyridine-3-carboxamide;
1-((2-((*R*)-3-Cyclohexyl-2-methylpropanoyl)-5-hydroxy-2-azaspiro[5.5]undecan-5-yl)methyl)-4-phenyl-5-(piperazine-1-carbonyl)pyridin-2(1*H*)-one;
1-(((*S*)-7-((*R*)-3-Cyclohexyl-2-methylpropanoyl)-10-hydroxy-7-azaspiro[4.5]decan-10-yl)methyl)-5-(morpholine-4-carbonyl)-4-phenylpyridin-2(1*H*)-one;
1-(((*S*)-7-((*R*)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1*H*)-one;
1-((1-((*R*)-3-Cyclohexyl-2-methylpropanoyl)-4-hydroxy-3,3-dimethylpiperidin-4-yl)methyl)-6-oxo-4-phenyl-1,6-dihydropyridine-3-carboxylic acid;
1-((7-((*R*)-3-Cyclohexyl-2-methylpropanoyl)-10-hydroxy-7-azaspiro[4.5]decan-10-yl)methyl)-4-methoxy-*N*,*N*-dimethyl-6-oxo-1,6-dihydropyridine-3-carboxamide;
1-((7-(4,4-Difluoro-2-methylbutanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((7-(4,4-Difluoro-2,2-dimethylbutanoyl)-10-hydroxy-7-azaspiro[4.5]decan-10-yl)methyl)-*N*,*N-*dimethyl-6-oxo-4-phenyl-1,6-dihydropyridine-3-carboxamide;
1-((10-Hydroxy-7-(4-(trifluoromethyl)thiazole-2-carbonyl)-7-azaspiro[4.5]decan-10-yl)methyl)-*N*,*N*-dimethyl-6-oxo-4-phenyl-1,6-dihydropyridine-3-carboxamide;
1-((10-Hydroxy-7-(2-(trifluoromethyl)thiazole-4-carbonyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*S*)-4,4,4-trifluoro-2-(hydroxymethyl)butanoyl)-7-azaspiro[4.5]decan-10-yl)methyl)-*N*,*N*-dimethyl-6-oxo-4-phenyl-1,6-dihydropyridine-3-carboxamide;
1-(((*S*)-10-Hydroxy-7-((*S*)-4,4,4-trifluoro-2-(hydroxymethyl)butanoyl)-7-azaspiro[4.5]decan-10-yl)methyl)-5-(morpholine-4-carbonyl)-4-phenylpyridin-2(1*H*)-one;
1-(((*S*)-10-Hydroxy-7-((*S*)-4,4,4-trifluoro-2-(hydroxymethyl)butanoyl)-7-azaspiro[4.5]decan-10-yl)methyl)-4-phenyl-5-(piperazine-1-carbonyl)pyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-5-(morpholine-4-carbonyl)-4-phenylpyridin-2(1*H*)-one;
1-(((*S*)-10-Hydroxy-7-((*R*)-4,4,4-trifluoro-2-methylbutanoyl)-7-azaspiro[4.5]decan-10-yl)methyl)-5-(4-methylpiperazine-1-carbonyl)-4-phenylpyridin-2(1*H*)-one;
Ethyl 1-(((*S*)-7-((*R*)-3-cyclohexyl-2-methylpropanoyl)-10-hydroxy-7-azaspiro[4.5]decan-10-yl)methyl)-6-oxo-4-phenyl-1,6-dihydropyridine-3-carboxylate;
1-(((*S*)-7-((*R*)-3-Cyclohexyl-2-methylpropanoyl)-10-hydroxy-7-azaspiro[4.5]decan-10-yl)methyl)-6-oxo-4-phenyl-1,6-dihydropyridine-3-carboxylic acid;
1-(((*R*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-phenyl-5-(pyrrolidine-1-carbonyl)pyridin-2(1*H*)-one;
1-(((*R*)-1-((*S*)-3-Cyclohexyl-2-(methoxymethyl)propanoyl)-4-hydroxy-3,3-dimethylpiperidin-4-yl)methyl)-*N*,*N*-dimethyl-6-oxo-4-phenyl-1,6-dihydropyridine-3-carboxamide;
1-(((*R*)-4-Hydroxy-3,3-dimethyl-1-((*R*)-4,4,4-trifluoro-2-methylbutanoyl)piperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-(2-fluorophenyl)-*N*,*N-*dimethyl-6-oxo-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
(*R*)-1-((4-Hydroxy-3,3-dimethyl-1-(4,4,4-trifluoro-2-(2,2,2-trifluoroethyl)butanoyl)piperidin-4-yl)methyl)-*N*,*N*-dimethyl-6-oxo-4-phenyl-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-5-((*S*)-2-(hydroxymethyl)piperazine-1-carbonyl)-4-phenylpyridin-2(1*H*)-one;
1-(((*R*)-1-((*R*)-3-Cyclohexyl-2-methylpropanoyl)-4-hydroxy-3,3-dimethylpiperidin-4-yl)methyl)-5-((*R*)-3-methylpiperazine-1-carbonyl)-4-phenylpyridin-2(1*H*)-one;
1-(((*R*)-7-((*R*)-3-Cyclobutyl-2-methylpropanoyl)-10-hydroxy-7-azaspiro[4.5]decan-10-yl)methyl)-*N*,*N*-dimethyl-6-oxo-4-phenyl-1,6-dihydropyridine-3-carboxamide;
1-(((*R*)-7-((*R*)-3-Cyclobutyl-2-methylpropanoyl)-10-hydroxy-7-azaspiro[4.5]decan-10-yl)methyl)-4-phenyl-5-(piperazine-1-carbonyl)pyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-*N*,*N*-dimethyl-6-oxo-4-phenyl-1,6-dihydropyridine-3-carboxamide;
1-(((*R*)-10-Hydroxy-7-((*R*)-4,4,4-trifluoro-2-methylbutanoyl)-7-azaspiro[4.5]decan-10-yl)methyl)-4-phenyl-5-(piperazine-1-carbonyl)pyridin-2(1*H*)-one;
(*R*)-1-((10-Hydroxy-7-(4,4,4-trifluoro-2-(2,2,2-trifluoroethyl)butanoyl)-7-azaspiro[4.5]decan-10-yl)methyl)-*N,N-*dimethyl-6-oxo-4-phenyl-1,6-dihydropyridine-3-carboxamide;
(*R*)-1-((10-Hydroxy-7-(4,4,4-trifluoro-2-(2,2,2-trifluoroethyl)butanoyl)-7-azaspiro[4.5]decan-10-yl)methyl)-4-phenyl-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-4-cyclopropyl-*N,N*-dimethyl-6-oxo-1,6-dihydropyridine-3-carboxamide;
1-(((*R*)-1-((*R*)-3-Cyclohexyl-2-methylpropanoyl)-4-hydroxy-3,3-dimethylpiperidin-4-yl)methyl)-4-cyclopropyl-5-(piperazine-1-carbonyl)pyridin-2(1*H*)-one;
1-(((*R*)-7-((*R*)-3-Cyclobutyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
4-(2-Fluorophenyl)-1-(((*R*)-10-hydroxy-7-((*R*)-4,4,4-trifluoro-2-methylbutanoyl)-7-azaspiro[4.5]decan-10-yl)methyl)-*N,N*-dimethyl-6-oxo-1,6-dihydropyridine-3-carboxamide;
1-(((*R*)-7-((*R*)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
4-(2-Fluorophenyl)-1-(((*R*)-10-hydroxy-7-((*R*)-4,4,4-trifluoro-2-methylbutanoyl)-7-azaspiro[4.5]decan-10-yl)methyl)-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-4-(2-fluorophenyl)-*N*,*N*-dimethyl-6-oxo-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-one;
1-(((*R*)-1-((*R*)-3-Cyclohexyl-2-methylpropanoyl)-4-hydroxy-3,3-dimethylpiperidin-4-yl)methyl)-5-(4-hydroxypiperidine-1-carbonyl)-4-phenylpyridin-2(1*H*)-one;
1-(((*R*)-7-((*R*)-3-Cyclobutyl-2-methylpropanoyl)-10-hydroxy-7-azaspiro[4.5]decan-10-yl)methyl)-4-cyclopropyl-*N,N-*dimethyl-6-oxo-1,6-dihydropyridine-3-carboxamide;
1-(((*R*)-7-((*R*)-3-Cyclobutyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-4-cyclopropyl-*N,N*-dimethyl-6-oxo-1,6-dihydropyridine-3-carboxamide;
1-(((*R*)-7-((*R*)-3-Cyclohexyl-2-methylpropanoyl)-10-hydroxy-7-azaspiro[4.5]decan-10-yl)methyl)-4-cyclopropyl-5-(piperazine-1-carbonyl)pyridin-2(1*H*)-one;
1-(((*R*)-1-((*R*)-3-Cyclobutyl-2-methylpropanoyl)-4-hydroxy-3,3-dimethylpiperidin-4-yl)methyl)-4-(2-fluorophenyl)-*N,N-*dimethyl-6-oxo-1,6-dihydropyridine-3-carboxamide;
1-(((*R*)-1-((*R*)-3-Cyclobutyl-2-methylpropanoyl)-4-hydroxy-3,3-dimethylpiperidin-4-yl)methyl)-4-(2-fluorophenyl)-5-(piperazine-1-carbonyl)pyridin-2(1*H*)-one;
1-(((*R*)-7-((*R*)-3-Cyclohexyl-2-methylpropanoyl)-10-hydroxy-7-azaspiro[4.5]decan-10-yl)methyl)-5-(morpholine-4-carbonyl)-4-phenylpyridin-2(1*H*)-one;
1-(((*R*)-7-((*R*)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1*H*)-one;
1-(((*R*)-10-Hydroxy-7-((*S*)-4,4,4-trifluoro-2-(hydroxymethyl)butanoyl)-7-azaspiro[4.5]decan-10-yl)methyl)-*N,N-*dimethyl-6-oxo-4-phenyl-1,6-dihydropyridine-3-carboxamide;
1-(((*R*)-10-Hydroxy-7-((*S*)-4,4,4-trifluoro-2-(hydroxymethyl)butanoyl)-7-azaspiro[4.5]decan-10-yl)methyl)-5-(morpholine-4-carbonyl)-4-phenylpyridin-2(1*H*)-one;
1-(((*R*)-10-Hydroxy-7-((*S*)-4,4,4-trifluoro-2-(hydroxymethyl)butanoyl)-7-azaspiro[4.5]decan-10-yl)methyl)-4-phenyl-5-(piperazine-1-carbonyl)pyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-5-(morpholine-4-carbonyl)-4-phenylpyridin-2(1*H*)-one;
1-(((*R*)-10-Hydroxy-7-((*R*)-4,4,4-trifluoro-2-methylbutanoyl)-7-azaspiro[4.5]decan-10-yl)methyl)-5-(4-methylpiperazine-1-carbonyl)-4-phenylpyridin-2(1*H*)-one;
Ethyl 1-(((*R*)-7-((*R*)-3-cyclohexyl-2-methylpropanoyl)-10-hydroxy-7-azaspiro[4.5]decan-10-yl)methyl)-6-oxo-4-phenyl-1,6-dihydropyridine-3-carboxylate;
1-(((*R*)-7-((*R*)-3-Cyclohexyl-2-methylpropanoyl)-10-hydroxy-7-azaspiro[4.5]decan-10-yl)methyl)-6-oxo-4-phenyl-1,6-dihydropyridine-3-carboxylic acid;
3-((10-Hydroxy-7-((3*R*,4*R*)-3-phenylpiperidine-4-carbonyl)-7-azaspiro[4.5]decan-10-yl)methyl)-6-phenylpyrimidin-4(3*H*)-one;
3-(((*S*)-10-Hydroxy-7-((*R*)-4,4,4-trifluoro-2-methylbutanoyl)-7-azaspiro[4.5]decan-10-yl)methyl)-6-phenylpyrimidin-4(3*H*)-one;
6-(2-Fluorophenyl)-3-(((*S*)-10-hydroxy-7-((*R*)-4,4,4-trifluoro-2-methylbutanoyl)-7-azaspiro[4.5]decan-10-yl)methyl)pyrimidin-4(3*H*)-one;
6-(3-Fluorophenyl)-3-(((*S*)-10-hydroxy-7-((*R*)-4,4,4-trifluoro-2-methylbutanoyl)-7-azaspiro[4.5]decan-10-yl)methyl)pyrimidin-4(3*H*)-one;
3-(((*S*)-7-((*R*)-4,4-Difluoro-2-methylpentanoyl)-10-hydroxy-7-azaspiro[4.5]decan-10-yl)methyl)-6-phenylpyrimidin-4(3*H*)-one;
3-(((*S*)-10-Hydroxy-7-((3*S*,4*R*)-4-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((3*R*,4*S*)-4-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((2*R*,3*R*)-2-phenylpyrrolidine-3-carbonyl)-7-azaspiro[4.5]decan-10-yl)methyl)-6-phenylpyrimidin-4(3*H*)-one;
3-(((*S*)-10-Hydroxy-7-((2*S*,3*S*)-2-phenylpyrrolidine-3-carbonyl)-7-azaspiro[4.5]decan-10-yl)methyl)-6-phenylpyrimidin-4(3*H*)-one.

18. A pharmaceutical composition comprising a compound, pharmaceutically acceptable salt, tautomer, stereoisomer or *N-*oxide derivative as defined in any one of claims 1-17, for use in treating obesity, insulin resistance, or type II diabetes, optionally wherein said pharmaceutical composition further comprises a pharmaceutically acceptable diluent, excipient or carrier.

## Patentansprüche

1. Verbindung oder ein pharmazeutisch akzeptables Salz, Tautomer, Stereoisomer oder *N*-Oxid-Derivat davon zur Verwendung in einem Verfahren zur Behandlung von Fettleibigkeit, Insulinresistenz oder Typ-II-Diabetes, wobei die Verbindung eine Verbindung gemäß der Formel (I): 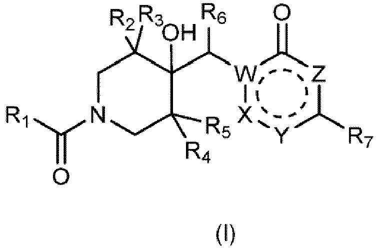 bei der:
R₁ eine optional substituierte Alkyl-, Alkenyl-, Alkynyl-, Ether-, Cycloalkyl-, Alkylcycloalkyl-, Heterocycloalkyl-, Alkylheterocycloalkyl-, Aryl-, Arylalkyl-, Heteroaryl- oder Heteroarylalkylgruppe ist;
R₂ H oder eine optional substituierte Alkylgruppe ist,
R₃ H oder eine optional substituierte Alkylgruppe ist,
R₄ H oder eine optional substituierte Alkylgruppe ist,
R₅ H oder eine optional substituierte Alkylgruppe ist,
R₂ und R₄ miteinander verbunden sein können, um ein optional substituiertes Cycloalkyl oder Heterocycloalkyl zu bilden, das den Kohlenstoff einschließt, an den sie gebunden sind,
R₄ und R₅ miteinander verbunden sein können, um ein optional substituiertes Cycloalkyl oder Heterocycloalkyl zu bilden, das das Kohlenstoffatom einschließt, an das sie gebunden sind;
R₆ H oder eine optional substituierte Alkylgruppe ist;
W C oder N ist;
X N oder CR₈ ist, wobei R₈ H oder optional substituiertes C1-C6-Alkyl ist,
Y N oder CR₉ ist;
Z CH oder NH ist,
wobei R₉ H oder optional substituiertes Alkyl, Amido, Amino, Aryl, Heteroaryl, Cycloalkyl, Cycloheteroalkyl, Halogen, Carbonyl, Ester, Aminoalkyl oder Cyano ist;
R₇ Wasserstoff, Halogen oder eine optional substituierte Alkyl-, Alkenyl-, Alkinyl-, Amino-, Aryl-, Cycloalkyl-, Cycloalkenyl-, Alkoxy-, Aryloxy-, Heteroaryl- oder Heterocycloalkylgruppe ist;
oder die Verbindung 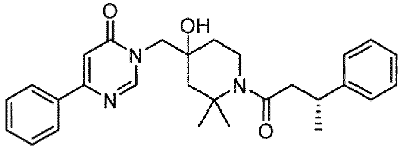
oder ein pharmazeutisch akzeptables Salz, Tautomer, Stereoisomer oder N Oxid-Derivat davon ist.

2. Verbindung, pharmazeutisch akzeptables Salz, Tautomer, Stereoisomer oder *N*-Oxid-Derivat zur Verwendung nach Anspruch 1, worin R₁ optional substituiertes C1-C6-Alkyl, optional substituiertes C4-C10-Alkylcycloalkyl, optional substituiertes C4-C5-Heterocycloalkyl, optional substituiertes C7-C10-Arylalkyl, optional substituiertes C3-C6-Cycloalkyl oder optional substituiertes C3-C6-Heteroaryl ist,
wobei jeder optionale Substituent unabhängig ausgewählt ist aus C1-C6-Alkyl, C2-C6-Alkenyl, Hydroxymethyl, Methoxymethyl, Benzyloxymethyl, Phenyl, C3-C6-Cycloalkyl, CF₃, CHF₂, OH oder Halogen.

3. Verbindung, pharmazeutisch akzeptables Salz, Tautomer, Stereoisomer oder *N*-Oxid-Derivat zur Verwendung nach Anspruch 1 oder Anspruch 2, worin R₁ optional substituiertes C7-C10-Cyclohexylalkan, optional substituiertes C4-C6-Cyclopropylalkan, optional substituiertes C5-C06-Cyclobutylalkan, optional substituiertes C7-C10-Alkylbenzol, optional substituiertes 2,2-Difluorbutan oder optional substituiertes 3, 3, 3-Trifluorpropan ist,
wobei jeder optionale Substituent unabhängig ausgewählt ist aus C1-C6-Alkyl, C2-C6-Alkenyl, Hydroxymethyl, Hydroxyethyl, Methoxymethyl, OH oder Halogen, wobei R₁ optional substituiert ist und der Substituent C1-C6-Alkyl, optional Methyl, ist.

4. Verbindung, pharmazeutisch akzeptables Salz, Tautomer, Stereoisomer oder *N*-Oxid-Derivat zur Verwendung nach einem der Ansprüche 1-3, wobei R₁ methylsubstituiertes Cyclohexylethan, methylsubstituiertes Ethylbenzol, methylsubstituiertes 2,2-Difluorbutan, methylsubstituiertes 3,3,3-Trifluorpropan, C4-C5-Heterocycloalkyl oder mit Phenyl substituiertes C4-C5-Heterocycloalkyl, optional phenylsubstituiertes Pyrrolidin oder phenylsubstituiertes Piperidin ist.

5. Verbindung, pharmazeutisch akzeptables Salz, Tautomer, Stereoisomer oder *N*-Oxid-Derivat zur Verwendung nach einem der Ansprüche 1-4, wobei R₇ optional substituiertes C6-C10-Aryl, C1-C12-Heteroaryl, C1-C10-Alkyl, C2-C10-Alkenyl, C3-C10-Cycloalkyl, Amino, C1-C3-Alkoxy oder Halogen ist,
wobei der optionale Substituent ausgewählt ist aus C1-C6-Alkyl, hydroxysubstituiertem C1-C6-Alkyl, C3-C6-Cycloalkyl, C1-C6-Heterocycloalkyl, C1-C6-Alkoxy, halogensubstituiertem C1-C6-Alkoxy, Amido, Cyano oder Halogen, wobei R₇ optional Cyclopropyl, Thiophen oder optional substituiertes Phenyl ist,
wobei der optionale Substituent aus C1-C6-Alkyl, hydroxysubstituiertem C1-C6-Alkyl, C1-C6-Alkylamin und Halogen ausgewählt ist, wobei R₇ optional Phenyl oder fluorsubstituiertes Phenyl ist.

6. Verbindung, pharmazeutisch akzeptables Salz, Tautomer, Stereoisomer oder *N*-Oxid-Derivat zur Verwendung nach einem der Ansprüche 1-5, worin R₂, R₃, R₄, R₅ unabhängig voneinander ausgewählt sind aus H und C1-C6-Alkyl, wobei optional R₂ und R₃ H sind und R₄ und R₅ Methyl sind.

7. Verbindung, pharmazeutisch akzeptables Salz, Tautomer, Stereoisomer oder *N*-Oxid-Derivat zur Verwendung nach einem der Ansprüche 1-5, wobei R₄ und R₅ miteinander verbunden sind, um ein optional substituiertes C3-C6-Cycloalkyl oder C3-C6-Heterocycloalkyl zu bilden, das den Kohlenstoff einschließt, an den sie gebunden sind, und R₂ und R₃ unabhängig voneinander aus H und C1-C6-Alkyl ausgewählt sind, wobei R₂ und R₃ optional H sind und R₄ und R₅ miteinander verbunden sind, um ein C3-C6-Cycloalkyl zu bilden, das den Kohlenstoff einschließt, an den sie gebunden sind, wobei optional R₄ und R₅ miteinander verbunden sind, um ein C4- oder C5-Cycloalkyl zu bilden, das den Kohlenstoff einschließt, an den sie gebunden sind.

8. Verbindung, pharmazeutisch akzeptables Salz, Tautomer, Stereoisomer oder *N*-Oxid-Derivat zur Verwendung nach einem der Ansprüche 1-7, wobei R₆ H oder C1-C6-Alkyl ist.

9. Verbindung, pharmazeutisch akzeptables Salz, Tautomer, Stereoisomer oder *N*-Oxid-Derivat zur Verwendung nach einem der Ansprüche 1-5, wobei R₂ und R₄ miteinander verbunden sind, um ein C5-Cycloalkyl zu bilden, das den Kohlenstoff einschließt, an die sie gebunden sind, und wobei R₃, R₅ und R₆ unabhängig voneinander H oder C1-C6-Alkyl sind.

10. Verbindung, pharmazeutisch akzeptables Salz, Tautomer, Stereoisomer oder *N*-Oxid-Derivat zur Verwendung nach einem der Ansprüche 1-9, wobei W N ist und X CR₈ ist, wobei R₈ H oder Methyl ist.

11. Verbindung, pharmazeutisch akzeptables Salz, Tautomer, Stereoisomer oder *N*-Oxid-Derivat zur Verwendung nach einem der Ansprüche 1-10, wobei Y N oder CR₉ ist, wobei R₉ H, C1-C6-Alkyl, NR'R", C(O)NR'R", Cyano, Carboxyl, Halogen, C1-C6-Alkylamin, C3-C6-Alkylester, optional substituiertes C6-C10-Aryl oder optional substituiertes C2-C6-Heteroaryl ist, wobei die ein oder mehreren Heteroatome aus N und O ausgewählt sind und die ein oder mehreren optionalen Substituenten des Aryls oder Heteroaryls aus C1-C6-Alkyl, C1-C6-Alkylamin, Amido und Cyano ausgewählt sind,
wobei R' und R" unabhängig voneinander ausgewählt sind aus H, optional mit OH substituiertem C1-C6-Alkyl, C3-C7-Cycloalkyl, C1-C7-Heterocycloalkyl, C4-C7-Alkylcycloalkyl, C3-C7-Alkylheterocycloalkyl, Benzyl, Phenyl und Methoxy, oder wobei R' und R" miteinander verbunden sind, um einen C2-C7-Heterocyclus zu bilden, der das N, an das sie gebunden sind, einschließt, wobei der Heterocyclus optional hydroxylsubstituiert, oxo-substituiert, methyl-substituiert, CH2OH-substituiert oder acetyl-substituiert ist.

12. Verbindung, pharmazeutisch akzeptables Salz, Tautomer, Stereoisomer oder *N*-Oxid-Derivat zur Verwendung nach einem der Ansprüche 1-11, wobei Y N oder CR₉ ist, wobei R₉ ausgewählt ist aus: Phenyl, optional substituiert mit Amido, Cyano oder Methylamin; Pyridin; Oxazol; Pyrazol; Carboxyl; C(O)NR'R"; oder NR'R";
wobei R' und R" unabhängig voneinander ausgewählt sind aus H, C1-C6-Alkyl, C3-C7-Cycloalkyl, C3-C7-Heterocycloalkyl, wobei das Heteroatom N oder O ist, oder wobei R' und R" miteinander verbunden sind, um ein C2-C7-Heterocycloalkyl zu bilden, das das N einschließt, an das sie gebunden sind, wobei das Heterocycloalkyl optional hydroxyl-substituiert, oxosubstituiert, methyl-substituiert, CH2OH-substituiert oder acetyl-substituiert ist.

13. Verbindung, pharmazeutisch akzeptables Salz, Tautomer, Stereoisomer oder *N*-Oxid-Derivat zur Verwendung nach Anspruch 1, wobei
R₁ ausgewählt ist aus optional substituiertem Ethylcyclohexan, optional substituiertem Ethylbenzol, optional substituiertem 2,2-Difluorbutan und optional substituiertem 3, 3, 3-Trifluorpropan, wobei der optionale Substituent einer oder mehrere von Methyl, Ethyl, Propyl, Propenyl, Hydroxymethyl und Methoxymethyl ist, oder wobei R₁ phenylsubstituiertes Pyrrolidin ist;
R₂ und R₃ unabhängig voneinander ausgewählt sind aus H, Methyl,
R₄ und R₅ unabhängig voneinander ausgewählt sind aus H, Methyl, oder miteinander verbunden sind, um ein C3-C6-Cycloalkyl oder Heterocycloalkyl zu bilden, das den Kohlenstoff einschließt, an den sie gebunden sind, optional ein C4- oder C5-Cycloalkyl, das den Kohlenstoff einschließt, an den sie gebunden sind,
R₆ H oder Methyl ist,
R₇ ausgewählt ist aus optional substituiertem Phenyl, Indazol, Thiophen oder Pyrazol, wobei der optionale Substituent ausgewählt ist aus C1-C6-Alkyl, hydroxysubstituiertem C1-C6-Alkyl, C3-C6-Cycloalkyl, C1-C6-Heterocycloalkyl, C1-C6-Alkoxy, halogensubstituiertem C1-C6-Alkoxy, C1-C6-Alkylamin, Amido, Cyano oder Halogen,
W N oder C ist,
X C H oder N ist,
Z C oder NH ist,
Y N oder CR₉ ist, wobei R₉ ausgewählt ist aus: Phenyl, optional substituiert mit Amido, Cyano oder Methylamin; Pyridin; Oxazol; Pyrazol; C(O)NR'R"; oder NR'R";
wobei R' und R" unabhängig voneinander ausgewählt sind aus H, C1-C6-Alkyl, C3-C7-Cycloalkyl, C3-C7-Heterocycloalkyl, wobei das Heteroatom N oder O ist, oder wobei R' und R" miteinander verbunden sind, um ein C2-C7-Heterocycloalkyl zu bilden, das das N einschließt, an das sie gebunden sind, wobei das Heterocycloalkyl optional hydroxylsubstituiert, oxosubstituiert, methylsubstituiert, hydroxymethylsubstituiert oder acetylsubstituiert ist.

14. Verbindung, pharmazeutisch akzeptables Salz, Tautomer, Stereoisomer oder *N*-Oxid-Derivat zur Verwendung nach einem der Ansprüche 11 bis 13, wobei Y CR₉ ist, wobei R₉ C(O)NR'R" ist und wobei R' und R" miteinander verbunden sind, um ein optional substituiertes Pyrrolidin, Piperidin, Piperazin oder Morpholin zu bilden, das das N einschließt, an das sie gebunden sind, wobei das Piperidin, Pyrrolidin, Piperazin oder Morpholin optional hydroxylsubstituiert, oxosubstituiert, methylsubstituiert, hydroxymethylsubstituiert oder acetylsubstituiert ist, optional wobei Y CR₉ ist, wobei R₉ C(O)NR'R" ist und wobei R' und R" miteinander verbunden sind, um einen Piperazinylring zu bilden.

15. Verbindung, pharmazeutisch akzeptables Salz, Tautomer, Stereoisomer oder *N*-Oxid-Derivat zur Verwendung nach einem der Ansprüche 1 bis 13, wobei W N ist und Y N ist, so dass der W, X, Y, Z umfassende Ring ein substituierter Pyrimidinonring ist.

16. Verbindung, pharmazeutisch akzeptables Salz, Tautomer, Stereoisomer oder *N*-Oxid-Derivat zur Verwendung nach einem der Ansprüche 1-15, wobei, wenn die Verbindung an der Hydroxylposition der Formel (I) chiral ist, die Verbindung das S-Enantiomer ist.

17. Verbindung oder pharmazeutisch akzeptables Salz, Tautomer, Stereoisomer oder *N*-Oxid-Derivat davon zur Verwendung nach Anspruch 1, wobei die Verbindung
(*R*)-5-Bromo-1-((4-hydroxy-1-(3-phenylbutanoyl)piperidin-4-yl)methyl)-4-phenylpyridin-2(1*H*)-on;
6-(2-Fluorphenyl)-3-((4-Hydroxy-1-(3-phenylpropanoyl)piperidin-4-yl)methyl)pyrimidin-4(3*H*)-on;
3-((1-(3-Cyclobutylpropanoyl)-4-hydroxypiperidin-4-yl)methyl)-6-(2-fluorphenyl)pyrimidin-4(3H)-on;
3-((1-(3-Cyclopentylpropanoyl)-4-hydroxypiperidin-4-yl)methyl)-6-(2-fluorphenyl)pyrimidin-4(3H)-on;
(*R,S*)-6-(2-Fluorphenyl)-3-((4-Hydroxy-1-(2-methyl-3-phenylproparioyl)piperidin-4-yl)methyl)pyrimidin-4(3*H*)-on;
(*R*)-*N-*(3-(1-((4-Hydroxy-1-(3-phenylbutanoyl)piperidin-4-yl)methyl)-6-oxo-4-phenyl-1,6-dihydropyridin-3-yl)phenyl)acetamid;
(*R*)-3-(1-((4-Hydroxy-1-(3-phenylbutanoyl)piperidin-4-yl)methyl)6-oxo-4-phenyl-1,6-dihydropyridin-3-yl)benzamid;
(*R*)-5-(Furan-2-yl)-1-((4-Hydroxy-1-(3-phenylbutanoyl)piperidin-4-yl)methyl)-4-phenylpyridin-2(1*H*)-on;
(*R*)-1-((4-Hydroxy-1-(3-phenylbutanoyl)piperidin-4-yl)methyl)-5-(1-methyl-1*H-*pyrazol-5-yl)-4-phenylpyridin-2(1*H*)-on;
(*R*)-4-Chlor-1-((4-hydroxy-1(3-phenylbutanoyl)piperidin-4-yl)methyl)-5-phenylpyridin-2(1*H*)-on;
(*R*)-4-(Dimethylamino)-1-((4-Hydroxy-1-(3-phenylbutanoyl)piperidin-4-yl)methyl)-5-phenylpyridin-2(1*H*)-on;
(*R*)-1-((4-Hydroxy-1-(3-phenylbutanoyl)piperidin-4-yl)methyl)-5-phenyl-4-(prop-1-en-2-yl)pyridin-2(1*H*)-on;
(*R*)-Ethyl-1-((4-hydroxy-1-(3-phenylbutanoyl)piperidin-4-yl)methyl)-6-oxo-4-phenyl-1,6-dihydropyridin-3-carboxylat;
(*R,S*)-4-(2-Fluorphenyl)-1-((4-hydroxy-1-(2-methyl-1,2,3,4-tetrahydronaphthalin-2-carbonyl)piperidin-4-yl)methyl)-*N-*isopropyl-*N*-methyl-6-oxo-1,6-dihydropyridin-3-carboxylat;
(*R*,*S*)-1-((1-(3-Cyclobutyl-2-methylpropanoyl)-4-hydroxypiperidin-4-yl)methyl)-4-phenyl-5-(piperidin-1-carbonyl)pyridin-2(1*H*)-on;
(*R*)-3-((4-Hydroxy-1-(3-phenylbutanoyl)piperidin-4-yl)methyl)-6-(l-methyl-1*H*-indazol-7-yl)pyrimidin-4(3*H*)-on;
(*R*)-3-((4-Hydroxy-1-(3-phenylbutanoyl)piperidin-4-yl)methyl)-6-(2-methoxyphenyl)pyrimidin-4(3*H*)-on;
(*R*)-3-((4-Hydroxy-1-(3-phenylbutanoyl)piperidin-4-yl)methyl)-2-methyl-6-phenylpyrimidin-4(3*H*)-on;
(*R*)-2-((4-Hydroxy-1-(3-phenylbutanoyl)piperidin-4-yl)methyl)-5,6-diphenylpyridazin-3(2*H*)-on;
(*R*)-3-((4-Hydroxy-1-(3-phenylbutanoyl)piperidin-4-yl)methyl)-6-(naphthalin-1-yl)pyrimidin-4(3*H*)-on;
(*R*)-6-(3-(1,3-Dioxolan-2-yl)phenyl)-3-((4-Hydroxy-1-(3-phenylbutanoyl)piperidin-4-yl)methyl)pyrimidin-4(3*H*)-on;
(*R*)-3-((4-Hydroxy-1-(3-phenylbutanoyl)piperidin-4-yl)methyl)-6-(thiophen-3-yl)pyrimidin-4(3*H*)-on;
4-(2-Fluorphenyl)-1-((4-Hydroxy-1-(1-methylcyclopentan-1-carbonyl)piperidin-4-yl)methyl)-*N-*isopropyl-*N-*methyl-6-oxo-1,6-dihydropyridin-3-carboxamid;
3-((1-(1-Ethylcyclohexan-1-carbonyl)-4-hydroxypiperidin-4-yl)methyl)-6-(2-Fluorphenyl)pyrimidin-4(3*H*)-on;
3-((1-(2-(Cyclohexylmethyl)-3-methylbutanoyl)-4-hydroxy-3,3-dimethylpiperidin-4-yl)methyl)-6-(2-fluorphenyl)pyrimidin-4(3*H*)-on;
(*R*)-6-(Furan-2-yl)-3-((4-Hydroxy-1-(3-phenylbutanoyl)piperidin-4-yl)methyl)pyrimidin-4(3*H*)-on;
(*R*)-1-((4-Hydroxy-1-(3-phenylbutanoyl)piperidin-4-yl)methyl)-4-phenylpyridin-2(1*H*)-on;
(*R*)-6-(Cyclohex-1-en-1-yl)-3-((4-Hydroxy-1-(3-phenylbutanoyl)piperidin-4-yl)methyl)pyrimidin-4(3*H*)-on;
(*R*)-3-((4-Hydroxy-1-(3-phenylbutanoyl)piperidin-4-yl)methyl)-6-(prop-1-en-2-yl)pyrimidin-4(3*H*)-on;
(*R*)-3-((4-Hydroxy-1-(3-phenylbutanoyl)piperidin-4-yl)methyl)-6-(2-(trifluoromethoxy)phenyl)pyrimidin-4(3*H*)-on;
3-(((1*R*,5*S*)-3-((*R*)-3-Cyclohexyl-2-methylpropanoyl)-8-hydroxy-3-azabicyclo[3.2.1]octan-8-yl)methyl)-6-(2-fluorphenyl)pyrimidin-4(3*H*)-on;
(*R*)-6-(2-Fluorphenyl)-3-((4-Hydroxy-1-(3-phenylbutanoyl)piperidin-4-yl)methyl)pyrimidin-4(3*H*)-on;
(*R*)-5-((4-Hydroxy-1-(3-phenylbutanoyl)piperidin-4-yl)methyl)-2-phenylpyrimidin-4(3*H*)-on;
3-((4-Hydroxy-1-(3-phenylpropanoyl)piperidin-4-yl)methyl)-2-methyl-6-phenylpyrimidin-4(3*H*)-on;
(*R*)-3-((4-Hydroxy-1-(3-phenylbutanoyl)piperidin-4-yl)methyl)-6-(isobutylamino)pyrimidin-4(3*H*)-on;
(*R*)-1-((1-(3-Cyclohexyl-2-methylpropanoyl)-4-hydroxypiperidin-4-yl)methyl)-4-phenyl-5-(piperidin-1 - carbonyl)pyridin-2(1*H*)-on;
(*R*)-2-((4-Hydroxy-1-(3-phenylbutanoyl)piperidin-4-yl)methyl)-5-phenylpyridazin-3(2*H*)-on;
(*R*)-4-(2-Cyanophenyl)-1-((4-Hydroxy-1-(3-phenylbutanoyl)piperidin-4-yl)methyl)-*N*-isopropyl-*N*-methyl-6-oxo-1,6-dihydropyridin-3-carboxamid;
(*R*)-3-((1-(3-Cyclohexyl-2-methylpropanoyl)-4-hydroxypiperidin-4-yl)methyl)-6-(2-fluorphenyl)pyrimidin-4(3*H*)-on;
3-((1-(2-(Cyclohexylmethyl)butanoyl)-4-hydroxypiperidin-4-yl)methyl)-6-(2-fluorphenyl)pyrimidin-4(3*H*)-on;
*rac*-6-(2-Fluorphenyl)-3-((4-Hydroxy-1-(*cis*-2-phenylcyclopropancarbonyl)piperidin-4-yl)methyl)pyrimidin-4(3*H*)-on;
6-(2-Fluorphenyl)-3-((4-Hydroxy-1-(1-methylcyclohexancarbonyl)piperidin-4-yl)methyl)pyrimidin-4(3*H*)-on;
3-((*S*)-1-(1-((*R*)-3-Cyclohexyl-2-methylpropanoyl)-4-hydroxypiperidin-4-yl)propyl)-6-phenylpyrimidin-4(3*H*)-on;
3-((*R*)-1-(1-((*R*)-3-Cyclohexyl-2-methylpropanoyl)-4-hydroxypiperidin-4-yl)propyl)-6-phenylpyrimidin-4(3*H*)-on;
(*R,S*)-3-((1-(3-Cyclohexyl-2-hydroxypropanoyl)-4-hydroxypiperidin-4-yl)methyl)-6-(2-fluorphenyl)pyrimidin-4(3*H*)-on;
(*R*)-1-((4-Hydroxy-1-(3-phenylbutanoyl)piperidin-4-yl)methyl)-6-oxo-4-phenyl-1,6-dihydropyridin-3-carbaldehyd;
(*R*)-5-((Dimethylamino)methyl)-1-((4-Hydroxy-1-(3-phenylbutanoyl)piperidin-4-yl)methyl)-4-phenylpyridin-2(1*H*)-on;
3-((*R*)- 1-(4-Hydroxy-1-((*R*)-3-phenylbutanoyl)piperidin-4-yl)ethyl)-6-phenylpyrimidin-4(3*H*)-on;
3-((*S*)-1-(4- Hydroxy-1-((*R*)-3-phenylbutanoyl)pipendin-4-yl)ethyl)-6-phenylpyrimidin-4(3*H*)-on;
(*R*)-6-(2-Fluorphenyl)-3-((4-Hydroxy-1-(4,4,4-trifluoro-3-phenylbutanoyl)piperidin-4-yl)methyl)pyrimidin-4(3*H*)-on;
(*R*,*S*)-3-((1-(3-Cyclohexyl-2-fluorpropanoyl)-4-hydroxypiperidin-4-yl)methyl)-6-(2-fluorphenyl)pyrimidin-4(3*H*)-on;
3-(((*R*)-1-((*R*)-3-Cyclohexyl-2-methylpropanoyl)-4-hydroxy-3,3-dimethylpiperidin-4-yl)methyl)-6-phenylpyrimidin-4(3*H*)-on;
3-(((*S*)-1-((*R*)-3-Cyclohexyl-2-methylpropanoyl)-4-hydroxy-3,3-dimethylpiperidin-4-yl)methyl)-6-phenylpyrimidin-4(3*H*)-on;
3-(((*R*)-6-((*R*)-3-Cyclohexyl-2-methylpropanoyl)-9-hydroxy-6-azaspiro[3.5]nonan-9-yl)methyl)-6-(2-fluorphenyl)pyrimidin-4(3*H*)-on;
3-(((*S*)-6-((*R*)*-*3-Cyclohexyl-2-methylpropanoyl)-9-hydroxy-6-azaspiro[3.5]nonan-9-yl)methyl)-6-(2-fluorphenyl)pyrimidin-4(3*H*)-on;
(*R*)-5-(3-(Aminomethyl)phenyl)-1-((1-(3-Cyclohexyl-2-methylpropanoyl)-4-hydroxypiperidin-4-yl)methyl)-4-(2-fluorphenyl)pyridin-2(1*H*)-on;
3-(((*S*)-4-Hydroxy-3,3-dimethyl-1-((*R*)-3-phenylbutanoyl)piperidin-4-yl)methyl)-6-phenylpyrimidin-4(3*H*)-on;
3-(((*R*)-4-Hydroxy-3,3<limethyl-1-((*R*)-3-phenylbutanoyl)piperidin-4-yl)methyl)-6-phenylpyrimidin-4(3*H*)-on;
(*R*)-6-(4-Fluorphenoxy)-3-((4-Hydroxy-1-(3-phenylbutanoyl)piperidin-4-yl)methyl)pyrimidin-4(3*H*)-on;
(*R*)-1-((4-Hydroxy-1-(3-phenylbutanoyl)piperidin-4-yl)methyl)-5-(2-oxopyrrolidin-1-yl)-4-phenylpyridin-2(1*H*)-on;
6-(2-Fluorphenyl)-3-((4-Hydroxy-1-(1-methyl-4-methylencyclohexancarbonyl)piperidin-4-yl)methyl)pyrimidin-4(3*H*)-on;
(*R*,*S*)-3-((1-(3-Cyclobutyl-2-methylpropanoyl)-4-hydroxypiperidin-4-yl)methyl)-6-(2-fluorphenyl)pyrimidin-4(3*H*)-on;
(*R*,*S*)-3-((1-(3-Cycloheptyl-2-methylpropanoyl)-4-hydroxypiperidin-4-yl)methyl)-6-(2-fluorphenyl)pyrimidin-4(3*H*)-on;
3-((1-(3-Cyclobutyl-2,2-dimethylpropanoyl)-4-hydroxypiperidin-4-yl)methyl)-6-(2-fluorphenyl)pyrimidin-4(3*H*)-on;
6-(2-Fluorphenyl)-3-(((1*R*,5*S*)-8-Hydroxy-3-(*H*3)-3-phenylbutanoyl)-3-azabicyclo[3.2.1]octan-8-yl)methyl)pyrimidin-4(3*H*)-on;
3-(((*R*)-4-Hydroxy-2,2-dimethyl-1-((*R*)-3-phenylbutanoyl)piperidin-4-yl)methyl)-6-phenylpyrimidin-4(3*H*)-on;
3-(((*S*)-4-Hydroxy-2,2-dimethyl-1-((*N*)-3-phenylbutanoyl)piperidin-4-yl)methyl)-6-phenylpyrimidin-4(3*H*)-on;
(*R*)-1-((4-Hydroxy-1-(3-phenylbutanoyl)piperidin-4-yl)methyl)-5-(4-hydroxypiperidin-1-carbonyl)-4-phenylpyridin-2(1*H*)-on;
(*R*)-1-((4-Hydroxy-1-(3-phenylbutanoyl)piperidin-4-yl)methyl)-4-pheriyl-5-(pyrrolidirie-1-carbonyl)pyridin-2(1*H*)-on;
(*R*)-1-((4-Hydroxy-1-(3-phenylbutanoyl)piperidin-4-yl)methyl)-4-phenyl-5-(1,4-dioxa-8-azaspiro[4.5]decane-8-carbonyl)pyridin-2(1*H*)-on;
(*R*)-*N-*Cyclohexyl-1-((4-hydroxy-1-(3-phenylbutanoyl)piperidin-4-yl)methyl)-6-oxo-4-phenyl-1,6-dihydropyridin-3-carboxamid;
6-(2-Fluorphenyl)-3-((4-Hydroxy-3,3-dimethyl-1-(cis-2-phenylcyclopropan-1-carbonyl)piperidin-4-yl)methyl)pyrimidin-4(3*H*)-on;
(*R,S*)-3-((1-(3-Cyclohexyl-1*H*-pyrazole-4-carbonyl)-4-hydroxy-3,3-dimethylpiperidin-4-yl)methyl)-6-(2-fluorophenyl)pyrimidin-4(3*H*)-on;
(*R*,*S*)-6-(2-Fluorphenyl)-3-((4-Hydroxy-3,3-dimethyl-1-(3-phenylproparioyl)piperidin-4-yl)methyl)pyrimidin-4(3*H*)-on;
(*R*,*S*)-6-(2-Fluorphenyl)-3-((4-Hydroxy-1-(1-isobutylcyclopropan-1-carbonyl)-3,3-dimethylpiperidin-4-yl)methyl)pyrimidin-4(3*H*)-on;
(*R,S*)-6-(2-Fluorphenyl)-3-((4-hydroxy-3,3-dimethyl-1-(3-methyl-3-phenylbutanoyl)piperidin-4-yl)methyl)pyrimidin-4(3*H*)-on;
(*S*)-1-((1-(4,4-Difluor-3-phenylbutanoyl)-4-hydroxypiperidin-4-yl)methyl)-*N*-isopropyl-*N*-methyl-6-oxo-4-phenyl-1,6-dihydropyridin-3-carboxamid;
1-((4-Hydroxy-1-(1-(thiophen-2-yl)cyclopropan-1 - carbonyl)piperidin-4-yl)methyl)-*N*-isopropyl-*N*-methyl-6-oxo-4-phenyl-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridin-3-carboxamid;
(*R*)-3-((4-Hydroxy-1-(3-phenylbutanoyl)piperidin-4-yl)methyl)-6-(2-(hydroxymethyl)phenyl)pyrimidin-4(3*H*)-on;
(*R*)-*N*,*N*-Diethyl-1-((4-hydroxy-1-(3-phenylbutanoyl)piperidin-4-yl)methyl)-6-oxo-4-phenyl-1,6-dihydropyridin-3-carboxamid;
3-((1-(3-Cyclohexylbutanoyl)-4-hydroxy-3,3-dimethylpiperidin-4-yl)methyl)-6-(2-fluorphenyl)pyrimidin-4(3*H*)-on;
(*R*)-1-((4-Hydroxy-1-(3-phenylbutanoyl)piperidin-4-yl)methyl)-5-(morpholin-4-carbonyl)-4-phenylpyridin-2(1*H*)-on;
(*R*,*S*)-3-((1-(3-Cyclohexylpropanoyl)-4-hydroxy-3,3-dimethylpiperidin-4-yl)methyl)-6-(2-fluorphenyl)pyrimidin-4(3*H*)-on;
(*R*,*S*)-1-((1-(3-Cyclobutylpropanoyl)-4-hydroxy-3,3-dimethylpiperidin-4-yl)methyl)-*N*-isopropyl-*N*-methyl-6-oxo-4-phenyl-1,6-dihydropyridin-3-carboxamid;
(*R*,*S*)-1-((1-(2-Ethylhexanoyl)-4-hydroxypiperidin-4-yl)methyl)-*N*-isopropyl-*N*-methyl-6-oxo-4-phenyl-1,6-dihydropyridin-3-carboxamid;
(*R*,*S*)-6-(2-Fluorphenyl)-3-((4-Hydroxy-3,3-dimethyl-1-(1-methylcyclohexan-1-carbonyl)piperidin-4-yl)methyl)pyrimidin-4(3*H*)-on;
6-(2-Fluorphenyl)-3-((1-(3-(4-fluorphenyl)propanoyl)-4-hydroxypiperidin-4-yl)methyl)pyrimidin-4(3*H*)-on;
(*R*,*S*)-3-((1-(3-Cyclopropylpropanoyl)-4-hydroxy-3,3-dimethylpiperidin-4-yl)methyl)-6-(2-fluorphenyl)pyrimidin-4(3*H*)-on;
1-((1-(2,2-Dimethylbutanoyl)-4-hydroxypiperidin-4-yl)methyl)-*N*-isopropyl-*N*-methyl-6-oxo-4-phenyl-1,6-dihydropyridin-3-carboxamid;
(*R*)*-N-*(2-(1-((4-Hydroxy-1-(3-phenylbutanoyl)piperidin-4-yl)methyl)-6-oxo-1,6-dihydropyrimidin-4-yl)phenyl)acetamid;
(*R*)-5-(4-(Aminomethyl)phenyl)-1-((4-hydroxy-1-(3-phenylbutanoyl)piperidin-4-yl)methyl)-4-phenylpyridin-2(1*H*)-on;
(*R*)-1-((1-(3-Cyclohexyl-2-methylpropanoyl)-4-hydroxypiperidin-4-yl)methyl)-*N*-isopropyl-*N*-methyl-6-oxo-4-phenyl-1,6-dihydropyridin-3-carboxamid;
(*R*)-1-((4-Hydroxy-1-(3-phenylbutanoyl)piperidin-4-yl)methyl)-4-phenyl-5-(piperidin-1-carbonyl)pyridin-2(1*H*)-on;
(*R*)-1-((4-Hydroxy-1-(3-phenylbutanoyl)piperidin-4-yl)methyl)-*N*-isopropyl-4-(2-methoxyphenyl)-*N*-methyl-6-oxo-1,6-dihydropyridin-3-carboxamid;
(*R*)-4-(2-Fluorphenyl)-1-((4-hydroxy-1-(3-phenylbutanoyl)piperidin-4-yl)methyl)-*N*-isopropyl-*N*-methyl-6-oxo-1,6-dihydropyridin-3-carboxamid;
(*R*)-1'-((4-Hydroxy-1-(3-phenylbutanoyl)piperidin-4-yl)methyl)-4'-phenyl-[2,3'-bipyridin]-6'(1'*H*)-on;
1-((1-(3-Cyclohexylpropanoyl)-4-hydroxypiperidin-4-yl)methyl)-*N*-isopropyl-*N*-methyl-6-oxo-4-phenyl-1,6-dihydropyridin-3-carboxamid;
(*R*)-tert-Butyl-4-(1-((4-Hydroxy-1-(3-phenylbutanoyl)piperidin-4-yl)methyl)-6-oxo-4-phenyl-1,6-dihydropyridin-3-carbonyl)piperazin-1 -carboxylat;
(*R*)-1-((4-Hydroxy-1-(3-phenylbutanoyl)piperidin-4-yl)methyl)-4-phenyl-5-(piperazin-1-carbonyl)pyridin-2(1*H*)-on;
(*R*)-1-((4-Hydroxy-1-(3-phenylbutanoyl)piperidin-4-yl)methyl)-*N*,*N*-dimethyl-6-oxo-4-phenyl-1,6-dihydropyridin-3-carboxamid;
(*R*)-1-((4-Hydroxy-1-(3-phenylbutanoyl)piperidin-4-yl)methyl)-*N*-isopropyl-*N*-methyl-6-oxo-4-(p-tolyl)-1,6-dihydropyridin-3-carboxamid;
(*R*)-1-((4-Hydroxy-1-(3-phenylbutanoyl)piperidin-4-yl)methyl)-*N-*methoxy-*N-*methyl-6-oxo-4-phenyl-1,6-dihydropyridin-3-carboxamid;
(*R*)-1-((4-Hydroxy-1-(3-phenylbutanoyl)piperidin-4-yl)methyl)-4,5-dipheriylpyridin-2(1*H*)-on;
(*R*)-1-((4-Hydroxy-1-(3-phenylbutanoyl)piperidin-4-yl)methyl)-4-phenyl-[3,3'-bipyridin]-6(1*H*)-on;
(*R*)-5-(3-(Aminomethyl)phenyl)-1-((4-hydroxy-1-(3-phenylbutanoyl)piperidin-4-yl)methyl)-4-phenylpyridin-2(1*H*)-on;
(*R*)-1-((4-Hydroxy-1-(3-phenylbutanoyl)piperidin-4-yl)methyl)-*N*-isopropyl-*N*-methyl-6-oxo-4-phenyl-1,6-dihydropyridin-3-carboxamid;
(*R*)-1-((4-Hydroxy-1-(3-phenylbutanoyl)piperidin-4-yl)methyl)-*N*-isopropyl-*N*-methyl-6-oxo-4-(m-tolyl)-1,6-dihydropyridin-3-carboxamid;
(*R*)-1-((4-Hydroxy-1-(3-phenylbutanoyl)piperidin-4-yl)methyl)-4-phenyl-5-(pyrimidin-5-yl)pyridin-2(1*H*)-on;
(*R*)-*N*-(Cyclopropylmethyl)-1-((4-Hydroxy-1-(3-phenylbutanoyl)piperidin-4-yl)methyl)-6-oxo-4-phenyl-1,6-dihydropyridin-3-carboxamid;
(*R*)-*N*-Benzyl-1-((4-hydroxy-1-(3-phenylbutanoyl)piperidin-4-yl)methyl)-*N*-methyl-6-oxo-4-phenyl-1,6-dihydropyridin-3-carboxamid;
(*R*)-2-(1-((4-Hydroxy-1-(3-phenylbutanoyl)piperidin-4-yl)methyl)-6-oxo-4-phenyl-1,6-dihydropyridin-3-yl)benzonitril;
(*R*)-1-((4-Hydroxy-1-(3-phenylbutanoyl)piperidin-4-yl)methyl)-*N*-methyl-6-oxo-*N*,4-diphenyl-1,6-dihydropyridin-3-carboxamid;
3-(1-(1-((*R*)-3-Cyclohexyl-2-methylpropanoyl)-4-hydroxypiperidin-4-yl)ethyl)-6-phenylpyrimidin-4(3*H*)-on;
(*R*)-1-((4-Hydroxy-1-(3-phenylbutanoyl)piperidin-4-yl)methyl)-5-(1-methyl-1H-pyrazol-4-yl)-4-phenylpyridin-2(1*H*)-on;
(*R*)-3-((1-(2-(Cyclohexylmethyl)pent-4-enoyl)-4-hydroxypiperidin-4-yl)methyl)-6-(2-fluorphenyl)pyrimidin-4(3*H*)-on;
(*R*)-1-((4-Hydroxy-1-(3-phenylbutanoyl)piperidin-4-yl)methyl)-5-methyl-4-phenylpyridin-2(1*H*)-on;
(*R*)-6-(1,5-Dimethyl-1*H*-pyrazol-4-yl)-3-((4-Hydroxy-1-(3-phenylbutanoyl)piperidin-4-yl)methyl)pyrimidin-4(3*H*)-on;
3-(((1*R*,5*S*)-3-(3-Cyclohexylpropanoyl)-8-hydroxy-3-azabicyclo[3.2.1]octan-8-yl)methyl)-6-(2-fluorphenyl)pyrimidin-4(3*H*)-on;
(*R*)-1-((4-Hydroxy-1-(3-phenylbutanoyl)piperidin-4-yl)methyl)-6-oxo-4-phenyl-1,6-dihydropyridin-3-carbonitril;
(*R*)-1-((4-Hydroxy-1-(3-phenylbutanoyl)piperidin-4-yl)methyl)-*N*-(2-hydroxyethyl)-6-oxo-4-phenyl-1,6-dihydropyridin-3-carboxamid;
1-((4-Hydroxy-1-((*R*)-3-phenylbutanoyl)piperidin-4-yl)methyl)-5-((*S*)-2-methylpyrrolidin-1-carbonyl)-4-phenylpyridin-2(1*H*)-on;
(*R*)-1-((4-Hydroxy-1-(3-phenylbutanoyl)piperidin-4-yl)methyl)-5-(4-(hydroxymethyl)piperidin-1-carbonyl)-4-phenylpyridin-2(1*H*)-on;
3-((1-(2-(Cyclohexylmethyl)butanoyl)-4-hydroxy-3,3-dimethylpiperidin-4-yl)methyl)-6-(2-fluorphenyl)pyrimidin-4(3*H*)-on;
3-((7-((*R*)-3-Cyclohexyl-2-methylpropanoyl)-10-hydroxy-7-azaspiro[4.5]decan-10-yl)methyl)-6-(2-fluorphenyl)pyrimidin-4(3*H*)-on;
1-(((*S*)-1-((*R*)-3-Cyclohexyl-2-methylpropanoyl)-4-hydroxy-3,3-dimethylpiperidin-4-yl)methyl)-*N*-isopropyl-*N*-methyl-6-oxo-4-phenyl-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-*N,N*-dimethyl-6-oxo-4-phenyl-1,6-dihydropyridin-3-carboxamid;
1-(((*R*)-1-((R)-3-Cyclohexyl-2-methylpropanoyl)-4-hydroxy-3,3-dimethylpiperidin-4-yl)methyl)-*N,N*-dimethyl-6-oxo-4-phenyl-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-4-phenyl-5-(pyrrolidin-1-carbonyl)pyridin-2(1*H*)-on;
1-(((*S*)-1-((*R*)-3-Cyclohexyl-2-methylpropanoyl)-4-hydroxy-3,3-dimethylpiperidin-4-yl)methyl)-4-phenyl-5-(piperazin-1-carbonyl)pyridin-2(1*H*)-on;
1-(((*R*)-1-((*R*)-3-Cyclohexyl-2-methylpropanoyl)-4-hydroxy-3,3-dimethylpiperidin-4-yl)methyl)-4-phenyl-5-(piperazin-1 - carbonyl)pyridin-2(1*H*)-on;
3-((1-(2-(Cyclohexyloxy)propanoyl)-4-hydroxy-3,3-dimethylpiperidin-4-yl)methyl)-6-(2-fluorphenyl)pyrimidin-4(3*H*)-on;
1-((7-((*R*)-3-Cyclohexyl-2-methylpropanoyl)-10-hydroxy-7-azaspiro[4.5]decan-10-yl)methyl)-*N,N*-dimethyl-6-oxo-1,6-dihydropyridin-3-carboxamid;
1-((1-((*R*)-3-Cyclobutyl-2-methylpropanoyl)-4-hydroxy-3,3-dimethylpiperidin-4-yl)methyl)-*N,N*-dimethyl-6-oxo-4-phenyl-1,6-dihydropyridin-3-carboxamid;
1-((1-((*R*)-3-Cyclopropyl-2-methylpropanoyl)-4-hydroxy-3,3-dimethylpiperidin-4-yl)methyl)-*N,N*-dimethyl-6-oxo-4-phenyl-1,6-dihydropyridin-3-carboxamid;
1-((1-((*R*)-3-Cyclohexyl-2-methylpropanoyl)-4-hydroxy-3,3-dimethylpiperidin-4-yl)methyl)-[4,5'-bipyrimidin]-6(1*H*)-on;
3-((1-((*R*)-3-Cyclohexyl-2-methylpropanoyl)-4-hydroxy-3,3-dimethylpiperidin-4-yl)methyl)-6-(1-methyl-1*H*-pyrazol-5-yl)pyrimidin-4(3*H*)-on;
3-((1-((*R*)-3-Cyclohexyl-2-methylpropanoyl)-4-hydroxy-3,3-dimethylpiperidin-4-yl)methyl)-6-(1*H*-pyrazol-4-yl)pyrimidin-4(3*H*)-on;
3-((1-((*R*)-3-Cyclohexyl-2-methylpropanoyl)-4-hydroxy-3,3-dimethylpiperidin-4-yl)methyl)-6-(1*H*-pyrazol-5-yl)pyrimidin-4(3*H*)-on;
3-((7-((*R*)-3-Cyclohexyl-2-methylpropanoyl)-10-hydroxy-7-azaspiro[4.5]decan-10-yl)methyl)pyrimidin-4(3*H*)-on;
3-((1-((*H*)-3-Cyclohexyl-2-methylpropanoyl)-4-hydroxy-3,3-dimethylpiperidin-4-yl)methyl)-6-(thiophen-3-yl)pyrimidin-4(3*H*)-on;
1-((1-((*R*)-3-Cyclohexyl-2-methylpropanoyl)-4-hydroxy-3,3-dimethylpiperidin-4-yl)methyl)-5-(oxazol-2-yl)-4-phenylpyridin-2(1*H*)-on;
3-((1-((*R*)-3-Cyclohexyl-2-methylpropanoyl)-4-hydroxy-3,3-dimethylpiperidin-4-yl)methyl)-6-(2-(hydroxymethyl)phenyl)pyrimidin-4(3*H*)-on;
3-((1-((*R*)-3-Cyclohexyl-2-methylpropanoyl)-4-hydroxy-3,3-dimethylpiperidin-4-yl)methyl)-6-(3-(hydroxymethyl)phenyl)pyrimidin-4(3*H*)-on;
3-((1-((F)-3-Cyclohexyl-2-methylpropanoyl)-4-hydroxy-3,3-dimethylpiperidin-4-yl)methyl)-6-(4-(hydroxymethyl)phenyl)pyrimidin-4(3*H*)-on;
6-(4-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-on;
6-(2-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3*H*)-on;
6-(2-Fluorphenyl)-3-((4-Hydroxy-3,3-dimethyl-1-(2,4,4-trimethylpentanoyl)piperidin-4-yl)methyl)pyrimidin-4(3*H*)-on;
6-(2-Fluorphenyl)-3-((4-Hydroxy-3,3-dimethyl-1-(4,4,4-trifluoro-2-methylbutanoyl)piperidin-4-yl)methyl)pyrimidin-4(3*H*)-on;
4-Chloro- 1-((7-((*R*)-3-cyclohexyl-2-methylpropanoyl)-10-hydroxy-7-azaspiro[4.5]decan-10-yl)methyl)-*N,N*-dimethyl-6-oxo-1,6-dihydropyridin-3-carboxamid;
1-((7-((*R*)-3-Cyclohexyl-2-methylpropanoyl)-10-hydroxy-7-azaspiro[4.5]decan-10-yl)methyl)-4-(dimethylamino)-*N*,*N-*dimethyl-6-oxo-1,6-dihydropyridin-3-carboxamid;
1-((1-((*S*)-3-(Benzyloxy)-2-(cyclohexylmethyl)propanoyl)-4-hydroxy-3,3-dimethylpiperidin-4-yl)methyl)-*N,N*-dimethyl-6-oxo-4-phenyl-1,6-dihydropyridin-3-carboxamid;
1-((10-Hydroxy-7-isobutyryl-7-azaspiro[4.5]decan-10-yl)methyl)-*N,N-*dimethyl-6-oxo-4-phenyl-1,6-dihydropyridin-3-carboxamid;
1-((10-Hydroxy-7-((*R*)-2-methylbutanoyl)-7-azaspiro[4.5]decan-10-yl)methyl)-*N,N*-dimethyl-6-oxo-4-phenyl-1,6-dihydropyridin-3-carboxamid;
1-((7-((*R*)-3-Cyclopropyl-2-methylpropanoyl)-10-hydroxy-7-azaspiro[4.5]decan-10-yl)methyl)-*N*,*N*-dimethyl-6-oxo-4-phenyl-1,6-dihydropyridin-3-carboxamid;
6-(2-Fluorophenyl)-3-((4-hydroxy-3,3-dimethyl-1-(2-methyl-3-(1*H*-pyrazol-1-yl)propanoyl)piperidin-4-yl)methyl)pyrimidin-4(3*H*)-on;
1-((7-((*R*)-3-Cyclohexyl-2-methylpropanoyl)-10-hydroxy-7-azaspiro[4.5]decan-10-yl)methyl)-4-(2-(hydroxymethyl)phenyl)-*N,N-*dimethyl-6-oxo-1,6-dihydropyridin-3-carboxamid;
1-(((*S*)-1-((*S*)-3-Cyclohexyl-2-(hydroxymethyl)propanoyl)-4-hydroxy-3,3-dimethylpiperidin-4-yl)methyl)-*N,N*-dimethyl-6-oxo-4-phenyl-1,6-dihydropyridin-3-carboxamid;
1-(((*R*)-1-((51-3-Cyclohexyl-2-(hydroxymethyl)propanoyl)-4-hydroxy-3,3-dimethylpiperidin-4-yl)methyl)-*N,N*-dimethyl-6-oxo-4-phenyl-1,6-dihydropyridin-3-carboxamid;
1'-((1-((*R*)-3-Cyclohexyl-2-methylpropanoyl)-4-hydroxy-3,3-dimethylpiperidin-4-yl)methyl)-4'-(2-fluorophenyl)-[2,3'-bipyridin]-6'(1'(1'*H*)-on;
1-((7-((*R*)-3-Cyclohexyl-2-methylpropanoyl)-10-hydroxy-7-azaspiro[4.5]decan-10-yl)methyl)-*N,N*-dimethyl-6-oxo-4-(thiophen-3-yl)-1,6-dihydropyridin-3-carboxamid;
1-((7-((F)-3-Cyclohexyl-2-methylpropanoyl)-10-hydroxy-7-azaspiro[4.5]decan-10-yl)methyl)-4-(3-(hydroxymethyl)phenyl)-*N,N-*dimethyl-6-oxo-1,6-dihydropyridin-3-carboxamid;
1-((7-(3-Cyclohexyl-2-hydroxypropanoyl)-10-hydroxy-7-azaspiro[4.5]decan-10-yl)methyl)-*N,N*-dimethyl-6-oxo-4-phenyl-1,6-dihydropyridin-3-carboxamid;
1-((10-Hydroxy-7 -(2-methyl-3-(piperidin-1-yl)propanoyl)-7-azaspiro[4.5]decan-10-yl)methyl)-*N,N*-dimethyl-6-oxo-4-phenyl-1,6-dihydropyridin-3-carboxamid;
6-(3-(Aminomethyl)phenyl)-3-((1-((*R*)-3-cyclohexyl-2-methylpropanoyl)-4-hydroxy-3,3-dimethylpiperidin-4-yl)methyl)pyrimidin-4(3H)-on;
1-(((*S*)-1-((*S*)-3-Cyclohexyl-2-(methoxymethyl)propanoyl)-4-hydroxy-3,3-dimethylpiperidin-4-yl)methyl)-*N,N*-dimethyl-6-oxo-4-phenyl-1,6-dihydropyridin-3-carboxamid;
4-(2-(Aminomethyl)phenyl)-1-((7-((*R*)-3-cyclohexyl-2-methylpropanoyl)-10-hydroxy-7-azaspiro[4.5]decan-10-yl)methyl)-*N,N-*dimethyl-6-oxo-1,6-dihydropyridin-3-carboxamid;
1-((7-((*R*)-3-Cyclohexyl-2-methylpropanoyl)-10-hydroxy-7-azaspiro[4.5]decan-10-yl)methyl)-4-(4-(hydroxymethyl)phenyl)-*N,N-*dimethyl-6-oxo-1,6-dihydropyridin-3-carboxamid;
1-((10-Hydroxy-7-(2-methyl-3-morpholinopropanoyl)-7-azaspiro[4.5]decan-10-yl)methyl)-*N,N-*dimethyl-6-oxo-4-phenyl-1,6-dihydropyridin-3-carboxamid;
1-(((*S*)-1-((*S*)-2-(Cyclohexylmethyl)-4-hydroxybutanoyl)-4-hydroxy-3,3-dimethylpiperidin-4-yl)methyl)-*N,N-*dimethyl-6-oxo-4-phenyl-1,6-dihydropyridin-3-carboxamid;
1-(((*R*)-1-((*S*)-2-(Cyclohexylmethyl)-4-hydroxybutanoyl)-4-hydroxy-3,3-dimethylpiperidin-4-yl)methyl)-*N,N*-dimethyl-6-oxo-4-phenyl-1,6-dihydropyridin-3-carboxamid;
1-(((*S*)-1-((*S*)-3-Cyclobutyl-2-(hydroxymethyl)propanoyl)-4-hydroxy-3,3-dimethylpiperidin-4-yl)methyl)-*N,N*-dimethyl-6-oxo-4-phenyl-1,6-dihydropyridin-3-carboxamid;
1-(((*R*)-1-((51-3-Cyclobutyl-2-(hydroxymethyl)propanoyl)-4-hydroxy-3,3-dimethylpiperidin-4-yl)methyl)-*N,N*-dimethyl-6-oxo-4-phenyl-1,6-dihydropyridin-3-carboxamid;
1-((1-((*H*)-3-Cyclohexyl-2-methylpropanoyl)-4-hydroxy-3,3-dimethylpiperidin-4-yl)methyl)-5-(morpholin-4-carbonyl)-4-phenylpyridin-2(1*H*)-on;
5-(4-Acetylpiperazine-1-carbonyl)-1-((1-((*R*)-3-cyclohexyl-2-methylpropanoyl)-4-hydroxy-3,3-dimethylpiperidin-4-yl)methyl)-4-phenylpyridin-2(1*H*)-on;
1-((1-((*R*)-3-Cyclohexyl-2-methylpropanoyl)-4-hydroxy-3,3-dimethylpiperidin-4-yl)methyl)-5-(4-methylpiperazin-1-carbonyl)-4-phenylpyridin-2(1*H*)-on;
1-((10-Hydroxy-7-(4,4,4-trifluoro-3-methylbutanoyl)-7-azaspiro[4.5]decan-10-yl)methyl)-*N,N*-dimethyl-6-oxo-4-phenyl-1,6-dihydropyridin-3-carboxamid;
1-((10-Hydroxy-7-(4,4,4-trifluorbutanoyl)-7-azaspiro[4.5]decan-10-yl)methyl)-*N,N*-dimethyl-6-oxo-4-phenyl-1,6-dihydropyridin-3-carboxamid;
1-((10-Hydroxy-7-(3-(tetrahydrofuran-3-yl)propanoyl)-7-azaspiro[4.5]decan-10-yl)methyl)-*N,N*-dimethyl-6-oxo-4-phenyl-1,6-dihydropyridin-3-carboxamid;
1-((10-Hydroxy-7-((1*R*,2*S*)-2-phenylcyclopropan-1-carbonyl)-7-azaspiro[4.5]decan-10-yl)methyl)-*N,N*-dimethyl-6-oxo-4-phenyl- 1,6-dihydropyridin-3-carboxamid;
1-((7-(Cyclopropancarbonyl)-10-hydroxy-7-azaspiro[4.5]decan-10-yl)methyl)-N,N-dimethyl-6-oxo-4-phenyl-1,6-dihydropyridin-3-carboxamid;
1-((10-Hydroxy-7 -(1-methylcyclohexan-1-carbonyl)-7-azaspiro[4.5]decan-10-yl)methyl)-N,N-dimethyl-6-oxo-4-phenyl-1,6-dihydropyridin-3-carboxamid;
1-((7-(3-Fluorocyclopentan-1-carbonyl)- 10-hydroxy-7-azaspiro[4.5]decan-10-yl)methyl)-N,N-dimethyl-6-oxo-4-phenyl-1,6-dihydropyridin-3-carboxamid;
1-((7-(1-(2,2-Difluoroethyl)cyclopropan-1 -carbonyl)-10-hydroxy-7 -azaspiro[4.5]decan-10-yl)methyl)- N,N-dimethyl-6-oxo-4-phenyl-1,6-dihydropyridin-3-carboxamid;
1-((7-(Bicyclo[2.2. 1]heptan- 1-carbonyl)- 10-hydroxy-7-azaspiro [4.5]decan-10-yl)methyl)-*N,N*-dimethyl-6-oxo-4-phenyl-1,6-dihydropyridin-3-carboxamid;
1-((7-((*S*)-4,4-Difluor-3-phenylbutanoyl)-10-hydroxy-7-azaspiro[4.5]decan-10-yl)methyl)-*N,N*-dimethyl-6-oxo-4-phenyl-1,6-dihydropyridin-3-carboxamid;
1-((7-((*R*)-4,4-Difluor-3-phenylbutanoyl)- 10-hydroxy-7-azaspiro[4.5]decan-10-yl)methyl)-*N,N*-dimethyl-6-oxo-4-phenyl-1,6-dihydropyridin-3-carboxamid;
1-((7-(3-Ethoxypropanoyl)-10-hydroxy-7-azaspiro[4.5]decan-10-yl)methyl)-*N,N*-dimethyl-6-oxo-4-phenyl-1,6-dihydropyridin-3-carboxamid;
1-((10-Hydroxy-7-((*S*)-3-phenylbutanoyl)-7-azaspiro[4.5]decan-10-yl)methyl)-*N,N*-dimethyl-6-oxo-4-phenyl-1,6-dihydropyridin-3-carboxamid;
1-((7-(3-(4-Fluorphenyl)propanoyl)-10-hydroxy-7-azaspiro[4.5]decan-10-yl)methyl)-*N,N*-Dmiethyl-6-oxo-4-phenyl-1,6-dihydropyridin-3-carboxamid;
1-((10-Hydroxy-7-((*R*)-3-phenylbutanoyl)-7-azaspiro[4.5]decan-10-yl)methyl)-*N,N*-dimethyl-6-oxo-4-phenyl-1,6-dihydropyridin-3-carboxamid;
1-((10-Hydroxy-7-(1-methylcyclopentan-1-carbonyl)-7-azaspiro [4.5]decan-10-yl)methyl)-*N,N*-dimethyl-6-oxo-4-phenyl-1,6-dihydropyridin-3-carboxamid;
1-((10-Hydroxy-7-(1*H*-pyrazol-3-carbonyl)-7-azaspiro[4.5]decan-10-yl)methyl)-*N,N*-dimethyl-6-oxo-4-phenyl-1,6-dihydropyridin-3-carboxamid;
1-((10-Hydroxy-7-(1*H*-indazol-3-carbonyl)-7-azaspiro[4.5]decan-10-yl)methyl)-*N,N*-dimethyl-6-oxo-4-phenyl-1,6-dihydropyridin-3-carboxamid;
1-((7-(3-Cyclohexyl-1*H*-pyrazol-4-carbonyl)-10-hydroxy-7-azaspiro[4.5]decan-10-yl)methyl)-*N*,*N*-dimethyl-6-oxo-4-phenyl-1,6-dihydropyridin-3-carboxamid;
1-((10-Hydroxy-7-(1*H*-indol-3-carbonyl)-7-azaspiro[4.5]decan-10-yl)methyl)-*N,N*-dimethyl-6-oxo-4-phenyl-1,6-dihydropyridin-3-carboxamid;
1-((7-(Bicyclo[1.1.1]pentan-1-carbonyl)-10-hydroxy-7-azaspiro[4.5]decan-10-yl)methyl)-*N*,*N*-dimethyl-6-oxo-4-phenyl-1,6-dihydropyridin-3-carboxamid;
1-((10-Hydroxy-7-(5-phenyloxazol-4-carbonyl)-7-azaspiro[4.5]decan-10-yl)methyl)-*N,N*-dimethyl-6-oxo-4-phenyl-1,6-dihydropyridin-3-carboxamid;
1-((7-(5-Cyclopropyloxazol-4-carbonyl)-10-hydroxy-7-azaspiro[4.5]decan-10-yl)methyl)-*N,N*-dimethyl-6-oxo-4-phenyl-1,6-dihydropyridin-3-carboxamid;
1-((7-(2-(Cyclohexyloxy)acetyl)-10-hydroxy-7-azaspiro[4.5]decan-10-yl)methyl)-*N,N-*dimethyl-6-oxo-4-phenyl-1,6-dihydropyridin-3-carboxamid;
1-((10-Hydroxy-7-(spiro[2.2]pentan-1-carbonyl)-7-azaspiro[4.5]decan-10-yl)methyl)-*N,N*-dimethyl-6-oxo-4-phenyl-1,6-dihydropyridin-3-carboxamid;
1-((10-hydroxy-7-(3-(trifluoromethyl)cyclopentan-1-carbonyl)-7-azaspiro[4.5]decan-10-yl)methyl)-*N,N*-dimethyl-6-oxo-4-phenyl-1,6-dihydropyridin-3-carboxamid;
1-((7-(2,4-Dimethylpentanoyl)-10-hydroxy-7-azaspiro[4.5]decan-10-yl)methyl)-*N,N*-dimethyl-6-oxo-4-phenyl-1,6-dihydropyridin-3-carboxamid;
1-((7-(1-Benzyl-1*H*-pyrrol-2-carbonyl)-10-hydroxy-7-azaspiro[4.5]decan-10-yl)methyl)-*N,N-*dimethyl-6-oxo-4-phenyl-1,6-dihydropyridin-3-carboxamid;
1-((7-(2-(Cyclohexyloxy)propanoyl)-10-hydroxy-7-azaspiro[4.5]decan-10-yl)methyl)-*N,N*-dimethyl-6-oxo-4-phenyl-1,6-dihydropyridin-3-carboxamid;
3-((1-((*S*)-3-Cyclobutyl-2-(hydroxymethyl)propanoyl)-4-hydroxy-3,3-dimethylpiperidin-4-yl)methyl)-6-(2-fluorphenyl)pyrimidin-4(3*H*)-on;
1-((7-(2-Cyclobutoxyacetyl)-10-hydroxy-7-azaspiro[4.5]decan-10-yl)methyl)-*N,N-*dimethyl-6-oxo-4-phenyl-1,6-dihydropyridin-3-carboxamid;
1-((7-(3,3-Difluorocyclopentane-1-carbonyl)-10-hydroxy-7-azaspiro[4.5]decan-10-yl)methyl)-*N,N*-dimethyl-6-oxo-4-phenyl-1,6-dihydropyridin-3-carboxamid;
1-((10-Hydroxy-7-(2-hydroxy-3-phenylpropanoyl)-7-azaspiro[4.5]decan-10-yl)methyl)-*N,N*-dimethyl-6-oxo-4-phenyl-1,6-dihydropyridin-3-carboxamid;
1-(((*S*)-4-Hydroxy-3,3-dimethyl-1-((*R*)-4,4,4-trifluoro-2-methylbutanoyl)piperidin-4-yl)methyl)-*N,N*-dimethyl-6-oxo-4-phenyl-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-4-(2-fluorphenyl)-*N,N* dimethyl-6-oxo-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-4-(2-fluorphenyl)-5-(piperazin-1-carbonyl)pyridin-2(1*H*)-on;
(*S*)-1-((4-Hydroxy-3,3-dimethyl-1-(4,4,4-trifluoro-2-(2,2,2-trifluoroethyl)butanoyl)piperidin-4-yl)methyl)-*N,N*-dimethyl-6-oxo-4-phenyl-1,6-dihydropyridin-3-carboxamid;
1-(((*S*)-1-((*R*)-3-Cyclohexyl-2-methylpropanoyl)-4-hydroxy-3,3-dimethylpiperidin-4-yl)methyl)-5-((*S*)-2-(hydroxymethyl)piperazin-1-carbonyl)-4-phenylpyridin-2(1H) -on;
1-(((*R*)-7-((*R*)-3-Cyclohexyl-2-methylpropanoyl)-10-hydroxy-7-azaspiro[4.5]decan-10-yl)methyl)-*N,N*-dimethyl-6-oxo-4-phenyl-1,6-dihydropyridin-3-carboxamid;
1-(((*R*)-7-((*R*)-3-Cyclohexyl-2-methylpropanoyl)-10-hydroxy-7-azaspiro[4.5]decan-10-yl)methyl)-4-phenyl-5-(piperazin-1 - carbonyl)pyridin-2(1*H*)-on;
1-(((*S*)-7-((*R*)-3-Cyclohexyl-2-methylpropanoyl)-10-hydroxy-7-azaspiro[4.5]decan-10-yl)methyl)-N,N-dimethyl-6-oxo-4-phenyl-1,6-dihydropyridin-3-carboxamid;
1-(((*S*)-7-((*R*)-3-Cyclohexyl-2-methylpropanoyl)-10-hydroxy-7-azaspiro[4.5]decan-10-yl)methyl)-4-phenyl-5-(piperazin-1 - carbonyl)pyridin-2(1*H*)-on;
1-(((*S*)-1-((*R*)-3-Cyclohexyl-2-methylpropanoyl)-4-hydroxy-3, 3-dimethylpiperidin-4-yl)methyl)-5-((*R*)-3-methylpiperazin-1-carbonyl)-4-phenylpyridin-2(1*H*)-on;
1-(((*S*)-7-((*R*)-3-Cyclobutyl-2-methylpropanoyl)-10-hydroxy-7-azaspiro[4.5]decan-10-yl)methyl)-N,N-dimethyl-6-oxo-4-phenyl-1,6-dihydropyridin-3-carboxamid;
1-(((*S*)-7-((fl)-3-Cyclobutyl-2-methylpropanoyl)-10-hydroxy-7-azaspiro[4.5]decan-10-yl)methyl)-4-phenyl-5-(piperazin-1 - carbonyl)pyridin-2(1*H*)-on;
1-(((*S*)-10-Hydroxy-7-((*R*)-4,4,4-trifluoro-2-methylbutanoyl)-7-azaspiro[4.5]decan-10-yl)methyl)-*N,N*-dimethyl-6-oxo-4-phenyl-1,6-dihydropyridin-3-carboxamid;
1-(((*S*)-10-Hydroxy-7 -((R)-4,4,4-trifluoro-2-methylbutanoyl)-7-azaspiro[4.5]decan-10-yl)methyl)-4-phenyl-5-(piperazin-1-carbonyl)pyridin-2(1*H*)-on;
(*S*)-1-((10-Hydroxy-7-(4,4,4-trifluoro-2-(2,2,2-trifluoroethyl)butanoyl)-7-azaspiro[4.5]decan-10-yl)methyl)-*N,N-*dimethyl-6-oxo-4-phenyl-1,6-dihydropyridin-3-carboxamid;
(*S*)-1-((10-Hydroxy-7-(4,4,4-trifluoro-2-(2,2,2-trifluoroethyl)butanoyl)-7-azaspiro [4. 5]decan-10-yl)methyl)-4-phenyl-5-(piperazine-1-carbonyl)pyridin-2(1*H*)-on;
1-(((*S*)-1-((*R*)-3-Cyclohexyl-2-methylpropanoyl)-4-hydroxy-3,3-dimethylpiperidiri-4-yl)methyl)-4-cyclopropyl-*N,N*-dimethyl-6-oxo-1,6-dihydropyridin-3-carboxamid;
1-(((*S*)-1-((R)-3-Cyclohexyl-2-methylpropanoyl)-4-hydroxy-3,3-dimethylpiperidin-4-yl)methyl)-4-cyclopropyl-5-(piperazin-1-carbonyl)pyridin-2(1*H*)-on;
1-(((*S*)-7-((*R*)-3-Cyclobutyl-2-methylpropanoyl)-10-hydroxy-7-azaspiro[4.5]decan-10-yl)methyl)-4-(2-fluorophenyl)*-N,N-*dimethyl-6-oxo-1,6-dihydropyridin-3-carboxamid;
4-(2-Fluorphenyl)-1-(((*S*)-10-hydroxy-7-((*R*)-4,4,4-trifluoro-2-methylbutanoyl)-7-azaspiro[4.5]decan-10-yl)methyl-*N,N-*dimethyl-6-oxo-1,6-dihydropyridin-3-carboxamid;
1-(((*S*)-7-((R)-3-Cyclobutyl-2-methylpropanoyl)-10-hydroxy-7-azaspiro[4.5]decan-10-yl)methyl)-4-(2-fluorophenyl)-5-(piperazin-1-carbonyl)pyridin-2(1*H*)-on;
4-(2-Fluorphenyl)-1-(((*S*)-10-Hydroxy-7-((*R*)-4,4,4-trifluoro-2-methylbutanoyl)-7-azaspiro[4.5]decan-10-yl)methyl)-5-(piperazin-1-carbonyl)pyridin-2(1*H*)-on;
1-(((*S*)-7-((*R*)-3-Cyclohexyl-2-methylpropanoyl)-10-hydroxy-7-azaspiro[4.5]decan-10-yl)methyl)-4-(2-fluorophenyl)-*N,N-*dimethyl-6-oxo-1,6-dihydropyridin-3-carboxamid;
1-(((*S*)-7-((R)-3-Cyclohexyl-2-methylpropanoyl)-10-hydroxy-7-azaspiro[4.5]decan-10-yl)methyl)-4-(2-fluorophenyl)-5-(piperazin-1-carbonyl)pyridin-2(1*H*)-on;
1-(((*S*)-1-((*R*)-3-Cyclohexyl-2-methylpropanoyl)-4-hydroxy-3,3-dimethylpiperidin-4-yl)methyl)-5-(4-hydroxypiperidin-1-carbonyl)-4-phenylpyridin-2(1*H*)-on;
1-((10-Hydroxy-7-(3-(trifluoromethyl)cyclobutan-1-carbonyl)-7-azaspiro[4.5]decan-10-yl)methyl)-*N,N*-dimethyl-6-oxo-4-phenyl-1,6-dihydropyridin-3-carboxamid;
1-(((*S*)-7-((*R*)-3-Cyclobutyl-2-methylpropanoyl)-10-hydroxy-7-azaspiro[4.5]decan-10-yl)methyl)-4-cyclopropyl-N,N-dimethyl-6-oxo-1,6-dihydropyridin-3-carboxamid;
1-(((*S*)-7-((*R*)-3-Cyclobutyl-2-methylpropanoyl)- 10-hydroxy-7-azaspiro[4.5]decan-10-yl)methyl)-4-cyclopropyl-5-(piperazin-1-carbonyl)pyridin-2(1*H*)-on;
1-(((*S*)-7-((*R*)-3-Cyclohexyl-2-methylpropanoyl)-10-hydroxy-7-azaspiro[4.5]decan-10-yl)methyl)-4-cyclopropyl-*N,N*-dimethyl-6-oxo-1,6-dihydropyridin-3-carboxamid;
1-(((*S*)-7-((*R*)-3-Cyclohexyl-2-methylpropanoyl)-10-hydroxy-7-azaspiro[4.5]decan-10-yl)methyl)-4-cyclopropyl-5-(piperazin-1-carbonyl)pyridin-2(1*H*)-on;
1-((2-((*R*)-3-Cyclohexyl-2-methylpropanoyl)-5-hydroxy-9-oxa-2-azaspiro[5.5]undecan-5-yl)methyl)-*N,N*-dimethyl-6-oxo-4-phenyl-1,6-dihydropyridin-3-carboxamid;
1-(((*S*)-1-((*R*)-3-Cyclobutyl-2-methylpropanoyl)-4-hydroxy-3,3-dimethylpiperidin-4-yl)methyl)-4-(2-fluorphenyl-*N,N-*dimethyl-6-oxo-1,6-dihydropyridin-3-carboxamid;
1-(((*S*)-1-((*R*)-3-Cyclobutyl-2-methylpropanoyl)-4-hydroxy-3,3-dimethylpiperidin-4-yl)methyl)-4-(2-fluorphenyl)-5-(piperazin-1-carbonyl)pyridin-2(1*H*)-on;
1-((2-((*R*)3-Cyclohexyl-2-methylpropanoyl)-5-hydroxy-2 - azaspiro[5.5]undecan-5-yl)methyl)-*N,N*-dimethyl-6-oxo-4-phenyl-1,6-dihydropyridin-3-carboxamid;
1-((2-((*R*)-3-Cyclohexyl-2-methylpropanoyl)-5-hydroxy-2-azaspiro[5.5]undecan-5-yl)methyl)-4-phenyl-5-(piperazin-1 - carbonyl)pyridin-2(1*H*)-on;
1-(((*S*)-7-((*R*)-3-Cyclohexyl-2-methylpropanoyl)-10-hydroxy-7-azaspiro[4.5]decan-10-yl)methyl)-5-(morpholin-4-carbonyl)-4-phenylpyridin-2(1*H*)-on;
1-(((*S*)-7-((*R*)-3-Cyclohexyl-2-methylpropanoyl)-10-hydroxy-7-azaspiro[4.5]decan-10-yl)methyl)-5-(4-methylpiperazin-1-carbonyl)-4-phenylpyridin-2(1*H*)-on;
1-((1-((*R*)-3-Cyclohexyl-2-methylpropanoyl)-4-hydroxy-3,3-dimethylpiperidin-4-yl)methyl)-6-oxo-4-phenyl-1,6-dihydropyridin-3-carbonsäure;
1-((7-((*R*)-3-Cyclohexyl-2-methylpropanoyl)-10-hydroxy-7-azaspiro[4.5]decan-10-yl)methyl)-4-methoxy-*N,N*-dimethyl-6-oxo-1,6-dihydropyridin-3-carboxamid;
1-((7-(4,4-Difluoro-2-methylbutanoyl)-10-hydroxy-7-azaspiro[4.5]decan-10-yl)methyl)-*N*,*N*-dimethyl-6-oxo-4-phenyl-1,6-dihydropyridin-3-carboxamid;
1-((7-(4,4-Difluoro-2,2-dimethylbutanoyl)- 10-hydroxy-7-azaspiro[4.5]decan-10-yl)methyl)-*N,N-*dimethyl-6-oxo-4-phenyl-1,6-dihydropyridin-3-carboxamid;
1-((10-Hydroxy-7-(4-(trifluoromethyl)thiazole-2-carbonyl)-7- azaspiro[4.5]decan-10-yl)methyl)-*N,N-*dimethyl-6-oxo-4-phenyl-1,6-dihydropyridin-3-carboxamid;
1-((10-Hydroxy-7-(2-(trifluoromethyl)thiazole-4-carbonyl)-7-azaspiro[4.5]decan-10-yl)methyl carboxamiddimethyl-6-oxo-4-phenyl-1,6-dihydropyridin-3-carboxamid;
1-(((*S*)-10-Hydroxy-7-((*S*)-4,4,4-trifluoro-2-(hydroxymethyl)butanoyl)-7-azaspiro[4.5]decan- 10-yl)methyl)-*N,N-*dimethyl-6-oxo-4-phenyl-1,6-dihydropyridin-3-carboxamid;
1-(((*S*)-10-Hydroxy-7-((*S*)-4,4,4-trifluor-2-(hydroxymethyl)butanoyl)-7-azaspiro[4.5]decan-10-yl)methyl)-5-(morpholin-4-carbonyl)-4-phenylpyridin-2(1*H*)-on;
1-(((*S*)-10-Hydroxy-7-((*S*)-4,4,4-trifluoro-2-(hydroxymethyl)butanoyl)-7-azaspiro[4.5]decan- 10-yl)methyl)-4-phenyl-5-(piperazin-1-carbonyl)pyridin-2(1H)-on;
1-(((S)-10-Hydroxy-7-((*R*)-4,4,4-trifluor-2-methylbutanoyl)-7-azaspiro[4.5]decan-10-yl)methyl)-5-(morpholin-4-carbonyl)-4-phenylpyridin-2(1*H*)-on;
1-(((*S*)-10-Hydroxy-7-((*R*)-4,4,4-trifluoro-2-methylbutanoyl)-7-azaspiro[4.5]decan-10-yl)methyl)-5-(4-methylpiperazin-1-carbonyl)-4-phenylpyridin-2(1*H*)-on;
Ethyl- 1-(((*S*)-7-((*R*)-3-cyclohexyl-2-methylpropanoyl)-10-hydroxy-7-azaspiro[4.5]decan-10-yl)methyl)-6-oxo-4-phenyl-1,6-dihydropyridin-3-carboxylat;
1-(((*S*)-7-((R)-3-Cyclohexyl-2-methylpropanoyl)-10-hydroxy-7-azaspiro[4.5]decan-10-yl)methyl)-6-oxo-4-phenyl-1,6-dihydropyridin-3-carbonsäure;
1-(((*R*)-1-((*R*)-3-Cyclohexyl-2-methylpropanoyl)-4-hydroxy-3,3-dimethylpiperidin-4-yl)methyl)-N-isopropyl-N-methyl-6-oxo-4-phenyl-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-4-phenyl-5-(pyrrolidin-1-carbonyl)pyridin-2(1*H*)-on;
1-(((*R*)-1-((51-3-Cyclohexyl-2-(methoxymethyl)propanoyl)-4-hydroxy-3,3-dimethylpiperidin-4-yl)methyl carboxamiddimethyl-6-oxo-4-phenyl-1,6-dihydropyridin-3-carboxamid;
1-(((*R*)-4-Hydroxy-3,3-dimethyl-1-((*R*)-4,4,4-trifluoro-2-methylbutanoyl)piperidin-4-yl)methyl carboxamiddimethyl-6-oxo-4-phenyl-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-4-(2-fluorphenyl carboxamiddimethyl-6-oxo-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-4-(2-fluorphenyl)-5-(piperazin-1-carbonyl)pyridin-2(1*H*)-on;
(*R*)-1-((4-Hydroxy-3,3-dimethyl-1-(4,4,4-trifluoro-2-(2,2,2-trifluoroethyl)butanoyl)piperidin-4-yl)methyl carboxamiddimethyl-6-oxo-4-phenyl-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-5-((*S*)-2-(hydroxymethyl)piperazin-1-carbonyl)-4-phenylpyridin-2(1*H*)-on;
1-(((*R*)-1-((*R*)-3-Cyclohexyl-2-methylpropanoyl)-4-hydroxy-3,3-dimethylpiperidin-4-yl)methyl)-5-((*R*)-3-methylpiperazin-1-carbonyl)-4-phenylpyridin-2(1*H*)-on;
1-(((*R*)-7-((*R*)-3-Cyclobutyl-2-methylpropanoyl)-10-hydroxy-7-azaspiro[4.5]decan-10-yl)methyl)-*N*,*N*-dimethyl-6-oxo-4-phenyl-1,6-dihydropyridin-3-carboxamid;
1-(((*R*)-7-((*R*)-3-Cyclobutyl-2-methylpropanoyl)-10-hydroxy-7-azaspiro[4.5]decan-10-yl)methyl)-4-phenyl-5-(piperazin-1-carbonyl)pyridin-2(1 R)-on;
1-(((*R*)-10-Hydroxy-7-((*R*)-4,4,4-trifluoro-2-methylbutanoyl)-7-azaspiro[4.5]decan-10-yl)methyl)-*N,N*-dimethyl-6-oxo-4-phenyl-1,6-dihydropyridin-3-carboxamid;
1-(((*R*)-10-Hydroxy-7-((*R*)-4,4,4-trifluoro-2-methylbutanoyl)-7-azaspiro[4.5]decan-10-yl)methyl)-4-phenyl-5-(piperazin-1-carbonyl)pyridin-2(1*H*)-on;
(*R*)-1-((10-Hydroxy-7-(4,4,4-trifluoro-2-(2,2,2-trifluoroethyl)butanoyl)-7-azaspiro[4.5]decan-10-yl)methyl)*-N,N-*dimethyl-6-oxo-4-phenyl-1,6-dihydropyridin-3-carboxamid;
(*R*)-1-((10-Hydroxy-7-(4,4,4-trifluoro-2-(2,2,2-trifluoroethyl)butanoyl)-7-azaspiro [4. 5]decan-10-yl)methyl)-4-phenyl-5-(piperazine-1-carbonyl)pyridin-2(1*H*)-on;
1-(((*R*)-1-((*R*)-3-Cyclohexyl-2-methylpropanoyl)-4-hydroxy-3,3-dimethylpiperidin-4-yl)methyl)-4-cyclopropyl-*N,N*-dimethyl-6-oxo-1,6-dihydropyridin-3-carboxamid;
1-(((*R*)-1-((*R*)-3-Cyclohexyl-2-methylpropanoyl)-4-hydroxy-3,3-dimethylpiperidin-4-yl)methyl)-4-cyclopropyl-5-(piperazin-1-carbonyl)pyridin-2(1*H*)-on;
1-(((R)-7-((R)-3-Cyclobutyl-2-methylpropanoyl)- 10-hydroxy-7-azaspiro[4.5]decan-10-yl)methyl)-4-(2-fluorophenyl)-*N,N-*dimethyl-6-oxo-1,6-dihydropyridin-3-carboxamid;
4-(2-Fluorophenyl)-1-(((*R*)-10-hydroxy-7-((*R*)-4,4,4-trifluoro-2-methylbutanoyl)-7-azaspiro[4.5]decan-10-yl)methyl)-*N,N-*dimethyl-6-oxo- 1,6-dihydropyridirie-3-carboxamid;
1-(((*R*)-7-((*R*)-3-Cyclobutyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-on;
4-(2-Fluorphenyl)-1-(((*R*)-10-Hydroxy-7-((*R*)-4,4,4-trifluor-2-methylbutanoyl)-7-azaspiro[4.5]decan-10-yl)methyl)-5-(piperazin-1-carbonyl)pyridin-2(1*H*)-on;
1-(((*R*)-7-((*R*)-3-Cyclohexyl-2-methylpropanoyl)-10-hydroxy-7-azaspiro[4.5]decan-10-yl)methyl)-4-(2-fluorophenyl)-*N,N-*dimethyl-6-oxo-1,6-dihydropyridin-3-carboxamid;
1-(((*R*)-7-((*R*)-3-Cyclohexyl-2-methylpropanoyl)-10-hydroxy-7-azaspiro[4.5]decan-10-yl)methyl)-4-(2-fluorophenyl)-5-(piperazine-1-carbonyl)pyridin-2(1*H*)-on;
1-(((*R*)-1-((*R*)-3-Cyclohexyl-2-methylpropanoyl)-4-hydroxy-3, 3-dimethylpiperidin-4-yl)methyl)-5-(4-hydroxypiperidin-1-carbonyl)-4-phenylpyridin-2(1*H*)-on;
1-(((*R*)-7-((*R*)-3-Cyclobutyl-2-methylpropanoyl)-10-hydroxy-7-azaspiro[4.5]decan-10-yl)methyl)-4-cyclopropyl-N,N-dimethyl-6-oxo-1,6-dihydropyridin-3-carboxamid;
1-(((*R*)-7-((*R*)-3-Cyclobutyl-2-methylpropanoyl)-10-hydroxy-7-azaspiro[4.5]decan-10-yl)methyl)-4-cyclopropyl-5-(piperazine-1-carbonyl)pyridin-2(1*H*)-on;
1-(((*R*)-7-((*R*)-3-Cyclohexyl-2-methylpropanoyl)-10-hydroxy-7-azaspiro[4.5]decan-10-yl)methyl)-4-cyclopropyl-N,N-dimethyl-6-oxo-1,6-dihydropyridin-3-carboxamid;
1-(((*R*)-7-((*R*)-3-Cyclohexyl-2-methylpropanoyl)-10-hydroxy-7-azaspiro[4.5]decan-10-yl)methyl)-4-cyclopropyl-5-(piperazine-1-carbonyl)pyridin-2(1*H*)-on;
1-(((*R*)-1-((*R*)-3-Cyclobutyl-2-methylpropanoyl)-4-hydroxy-3,3-dimethylpiperidin-4-yl)methyl)-4-(2-fluorphenyl)-*N,N-*dimethyl-6-oxo-1,6-dihydropyridirie-3-carboxamid;
1-(((*R*)-1-((*R*)-3-Cyclobutyl-2-methylpropanoyl)-4-hydroxy-3,3-dimethylpiperidin-4-yl)methyl)-4-(2-fluorphenyl)-5-(piperazin-1-carbonyl)pyridin-2(1*H*)-on;
1-(((*R*)-7-((*R*)-3-Cyclohexyl-2-methylpropanoyl)-10-hydroxy-7-azaspiro[4.5]decan-10-yl)methyl)-5-(morpholine-4-carbonyl)-4-phenylpyridin-2(1*H*)-on;
1-(((*R*)-7-((*R*)-3-Cyclohexyl-2-methylpropanoyl)-10-hydroxy-7-azaspiro[4.5]decan-10-yl)methyl)-5-(4-methylpiperazine-1-carbonyl)-4-phenylpyridin-2(1*H*)-on;
1-(((*R*)-10-Hydroxy-7-((*S*)-4,4,4-trifluoro-2-(hydroxymethyl)butanoyl)-7-azaspiro[4.5]decan-10-yl)methyl)-*N,N-*dimethyl-6-oxo-4-phenyl-1,6-dihydropyridin-3-carboxamid;
1-(((*R*)-10-Hydroxy-7-((*S*)-4,4,4-trifluor-2-(hydroxymethyl)butanoyl)-7-azaspiro[4.5]decan-10-yl)methyl)-5-(morpholin-4-carbonyl)-4-phenylpyridin-2(1*H*)-on;
1-(((*R*)-10-Hydroxy-7-((*S*)-4,4,4-trifluoro-2-(hydroxymethyl)butanoyl)-7-azaspiro[4.5]decan-10-yl)methyl)-4-phenyl-5-(piperazin-1-carbonyl)pyridin-2(1*H*)-on;
1-(((*R*)-10-Hydroxy-7-((*R*)-4,4,4-trifluoro-2-methylbutanoyl)-7-azaspiro[4.5]decan-10-yl)methyl)-5-(morpholine-4-carbonyl)-4-phenylpyridin-2(1*H*)-on;
1-(((*R*)-10-Hydroxy-7-((*R*)-4, 4, 4-trifluoro-2-methylbutanoyl)-7-azaspiro[4.5]decan-10-yl)methyl)-5-(4-methylpiperazin-1-carbonyl)-4-phenylpyridin-2(1*H*)-on;
Ethyl-1-(((*R*)-7-((*R*)-3-cyclohexyl-2-methylpropanoyl)-10-hydroxy-7-azaspiro[4.5]decan-10-yl)methyl)-6-oxo-4-phenyl-1,6-dihydropyridin-3-carboxylat;
1-(((*R*)-7-((*R*)-3-Cyclohexyl-2-methylpropanoyl)-10-hydroxy-7-azaspiro[4.5]decan-10-yl)methyl)-6-oxo-4-phenyl-1,6-dihydropyridin-3-carbonsäure;
3-((10-Hydroxy-7-((3*R*,4*R*)-3-phenylpiperidine-4-carbonyl)-7-azaspiro[4.5]decan-10-yl)methyl)-6-phenylpyrimidin-4(3*H*)-on;
3-(((*S*)-10-Hydroxy-7-((*R*)-4,4,4-trifluoro-2-methylbutanoyl)-7-azaspiro[4.5]decan-10-yl)methyl)-6-phenylpyrimidin-4(3*H*)-on;
6-(2-Fluorphenyl)-3-(((*S*)-10-Hydroxy-7-((*R*)-4,4,4-trifluor-2-methylbutanoyl)-7-azaspiro[4.5]decan-10-yl)methyl)pyrimidin-4(3*H*)-on;
6-(3-Fluorphenyl)-3-(((*S*)-10-Hydroxy-7-((*R*)-4,4,4-trifluor-2-methylbutanoyl)-7-azaspiro[4.5]decan-10-yl)methyl)pyrimidin-4(3*H*)-on;
3-(((*S*)-7-((*R*)-4,4-Difluor-2-methylpentanoyl)-10-hydroxy-7-azaspiro[4.5]decan-10-yl)methyl)-6-phenylpyrimidin-4(3*H*)-on;
3-(((*S*)-10-Hydroxy-7-((3*S*,4*R*)-4-phenylpyrrolidin-3-carbonyl)-7-azaspiro[4.5]decan-10-yl)methyl)-6-phenylpyrimidin-4(3*H*)-on;
3-(((*S*)-10-Hydroxy-7-((3*R*,4*S*)-4-phenylpyrrolidin-3-carbonyl)-7-azaspiro[4.5]decan-10-yl)methyl)-6-phenylpyrimidin-4(3*H*)-on;
3-(((*S*)-10-Hydroxy-7-((2*R*,3*R*)-2-phenylpyrrolidin-3-carbonyl)-7-azaspiro[4.5]decan-10-yl)methyl)-6-phenylpyrimidin-4(3*H*)-on;
3-(((*S*)-10-Hydroxy-7-((2S,3S)-2-phenylpyrrolidin-3-carbonyl)-7-azaspiro[4.5]decan-10-yl)methyl)-6-phenylpyrimidin-4(3*H*)-on.

18. Pharmazeutische Zusammensetzung, umfassend eine Verbindung, ein pharmazeutisch akzeptables Salz, Tautomer, Stereoisomer oder N-Oxid-Derivat, wie in einem der Ansprüche 1 bis 17 definiert, zur Verwendung bei der Behandlung von Fettleibigkeit, Insulinresistenz oder Typ-II-Diabetes, wobei die pharmazeutische Zusammensetzung ferner ein pharmazeutisch akzeptables Verdünnungsmittel, einen Exzipienten oder Träger umfasst.

## Revendications

1. Composé, ou sel, tautomère, stéréoisomère ou dérivé de N-oxyde pharmaceutiquement acceptable de celui-ci, destiné à être utilisé dans un procédé de traitement de l'obésité, résistance à l'insuline, ou diabète de type II, dans lequel le composé est un composé selon la formule (I) : 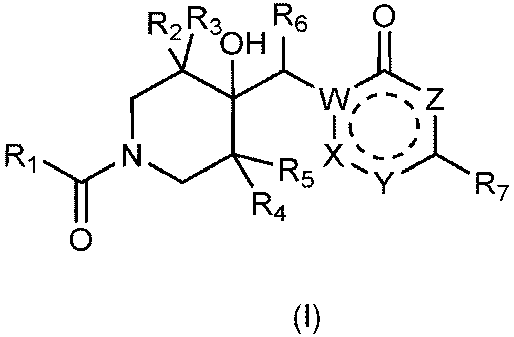 dans lequel :
R₁ est un groupe alkyle, alcényle, alcynyle, éther, cycloalkyle, alkylcycloalkyle, hétérocycloalkyle, alkylhétérocycloalkyle, aryle, arylalkyle, hétéroaryle ou hétéroarylalkyle facultativement substitué ;
R₂ est H ou un groupe alkyle facultativement substitué,
R₃ est H ou un groupe alkyle facultativement substitué,
R₄ est H ou un groupe alkyle facultativement substitué,
R₅ est H ou un groupe alkyle facultativement substitué,
R₂ et R₄ peuvent être joints l'un à l'autre pour former un cycloalkyle ou hétérocycloalkyle facultativement substitué qui comprend le carbone auquel ils sont attachés,
R₄ et R₅ peuvent être joints l'un à l'autre pour former un cycloalkyle ou hétérocycloalkyle facultativement substitué qui comprend le carbone auquel ils sont attachés ;
R₆ est H ou un groupe alkyle facultativement substitué ;
W est C ou N ;
X est N ou CR₈, dans lequel R₈ est H ou un alkyle en C1-C6 facultativement substitué,
Y est N ou CR₉
Z est CH, ou NH,
dans lequel R₉ est H ou un alkyle, amido, amino, aryle, hétéroaryle, cycloalkyle, cyclohétéroalkyle, halo, carbonyle, ester, aminoalkyle ou cyano facultativement substitué ;
R7 est un hydrogène, un halo ou un groupe alkyle, alcényle, alcynyle, amino, aryle, cycloalkyle, cycloalcényle, alcoxy, aryloxy, hétéroaryle ou hétérocycloalkyle facultativement substitué ;
ou le composé 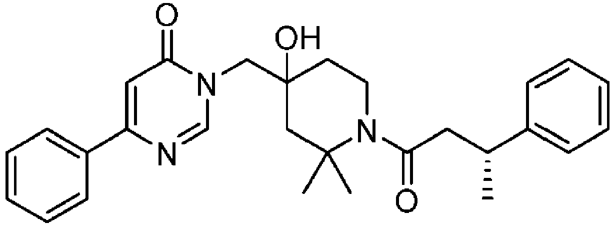
ou un sel, tautomère, stéréoisomère ou dérivé N-oxyde pharmaceutiquement acceptable de celui-ci.

2. Composé, sel, tautomère, stéréoisomère ou dérivé de N-oxyde pharmaceutiquement acceptable destiné à être utilisé selon la revendication 1, dans lequel R₁ est un alkyle en C1-C6 facultativement substitué, un alkylcycloalkyle en C4-C10 facultativement substitué, un hétérocycloalkyle en C4-C5 facultativement substitué, un arylalkyle en C7-C10 facultativement substitué, un cycloalkyle en C3-C6 facultativement substitué, ou un hétéroaryle en C3-C6 facultativement substitué,
dans lequel chaque substituant facultatif est indépendamment choisi parmi un alkyle en C1-C6, un alcényle en C2-C6, un hydroxyméthyle, un méthoxyméthyle, un benzyloxyméthyle, un phényle, un cycloalkyle en C3-C6, CF₃, CHF₂, OH, ou halo.

3. Composé, sel, tautomère, stéréoisomère ou dérivé de N-oxyde pharmaceutiquement acceptable destiné à être utilisé selon la revendication 1 ou la revendication 2, dans lequel R₁ est un cyclohexylalcane en C7-C10 facultativement substitué, un cyclopropylalcane en C4-C6 facultativement substitué, un cyclobutylalcane en C5-C6 facultativement substitué, un alkylbenzène en C7-C10 facultativement substitué, un 2,2-difluorobutane facultativement substitué, ou un 3,3,3-trifluoropropane facultativement substitué,
dans lequel chaque substituant facultatif est indépendamment choisi parmi un alkyle en C1-C6, un alcényle en C2-C6, un hydroxyméthyle, un hydroxyéthyle, un méthoxyméthyle, OH ou halo, facultativement dans lequel R₁ est substitué et le substituant est un alkyle en C1-C6, facultativement un méthyle.

4. Composé, sel, tautomère, stéréoisomère ou dérivé de N-oxyde pharmaceutiquement acceptable destiné à être utilisé selon l'une quelconque des revendications 1 à 3, dans lequel R₁ est un cyclohexyléthane à substitution méthyle, un éthylbenzène à substitution méthyle, un 2,2-difluorobutane à substitution méthyle, un 3,3,3-trifluoropropane à substitution méthyle, un hétérocycloalkyle en C4-C5, ou un hétérocycloalkyle C4-C5 substitué par phényle, facultativement une pyrrolidine à substitution phényle ou une pipéridine à substitution phényle.

5. Composé, sel, tautomère, stéréoisomère ou dérivé de *N*-oxyde pharmaceutiquement acceptable destiné à être utilisé selon l'une quelconque des revendications 1 à 4, dans lequel R₇ est un aryle en C6-C10 facultativement substitué, hétéroaryle en C1-C12, alkyle en C1-C10, alcényle en C2-C10, cycloalkyle en C3-C10, amino, alcoxy en C1-C3, ou halo,
dans lequel le substituant facultatif est choisi parmi un alkyle en C1-C6, un alkyle en C1-C6 à substitution hydroxy, un cycloalkyle en C3-C6, un hétérocycloalkyle en C1-C6, un alcoxy en C1-C6, un alcoxy en C1-C6 à substitution halo, un amido, un cyano ou un halo, facultativement dans lequel R₇ est un cyclopropyle, un thiophène, ou un phényle facultativement substitué,
dans lequel le substituant facultatif est choisi parmi un alkyle en C1-C6, un alkyle en C1-C6 à substitution hydroxy, une alkylamine en C1-C6, et un halo, facultativement dans lequel R₇ est un phényle ou un phényle à substitution fluoro.

6. Composé, sel, tautomère, stéréoisomère ou dérivé de N-oxyde pharmaceutiquement acceptable destiné à être utilisé selon l'une quelconque des revendications 1 à 5, dans lequel R₂, R₃, R₄, R₅ sont choisis indépendamment parmi H et un alkyle en C1-C6, facultativement dans lequel R₂ et R₃ sont H, et R₄ et R₅ sont un méthyle.

7. Composé, sel, tautomère, stéréoisomère ou dérivé de N-oxyde pharmaceutiquement acceptable destiné à être utilisé selon l'une quelconque des revendications 1 à 5, dans lequel R₄ et R₅ sont joints l'un à l'autre pour former un cycloalkyle en C3-C6 ou un hétérocycloalkyle en C3-C6 facultativement substitué qui comprend le carbone auquel ils sont attachés et R₂ et R₃ sont indépendamment choisis parmi H et un alkyle en C1-C6, facultativement dans lequel R₂ et R₃ sont H et R₄ et R₅ sont joints l'un à l'autre pour former un cycloalkyle en C3-C6 qui comprend le carbone auquel ils sont attachés, facultativement dans lequel R₄ et R₅ sont joints l'un à l'autre pour former un cycloalkyle en C4 ou C5 qui comprend le carbone auquel ils sont attachés.

8. Composé sel, tautomère, stéréoisomère ou dérivé de N-oxyde pharmaceutiquement acceptable destiné à être utilisé selon l'une quelconque des revendications 1 à 7, dans lequel R₆ est H ou un alkyle en C1-C6.

9. Composé, sel, tautomère, stéréoisomère ou dérivé de N-oxyde pharmaceutiquement acceptable destiné à être utilisé selon l'une quelconque des revendications 1 à 5, dans lequel R₂ et R₄ sont joints l'un à l'autre pour former un cycloalkyle en C5 qui comprend les carbones auxquels ils sont attachés, et dans lequel R₃, R₅ et R₆ sont indépendamment H ou un alkyle en C1-C6.

10. Composé, sel, tautomère, stéréoisomère ou dérivé de N-oxyde pharmaceutiquement acceptable destiné à être utilisé selon l'une quelconque des revendications 1 à 9, dans lequel W est N et X est CR₈, dans lequel R₈ est H ou un méthyle.

11. Composé, sel, tautomère, stéréoisomère ou dérivé de N-oxyde pharmaceutiquement acceptable destiné à être utilisé selon l'une quelconque des revendications 1 à 10, dans lequel Y est N ou CR₉, dans lequel R₉ est H, alkyle en C1-C6, NR'R", C(O)NR'R", cyano, carboxyle, halo, alkylamine en C1-C6, alkylester en C3-C6, aryle en C6-C10 facultativement substitué ou hétéroaryle en C2-C6 facultativement substitué, dans lequel les un ou plusieurs hétéroatomes sont choisis parmi N et O, et les un ou plusieurs substituants facultatifs de l'aryle ou de l'hétéroaryle sont choisis parmi un alkyle en C1-C6, alkylamine en C1-C6, amido, et cyano,
dans lequel R' et R" sont indépendamment choisis parmi H, un alkyle en C1-C6 facultativement substitué par OH, un cycloalkyle en C3-C7, un hétérocycloalkyle en C1-C7, un alkylcycloalkyle en C4-C7, un alkylhétérocycloalkyle en C3-C7, un benzyle, un phényle, et un méthoxy, ou dans lequel R' et R" sont joints l'un à l'autre pour former un hétérocycle en C2-C7 qui comprend le N auquel ils sont attachés, l'hétérocycle étant facultativement substitué par hydroxyle, substitué par oxo, substitué par méthyle, substitué par CH2OH, ou substitué par acétyle.

12. Composé, sel, tautomère, stéréoisomère ou dérivé de N-oxyde pharmaceutiquement acceptable destiné à être utilisé selon l'une quelconque des revendications 1 à 11, dans lequel Y est N ou CR₉, dans lequel R₉ est choisi parmi : phényle facultativement substitué par amido, cyano ou méthylamine ; pyridine ; oxazole ; pyrazole ; carboxyle ; C(O)NR'R" ; ou NR'R" ;
dans lequel R' et R" sont indépendamment choisis parmi H, un alkyle en C1-C6, un cycloalkyle en C3-C7, un hétérocycloalkyle en C3-C7 dans lequel l'hétéroatome est N ou O, ou dans lequel R' et R" sont joints l'un à l'autre pour former un hétérocycloalkyle en C2-C7 qui comprend le N auquel ils sont attachés, l'hétérocycloalkyle étant facultativement substitué par hydroxyle, substitué par oxo, substitué par méthyle, substitué par CH2OH, ou substitué par acétyle.

13. Composé, sel, tautomère, stéréoisomère ou dérivé de N-oxyde pharmaceutiquement acceptable destiné à être utilisé selon la revendication 1, dans lequel
R₁ est choisi parmi un éthylcyclohexane facultativement substitué, éthylbenzène facultativement substitué, 2,2-difluorobutane facultativement substitué, et 3,3,3-trifluoropropane facultativement substitué, dans lequel le substituant facultatif est un ou plusieurs parmi un méthyle, un éthyle, un propyle, un propényle, un hydroxyméthyle et un méthoxyméthyle, ou dans lequel R₁ est une pyrrolidine à substitution phényle ;
R₂ et R₃ sont indépendamment choisis parmi H, méthyle,
R₄ et R₅ sont indépendamment choisis parmi H, méthyle, ou sont joints l'un à l'autre pour former un cycloalkyle ou hétérocycloalkyle en C3-C6 qui comprend le carbone auquel ils sont attachés, facultativement un cycloalkyle en C4 ou C5 qui comprend le carbone auquel ils sont attachés
R₆ est H ou méthyle,
R7 est choisi parmi un phényle, un indazole, un thiophène ou un pyrazole facultativement substitué, le substituant facultatif étant choisi parmi un alkyle en C1-C6, un alkyle en C1-C6 à substitution hydroxy, un cycloalkyle en C3-C6, un hétérocycloalkyle en C1-C6, un alcoxy en C1-C6, un alcoxy en C1-C6 à substitution halo, une alkylamine en C1-C6, un amido, un cyano ou un halo,
W est N ou C
X est CH ou N
Z est C ou NH
Y est N ou CR₉, où R₉ est choisi parmi : phényle facultativement substitué par amido, cyano ou méthylamine ; pyridine ; oxazole ; pyrazole ; C(O)NR'R" ; ou NR'R" ;
dans lequel R' et R" sont indépendamment choisis parmi H, un alkyle en C1-C6, un cycloalkyle en C3-C7, un hétérocycloalkyle en C3-C7 dans lequel l'hétéroatome est N ou O, ou dans lequel R' et R" sont joints l'un à l'autre pour former un hétérocycloalkyle en C2-C7 qui comprend le N auquel ils sont attachés, dans lequel l'hétérocycloalkyle est facultativement substitué par hydroxyle, substitué par oxo, substitué par méthyle, substitué par hydroxyméthyle, ou substitué par acétyle.

14. Composé, sel, tautomère, stéréoisomère ou dérivé de N-oxyde pharmaceutiquement acceptable destiné à être utilisé selon l'une quelconque des revendications 11 à 13, dans lequel Y est CR₉, dans lequel R₉ est C(O)NR'R" et dans lequel R' et R" sont joints l'un à l'autre pour former une pyrrolidine, une pipéridine, une pipérazine ou une morpholine facultativement substituée qui comprend le N auquel elles sont attachées, dans lequel la pipéridine, la pyrrolidine, la pipérazine ou la morpholine est facultativement substituée par hydroxyle, substituée par oxo, substituée par méthyle, substituée par hydroxyméthyle, ou substituée par acétyle, facultativement dans lequel Y est CR₉, dans lequel R₉ est C(O)NR'R" et dans lequel R' et R" sont joints l'un à l'autre pour former un cycle pipérazinyle.

15. Composé, sel, tautomère, stéréoisomère ou dérivé de N-oxyde pharmaceutiquement acceptable destiné à être utilisé selon l'une quelconque des revendications 1 à 13, dans lequel W est N et Y est N, de telle sorte que le cycle comprenant W, X, Y, Z est un cycle pyrimidinone substitué.

16. Composé, sel, tautomère, stéréoisomère ou dérivé de N-oxyde pharmaceutiquement acceptable destiné à être utilisé selon l'une quelconque des revendications 1 à 15, dans lequel lorsque le composé est chiral en position hydroxyle de la formule (I), le composé est l'énantiomère S.

17. Composé, ou sel, tautomère, stéréoisomère ou dérivé de *N*-oxyde pharmaceutiquement acceptable de celui-ci, destiné à être utilisé selon la revendication 1, dans lequel le composé est :
(*R*)-5-Bromo-1-((4-hydroxy-1-(3-phénylbutanoyl)pipéridin-4-yl)méthyl)-4-phénylpyridin-2(1*H*)-one ;
6-(2-fluorophényl)-3-((4-hydroxy-1-(3-phénylpropanoyl)pipéridin-4-yl)méthyl)pyrimidin-4(3*H*)-one ;
3-((1-(3-Cyclobutylpropanoyl)-4-hydroxypipéridin-4-yl)méthyl)-6-(2-fluorophényl)pyrimidin-4(3*H*)-one ;
3-((1-(3-cyclopentylpropanoyl)-4-hydroxypipéridin-4-yl)méthyl)-6-(2-fluorophényl)pyrimidin-4(3*H*)-one ;
(*R,S*)-6-(2-Fluorophényl)-3-((4-hydroxy-1-(2-méthyl-3-phénylpropanoyl)pipéridin-4-yl)méthyl)pyrimidin-4(3*H*)-one ;
(*R*)-*N*-(3-(1-((4-Hydroxy-1-(3-phénylbutanoyl)pipéridin-4-yl)méthyl)-6-oxo-4-phényl-1,6-dihydropyridin-3-yl)phényl)acétamide ;
(*R*)-3-(1-((4-Hydroxy-1-(3-phénylbutanoyl)pipéridin-4-yl)méthyl)6-oxo-4-phényl-1,6-dihydropyridin-3-yl)benzamide ;
(*R*)-5-(Furan-2-yl)-1-((4-hydroxy-1-(3-phénylbutanoyl)pipéridin-4-yl)méthyl)-4-phénylpyridin-2(1*H*)-one ;
(*R*)-1-((4-Hydroxy-1-(3-phénylbutanoyl)pipéridin-4-yl)méthyl)-5-(1-méthyl-1*H*-pyrazol-5-yl)-4-phénylpyridin-2(1*H*)-one ;
(*R*)-4-Chloro-1-((4-hydroxy-1(3-phénylbutanoyl)pipéridin-4-yl)méthyl)-5-phénylpyridin-2(1*H*)-one ;
(*R*)-4-(Diméthylamino)-1-((4-hydroxy-1-(3-phénylbutanoyl)pipéridin-4-yl)méthyl)-5-phénylpyridin-2(1*H*)-one ;
(*R*)-1-((4-Hydroxy-1-(3-phénylbutanoyl)pipéridin-4-yl)méthyl)-5-phényl-4-(prop-1-en-2-yl)pyridine-2(1*H*)-one ;
(*R*)-éthyle 1-((4-hydroxy-1-(3-phénylbutanoyl)pipéridin-4-yl)méthyl)-6-oxo-4-phényl-1,6-dihydropyridine-3-carboxylate ;
(*R,S*)-4-(2-Fluorophényl)-1-((4-hydroxy-1-(2-méthyl-1,2,3,4-tétrahydronaphtalène-2-carbonyl)pipéridin-4-yl)méthyl)-*N*-isopropyl-*N*-méthyl-6-oxo-1,6-dihydropyridine-3-carboxamide ;
(*R,S*)-1-((1-(3-Cyclobutyl-2-méthylpropanoyl)-4-hydroxypipéridin-4-yl)méthyl)-4-phényl-5-(pipéridine-1-carbonyl)pyridin-2(1*H*)-one ;
(*R*)-3-((4-Hydroxy-1-(3-phénylbutanoyl)pipéridin-4-yl)méthyl)-6-(1-méthyl-1*H*-indazol-7-yl)pyrimidin-4(3*H*)-one ;
(*R*)-3-((4-Hydroxy-1-(3-phénylbutanoyl)pipéridin-4-yl)méthyl)-6-(2-méthoxyphényl)pyrimidin-4(3*H*)-one ;
(*R*)-3-((4-Hydroxy-1-(3-phénylbutanoyl)pipéridin-4-yl)méthyl)-2-méthyl-6-phénylpyrimidin-4(3*H*)-one ;
(*R*)-2-((4-Hydroxy-1-(3-phénylbutanoyl)pipéridin-4-yl)méthyl)-5,6-diphénylpyridazin-3(2*H*)-one ;
(*R*)-3-((4-Hydroxy-1-(3-phénylbutanoyl)pipéridin-4-yl)méthyl)-6-(naphtalèn-1-yl)pyrimidin-4(3*H*)-one ;
(*R*)-6-(3-(1,3-Dioxolan-2-yl)phényl)-3-((4-hydroxy-1-(3-phénylbutanoyl)pipéridin-4-yl)méthyl)pyrimidin-4(3*H*)-one ;
(*R*)-3-((4-Hydroxy-1-(3-phénylbutanoyl)pipéridin-4-yl)méthyl)-6-(thiophèn-3-yl)pyrimidin-4(3*H*)-one ;
4-(2-Fluorophényl)-1-((4-hydroxy-1-(1-méthylcyclopentane-1-carbonyl)pipéridin-4-yl)méthyl)-*N-*isopropyl-*N*-méthyl-6-oxo-1,6-dihydropyridine-3-carboxamide ;
3-((1-(1-Éthylcyclohexane-1-carbonyl)-4-hydroxypipéridin-4-yl)méthyl)-6-(2-fluorophényl)pyrimidin-4(3*H*)-one ;
3-((1-(2-(Cyclohexylméthyl)-3-méthylbutanoyl)-4-hydroxy-3,3-diméthylpipéridin-4-yl)méthyl)-6-(2-fluorophényl)pyrimidin-4(3*H*)-one ;
(*R*)-6-(Furan-2-yl)-3-((4-hydroxy-1-(3-phénylbutanoyl)pipéridin-4-yl)méthyl)pyrimidin-4(3*H*)-one ;
(*R*)-1-((4-Hydroxy-1-(3-phénylbutanoyl)pipéridin-4-yl)méthyl)-4-phénylpyridin-2(1*H*)-one ;
(*R*)-6-(Cyclohex-1-en-1-yl)-3-((4-hydroxy-1-(3-phénylbutanoyl)pipéridin-4-yl)méthyl)pyrimidin-4(3*H*)-one ;
(*R*)-3-((4-Hydroxy-1-(3-phénylbutanoyl)pipéridin-4-yl)méthyl)-6-(prop-1-en-2-yl)pyrimidin-4(3*H*)-one ;
(*R*)-3-((4-Hydroxy-1-(3-phénylbutanoyl)pipéridin-4-yl)méthyl)-6-(2-(trifluorométhoxy)phényl)pyrimidin-4(3*H*)-one ;
3-(((1*R*,5*S*)-3-((R)-3-Cyclohexyl-2-méthylpropanoyl)-8-hydroxy-3-azabicyclo[3.2.1]octan-8-yl)méthyl)-6-(2-fluorophényl)pyrimidin-4(3*H*)-one ;
(*R*)-6-(2-Fluorophényl)-3-((4-hydroxy-1-(3-phénylbutanoyl)pipéridin-4-yl)méthyl)pyrimidin-4(3*H*)-one ;
(*R*)-5-((4-Hydroxy-1-(3-phénylbutanoyl)pipéridin-4-yl)méthyl)-2-phénylpyrimidin-4(3*H*)-one ;
3-((4-Hydroxy-1-(3-phénylpropanoyl)pipéridin-4-yl)méthyl)-2-méthyl-6-phénylpyrimidin-4(3*H*)-one ;
(*R*)-3-((4-Hydroxy-1-(3-phénylbutanoyl)pipéridin-4-yl)méthyl)-6-(isobutylamino)pyrimidin-4(3*H*)-one ;
(*R*)-1-((1-(3-Cyclohexyl-2-méthylpropanoyl)-4-hydroxypipéridin-4-yl)méthyl)-4-phényl-5-(pipéridine-1-carbonyl)pyridin-2(1*H*)-one ;
(*R*)-2-((4-Hydroxy-1-(3-phénylbutanoyl)pipéridin-4-yl)méthyl)-5-phénylpyridazin-3(2*H*)-one ;
(*R*)-4-(2-Cyanophényl)-1-((4-hydroxy-1-(3-phénylbutanoyl)pipéridin-4-yl)méthyl)-*N*-isopropyl-*N*-méthyl-6-oxo-1,6-dihydropyridine-3-carboxamide ;
(*R*)-3-((1-(3-Cyclohexyl-2-méthylpropanoyl)-4-hydroxypipéridin-4-yl)méthyl)-6-(2-fluorophényl)pyrimidin-4(3*H*)-one ;
3-((1-(2-(cyclohexylméthyl)butanoyl)-4-hydroxypipéridin-4-yl)méthyl)-6-(2-fluorophényl)pyrimidin-4(3*H*)-one ;
*rac*-6-(2-Fluorophényl)-3-((4-hydroxy-1-(cis-2-phénylcyclopropanecarbonyl)pipéridin-4-yl)méthyl)pyrimidin-4(3*H*)-one ;
6-(2-fluorophényl)-3-((4-hydroxy-1-(1-méthylcyclohexanecarbonyl)pipéridin-4-yl)méthyl)pyrimidin-4(3*H*)-one ;
3-((*S*)-1-(1-((R)-3-Cyclohexyl-2-méthylpropanoyl)-4-hydroxypipéridin-4-yl)propyl)-6-phénylpyrimidin-4(3*H*)-one ;
3-((*R*)-1-(1-((*R*)-3-Cyclohexyl-2-méthylpropanoyl)-4-hydroxypipéridin-4-yl)propyl)-6-phénylpyrimidin-4(3*H*)-one ;
(*R,S*)-3-((1-(3-cyclohexyl-2-hydroxypropanoyl)-4-hydroxypipéridin-4-yl)méthyl)-6-(2-fluorophényl)pyrimidin-4(3*H*)-one ;
(*R*)-1-((4-Hydroxy-1-(3-phénylbutanoyl)pipéridin-4-yl)méthyl)-6-oxo-4-phényl-1,6-dihydropyridine-3-carbaldéhyde ;
(*R*)-5-((Diméthylamino)méthyl)-1-((4-hydroxy-1-(3-phénylbutanoyl)pipéridin-4-yl)méthyl)-4-phénylpyridin-2(1*H*)-one ;
3-((*R*)-1-(4-Hydroxy-1-((*R*)-3-phénylbutanoyl)pipéridin-4-yl)éthyl)-6-phénylpyrimidin-4(3*H*)-one ;
3-((*S*)-1-(4-Hydroxy-1-((*R*)-3-phénylbutanoyl)pipéridin-4-yl)éthyl)-6-phénylpyrimidin-4(3*H*)-one ;
(*R*)-6-(2-Fluorophényl)-3-((4-hydroxy-1-(4,4,4-trifluoro-3-phénylbutanoyl)pipéridin-4-yl)méthyl)pyrimidin-4(3*H*)-one ;
(*R,S*)-3-((1-(3-Cyclohexyl-2-fluoropropanoyl)-4-hydroxypipéridin-4-yl)méthyl)-6-(2-fluorophényl)pyrimidin-4(3*H*)-one ;
3-(((*R*)-1-((*R*)-3-Cyclohexyl-2-méthylpropanoyl)-4-hydroxy-3,3-diméthylpipéridin-4-yl)méthyl)-6-phénylpyrimidin-4(3*H*)-one ;
3-(((*S*)-1-((*R*)-3-Cyclohexyl-2-méthylpropanoyl)-4-hydroxy-3,3-diméthylpipéridin-4-yl)méthyl)-6-phénylpyrimidin-4(3*H*)-one ;
3-(((*R*)-6-((*R*)-3-Cyclohexyl-2-méthylpropanoyl)-9-hydroxy-6-azaspiro[3.5]nonan-9-yl)méthyl)-6-(2-fluorophényl)pyrimidin-4(3*H*)-one ;
3-(((S)-6-((*R*)-3-Cyclohexyl-2-méthylpropanoyl)-9-hydroxy-6-azaspiro[3.5]nonan-9-yl)méthyl)-6-(2-fluorophényl)pyrimidin-4(3*H*)-one ;
(*R*)-5-(3-(Aminométhyl)phényl)-1-((1-(3-cyclohexyl-2-méthylpropanoyl)-4-hydroxypipéridin¬4-yl)méthyl)-4-(2-fluorophényl)pyridine-2(1*H*)-one ;
3-(((S)-4-Hydroxy-3,3-diméthyl-1-((*R*)-3-phénylbutanoyl)pipéridin-4-yl)méthyl)-6-phénylpyrimidin-4(3*H*)-one ;
3-(((*R*)-4-Hydroxy-3,3-diméthyl-1-((*R*)-3-phénylbutanoyl)pipéridin-4-yl)méthyl)-6-phénylpyrimidin-4(3*H*)-one ;
(*R*)-6-(4-fluorophénoxy)-3-((4-hydroxy-1-(3-phénylbutanoyl)pipéridin-4-yl)méthyl)pyrimidin-4(3*H*)-one ;
(*R*)-1-((4-Hydroxy-1-(3-phénylbutanoyl)pipéridin-4-yl)méthyl)-5-(2-oxopyrrolidin-1-yl)-4-phénylpyridin-2(1*H*)-one ;
6-(2-fluorophényl)-3-((4-hydroxy-1-(1-méthyl-4-méthylènecyclohexanecarbonyl)pipéridin-4-yl)méthyl)pyrimidin-4(3*H*)-one ;
(*R*,*S*)-3-((1-(3-cyclobutyl-2-méthylpropanoyl)-4-hydroxypipéridin-4-yl)méthyl)-6-(2-fluorophényl)pyrimidin-4(3*H*)-one ;
(*R*,*S*)-3-((1-(3-Cycloheptyl-2-méthylpropanoyl)-4-hydroxypipéridin-4-yl)méthyl)-6-(2-fluorophényl)pyrimidin-4(3*H*)-one ;
3-((1-(3-cyclobutyl-2,2-diméthylpropanoyl)-4-hydroxypipéridin-4-yl)méthyl)-6-(2-fluorophényl)pyrimidin-4(3*H*)-one ;
6-(2-Fluorophényl)-3-(((1*R*,5*S*)-8-hydroxy-3-((R)-3-phénylbutanoyl)-3-azabicyclo[3.2.1]octan-8-yl)méthyl)pyrimidin-4(3*H*)-one ;
3-(((R)-4-Hydroxy-2,2-diméthyl-1-((*R*)-3-phénylbutanoyl)pipéridin-4-yl)méthyl)-6-phénylpyrimidin-4(3*H*)-one ;
3-(((S)-4-Hydroxy-2,2-diméthyl-1-((*R*)-3-phénylbutanoyl)pipéridin-4-yl)méthyl)-6-phénylpyrimidin-4(3*H*)-one ;
(*R*)-1-((4-Hydroxy-1-(3-phénylbutanoyl)pipéridin-4-yl)méthyl)-5-(4-hydroxypipéridine-1-carbonyl)-4-phénylpyridin-2(1*H*)-one ;
(*R*)-1-((4-Hydroxy-1-(3-phénylbutanoyl)pipéridin-4-yl)méthyl)-4-phényl-5-(pyrrolidine-1-carbonyl)pyridin-2(1*H*)-one ;
(*R*)-1-((4-Hydroxy-1-(3-phénylbutanoyl)pipéridin-4-yl)méthyl)-4-phényl-5-(1,4-dioxa-8-azaspiro[4.5]décane-8-carbonyl)pyridine-2(1*H*)-one ;
(*R*)-*N*-Cyclohexyl-1-((4-hydroxy-1-(3-phénylbutanoyl)pipéridin-4-yl)méthyl)-6-oxo-4-phényl-1,6-dihydropyridine-3-carboxamide ;
6-(2-Fluorophényl)-3-((4-hydroxy-3,3-diméthyl-1-(*cis-*2-phénylcyclopropane-1-carbonyl)pipéridin-4-yl)méthyl)pyrimidin-4(3*H*)-one ;
(*R*,*S*)-3-((1-(3-Cyclohexyl-1H-pyrazole-4-carbonyl)-4-hydroxy-3,3-diméthylpipéridin-4-yl)méthyl)-6-(2-fluorophényl)pyrimidin-4(3*H*)-one ;
(*R*,*S*)-6-(2-Fluorophényl)-3-((4-hydroxy-3,3-diméthyl-1-(3-phénylpropanoyl)pipéridin-4-yl)méthyl)pyrimidin-4(3*H*)-one ;
(*R*,*S*)-6-(2-Fluorophényl)-3-((4-hydroxy-1-(1-isobutyle cyclopropane-1-carbonyl)-3,3-diméthylpipéridin-4-yl)méthyl)pyrimidin-4(3*H*)-one ;
(*R*,*S*)-6-(2-Fluorophényl)-3-((4-hydroxy-3,3-diméthyl-1 -(3-méthyl-3-phénylbutanoyl)pipéridin-4-yl)méthyl)pyrimidin-4(3*H*)-one ;
(*S*)-1-((1-(4,4-Difluoro-3-phénylbutanoyl)-4-hydroxypipéridin-4-yl)méthyl)-*N*-isopropyl-*N*-méthyl-6-oxo-4-phényl-1,6-dihydropyridine-3-carboxamide ;
1-((4-Hydroxy-1-(1-(thiophén-2-yl)cyclopropane-1-carbonyl)pipéridin-4-yl)méthyl)-*N*-isopropyl-*N*-méthyl-6-oxo-4-phényl-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-*N*-isopropyl-*N-*méthyl-6-oxo-4-phényl-1,6-dihydropyridine-3-carboxamide ;
1-(((S)-1-((*R*)-3-Cyclohexyl-2-méthylpropanoyl)-4-hydroxy-3,3-diméthylpipéridin-4-yl)méthyl)-*N*-isopropyl-*N-*méthyl-6-oxo-4-phényl-1,6-dihydropyridine-3-carboxamide ;
(*R*)-3-((4-Hydroxy-1-(3-phénylbutanoyl)pipéridin-4-yl)méthyl)-6-(2-(hydroxyméthyl)phényl)pyrimidin-4(3*H*)-one ;
(*R*)-*N,N*-Diéthyl-1-((4-hydroxy-1-(3-phénylbutanoyl)pipéridin-4-yl)méthyl)-6-oxo-4-phényl-1,6-dihydropyridine-3-carboxamide ;
3-((1-(3-Cyclohexylbutanoyl)-4-hydroxy-3,3-diméthylpipéridin-4-yl)méthyl)-6-(2-fluorophényl)pyrimidin-4(3*H*)-one ;
(*R*)-1-((4-Hydroxy-1-(3-phénylbutanoyl)pipéridin-4-yl)méthyl)-5-(morpholine-4-carbonyl)-4-phénylpyridin-2(1*H*)-one ;
(*R*,*S*)-3-((1-(3-cyclohexylpropanoyl)-4-hydroxy-3,3-diméthylpipéridin-4-yl)méthyl)-6-(2-fluorophényl)pyrimidin-4(3*H*)-one ;
(*R*,*S*)-1-((1-(3-Cyclobutylpropanoyl)-4-hydroxy-3,3-diméthylpipéridin-4-yl)méthyl)-*N*-isopropyl-*N*-méthyl-6-oxo-4-phényl-1,6-dihydropyridine-3-carboxamide ;
(R,S)-1-((1-(2-Éthylhexanoyl)-4-hydroxypiperidin-4-yl)méthyl)-*N*-isopropyl-*N*-méthyl-6-oxo-4-phényl-1,6-dihydropyridine-3-carboxamide ;
(*R*,*S*)-6-(2-Fluorophényl)-3-((4-hydroxy-3,3-diméthyl-1-(1-méthylcyclohexane-1-carbonyl)pipéridin-4-yl)méthyl)pyrimidin-4(3*H*)-one ;
6-(2-Fluorophényl)-3-((1-(3-(4-fluorophényl)propanoyl)-4-hydroxypipéridin-4-yl)méthyl)pyrimidin-4(3*H*)-one ;
(*R*,*S*)-3-((1-(3-Cyclopropylpropanoyl)-4-hydroxy-3,3-diméthylpipéridin-4-yl)méthyl)-6-(2-fluorophényl)pyrimidin-4(3*H*)-one ;
1-((1-(2,2-Diméthylbutanoyl)-4-hydroxypipéridin-4-yl)méthyl)-*N*-isopropyl-*N*-méthyl-6-oxo-4-phényle-1,6-dihydropyridine-3-carboxamide ;
(*R*)-*N*-(2-(1-((4-Hydroxy-1-(3-phénylbutanoyl)pipéridin-4-yl)méthyl)-6-oxo-1,6-dihydropyrimidin-4-yl)phényl)acétamide ;
(*R*)-5-(4-(Aminométhyl)phényl)-1-((4-hydroxy-1-(3-phénylbutanoyl)pipéridin-4-yl)méthyl)-4-phénylpyridin-2(1*H*)-one ;
(*R*)-1-((1-(3-Cyclohexyl-2-méthylpropanoyl)-4-hydroxypipéridin-4-yl)méthyl)-*N*-isopropyl-*N*-méthyl-6-oxo-4-phényl-1,6-dihydropyridine-3-carboxamide ;
(*R*)-1-((4-Hydroxy-1-(3-phénylbutanoyl)pipéridin-4-yl)méthyl)-4-phényl-5-(pipéridine-1-carbonyl)pyridin-2(1*H*)-one ;
(*R*)-1-((4-Hydroxy-1-(3-phénylbutanoyl)pipéridin-4-yl)méthyl)-*N*-isopropyl-4-(2-méthoxyphényl)-*N*-méthyl-6-oxo-1,6-dihydropyridine-3-carboxamide ;
(*R*)-4-(2-Fluorophényl)-1-((4-hydroxy-1-(3-phénylbutanoyl)pipéridin-4-yl)méthyl)-*N*-isopropyl-*N*-méthyl-6-oxo-1,6-dihydropyridine-3-carboxamide ;
(*R*)-1'-((4-Hydroxy-1-(3-phénylbutanoyl)pipéridin-4-yl)méthyl)-4'-phényl-[2,3'-bipyridin]-6'(1'*H*)-one ;
1-((1-(3-cyclohexylpropanoyl)-4-hydroxypipéridin-4-yl)méthyl)-*N*-isopropyl-*N*-méthyl-6-oxo-4-phényl-1,6-dihydropyridine-3-carboxamide ;
(*R*)-*tert*-Butyle 4-(1-((4-hydroxy-1-(3-phénylbutanoyl)pipéridin-4-yl)méthyl)-6-oxo-4-phényl-1,6-dihydropyridine-3-carbonyl)pipérazine-1-carboxylate ;
(*R*)-1-((4-Hydroxy-1-(3-phénylbutanoyl)pipéridin-4-yl)méthyl)-4-phényl-5-(pipérazine-1-carbonyl)pyridin-2(1*H*)-one ;
(*R*)-1-((4-Hydroxy-1-(3-phénylbutanoyl)pipéridin-4-yl)méthyl)-*N*,*N*-diméthyl-6-oxo-4-phényl-1,6-dihydropyridine-3-carboxamide ;
(*R*)-1-((4-Hydroxy-1-(3-phénylbutanoyl)pipéridin-4-yl)méthyl)-*N*-isopropyl-*N*-méthyl-6-oxo-4-(p-tolyl)-1,6-dihydropyridine-3-carboxamide ;
(*R*)-1-((4-Hydroxy-1-(3-phénylbutanoyl)pipéridin-4-yl)méthyl)-*N*-méthoxy-*N*-méthyl-6-oxo-4-phényl-1,6-dihydropyridine-3-carboxamide ;
(*R*)-1-((4-Hydroxy-1-(3-phénylbutanoyl)pipéridin-4-yl)méthyl)-4,5-diphénylpyridin-2(1*H*)-one ;
(*R*)-1-((4-Hydroxy-1-(3-phénylbutanoyl)pipéridin-4-yl)méthyl)-4-phényl-[3,3'-bipyridin]-6(1*H*)-one ;
(*R*)-5-(3-(Aminométhyl)phényl)-1-((4-hydroxy-1-(3-phénylbutanoyl)pipéridin-4-yl)méthyl)-4-phénylpyridin-2(1*H*)-one ;
(*R*)-1-((4-Hydroxy-1-(3-phénylbutanoyl)pipéridin-4-yl)méthyl)-*N*-isopropyl-*N*-méthyl-6-oxo-4-phényl-1,6-dihydropyridine-3-carboxamide ;
(*R*)-1-((4-Hydroxy-1-(3-phénylbutanoyl)pipéridin-4-yl)méthyl)-*N*-isopropyl-*N*-méthyl-6-oxo-4-(m-tolyl)-1,6-dihydropyridine-3-carboxamide ;
(*R*)-1-((4-Hydroxy-1-(3-phénylbutanoyl)pipéridin-4-yl)méthyl)-4-phényl-5-(pyrimidin-5-yl)pyridin-2(1*H*)-one ;
(*R*)-*N*-(Cyclopropylméthyl)-1-((4-hydroxy-1-(3-phénylbutanoyl)pipéridin-4-yl)méthyl)-6-oxo-4-phényl-1,6-dihydropyridine-3-carboxamide ;
(*R*)-*N*-Benzyl-1-((4-hydroxy-1-(3-phénylbutanoyl)pipéridin-4-yl)méthyl)-*N*-méthyl-6-oxo-4-phényl-1,6-dihydropyridine-3-carboxamide ;
(*R*)-2-(1-((4-Hydroxy-1-(3-phénylbutanoyl)pipéridin-4-yl)méthyl)-6-oxo-4-phényl-1,6-dihydropyridin-3-yl)benzonitrile ;
(*R*)-1-((4-Hydroxy-1-(3-phénylbutanoyl)pipéridin-4-yl)méthyl)-*N*-méthyl-6-oxo-*N*,4-diphényl-1,6-dihydropyridine-3-carboxamide ;
3-(1-(1-((*R*)-3-cyclohexyl-2-méthylpropanoyl)-4-hydroxypipéridin-4-ypéthyl)-6-phénylpyrimidin-4(3*H*)-one ;
(*R*)-1-((4-Hydroxy-1-(3-phénylbutanoyl)pipéridin-4-yl)méthyl)-5-(1-méthyl-1H-pyrazol-4-yl)-4-phénylpyridin-2(1*H*)-one ;
(*R*)-3-((1-(2-(Cyclohexylméthyl)pent-4-enoyl)-4-hydroxypipéridin-4-yl)méthyl)-6-(2-fluorophényl)pyrimidin-4(3*H*)-one ;
(*R*)-1-((4-Hydroxy-1-(3-phénylbutanoyl)pipéridin-4-yl)méthyl)-5-méthyl-4-phénylpyridin-2(1*H*)-one ;
(*R*)-6-(1,5-Diméthyl-1H-pyrazol-4-yl)-3-((4-hydroxy-1-(3-phénylbutanoyl)pipéridin-4-yl)méthyl)pyrimidin-4(3*H*)-one ;
3-(((1*R*,5*S*)-3-(3-Cyclohexylpropanoyl)-8-hydroxy-3-azabicyclo[3.2.1]octan-8-yl)méthyl)-6-(2-fluorophényl)pyrimidin-4(3*H*)-one ;
(*R*)-1-((4-Hydroxy-1-(3-phénylbutanoyl)pipéridin-4-yl)méthyl)-6-oxo-4-phényl-1,6-dihydropyridine-3-carbonitrile ;
(*R*)-1-((4-Hydroxy-1-(3-phénylbutanoyl)pipéridin-4-yl)méthyl)-*N*-(2-hydroxyéthyl)-6-oxo-4-phényl-1,6-dihydropyridine-3-carboxamide ;
1-((4-Hydroxy-1-((*R*)-3-phénylbutanoyl)pipéridin-4-yl)méthyl)-5-((S)-2-méthylpyrrolidine-1-carbonyl)-4-phénylpyridin-2(1*H*)-one ;
(*R*)-1-((4-Hydroxy-1-(3-phénylbutanoyl)pipéridin-4-yl)méthyl)-5-(4-(hydroxyméthyl)pipéridine-1-carbonyl)-4-phénylpyridin-2(1*H*)-one ;
3-((1-(2-(cyclohexylméthyl)butanoyl)-4-hydroxy-3,3-diméthylpipéridin-4-yl)méthyl)-6-(2-fluorophényl)pyrimidin-4(3*H*)-one ;
3-((7-((*R*)-3-Cyclohexyl-2-méthylpropanoyl)-10-hydroxy-7-azaspiro[4.5]décan-10-yl)méthyl)-6-(2-fluorophényl)pyrimidin-4(3*H*)-one ;
1-(((S)-1-((*R*)-3-Cyclohexyl-2-méthylpropanoyl)-4-hydroxy-3,3-diméthylpipéridin-4-yl)méthyl)-*N*-isopropyl-*N-*méthyl-6-oxo-4-phényl-1,6-dihydropyridine-3-carboxamide ;
1-(((S)-1-((*R*)-3-Cyclohexyl-2-méthylpropanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((S)-1-((*R*)-3-Cyclohexyl-2-méthylpropanoyl)-4-hydroxy-3,3-diméthylpipéridin-4-yl)méthyl)-4-phényl-5-(pyrrolidine-1-carbonyl)pyridin-2(1*H*)-one ;
1-(((S)-1-((*R*)-3-Cyclohexyl-2-méthylpropanoyl)-4-hydroxy-3,3-diméthylpipéridin-4-yl)méthyl)-4-phényl-5-(pipérazine-1-carbonyl)pyridin-2(1*H*)-one ;
1-(((*R*)-1-((*R*)-3-Cyclohexyl-2-méthylpropanoyl)-4-hydroxy-3,3-diméthylpipéridin-4-yl)méthyl)-4-phényl-5-(pipérazine-1-carbonyl)pyridin-2(1*H*)-one ;
3-((1-(2-(Cyclohexyloxy)propanoyl)-4-hydroxy-3,3-diméthylpipéridin-4-yl)méthyl)-6-(2-fluorophényl)pyrimidin-4(3*H*)-one ;
1-((7-((*R*)-3-Cyclohexyl-2-méthylpropanoyl)-10-hydroxy-7-azaspiro[4.5]décan-10-yl)méthyl)-*N*,*N*-diméthyl-6-oxo-1,6-dihydropyridine-3-carboxamide ;
1-((1-((*R*)-3-Cyclobutyl-2-méthylpropanoyl)-4-hydroxy-3,3-diméthylpipéridin-4-yl)méthyl)-*N*,*N*-diméthyl-6-oxo-4-phényl-1,6-dihydropyridine-3-carboxamide ;
1-((1-((*R*)-3-Cyclopropyl-2-méthylpropanoyl)-4-hydroxy-3,3-diméthylpipéridin-4-yl)méthyl)-*N*,*N*-diméthyl-6-oxo-4-phényle-1,6-dihydropyridine-3-carboxamide ;
1-((1-((*R*)-3-Cyclohexyl-2-méthylpropanoyl)-4-hydroxy-3,3-diméthylpipéridin-4-yl)méthyl)-[4,5'-bipyrimidin]-6(1*H*)-one ;
3-((1-((*R*)-3-Cyclohexyl-2-méthylpropanoyl)-4-hydroxy-3,3-diméthylpipéridin-4-yl)méthyl)-6-(1-méthyl-1*H*-pyrazol-5-yl)pyrimidin-4(3*H*)-one ;
3-((1-((*R*)-3-Cyclohexyl-2-méthylpropanoyl)-4-hydroxy-3,3-diméthylpipéridin-4-yl)méthyl)-6-(1*H*-pyrazol-4-yl)pyrimidin-4(3*H*)-one ;
3-((1-((*R*)-3-Cyclohexyl-2-méthylpropanoyl)-4-hydroxy-3,3-diméthylpipéridin-4-yl)méthyl)-6-(1*H*-pyrazol-5-yl)pyrimidin-4(3*H*)-one ;
3-((7-((*R*)-3-Cyclohexyl-2-méthylpropanoyl)-10-hydroxy-7-azaspiro[4.5]décan-10-yl)méthyl)pyrimidin-4(3*H*)-one ;
3-((1-((*R*)-3-Cyclohexyl-2-méthylpropanoyl)-4-hydroxy-3,3-diméthylpipéridin-4-yl)méthyl)-6-(thiophèn-3-yl)pyrimidin-4(3*H*)-one ;
1-((1-((*R*)-3-Cyclohexyl-2-méthylpropanoyl)-4-hydroxy-3,3-diméthylpipéridin-4-yl)méthyl)-5-(oxazol-2-yl)-4-phénylpyridine-2(1*H*)-one ;
3-((1-((*R*)-3-Cyclohexyl-2-méthylpropanoyl)-4-hydroxy-3,3-diméthylpipéridin-4-yl)méthyl)-6-(2-(hydroxyméthyl)phényl)pyrimidin-4(3*H*)-one ;
3-((1-((*R*)-3-Cyclohexyl-2-méthylpropanoyl)-4-hydroxy-3,3-diméthylpipéridin-4-yl)méthyl)-6-(3-(hydroxyméthyl)phényl)pyrimidin-4(3*H*)-one ;
3-((1-((*R*)-3-Cyclohexyl-2-méthylpropanoyl)-4-hydroxy-3,3-diméthylpipéridin-4-yl)méthyl)-6-(4-(hydroxyméthyl)phényl)pyrimidin-4(3*H*)-one ;
6-(4-(Aminométhyl)phényl)-3-((1-((*R*)-3-cyclohexyl-2-méthylpropanoyl)-4-hydroxy-3,3-diméthylpipéridin-4-yl)méthyl)pyrimidin-4(3*H*)-one ;
6-(2-(Aminométhyl)phényl)-3-((1-((*R*)-3-cyclohexyl-2-méthylpropanoyl)-4-hydroxy-3,3-diméthylpipéridin-4-yl)méthyl)pyrimidin-4(3*H*)-one ;
6-(2-Fluorophényl)-3-((4-hydroxy-3,3-diméthyl-1-(2,4,4-triméthylpentanoyl)pipéridin-4-yl)méthyl)pyrimidin-4(3*H*)-one ;
6-(2-Fluorophényl)-3-((4-hydroxy-3,3-diméthyl-1-(4,4,4-trifluoro-2-méthylbutanoyl)pipéridin-4-yl)méthyl)pyrimidin-4(3*H*)-one ;
4-Chloro-1-((7-((*R*)-3-cyclohexyl-2-méthylpropanoyl)-10-hydroxy-7-azaspiro[4.5]décan-10-yl)méthyl)-*N*,*N*-diméthyl-6-oxo-1,6-dihydropyridine-3-carboxamide ;
1-((7-((*R*)-3-Cyclohexyl-2-méthylpropanoyl)-10-hydroxy-7-azaspiro[4.5]décan-10-yl)méthyl)-4-(diméthylamino)-*N,N-*diméthyl-6-oxo-1,6-dihydropyridine-3-carboxamide ;
1-((1-((*S*)-3-(Benzyloxy)-2-(cyclohexylméthyl)propanoyl)-4-hydroxy-3,3-diméthylpipéridin-4-yl)méthyl)-*N*,*N*-diméthyl-6-oxo-4-phényl-1,6-dihydropyridine-3-carboxamide ;
1-((10-Hydroxy-7-isobutyryl-7-azaspiro[4.5]décan-10-yl)méthyl)-*N*,*N*-diméthyl-6-oxo-4-phényl-1,6-dihydropyridine-3-carboxamide ;
1-((10-Hydroxy-7-((*R*)-2-méthylbutanoyl)-7-azaspiro[4.5]décan-10-yl)méthyl)-*N*,*N*-diméthyl-6-oxo-4-phényl-1,6-dihydropyridine-3-carboxamide ;
1-((7-((*R*)-3-Cyclopropyl-2-méthylpropanoyl)-10-hydroxy-7-azaspiro[4.5]décan-10-yl)méthyl)-*N,N*-diméthyl-6-oxo-4-phényl-1,6-dihydropyridine-3-carboxamide ;
6-(2-Fluorophényl)-3-((4-hydroxy-3,3-diméthyl-1-(2-méthyl-3-(1*H*-pyrazol-1-yl)popanoyl)pipéridin-4-yl)méthyl)pyrimidin-4(3*H*)-one ;
1-((7-((*R*)-3-Cyclohexyl-2-méthylpropanoyl)-10-hydroxy-7-azaspiro[4.5]décan-10-yl)méthyl)-4-(2-(hydroxyméthyl)phényl)-*N*,*N*-diméthyl-6-oxo-1,6-dihydropyridine-3-carboxamide ;
1-(((S)-1-((S)-3-Cyclohexyl-2-(hydroxyméthyl)propanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((*R*)-1-((S)-3-Cyclohexyl-2-(hydroxyméthyl)propanoyl)-4-hydroxy-3,3-diméthylpipéridin-4-yl)méthyl)-*N*,*N*-diméthyl-6-oxo-4-phényl-1,6-dihydropyridine-3-carboxamide ;
1'-((1-((*R*)-3-Cyclohexyl-2-méthylpropanoyl)-4-hydroxy-3,3-diméthylpipéridin-4-yl)méthyl)-4'-(2-fluorophényl)-[2,3'-bipyridine]-6'(1'*H*)-one ;
1-((7-((*R*)-3-Cyclohexyl-2-méthylpropanoyl)-10-hydroxy-7-azaspiro[4.5]décan-10-yl)méthyl)-*N*,*N*-diméthyl-6-oxo-4-(thiophèn-3-yl)-1,6-dihydropyridine-3-carboxamide ;
1-((7-((*R*)-3-Cyclohexyl-2-méthylpropanoyl)-10-hydroxy-7-azaspiro[4.5]décan-10-yl)méthyl)-4-(3-(hydroxyméthyl)phényl)-*N*,*N*-diméthyl-6-oxo-1,6-dihydropyridine-3-carboxamide ;
1-((7-(3-Cyclohexyl-2-hydroxypropanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(2-méthyl-3-(pipéridin-1-yl)propanoyl)-7-azaspiro[4.5]décan-10-yl)méthyl)-*N*,*N-*diméthyl-6-oxo-4-phényl-1,6-dihydropyridine-3-carboxamide ;
6-(3-(Aminométhyl)phényl)-3-((1-((*R*)-3-cyclohexyl-2-méthylpropanoyl)-4-hydroxy-3,3-diméthylpipéridin-4-yl)méthyl)pyrimidin-4(3*H*)-one ;
1-(((S)-1-((S)-3-Cyclohexyl-2-(méthoxyméthyl)propanoyl)-4-hydroxy-3,3-diméthylpipéridin¬4-yl)méthyl)-*N,N*-diméthyl-6-oxo-4-phényl-1,6-dihydropyridine-3-carboxamide ;
4-(2-(Aminométhyl)phényl)-1-((7-((*R*)-3-cyclohexyl-2-méthylpropanoyl)-10-hydroxy-7-azaspiro[4.5]décan-10-yl)méthyl)-*N*,*N*-diméthyl-6-oxo-1,6-dihydropyridine-3-carboxamide ;
1-((7-((*R*)-3-Cyclohexyl-2-méthylpropanoyl)-10-hydroxy-7-azaspiro[4.5]décan-10-yl)méthyl)-4-(4-(hydroxyméthyl)phényl)-*N*,*N*-diméthyl-6-oxo-1,6-dihydropyridine-3-carboxamide ;
1-((10-Hydroxy-7-(2-méthyl-3-morpholinopropanoyl)-7-azaspiro[4.5]décan-10-yl)méthyl)-*N*,*N*-diméthyl-6-oxo-4-phényl-1,6-dihydropyridine-3-carboxamide ;
1-(((*S*)-1-((*S*)-2-(Cyclohexylméthyl)-4-hydroxybutanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((*R*)-1-((S)-2-(Cyclohexylméthyl)-4-hydroxybutanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((S)-1-((*S*)-3-Cyclobutyl-2-(hydroxyméthyl)propanoyl)-4-hydroxy-3,3-diméthylpipéridin-4-yl)méthyl)-*N,N*-diméthyl-6-oxo-4-phényl-1,6-dihydropyridine-3-carboxamide ;
1-(((*R*)-1-((*S*)-3-Cyclobutyl-2-(hydroxyméthyl)propanoyl)-4-hydroxy-3,3-diméthylpipéridin-4-yl)méthyl)-*N,N*-diméthyl-6-oxo-4-phényl-1,6-dihydropyridine-3-carboxamide ;
1-((1-((*R*)-3-Cyclohexyl-2-méthylpropanoyl)-4-hydroxy-3,3-diméthylpipéridin-4-yl)méthyl)-5-(morpholine-4-carbonyl)-4-phénylpyridine-2(1*H*)-one ;
5-(4-acétylpipérazine-1-carbonyl)-1-((1-((*R*)-3-cyclohexyl-2-méthylpropanoyl)-4-hydroxy-3,3-diméthylpipéridin-4-yl)méthyl)-4-phénylpyridine-2(1*H*)-one ;
1-((1-((*R*)-3-Cyclohexyl-2-méthylpropanoyl)-4-hydroxy-3,3-diméthylpipéridin-4-yl)méthyl)-5-(4-méthylpipérazine-1-carbonyl)-4-phénylpyridine-2(1*H*)-one ;
1-((10-Hydroxy-7-(4,4,4-trifluoro-3-méthylbutanoyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(4,4,4-trifluorobutanoyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(3-(tétrahydrofuran-3-yl)propanoyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-((1*R*,2*S*)-2-phénylcyclopropane-1-carbonyl)-7-azaspiro[4.5]décan-10-yl)méthyl)-*N*,*N*-diméthyl-6-oxo-4-phényl-1,6-dihydropyridine-3-carboxamide ;
1-((7-(Cyclopropanecarbonyl)-10-hydroxy-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(1-méthylcyclohexane-1-carbonyI)-7-azaspiro[4.5]décan-10-yl)méthyl)-*N*,*N*-diméthyl-6-oxo-4-phényl-1,6-dihydropyridine-3-carboxamide ;
1-((7-(3-Fluorocyclopentane-1-carbonyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(1-(2,2-Difluoroéthyl)cyclopropane-1-carbonyl)-10-hydroxy-7-azaspiro[4.5]décan-10-yl)méthyl)-*N,N*-diméthyl-6-oxo-4-phényl-1,6-dihydropyridine-3-carboxamide ;
1-((7-(Bicyclo[2.2.1]heptane-1-carbonyI)-10-hydroxy-7-azaspiro[4.5]décan-10-yl)méthyl)-*N*,*N*-diméthyl-6-oxo-4-phényl-1,6-dihydropyridine-3-carboxamide ;
1-((7-((*S*)-4,4-Difluoro-3-phénylbutanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-((*R*)-4,4-Difluoro-3-phénylbutanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(3-Ethoxypropanoyl)-10-hydroxy-7-azaspiro[4.5]décan-10-yl)méthyl)-*N*,*N*-diméthyl-6-oxo-4-phényl-1,6-ledihydropyridine-3-carboxamide ;
1-((10-Hydroxy-7-((S)-3-phénylbutanoyl)-7-azaspiro[4.5]décan-10-yl)méthyl)-*N*,*N*-diméthyl-6-oxo-4-phényl-1,6-dihydropyridine-3-carboxamide ;
1-((7-(3-(4-Fluorophényl)propanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((10-Hydroxy-7-((*R*)-3-phénylbutanoyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(1-méthylcyclopentane-1-carbonyl)-7-azaspiro[4.5]décan-10-yl)méthyl)-*N*,*N*-diméthyl-6-oxo-4-phényl1-1,6-dihydropyridine-3-carboxamide ;
1-((10-Hydroxy-7-(1*H*-pyrazole-3-carbonyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(1*H*-indazole-3-carbonyl)-7-azaspiro[4.5]décan-10-yl)méthyl)-*N*,*N*-diméthyl-6-oxo-4-phényl-1,6-dihydropyridine-3-carboxamide ;
1-((7-(3-Cyclohexyl-1*H*-pyrazole-4-carbonyl)-10-hydroxy-7-azaspiro[4.5]décan-10-yl)méthyl)-*N*,*N*-diméthyl-6-l'oxo-4-phényl-1,6-dihydropyridine-3-carboxamide ;
1-((10-Hydroxy-7-(1*H*-indole-3-carbonyl)-7-azaspiro[4.5]décan-10-yl)méthyl)-*N*,*N*-diméthyl-6-oxo-4-phényl-1,6-dihydropyridine-3-carboxamide ;
1-((7-(Bicyclo[1.1.1]pentane-1-carbonyl)-10-hydroxy-7-azaspiro[4.5]décan-10-yl)méthyl)-*N*,*N*-diméthyl-6-oxo-4-phényl-1,6-dihydropyridine-3-carboxannide ;
1-((10-Hydroxy-7-(5-phényloxazole-4-carbonyl)-7-azaspiro[4.5]décan-10-yl)méthyl)-*N*,*N*-diméthyl-6-oxo-4-phényl-1,6-dihydropyridine-3-carboxamide ;
1-((7-(5-Cyclopropyloxazole-4-carbonyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(2-(Cyclohexyloxy)acétyl)-10-hydroxy-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(spiro[2.2]pentane-1-carbonyl)-7-azaspiro[4.5]décan-10-yl)méthyl)-*N,N*¬diméthyl-6-oxo-4-phényl-1,6-dihydropyridine-3-carboxamide ;
1-((10-Hydroxy-7-(3-(trifluorométhyl)cyclopentane-1-carbonyl)-7-azaspiro[4.5]décan-10-yl)méthyl)-*N*,*N*-diméthyl-6-oxo-4-phényl-1,6-dihydropyridine-3-carboxamide ;
1-((7-(2,4-Diméthylpentanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(1-Benzyl-1*H*-pyrrole-2-carbonyl)-10-hydroxy-7-azaspiro[4.5]décan-10-yl)méthyl)-N,N-diméthyl-6-oxo-4-phényl-1,6-dihydropyridine-3-carboxamide ;
1-((7-(2-(Cyclohexyloxy)propanoyl)-10-hydroxy-7-azaspiro[4.5]décan-10-yl)méthyl)-*N*,*N*-diméthyl-6-oxo-4-phényl-1,6-dihydropyridine-3-carboxamide ;
3-((1-((*S*)-3-Cyclobutyl-2-(hydroxyméthyl)propanoyl)-4-hydroxy-3,3-diméthylpipéridin-4-yl)méthyl)-6-(2-fluorophényl)pyrimidin-4(3*H*)-one ;
1-((7-(2-Cyclobutoxyacetyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(3,3-Difluorocyclopentane-1-carbonyl)-10-hydroxy-7-azaspiro[4.5]décan-10-yl)méthyl)-*N,N*-diméthyl-6-oxo-4-phényl-1,6-dihydropyridine-3-carboxamide ;
1-((10-Hydroxy-7-(2-hydroxy-3-phénylpropanoyl)-7-azaspiro[4.5]décan-10-yl)méthyl)-*N*,*N*-diméthyl-6-oxo-4-phényl-1,6-dihydropyridine-3-carboxamide ;
1-(((*S*)-4-Hydroxy-3,3-diméthyl-1-((R)-4,4,4-trifluoro-2-méthylbutanoyl)pipéridin-4-yl)méthyl)-*N*,*N*-diméthyl-6-oxo-4-phényl-1,6-dihydropyridine-3-carboxamide ;
1-(((*S*)-1-((*R*)-3-Cyclohexyl-2-méthylpropanoyl)-4-hydroxy-3,3-diméthylpipéridin-4-yl)méthyl)-4-(2-fluorophényl)-*N*,*N*-diméthyl-6-oxo-1,6-dihydropyridine-3-carboxamide ;
1-(((*S*)-1-((*R*)-3-Cyclohexyl-2-méthylpropanoyl)-4-hydroxy-3,3-diméthylpipéridin-4-yl)méthyl)-4-(2-fluorophényl)-5-(pipérazine-1-carbonyl)pyridin-2(1*H*)-one ;
(*S*)-1-((4-Hydroxy-3,3-diméthyl-1-(4,4,4-trifluoro-2-*(2,2,2-trifluoroéthyl)butanoyl)pipéridin-4-yl)méthyl)-N,N-*diméthyl-6-oxo-4-phényl-1,6-dihydropyridine-3-carboxamide ;
1-(((S)-1-((*R*)-3-Cyclohexyl-2-méthylpropanoyl)-4-hydroxy-3,3-diméthylpipéridin-4-yl)méthyl)-5-((S)-2-(hydroxyméthyl)pipérazine-1-carbonyl)-4-phénylpyridin-2(1*H*)-one ;
1-(((*R*)-7-((*R*)-3-Cyclohexyl-2-méthylpropanoyl)-10-hydroxy-7-azaspiro[4.5]décan-10-yl)méthyl)-*N,N*-diméthyl-6-oxo-4-phényl-1,6-dihydropyridine-3-carboxamide ;
1-(((*R*)-7-((*R*)-3-Cyclohexyl-2-méthylpropanoyl)-10-hydroxy-7-azaspiro[4.5]décan-10-yl)méthyl)-4-phényl-5-(pipérazine-1-carbonyl)pyridin-2(1*H*)-one ;
1-(((S)-7-((*R*)-3-Cyclohexyl-2-méthylpropanoyl)-10-hydroxy-7-azaspiro[4.5]décan-10-yl)méthyl)-*N,N*-diméthyl-6-oxo-4-phényl-1,6-dihydropyridine-3-carboxamide ;
1-(((S)-7-((*R*)-3-Cyclohexyl-2-méthylpropanoyl)-10-hydroxy-7-azaspiro[4.5]décan-10-yl)méthyl)-4-phényl-5-(pipérazine-1-carbonyl)pyridin-2(1*H*)-one ;
1-(((S)-1-((*R*)-3-Cyclohexyl-2-méthylpropanoyl)-4-hydroxy-3,3-diméthylpipéridin-4-yl)méthyl)-5-((*R*)-3-méthylpipérazine-1-carbonyl)-4-phénylpyridin-2(1*H*)-one ;
1-(((S)-7-((*R*)-3-Cyclobutyl-2-méthylpropanoyl)-10-hydroxy-7-azaspiro[4.5]décan-10-yl)méthyl)-*N,N*-diméthyl-6-oxo-4-phényl-1,6-dihydropyridine-3-carboxamide ;
1-(((S)-7-((*R*)-3-Cyclobutyl-2-méthylpropanoyl)-10-hydroxy-7-azaspiro[4.5]décan-10-yl)méthyl)-4-phényl-5-(pipérazine-1-carbonyl)pyridin-2(1*H*)-one ;
1-(((S)-10-Hydroxy-7-((*R*)-4,4,4-trifluoro-2-méthylbutanoyl)-7-azaspiro[4.5]décan-10-yl)méthyl)-*N*,*N-*diméthyl-6-oxo-4-phényl-1,6-dihydropyridine-3-carboxamide ;
1-(((S)-10-Hydroxy-7-((*R*)-4,4,4-trifluoro-2-méthylbutanoyl)-7-azaspiro[4.5]décan-10-yl)méthyl)-4-phényl-5-(pipérazine-1-carbonyl)pyridin-2(1H)-one ;
(S)-1-((10-Hydroxy-7-(4,4,4-trifluoro-2-(2,2,2-trifluoroéthyl)butanoyl)-7-azaspiro[4.5]décan-10-yl)méthyl)-*N*,*N*-diméthyl-6-oxo-4-phényl-1,6-dihydropyridine-3-carboxamide ;
(S)-1-((10-Hydroxy-7-(4,4,4-trifluoro-2-(2,2,2-trifluoroéthyl)butanoyl)-7-azaspiro[4.5]décan-10-yl)méthyl)-4-phényl-5-(pipérazine-1-carbonyl)pyridin-2(1*H*)-one ;
1-(((S)-1-((*R*)-3-Cyclohexyl-2-méthylpropanoyl)-4-hydroxy-3,3-diméthylpipéridin-4-yl)méthyl)-4-cyclopropyl-*N*,*N*-diméthyl-6-oxo-1,6-dihydropyridine-3-carboxamide ;
1-(((*S*)-1-((*R*)-3-Cyclohexyl-2-méthylpropanoyl)-4-hydroxy-3,3-diméthylpipéridin-4-yl)méthyl)-4-cyclopropyl-5-(pipérazine-1-carbonyl)pyridin-2(1*H*)-one ;
1-(((*S*)-7-((*R*)-3-Cyclobutyl-2-méthylpropanoyl)-10-hydroxy-7-azaspiro[4.5]décan-10-yl)méthyl)-4-(2-fluorophényl)-*N*,*N*-diméthyl-6-oxo-1,6-dihydropyridine-3-carboxamide ;
4-(2-Fluorophényl)-1-(((S)-10-hydroxy-7-((*R*)-4,4,4-trifluoro-2-méthylbutanoyl)-7-azaspiro[4.5]décan-10-yl)méthyl)-*N*,*N*-diméthyl-6-oxo-1,6-dihydropyridine-3-carboxamide ;
1-(((S)-7-((*R*)-3-Cyclobutyl-2-méthylpropanoyl)-10-hydroxy-7-azaspiro[4.5]décan-10-yl)méthyl)-4-(2-fluorophényl)-5-(pipérazine-1-carbonyl)pyridin-2(1*H*)-one ;
4-(2-Fluorophényl)-1-(((S)-10-hydroxy-7-((*R*)-4,4,4-trifluoro-2-méthylbutanoyl)-7-azaspiro[4.5]décan-10-yl)méthyl)-5-(pipérazine-1-carbonyl)pyridin-2(1*H*)-one ;
1-(((*S*)-7-((*R*)-3-Cyclohexyl-2-méthylpropanoyl)-10-hydroxy-7-azaspiro[4.5]décan-10-yl)méthyl)-4-(2-fluorophényl)-*N*,*N*-diméthyl-6-oxo-1,6-dihydropyridine-3-carboxamide ;
1-(((*S*)-7-((*R*)-3-Cyclohexyl-2-méthylpropanoyl)-10-hydroxy-7-azaspiro[4.5]décan-10-yl)méthyl)-4-(2-fluorophényl)-5-(pipérazine-1-carbonyl)pyridin-2(1*H*)-one ;
1-(((S)-1-((*R*)-3-Cyclohexyl-2-méthylpropanoyl)-4-hydroxy-3,3-diméthylpipéridin-4-yl)méthyl)-5-(4-hydroxypipéridine-1-carbonyl)-4-phénylpyridin-2(1*H*)-one ;
1-((10-Hydroxy-7-(3-(trifluorométhyl)cyclobutane-1-carbonyl)-7-azaspiro[4.5]décan-10-yl)méthyl)-*N*,*N*-diméthyl-6-oxo-4-phényl-1,6-dihydropyridine-3-carboxamide ;
1-(((*S*)-7-((R)-3-Cyclobutyl-2-méthylpropanoyl)-10-hydroxy-7-azaspiro[4.5]décan-10-yl)méthyl)-4-cyclopropyl-N,N-diméthyl-6-oxo-1,6-dihydropyridine-3-carboxamide ;
1-(((*S*)-7-((R)-3-Cyclobutyl-2-méthylpropanoyl)-10-hydroxy-7-azaspiro[4.5]décan-10-yl)méthyl)-4-cyclopropyl-5-(pipérazine-1-carbonyl)pyridin-2(1*H*)-one ;
1-(((*S*)-7-((R)-3-Cyclohexyl-2-méthylpropanoyl)-10-hydroxy-7-azaspiro[4.5]décan-10-yl)méthyl)-4-cyclopropyl-*N*,*N*-diméthyl-6-oxo-1,6-dihydropyridine-3-carboxamide ;
1-(((*S*)-7-((*R*)-3-Cyclohexyl-2-méthylpropanoyl)-10-hydroxy-7-azaspiro[4.5]décan-10-yl)méthyl)-4-cyclopropyl-5-(pipérazine-1-carbonyl)pyridin-2(1*H*)-one ;
1-((2-((*R*)-3-Cyclohexyl-2-méthylpropanoyl)-5-hydroxy-9-oxa-2-azaspiro[5.5]undécan-5-yl)méthyl)-*N*,*N*-diméthyl-6-oxo-4-phényl-1,6-dihydropyridine-3-carboxamide ;
1-(((*S*)-1-((*R*)-3-Cyclobutyl-2-méthylpropanoyl)-4-hydroxy-3,3-diméthylpipéridin-4-yl)méthyl)-4-(2-fluorophényl)-*N*,*N*-diméthyl-6-oxo-1,6-dihydropyridine-3-carboxamide ;
1-(((*S*)-1-((*R*)-3-Cyclobutyl-2-méthylpropanoyl)-4-hydroxy-3,3-diméthylpipéridin-4-yl)méthyl)-4-(2-fluorophényl)-5-(pipérazine-1-carbonyl)pyridin-2(1*H*)-one ;
1-((2-((*R*)-3-Cyclohexyl-2-méthylpropanoyl)-5-hydroxy-2-azaspiro[5.5]undécan-5-yl)méthyl)-*N*,*N*-diméthyl-6-oxo-4-phényl-1,6-dihydropyridine-3-carboxamide ;
1-((2-((*R*)-3-Cyclohexyl-2-méthylpropanoyl)-5-hydroxy-2-azaspiro[5.5]undécan-5-yl)méthyl)-4-phényl-5-(pipérazine-1-carbonyl)pyridin-2(1*H*)-one ;
1-(((*S*)-7-((*R*)-3-Cyclohexyl-2-méthylpropanoyl)-10-hydroxy-7-azaspiro[4.5]décan-10-yl)méthyl)-5-(morpholine-4-carbonyl)-4-phénylpyridin-2(1*H*)-one ;
1-(((*S*)-7-((*R*)-3-Cyclohexyl-2-méthylpropanoyl)-10-hydroxy-7-azaspiro[4.5]décan-10-yl)méthyl)-5-(4-méthylpipérazine-1-carbonyl)-4-phénylpyridin-2(1*H*)-one ;
Acide 1-((1-((*R*)-3-cyclohexyl-2-méthylpropanoyl)-4-hydroxy-3,3-diméthylpipéridin-4-yl)méthyl)-6-oxo-4-phényl-1,6-dihydropyridineacide-3-carboxylique ;
1-((7-((*R*)-3-Cyclohexyl-2-méthylpropanoyl)-10-hydroxy-7-azaspiro[4.5]décan-10-yl)méthyl)-4-méthoxy-*N*,*N*-diméthyl-6-oxo-1,6-dihydropyridine-3-carboxamide ;
1-((7-(4,4-Difluoro-2-méthylbutanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((7-(4,4-Difluoro-2,2-diméthylbutanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(4-(trifluorométhyl)thiazole-2-carbonyl)-7-azaspiro[4.5]décan-10-yl)méthyl)-*N*,*N*-diméthyl-6-oxo-4-phényl-1,6-dihydropyridine-3-carboxamide ;
1-((10-Hydroxy-7-(2-(trifluorométhyl)thiazole-4-carbonyl)-7-azaspiro[4.5]décan-10-yl)méthyl)-*N*,*N*-diméthyl-6-oxo-4-phényl-1,6-dihydropyridine-3-carboxamide ;
1-(((S)-10-Hydroxy-7-((*S*)-4,4,4-trifluoro-2-(hydroxyméthyl)butanoyl)-7-azaspiro[4.5]décan-10-yl)méthyl)-*N*,*N*-diméthyl-6-oxo-4-phényl-1,6-dihydropyridine-3-carboxamide ;
1-(((S)-10-Hydroxy-7-((S)-4,4,4-trifluoro-2-(hydroxyméthyl)butanoyl)-7-azaspiro[4.5]décan-10-yl)méthyl)-5-(morpholine-4-carbonyl)-4-phénylpyridin-2(1*H*)-one ;
1-(((*S*)-10-Hydroxy-7-((S)-4,4,4-trifluoro-2-(hydroxyméthyl)butanoyl)-7-azaspiro[4.5]décan-10-yl)méthyl)-4-phényl-5-(pipérazine-1-carbonyl)pyridin-2(1*H*)-one ;
1-(((*S*)-10-Hydroxy-7-((R)-4,4,4-trifluoro-2-méthylbutanoyl)-7-azaspiro[4.5]décan-10-yl)méthyl)-5-(morpholine-4-carbonyl)-4-phénylpyridin-2(1*H*)-one ;
1-(((*S*)-10-Hydroxy-7-((R)-4,4,4-trifluoro-2-méthylbutanoyl)-7-azaspiro[4.5]décan-10-yl)méthyl)-5-(4-méthylpipérazine-1-carbonyl)-4-phénylpyridin-2(1*H*)-one ;
Éthyle 1-(((*S*)-7-((*R*)-3-cyclohexyl-2-méthylpropanoyl)-10-hydroxy-7-azaspiro[4.5]décan-10-yl)méthyl)-6-oxo-4-phényl-1,6-dihydropyridine-3-carboxylate ;
Acide 1-(((*S*)-7-((*R*)-3-cyclohexyl-2-méthylpropanoyl)-10-hydroxy-7-azaspiro[4.5]décan-10-yl)méthyl)-6-oxo-4-phényl-1,6-dihydropyridine-3-carboxylique ;
1-(((*R*)-1-((*R*)-3-Cyclohexyl-2-méthylpropanoyl)-4-hydroxy-3,3-diméthylpipéridin-4-yl)méthyl)-*N*-isopropyl-*N-*méthyl-6-oxo-4-phényl-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-4-phényl-5-(pyrrolidine-1-carbonyl)pyridin-2(1*H*)-one ;
1-(((*R*)-1-((*S*)-3-Cyclohexyl-2-(méthoxyméthyl)propanoyl)-4-hydroxy-3,3-diméthylpipéridin-4-yl)méthyl)-*N*,*N*-diméthyl-6-oxo-4-phényl-1,6-dihydropyridine-3-carboxamide ;
1-(((*R*)-4-Hydroxy-3,3-diméthyl-1-((*R*)-4,4,4-trifluoro-2-méthylbutanoyl)pipéridin-4-yl)méthyl)-*N*,*N*-diméthyl-6-oxo-4-phényl-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-4-(2-fluorophényl)-*N*,*N*-diméthyl-6-oxo-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-4-(2-fluorophényl)-5-(pipérazine-1-carbonyl)pyridin-2(1*H*)-one ;
(*R*)-1-((4-Hydroxy-3,3-diméthyl-1-(4,4,4-trifluoro-2-*(2,2,2-trifluoroéthyl)butanoyl)pipéridin-4-yl)méthyl)-N,N-*diméthyl-6-oxo-4-phényl-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-5-((*S*)-2-(hydroxyméthyl)pipérazine-1-carbonyl)-4-phénylpyridin-2(1*H*)-one ;
1-(((*R*)-1-((*R*)-3-Cyclohexyl-2-méthylpropanoyl)-4-hydroxy-3,3-diméthylpipéridin-4-yl)méthyl)-5-((*R*)-3-méthylpipérazine-1-carbonyl)-4-phénylpyridin-2(1*H*)-one ;
1-(((*R*)-7-((R)-3-Cyclobutyl-2-méthylpropanoyl)-10-hydroxy-7-azaspiro[4.5]décan-10-yl)méthyl)-*N*,*N*-diméthyl-6-oxo-4-phényl-1,6-dihydropyridine-3-carboxamide ;
1-(((*R*)-7-((*R*)-3-Cyclobutyl-2-méthylpropanoyl)-10-hydroxy-7-azaspiro[4.5]décan-10-yl)méthyl)-4-phényl-5-(pipérazine-1-carbonyl)pyridin-2(1*H*)-one ;
1-(((R)-10-Hydroxy-7-((*R*)-4,4,4-trifluoro-2-méthylbutanoyl)-7-azaspiro[4.5]décan-10-yl)méthyl)-*N*,*N-*diméthyl-6-oxo-4-phényl-1,6-dihydropyridine-3-carboxamide ;
1-(((*R*)-10-Hydroxy-7-((R)-4,4,4-trifluoro-2-méthylbutanoyl)-7-azaspiro[4.5]décan-10-yl)méthyl)-4-phényl-5-(pipérazine-1-carbonyl)pyridin-2(1*H*)-one ;
(*R*)-1-((10-Hydroxy-7-(4,4,4-trifluoro-2-(2,2,2-trifluoroéthyl)butanoyl)-7-azaspiro[4.5]décan-10-yl)méthyl)-*N*,*N*-diméthyl-6-oxo-4-phényl-1,6-dihydropyridine-3-carboxamide ;
(*R*)-1-((10-Hydroxy-7-(4,4,4-trifluoro-2-(2,2,2-trifluoroéthyl)butanoyl)-7-azaspiro[4.5]décan-10-yl)méthyl)-4-phényl-5-(pipérazine-1-carbonyl)pyridin-2(1*H*)-one ;
1-(((*R*)-1-((*R*)-3-Cyclohexyl-2-méthylpropanoyl)-4-hydroxy-3,3-diméthylpipéridin-4-yl)méthyl)-4-cyclopropyl-*N*,*N*-diméthyl-6-oxo-1,6-dihydropyridine-3-carboxamide ;
1-(((*R*)-1-((*R*)-3-Cyclohexyl-2-méthylpropanoyl)-4-hydroxy-3,3-diméthylpipéridin-4-yl)méthyl)-4-cyclopropyl-5-(pipérazine-1-carbonyl)pyridin-2(1*H*)-one ;
1-(((*R*)-7-((*R*)-3-Cyclobutyl-2-méthylpropanoyl)-10-hydroxy-7-azaspiro[4.5]décan-10-yl)méthyl)-4-(2-fluorophényl)-*N*,*N*-diméthyl-6-oxo-1,6-dihydropyridine-3-carboxamide ;
4-(2-FluorophényI)-1-(((*R*)-10-hydroxy-7-((*R*)-4,4,4-trifluoro-2-méthylbutanoyl)-7-azaspiro[4.5]décan-10-yl)méthyl)-*N*,*N*-diméthyl-6-oxo-1,6-dihydropyridine-3-carboxamide ;
1-(((*R*)-7-((*R*)-3-Cyclobutyl-2-méthylpropanoyl)-10-hydroxy-7-azaspiro[4.5]décan-10-yl)méthyl)-4-(2-fluorophényl)-5-(pipérazine-1-carbonyl)pyridin-2(1*H*)-one ;
4-(2-FluorophényI)-1-(((*R*)-10-hydroxy-7-((*R*)-4,4,4-trifluoro-2-méthylbutanoyl)-7-azaspiro[4.5]décan-10-yl)méthyl)-5-(pipérazine-1-carbonyl)pyridin-2(1*H*)-one ;
1-(((*R*)-7-((*R*)-3-Cyclohexyl-2-méthylpropanoyl)-10-hydroxy-7-azaspiro[4.5]décan-10-yl)méthyl)-4-(2-fluorophényl)-*N*,*N*-diméthyl-6-oxo-1,6-dihydropyridine-3-carboxamide ;
1-(((*R*)-7-((*R*)-3-Cyclohexyl-2-méthylpropanoyl)-10-hydroxy-7-azaspiro[4.5]décan-10-yl)méthyl)-4-(2-fluorophényl)-5-(pipérazine-1-carbonyl)pyridin-2(1*H*)-one ;
1-(((*R*)-1-((*R*)-3-Cyclohexyl-2-méthylpropanoyl)-4-hydroxy-3,3-diméthylpipéridin-4-yl)méthyl)-5-(4-hydroxypipéridine-1-carbonyl)-4-phénylpyridin-2(1*H*)-one ;
1-(((*R*)-7-((*R*)-3-Cyclobutyl-2-méthylpropanoyl)-10-hydroxy-7-azaspiro[4.5]décan-10-yl)méthyl)-4-cyclopropyl-*N*,*N*-diméthyl-6-oxo-1,6-dihydropyridine-3-carboxamide ;
1-(((*R*)-7-((*R*)-3-Cyclobutyl-2-méthylpropanoyl)-10-hydroxy-7-azaspiro[4.5]décan-10-yl)méthyl)-4-cyclopropyl-5-(pipérazine-1-carbonyl)pyridin-2(1*H*)-one ;
1-(((*R*)-7-((*R*)-3-Cyclohexyl-2-méthylpropanoyl)-10-hydroxy-7-azaspiro[4.5]décan-10-yl)méthyl)-4-cyclopropyl-*N*,*N*-diméthyl-6-oxo-1,6-dihydropyridine-3-carboxamide ;
1-(((*R*)-7-((*R*)-3-Cyclohexyl-2-méthylpropanoyl)-10-hydroxy-7-azaspiro[4.5]décan-10-yl)méthyl)-4-cyclopropyl-5-(pipérazine-1-carbonyl)pyridin-2(1*H*)-one ;
1-(((*R*)-1-((*R*)-3-Cyclobutyl-2-méthylpropanoyl)-4-hydroxy-3,3-diméthylpipéridin-4-yl)méthyl)-4-(2-fluorophényl)-*N*,*N*-diméthyl-6-oxo-1,6-dihydropyridine-3-carboxamide ;
1-(((*R*)-1-((*R*)-3-Cyclobutyl-2-méthylpropanoyl)-4-hydroxy-3,3-diméthylpipéridin-4-yl)méthyl)-4-(2-fluorophényl)-5-(pipérazine-1-carbonyl)pyridin-2(1*H*)-one ;
1-(((*R*)-7-((*R*)-3-Cyclohexyl-2-méthylpropanoyl)-10-hydroxy-7-azaspiro[4.5]décan-10-yl)méthyl)-5-(morpholine-4-carbonyl)-4-phénylpyridin-2(1*H*)-one ;
1-(((R)-7-((*R*)-3-Cyclohexyl-2-méthylpropanoyl)-10-hydroxy-7-azaspiro[4.5]décan-10-yl)méthyl)-5-(4-méthylpipérazine-1-carbonyl)-4-phénylpyridin-2(1*H*)-one ;
1-(((*R*)-10-Hydroxy-7-((S)-4,4,4-trifluoro-2-(hydroxyméthyl)butanoyl)-7-azaspiro[4.5]décan-10-yl)méthyl)-*N*,*N*-diméthyl-6-oxo-4-phényl-1,6-dihydropyridine-3-carboxamide ;
1-(((*R*)-10-Hydroxy-7-((S)-4,4,4-trifluoro-2-(hydroxyméthyl)butanoyl)-7-azaspiro[4.5]décan-10-yl)méthyl)-5-(morpholine-4-carbonyl)-4-phénylpyridin-2(1*H*)-one ;
1-(((*R*)-10-Hydroxy-7-((S)-4,4,4-trifluoro-2-(hydroxyméthyl)butanoyl)-7-azaspiro[4.5]décan-10-yl)méthyl)-4-phényl-5-(pipérazine-1-carbonyl)pyridin-2(1*H*)-one ;
1-(((*R*)-10-Hydroxy-7-((*R*)-4,4,4-trifIuoro-2-méthylbutanoyI)-7-azaspiro[4.5]décan-10-yl)méthyl)-5-(morpholine-4-carbonyl)-4-phénylpyridin-2(1*H*)-one ;
1-(((*R*)-10-Hydroxy-7-((*R*)-4,4,4-trifIuoro-2-méthylbutanoyl)-7-azaspiro[4.5]décan-10-yl)méthyl)-5-(4-méthylpipérazine-1-carbonyl)-4-phénylpyridin-2(1*H*)-one ;
Éthyle 1-(((*R*)-7-((R)-3-cyclohexyl-2-méthylpropanoyl)-10-hydroxy-7-azaspiro[4.5]décan-10-yl)méthyl)-6-oxo-4-phényl-1,6-dihydropyridine-3-carboxylate ;
Acide 1-(((*R*)-7-((*R*)-3-Cyclohexyl-2-méthylpropanoyl)-10-hydroxy-7-azaspiro[4.5]décan-10-yl)méthyl)-6-oxo-4-phényl-1,6-dihydropyridine-3-carboxylique ;
3-((10-Hydroxy-7-((3*R*,4*R*)-3-phénylpipéridine-4-carbonyl)-7-azaspiro[4.5]décan-10-yl)méthyl)-6-phénylpyrimidin-4(3*H*)-one ;
3-(((S)-10-Hydroxy-7-((R)-4,4,4-trifluoro-2-méthylbutanoyl)-7-azaspiro[4.5]décan-10-yl)méthyl)-6-phénylpyrimidin-4(3*H*)-one ;
6-(2-Fluorophényl)-3-(((S)-10-hydroxy-7-((*R*)-4,4,4-trifIuoro-2-méthylbutanoyl)-7-azaspiro[4.5]décan-10-yl)méthyl)pyrimidin-4(3*H*)-one ;
6-(3-Fluorophényl)-3-(((*S*)-10-hydroxy-7-((*R*)-4,4,4-trifIuoro-2-méthylbutanoyl)-7-azaspiro[4.5]décan-10-yl)méthyl)pyrimidin-4(3*H*)-one ;
3-(((S)-7-((R)-4,4-Difluoro-2-méthylpentanoyl)-10-hydroxy-7-azaspiro[4.5]décan-10-yl)méthyl)-6-phénylpyrimidin-4(3*H*)-one ;
3-(((S)-10-Hydroxy-7-((3*S*,4*R*)-4-phénylpyrrolidine-3-carbonyl)-7-azaspiro[4.5]décan-10-yl)méthyl)-6-phénylpyrimidin-4(3*H*)-one ;
3-(((S)-10-Hydroxy-7-((3*R*,4*S*)-4-phénylpyrrolidine-3-carbonyl)-7-azaspiro[4.5]décan-10-yl)méthyl)-6-phénylpyrimidin-4(3*H*)-one ;
3-(((S)-10-Hydroxy-7-((2*R*,3*R*)-2-phénylpyrrolidine-3-carbonyl)-7-azaspiro[4.5]décan-10-yl)méthyl)-6-phénylpyrimidin-4(3*H*)-one ;
3-(((S)-10-Hydroxy-7-((2*S*,3*S*)-2-phénylpyrrolidine-3-carbonyl)-7-azaspiro[4.5]décan-10-yl)méthyl)-6-phénylpyrimidin-4(3*H*)-one.

18. Composition pharmaceutique comprenant un composé, sel, tautomère, stéréoisomère ou dérivé de *N*-oxyde pharmaceutiquement acceptable tel que défini selon l'une quelconque des revendications 1 à 17, destinée à être utilisée dans le traitement de l'obésité, résistance à l'insuline, ou diabète de type II, facultativement dans laquelle ladite composition pharmaceutique comprend en outre un diluant, excipient ou support pharmaceutiquement acceptable.
